# EUROPEAN PATENT APPLICATION

(11) **EP 2 764 866 A1**
(43) Date of publication of application: **13.08.2014**
(21) Application number: 14153732.4
(22) Date of filing: 04.02.2014
(51) Int. Cl.: A61K 31/505, C07D 239/46, A61P 35/00

(54) **Inhibitors of nedd8-activating enzyme**

(30) Priority: 07.02.2013 EP 13154479; 07.02.2013 EP 13154497; 14.02.2013 EP 13155308; 28.02.2013 EP 13157312; 28.02.2013 EP 13157322; 07.03.2013 EP 13158148; 07.03.2013 EP 13158123; 21.03.2013 EP 13160513; 21.03.2013 EP 13160508; 21.03.2013 EP 13160501
(71) Applicant: IP Gesellschaft für Management mbH, 50931 Köln (DE)
(72) Inventor: Trinius, Frank, 50931 Köln (DE)

(57) **Abstract**

The invention relates to an administration unit comprising crystalline form I of {(1 S,2S,4R)-4-[(6-{[(1R,2S)-5-chloro-2methoxy-2,3-dihydro-1H-inden-1-yl]amino}pyrimidin-4-yl)oxy]-2-hydroxycyclopentyl}methyl sulfamate (I-216) hydrochloride salt and to a packaging comprising the administration unit according to the invention.

## Description

### CROSSREFERENCES

The present application claims priorities of European patent application no. EP13154479.3, filed on 07 February 2013; European patent application no. EP13154497.5, filed on 07 February 2013; European patent application no. EP13155308.3, filed on 14 February 2013; European patent application no. EP13157312.3, filed on 28 February 2013; European patent application no. EP13157322.2, filed on 28 February 2013; European patent application no. EP13158148.0, filed on 07 March 2013; European patent application no. EP13158123.3, filed on 07 March 2013; European patent application no. EP13160513.1, filed on 21 March 2013; European patent application no. EP13160508.1, filed on 21 March 2013; and European patent application no. EP13160501.6, filed on 21 March 2013.

### BACKGROUND ART

Many pharmaceuticals are marketed without any packaging. In various countries, small drug shops are a common source of medicine where stocking of prescription-only medicines and unpackaged tablets is the norm (Goodman C et al., Health Policy Plan. 2007 Nov;22(6):393-403). Furthermore, self-medication is common practice in various countries. For example, the most frequently proposed drugs to treat male urethritis are: ampicillin 250-mg capsules (44%); oxytetracyline 250-mg capsules (24%); and cotrimoxazole 450-mg tablets (12%), and these drugs are frequently sold unpackaged (Sow PS et al., Int J Infect Dis. 2002 Jun;6(2):108-12).

In Germany and other European countries, numerous pharmaceuticals are marketed in packaging wherein every single administration unit is individually packaged in a single packaging. Examples include but are not limited to Frubiase^{®} Calcium (Boehringer Ingelheim) marketed as a liquid administration unit that is individually packaged in a glass ampoule; Orthomol Immun^{®} Direktgranulat (Orthomol) marketed as administration unit in granulate form that is individually packaged in a bag; Orthomol Vital m^{®} (Orthomol) marketed as a liquid administration unit that is individually packaged in a bottle having a lid containing an individually packaged tablet; Vitasprint^{®} (Pfizer) and Celestamine^{®} (MSD Sharp & Dohme) each marketed as a liquid administration unit that is individually packaged in a bottle; Alka-Seltzer^{®} (Bayer) marketed as effervescent tablet that is individually packaged in a bag; Aspirin^{®} Complex (Bayer), Aspirin^{®} Effect (Bayer), Helmex^{®} (Infectopharm), Monuril^{®} Granulat (Pierre Fabre Pharma) each marketed as administration unit in granulate form that individually packaged in a bag; ellaOne^{®} (HRA Pharma), Levonelle^{®} (Bayer Schering Pharma); Pidana^{®} (HRA Pharma), Fungata^{®} (Pfizer), and Canesten^{®} Gyn once (Bayer) each marketed as a single tablet that is individually packaged in a blister. Furthermore, numerous single-to-use syringes are marketed individually packaged.

It is an object of the invention to provide administration units containing drugs that have advantages compared to conventional administration units and conventional drugs, respectively. It is also an object of the invention to provide packaging containing administration units containing drugs that have advantages compared to conventional packaging. These objects have been achieved by the present invention.

### SUMMARY OF THE INVENTION

The present invention relates to different drugs (active pharmaceutical ingredients) and certain forms of said drugs, such as stereoisomers, salts and/or polymorphs thereof. The present invention covers various aspects that are numbered (i), (ii), (iii), (iv), (v), (vi), (vii), (viii), (ix), and (x). Aspects (i), (ii), (iii), (iv), (v), (vi), (vii), (viii), (ix), and (x) are separate from one another.

The present invention relates to
⊙ an administration unit comprising (i) {drug1a} or {drug1b}; (ii) {drug2a}; (iii) {drug3a}; (iv) {drug4a}, {drug4b}, (drug4c), {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b};
⊙ a packaging comprising one or more of such administration units;
⊙ a method for manufacturing such an administration unit;
⊙ a method for manufacturing such a packaging; and
⊙ particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or(x) {drug10a} or {drug10b}.

Certain embodiments of the invention are also disclosed in WO2013/018362, WO2013/017989, WO2013/021309, WO2013/028465, WO2013/028832, WO2013/030374, WO2013/030803, WO2013/040120, WO2013/039764, and WO2013/038351, respectively. Any embodiment disclosed in any of these references is incorporated herein by reference as a preferred embodiment of the invention.

### GENERAL DEFINITIONS

To avoid unnecessary repetitions, the description refers to the following abbreviations the meaning of which is defined hereinafter:
Aspect (i) of the present invention relates to 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt, abbreviated as '{drug1}'. In particular, the present invention relates to crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt, abbreviated as '{drug1a}', and to 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt C type crystals, abbreviated as '{drug1b}'. {drug1a} and {drug1b} are a specific forms falling within the definition of {drug1}. Aspect (i) of the invention is separate from aspects (ii), (iii), (iv), (v), (vi), (vii), (viii), (ix), and (x) of the invention. Thus, all embodiments of {drug1a} and {drug1b}, respectively, are only related to {drug1}, but neither to {drug2}, nor to {drug3}, nor to {drug4}, nor to {drug5}, nor to {drug6}, nor to {drug7}, nor to {drug8}, nor to {drug9}, nor to {drug10}.
Aspect (ii) of the present invention relates to Crizotinib for treatment of cancer, abbreviated as '{drug2}'. In particular, the present invention relates to a solid form of Crizotinib for treatment of cancer, abbreviated as '{drug2a}'. {drug2a} is a specific form falling within the definition of {drug2}. Aspect (ii) of the invention is separate from aspects (i), (iii), (iv), (v), (vi), (vii), (viii), (ix), and (x) of the invention. Thus, all embodiments of {drug2a} are only related to {drug2}, but neither to {drug1}, nor to {drug3}, nor to {drug4}, nor to {drug5}, nor to {drug6}, nor to {drug7}, nor to {drug8}, nor to {drug9}, nor to {drug10}.
Aspect (iii) of the present invention relates to 2-chloro-N-{2-[3-(2-{[(4'-hydroxybiphenyl-3-yl)methyl]amino}-2-oxoethyl)phenyl]-1,1-dimethylethyl}-acetamide, abbreviated as '{drug3}'. In particular, the present invention relates to crystalline form of 2-chloro-N-{2-[3-(2-{[(4'-hydroxybiphenyl-3-yl)methyl]amino}-2-oxoethyl)phenyl]-1,1-dimethylethyl}-acetamide, abbreviated as '{drug3a}'. {drug3a} is a specific form falling within the definition of {drug3}. Aspect (iii) of the invention is separate from aspects (i), (ii), (iv), (v), (vi), (vii), (viii), (ix), and (x) of the invention. Thus, all embodiments of {drug3a} are only related to {drug3}, but neither to {drug1}, nor to {drug2}, nor to {drug4}, nor to {drug5}, nor to {drug6}, nor to {drug7}, nor to {drug8}, nor to {drug9}, nor to {drug10}.
Aspect (iv) of the present invention relates to Compound A, also abbreviated as '{drug4}': 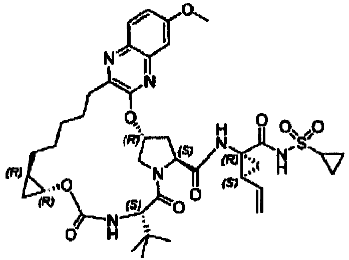 In particular, the present invention relates to crystalline and/or hydrated form of Compound A, abbreviated as '{drug4a}'; hydrate III of compound A, abbreviated as '{drug4b}'; hydrate II of compound A, abbreviated as '{drug4c}'; crystalline Na salt of compound A, abbreviated as '{drug4d}'; crystalline K salt of compound a, abbreviated as '{drug4e}'; hydrate I of compound A, abbreviated as '{drug4f}'; hydrate IV of compound A, abbreviated as '{drug4g}'; hydrate V of compound A, abbreviated as '{drug4h}'; and hydrate VI of compound A, abbreviated as '{drug4i}'. {drug4a}, {drug4b}, {drug4c}, {drug4d}, {drug4e}, {drug4f}, {drug4g}, {drug4h} and {drug4i}, respectively, are specific forms falling within the definition of {drug4}. Hydrate III of compound A {drug4b} is preferably characterized by either (i) an X-ray powder diffraction pattern obtained using copper Kₐ radiation which comprises 2Θ values in degrees of about 20.5, 5.0, and 18.2; or (ii) a solid state carbon-13 CPMAS NMR comprising peaks at about 5.14, 6.31, 12.49, 18.35, 26.81, 28.03, 30.33, 31.27, 34.95, 35.99, 38.68, 42.01, 54.93, 56.39, 60.14, 74.20, 107.02, 120.11, 121.60, 129.73, 134.35, 135.95, 142.89, 148.47, 155.37, 157.32, 160.90, 168.32, 172.17, and 175.53 ppm. Hydrate II of compound A {drug4c} is preferably characterized by an X-ray powder diffraction pattern obtained using copper Kₙ radiation which comprises 2Θ values in degrees of about 11.7, 16.6, and 11.2. The crystalline Na salt of compound A {drug4d} is preferably characterized by an X-ray powder diffraction pattern obtained using copper Kₐ radiation which comprises 2Θ values in degrees of about 18.4, 9.1, and 9.8. The crystalline K salt of compound A {drug4e} is preferably characterized by an X-ray powder diffraction pattern obtained using copper Kₐ radiation which comprises 2Θ values in degrees of about 18.2, 8.9, and 20.3. Hydrate I of compound A {drug4f} is preferably characterized by either (i) an X-ray powder diffraction pattern obtained using copper Kₐ radiation which comprises 2Θ values in degrees of about 8.6, 20.6, and 26.6; or (ii) a solid state carbon-13 CPMAS NMR comprising peaks at about 4.22, 7.23, 11.45, 17.79, 24.04, 26.95, 28.29, 31.15, 32.47, 32.47, 33.46, 34.03, 35.74, 42.32, 53.50, 56.05, 56.96, 77.49, 108.95, 1 19.65, 122.55, 131.05, 133.13, 135.38, 142.28, 150.78, 156.03, 157.99, 161.36, 171.40, 173.42, 174.30 ppm. Hydrate IV of compound A {drug4g} is preferably characterized by either (i) an X-ray powder diffraction pattern obtained using copper Kₐ radiation which comprises 2Θ values in degrees of about 14.7, 11.5, and 7.1; or (ii) a solid state carbon-13 CPMAS NMR comprising peaks at about 3.90, 5.30, 6.99, 10.49, 13.13, 17.81, 24.73, 27.52, 28.14, 29.42, 31.02, 32.80, 36.08, 39.22, 42.45, 53.62, 55.93, 59.14, 60.76, 74.77, 109.22, 111.19, 11.38, 120.24, 122.50, 133.96, 139.74, 147.2, 148.90, 154.65, 158.25, 159.53, 160.12, 170.14, 171.05, 172.08, 173.47, and 174.46 ppm. Hydrate V of compound A {drug4h} is preferably characterized by either (i) an X-ray powder diffraction pattern obtained using copper Kₐ radiation which comprises 2Θ values in degrees of about 9.1, 18.3, and 19.8; or (ii) a solid state carbon-13 CPMAS NMR comprising peaks at about 7.86, 8.92, 13.10, 18.31, 23.72, 27.44, 28.47, 30.77, 35.79, 36.25, 37.15, 37.15, 42.95, 53.13, 55.67, 57.31, 60.47, 62.06, 75.09, 110.59, 1 12.24, 118.32, 132.18, 134.05, 135.83, 139.88, 148.30, 155.19, 157.97, 159.41, 170.31 and 175.20. Hydrate VI of compound A {drug4i} is preferably characterized by either (i) an X-ray powder diffraction pattern obtained using copper Kₐ radiation which comprises 2Θ values in degrees of about 20.5, 12.8, and 19.4; or (ii) a solid state carbon-13 CPMAS NMR comprising peaks at about 4.87, 6.24, 11.70, 12.85, 18.36, 26.55, 28.31m 31.51, 34.98, 38.47, 42.09, 54.27, 56.12, 60.10, 73.49, 73.97, 105.91, 108.04, 118.39, 1 19.75, 121.33, 129.96, 133.87, 136.13, 142.26, 142.97, 146.85, 148.36, 154.97, 157.32, 160.71, 168.23, 172.21 and 175.34 ppm. Aspect (iv) of the invention is separate from aspects (i), (ii), (iii), (v), (vi), (vii), (viii), (ix), and (x) of the invention. Thus, all embodiments of {drug4a} are only related to {drug4}, but neither to {drug1}, nor to {drug2}, nor to {drug3}, nor to {drug5}, nor to {drug6}, nor to {drug7}, nor to {drug8}, nor to {drug9}, nor to {drug10}.
Aspect (v) of the present invention relates to {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) and pharmaceutically acceptable salts thereof, and prodrugs thereof, abbreviated as '{drug5}'. In particular, the present invention relates to crystalline form I of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclo-pentyl}methyl sulfamate (I-216) hydrochloride salt, abbreviated as '{drug5a}'. {drug5a} is a specific form falling within the definition of {drug5}. Aspect (v) of the invention is separate from aspects (i), (ii), (iii), (iv), (vi), (vii), (viii), (ix), and (x) of the invention. Thus, all embodiments of {drug5a} are only related to {drug5}, but neither to {drug1}, nor to {drug2}, nor to {drug3}, nor to {drug4}, nor to {drug6}, nor to {drug7}, nor to {drug8}, nor to {drug9}, nor to {drug10}.
Aspect (vi) of the present invention relates to Rilapladib, abbreviated as '{drug6}'. In particular, the present invention relates to crystalline form of Rilapladib, abbreviated as '{drug6a}'. {drug6a} is a specific form falling within the definition of {drug6}. Aspect (vi) of the invention is separate from aspects (i), (ii), (iii), (iv), (v), (vii), (viii), (ix), and (x) of the invention. Thus, all embodiments of {drug6a} are only related to {drug6}, but neither to {drug1}, nor to {drug2}, nor to {drug3}, nor to {drug4}, nor to {drug5}, nor to {drug7}, nor to {drug8}, nor to {drug9}, nor to {drug10}.
Aspect (vii) of the present invention relates to 6-chloro-3-[2-(1-ethyl-propylamino)-3,4-dioxo-cyclobut-1-enylamino]-2-hydroxy-N-methoxy-N-methyl-benzenesulfonamide choline, abbreviated as '{drug7}'. In particular, the present invention relates to solid form of 6-chloro-3-[2-(1-ethyl-propylamino)-3,4-dioxo-cyclobut-1-enylamino]-2-hydroxy-N-methoxy-N-methyl-benzenesulfonamide choline, abbreviated as '{drug7a)'. {drug7a} is a specific form falling within the definition of {drug7}. Aspect (vii) of the invention is separate from aspects (i), (ii), (iii), (iv), (v), (vi), (viii), (ix), and (x) of the invention. Thus, all embodiments of {drug7a} are only related to {drug7}, but neither to {drug1}, nor to {drug2}, nor to {drug3}, nor to {drug4}, nor to {drug5}, nor to {drug6}, nor to {drug8}, nor to {drug9}, nor to {drug10}.
Aspect (viii) of the present invention relates to an oral dosage form comprising: 1) a dispersion of a compound of formula (I) or a pharmaceutically acceptable salt thereof, in a hydrophilic polymer; and 2) a pharmaceutically acceptable carrier or excipient, abbreviated as '{drug8}'. In particular, the present invention relates to solid form of an oral dosage form comprising: 1) a dispersion of a compound of formula (I) or a pharmaceutically acceptable salt thereof, in a hydrophilic polymer; and 2) a pharmaceutically acceptable carrier or excipient, abbreviated as '{drug8a}'. {drug8a} is a specific form falling within the definition of {drug8}. Aspect (viii) of the invention is separate from aspects (i), (ii), (iii), (iv), (v), (vi), (vii), (ix), and (x) of the invention. Thus, all embodiments of {drug8a} are only related to {drug8}, but neither to {drug1}, nor to {drug2}, nor to {drug3}, nor to {drug4}, nor to {drug5}, nor to {drug6}, nor to {drug7}, nor to {drug9}, nor to {drug10}.
Aspect (ix) of the present invention relates to 4-amino-5-fluoro-3-[6-(4-methylpiperazinyl-1-yl)-1H-benzimidazol-2-yl]-1H-quinolin-2-one for the treatment of cancer in moderate hepatic impaired patients, abbreviated as '{drug9}'.In particular, the present invention relates to crystalline Form B of lactate salt form of 4-amino-5-fluoro-3-[6-(4-methylpiperazinyl-1-yl)-1H-benzimidazol-2-yl]-1H-quinolin-2-one for the treatment of cancer in moderate hepatic impaired patients, abbreviated as '{drug9a}'. {drug9a} is a specific form falling within the definition of {drug9}. Aspect (ix) of the invention is separate from aspects (i), (ii), (iii), (iv), (v), (vi), (vii), (viii), and (x) of the invention. Thus, all embodiments of {drug9a} are only related to {drug9}, but neither to {drug1}, {drug2}, nor to {drug3}, nor to {drug4}, nor to {drug5}, nor to {drug6}, nor to {drug7}, nor to {drug8}, nor to {drug10}.
Aspect (x) of the present invention relates to 6-carboxy-2-(3,5-dichlorophenyl)-benzoxazole meglumine, abbreviated as '{drug10}'. In particular, the present invention relates to crystalline form of 6-carboxy-2-(3,5-dichlorophenyl)-benzoxazole meglumine, abbreviated as '{drug10a}'. Preferably, the crystalline form of 6-carboxy-2-(3,5-dichlorophenyl)-benzoxazole meglumine has a powder X-ray diffraction pattern comprising peaks at diffraction angles (2Θ) of 10.7 ± 0.2, 11.8 ± 0.2, and 13.3 ± 0.2, abbreviated as '{drug10b}'. {drug10a} and {drug10b} are specific forms falling within the definition of {drug10}. Aspect (x) of the invention is separate from aspects (i), (ii), (iii), (iv), (v), (vi), (vii), (viii), and (ix) of the invention. Thus, all embodiments of {drug10a} and {drug10b}, respectively, are only related to {drug10}, but neither to {drug1}, {drug2}, nor to {drug3}, nor to {drug4}, nor to {drug5}, nor to {drug6}, nor to {drug7}, nor to {drug8}, nor to {drug9}.
   Summarizing therefore the following abbreviations have the following meanings:

### Aspect (i):

- {drug1} =: 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt;
- {drug1a} =: crystals of 1-(3-chloro-5-1[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt;
- {drug1b} =: 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt C type crystals.

### Aspect (ii):

- {drug2} =: Crizotinib for treatment of cancer;
- {drug2a} =: solid form of Crizotinib for treatment of cancer.

### Aspect (iii):

- {drug3} =: 2-chloro-N-{2-[3-(2-{[(4'-hydroxybiphenyl-3-yl)methyl]amino}-2-oxoethyl)phenyl)-1,1-dimethylethyl}-acetamide;
- {drug3a} =: crystalline form of 2-chloro-N-{2-[3-(2-{[(4'-hydroxybiphenyl-3-yl)methyl]amino}-2-oxoethyl)phenyl]-1,1-dimethylethyl }-acetamide.

### Aspect (iv):

- {drug4} =: 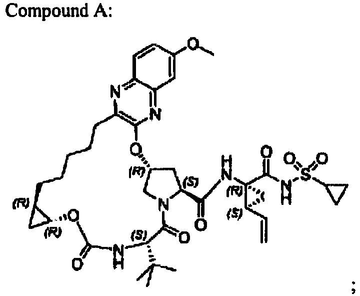
- {drug4a} =: crystalline and/or hydrated form of Compound A;
- {drug4b} =: Hydrate III of compound A which is preferably characterized by either (i) an X-ray powder diffraction pattern obtained using copper Kₐ radiation which comprises 2Θ values in degrees of about 20.5, 5.0, and 18.2; or (ii) a solid state carbon-13 CPMAS NMR comprising peaks at about 5.14, 6.31, 12.49, 18.35, 26.81, 28.03, 30.33, 31.27, 34.95, 35.99, 38.68, 42.01, 54.93, 56.39, 60.14, 74.20, 107.02, 120.11, 121.60, 129.73, 134.35, 135.95, 142.89, 148.47, 155.37, 157.32, 160.90, 168.32, 172.17, and 175.53 ppm.
- {drug4c} =: Hydrate II of compound A which is preferably characterized by an X-ray powder diffraction pattern obtained using copper Kₐ radiation which comprises 2Θ values in degrees of about 11.7, 16.6, and 11.2.
- {drug4d} =: Crystalline Na salt of compound A which is preferably characterized by an X-ray powder diffraction pattern obtained using copper Kₐ radiation which comprises 2Θ values in degrees of about 18.4, 9.1, and 9.8.
- {drug4e} =: Crystalline K salt of compound A which is preferably characterized by an X-ray powder diffraction pattern obtained using copper Kₐ radiation which comprises 2Θ values in degrees of about 18.2, 8.9, and 20.3.
- {drug4f} =: Hydrate I of compound A which is preferably characterized by either (i) an X-ray powder diffraction pattern obtained using copper Kₐ radiation which comprises 2Θ values in degrees of about 8.6, 20.6, and 26.6; or (ii) a solid state carbon-13 CPMAS NMR comprising peaks at about 4.22, 7.23, 11.45, 17.79, 24.04, 26.95, 28.29, 31.15, 32.47, 32.47, 33.46, 34.03, 35.74, 42.32, 53.50, 56.05, 56.96, 77.49, 108.95, 1 19.65, 122.55, 131.05, 133.13, 135.38, 142.28, 150.78, 156.03, 157.99, 161.36, 171.40, 173.42, 174.30 ppm.
- {drug4g} =: Hydrate IV of compound A which is preferably characterized by either (i) an X-ray powder diffraction pattern obtained using copper Kₐ radiation which comprises 2Θ values in degrees of about 14.7, 11.5, and 7.1; or (ii) a solid state carbon-13 CPMAS NMR comprising peaks at about 3.90, 5.30, 6.99, 10.49, 13.13, 17.81, 24.73, 27.52, 28.14, 29.42, 31.02, 32.80, 36.08, 39.22, 42.45, 53.62, 55.93, 59.14, 60.76, 74.77, 109.22, 111.19, 11.38, 120.24, 122.50, 133.96, 139.74, 147.2, 148.90, 154.65, 158.25, 159.53, 160.12, 170.14, 171.05, 172.08, 173.47, and 174.46 ppm.
- {drug4h} =: Hydrate V of compound A which is preferably characterized by either (i) an X-ray powder diffraction pattern obtained using copper Kₐ radiation which comprises 2Θ values in degrees of about 9.1, 18.3, and 19.8; or (ii) a solid state carbon-13 CPMAS NMR comprising peaks at about 7.86, 8.92, 13.10, 18.31, 23.72, 27.44, 28.47, 30.77, 35.79, 36.25, 37.15, 37.15, 42.95, 53.13, 55.67, 57.31, 60.47, 62.06, 75.09, 110.59, 1 12.24, 118.32, 132.18, 134.05, 135.83, 139.88, 148.30, 155.19, 157.97, 159.41, 170.31 and 175.20.
- {drug4i} =: Hydrate VI of compound A which is preferably characterized by either (i) an X-ray powder diffraction pattern obtained using copper Kₐ radiation which comprises 2Θ values in degrees of about 20.5, 12.8, and 19.4; or (ii) a solid state carbon-13 CPMAS NMR comprising peaks at about 4.87, 6.24, 11.70, 12.85, 18.36, 26.55, 28.31m 31.51, 34.98, 38.47, 42.09, 54.27, 56.12, 60.10, 73.49, 73.97, 105.91, 108.04, 118.39, 1 19.75, 121.33, 129.96, 133.87, 136.13, 142.26, 142.97, 146.85, 148.36, 154.97, 157.32, 160.71, 168.23, 172.21 and 175.34 ppm.

### Aspect (v):

- {drug5} =: {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) and pharmaceutically acceptable salts thereof, and prodrugs thereof;
- {drug5a} =: crystalline form I of {(1S,2S,4R)-4-[(6-{[(1R,2S)-5-chloro-2-methoxy-2,3-dihydro-1H-inden-1-yl]amino}-pyrimidin-4-yl)oxy]-2-hydroxycyclopentyl}methyl sulfamate (I-216) hydrochloride salt.

### Aspect (vi):

- {drug6} =: Rilapladib;
- {drug6a} =: crystalline form of Rilapladib.

### Aspect (vii):

- {drug7} =: 6-chloro-3-[2-(1-ethyl-propylamino)-3,4-dioxo-cyclobut-1-enylamino]-2-hydroxy-N-methoxy-N-methyl-benzenesulfonamide choline;
- {drug7a} =: solid form of 6-chloro-3-[2-(1-ethyl-propylamino)-3,4-dioxo-cyclobut-1-enylamino]-2-hydroxy-N-methoxy-N-methyl-benzenesulfonamide choline.

### Aspect (viii):

- {drug8} =: an oral dosage form comprising: 1) a dispersion of a compound of formula (I) or a pharmaceutically acceptable salt thereof, in a hydrophilic polymer; and 2) a pharmaceutically acceptable carrier or excipient;
- {drug8a} =: solid form of an oral dosage form comprising: 1) a dispersion of a compound of formula (I) or a pharmaceutically acceptable salt thereof, in a hydrophilic polymer; and 2) a pharmaceutically acceptable carrier or excipient.

### Aspect (ix):

- {drug9} =: 4-amino-5-fluoro-3-[6-(4-methylpiperazinyl-l-yl)-1H-benzimidazol-2-yl]-1H-quinolin-2-one for the treatment of cancer in moderate hepatic impaired patients;
- {drug9a} =: crystalline Form B of lactate salt form of 4-amino-5-fluoro-3-[6-(4-methylpiperazinyl-1-yl)-1H-benzimidazol-2-yl]-1H-quinolin-2-one for the treatment of cancer in moderate hepatic impaired patients.

### Aspect (x):

- {drug10} =: 6-carboxy-2-(3,5-dichlorophenyl)-benzoxazole meglumine;
- {drug10a} =: crystalline form of 6-carboxy-2-(3,5-dichlorophenyl)-benzoxazole meglumine;
- {drug10b} =: crystalline form of 6-carboxy-2-(3,5-dichlorophenyl)-benzoxazole meglumine having a powder X-ray diffraction pattern comprising peaks at diffraction angles (2Θ) of 10.7 ± 0.2, 11.8 ± 0.2, and 13.3 ± 0.2.

### SPECIFIC INORMATION CONCERNING ASPECT (I) OF THE INVENTION

Aspect (i) of the invention concerns forms of {drug1}, especially a solid form of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt ({drug1}), namely crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt ({drug1a}) (in the following also referred to as " compound X maleic acid salt crystals"), which are useful for treating thrombocytopenia, especially C type crystals ({drug1b}). 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt can be described as maleic acid salt of formula:

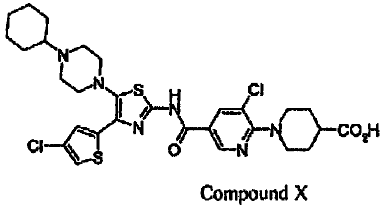

Crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt are described in WO2013018362, which is incorporated by reference.

As used herein, crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt ({drug1a}) is a crystalline form of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt ({drug1}) characterized by the following description according to WO2013/018362:

Compound X maleic acid salt crystals ({drug1a}) exhibit crystal polymorphism, and of these forms, C type crystals ({drug1b}) (hereinafter referred to as "compound X maleic acid salt C type crystals") have excellent oral absorption, ensure constant quality even in mass production such as industrial production, and can be stably supplied, and thus the present invention was achieved. In other words, the present invention relates to compound X maleic acid salt C type crystals ({drug1b}), and to a pharmaceutical composition containing compound X maleic acid salt C type crystals ({drug1b}) and excipients. As illustrated in FIG. 1 of WO2013/018362 through FIG. 6 of WO2013/018362, compound X maleic acid salt C type crystals ({drug1b}) are characterized by the following parameters:

*Differential Scanning Calorimeter Analysis:* The endothermic peak top temperatures are at approximately 229 to 232 °C and at approximately 300 to 303 °C. Other embodiments include the differential scanning calorimeter analysis charts shown in FIG. 1 of WO2013/018362, FIG. 3 of WO2013/018362, FIG. 5 of WO2013/018362, as well as other charts that are recognized by one skilled in the art as being essentially identical to them. *Powder X-Ray Crystal Diffraction:* When measured using a Cu-K alpha beam, peaks are observed at approximately 8.5 degree, approximately 9.5 degree, approximately 12.1 degree, approximately 20.4 degree, approximately 22.7 degree, and approximately 23.3 degree, for a diffraction angle of 2-theta. As another embodiment, when measured using a Cu-K alpha beam, peaks are observed at approximately 5.8 degree, approximately 8.5 degree, approximately 9.5 degree, approximately 12.1 degree, approximately 16.9 degree, approximately 17.4 degree, approximately 17.8 degree, approximately 19.3 degree, approximately 20.4 degree, approximately 22.7 degree, and approximately 23.3 degree for a diffraction angle of 2-theta. As yet another embodiment, when measured using a Cu-K alpha beam, peaks are observed at 8.4 degree - 8.8 degree, 9.3 degree - 9.7 degree, 11.9 degree - 12.3 degree, 20.2 degree - 20.6 degree, 22.5 degree - 22.9 degree, 23.1 degree ∼ 23.5 degree, for a diffraction angle of 2-theta. As yet another embodiment, when measured using a Cu-K alpha beam, peaks are observed at 5.6 degree - 6.0 degree, 8.4 degree - 8.8 degree, 9.3 degree - 9.7 degree, 11.9 degree- 12.3 degree, 16.7 degree - 17.1 degree, 17.2 degree - 17.6 degree, 17.6 degree - 18.0 degree, 19.1 degree ∼ 19.5 degree, 20.2 degree - 20.6 degree, 22.5 degree - 22.9 degree, 23.1 degree ∼ 23.5 degree, for a diffraction angle of 2-theta. As yet another embodiment, when measured using a Cu-K alpha beam, peaks are observed at 8.5 degree - 8.7 degree, 9.4 degree - 9.6 degree, 12.0 degree- 12.2 degree, 20.3 degree - 20.5 degree, 22.5 degree- 22.8 degree, 23.2 degree ∼ 23.4 degree, for a diffraction angle of 2-theta. As yet another embodiment, when measured using a Cu-K alpha beam, peaks are observed at 5.7 degree - 5.9 degree, 8.5 degree - 8.7 degree, 9.4 degree - 9.6 degree, 12.0 degree-12.2 degree, 16.8 degree - 17.0 degree, 17.3 degree - 17.5 degree, 17.7 degree - 17.9 degree, 19.2 degree ∼ 19.4 degree, 20.3 degree - 20.5 degree, 22.5 degree - 22.8 degree, 23.2 degree - 23.4 degree, for a diffraction angle of 2-theta. Other embodiments include the powder x-ray crystal diffraction patterns shown in FIG. 2 of WO2013/018362, FIG. 4 of WO2013/018362, FIG. 6 of WO2013/018362, as well as other patterns that are recognized by one skilled in the art as being essentially identical to them. With powder x-ray crystal diffraction, the crystal diffraction angle and the overall pattern are important data characteristics when verifying the identity of crystals, and the relative strength can change somewhat depending on the direction of crystal growth, particle size, and measurement conditions, and therefore should not be interpreted strictly. Furthermore, the present invention relates to pure compound X maleic acid salt C type crystals ({drug1b}), but mixtures that can appear essentially identical to pure compound X maleic acid salt C type crystals ({drug1b}) are also included in the present invention.

Therefore, aspect (i) of the present invention relates to compound X maleic acid salt crystals that are characterized in differential scanning calorimeter analysis by having endothermic peak top temperatures at approximately 229 to 232 °C and approximately 300 to 303 °C. As another embodiment, aspect (i) of the present invention relates to compound X maleic acid salt crystals that are characterized in powder x-ray crystal diffraction using a Cu-K alpha beam by having peaks at approximately 8.5 degree, approximately 9.5 degree, approximately 12.1 degree, approximately 20.4 degree, approximately 22.7 degree, and approximately 23.3 degree, for a diffraction angle of 2-theta. As yet another embodiment, aspect (i) of the present invention relates to compound X maleic acid salt crystals that are characterized in powder x-ray crystal diffraction using a Cu-K alpha beam by having peaks at approximately 5.8 degree, approximately 8.5 degree, approximately 9.5 degree, approximately 12.1 degree, approximately 16.9 degree, approximately 17.4 degree, approximately 17.8 degree, approximately 19.3 degree, approximately 20.4 degree, approximately 22.7 degree, and approximately 23.3 degree, for a diffraction angle of 2-theta. As another embodiment, aspect (i) of the present invention relates to compound X maleic acid salt crystals that are characterized in differential scanning calorimeter analysis by having endothermic peak top temperatures between approximately 229 and 232 °C and between approximately 300 and 303 °C, and in powder x-ray crystal diffraction using a Cu-K alpha beam by having peaks at approximately 8.5 degree, approximately 9.5 degree, approximately 12.1 degree, approximately 20.4 degree, approximately 22.7 degree, and approximately 23.3 degree, for a diffraction angle of 2-theta. As yet another embodiment, aspect (i) of the present invention relates to compound X maleic acid salt crystals that are characterized in differential scanning calorimeter analysis by having endothermic peak top temperatures between approximately 229 and 232 °C and between approximately 300 and 303 °C, and in powder x-ray crystal diffraction using a Cu-K alpha beam by having peaks at approximately 5.8 degree, approximately 8.5 degree, approximately 9.5 degree, approximately 12.1 degree, approximately 16.9 degree, approximately 17.4 degree, approximately 17.8 degree, approximately 19.3 degree, approximately 20.4 degree, approximately 22.7 degree, and approximately 23.3 degree, for a diffraction angle of 2-theta.

Furthermore, aspect (i) of the present invention relates to a pharmaceutical composition for treating thrombocytopenia that contains compound X maleic acid salt C type crystals ({drug1b}). This pharmaceutical composition includes thrombocytopenia treatment agents that contain compound X maleic acid salt C type crystals ({drug1b}). Furthermore, this pharmaceutical composition can include any pharmaceutical compositions containing compound X maleic acid salt C type crystals ({drug1b}) while maintaining the crystal form, and for example, a solid drug composition can be exemplified. In addition, this pharmaceutical composition also includes liquid pharmaceutical compositions containing the compound X maleic acid salt C type crystals ({drug1b}) while maintaining the crystal form, such as a suspension agent.

Furthermore, the present invention relates to the use of compound X maleic acid salt C type crystals ({drug1b}) for manufacturing a pharmaceutical composition for treating thrombocytopenia, the use of compound X maleic acid salt C type crystals for treating thrombocytopenia, compound X maleic acid salt C type crystals for treating thrombocytopenia, and a method of treating thrombocytopenia by administering to a subject an effective dose of compound X maleic acid salt C type crystals. A "subject" refers to a human or other animal that requires treatment, and one embodiment is a human that requires treatment.

*Advantageous Effects of Invention:* Compound X maleic acid salt C type crystals ({drug1b}) exhibit excellent oral absorption, ensure constant quality even during mass production such as industrial production and the like, and can be stably supplied. Furthermore, compound X maleic acid salt C type crystals ({drug1b}) can be used as an active ingredient for a pharmaceutical composition for treating thrombocytopenia.

Crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt ({drug1a}) and Compound X maleic acid salt C type crystals ({drug1b}), respectively, can be obtained by the following procedures as described in WO2013/018362:

When manufacturing the compound X maleic acid salt crystals, the necessary starting compounds can be obtained as described below. In other words, compound X or a pharmaceutically acceptable salt thereof can be obtained by the method according to patent literature, a variation method thereof, or an obvious method to one skilled in the art.

Compound X maleic acid salt A type crystals and compound X maleic acid salt B type crystals can be obtained by crystallizing in a mixture of water and alcohol such as methanol, ethanol, isopropanol, and the like, and in another embodiment, can be obtained by crystallizing in 80% ethanol water, but on the other hand, compound X maleic acid salt C type crystal ({drug1b}) can be obtained by the following method. In other words, compound X maleic acid salt C type crystal ({drug1b}) can be obtained by crystallizing in: a mixture of water and acetone, methyl ethyl ketone, or acetonitrile, which may also contain dimethylsulfoxide (DMSO): or in another embodiment, in 50% or higher acetone water which may contain DMSO; or in yet another embodiment, in 60% or higher acetone water which may contain DMSO; or in yet another embodiment, in a mixture where acetone: water: DMSO = 1 ∼ 3:0.1 ∼ 2:1 ∼ 3, or in another embodiment, in a mixture where acetone: water: DMSO = 2:1:2.

The pharmacological activity of the compound X maleic acid salt C type crystals ({drug1b}) was confirmed by the following tests:

*Test Example I Cell Proliferation Test for Human c-mpl-Ba*/*F3 Cells:* Human c-mpl-Ba/F3 cells at a concentration of 2 x 10⁵ cells/mL were cultured at 37 °C on a 96 well microplate using RPMI 1640 culture medium (100 microliter/well) containing 10% fetal calf serum to which various concentrations of the test compound was added. After 24 hours from the start of culturing, 10 microliter/well of WST-1/1-methoxy PMS (cell measuring kit, Dojin) was added. Immediately after the addition and two hours later, the A450/A650 absorbance was measured using a microplate reader (Model 3350, Bio-Rad), and the increase in absorbance after 2 hours was interpreted to represent the proliferation activity of each test compound. The results showed that the cell proliferation effect of compound X maleic acid salt C type crystal ({drug1b}) on human c-mpl-Ba/F3 cells was 2.0 nM at EC₅₀. Furthermore, the maximum cell proliferation activity of compound X maleic acid salt C type crystals ({drug1b}) was 102%, based on the maximum cell proliferation activity of rhTPO being 100%. Furthermore, when Ba/F3 cells where c-mpl was not introduced were used, a cell proliferation effect was not observed, and therefore it was confirmed that compound X maleic acid salt C type crystals ({drug1b}) affect the proliferation of Ba/F3 cells through human c-mpl. The results of the aforementioned test clearly showed that, compound X maleic acid salt C type crystals ({drug1b}) have a thrombocytosis effect. Therefore, compound X maleic acid salt C type crystals ({drug1b}) are useful for treating and preventing various types of thrombocytopenia by having a thrombocytosis effect. Herein, the various types of thrombocytopenia include anaplastic anemia, thrombocytopenia during myelodysplastic syndrome, thrombocytopenia due to chemotherapy or radiation therapy of malignant tumors, idiopathic thrombocytopenic purpura, thrombocytopenia during hepatic disease, thrombocytopenia due to HIV, and the like. Furthermore, compound X maleic acid salt C type crystals ({drug1b}) have thrombocytosis effects, and therefore can prevent thrombocytopenia by being administered before performing various treatments including chemotherapy or radiation therapy where there is a possibility of causing thrombocytopenia.

A pharmaceutical composition containing compound X maleic acid salt C type crystals ({drug1b}) as an active ingredient can be prepared by a commonly used method using excipients that are normally used in this field, or in other words using excipients for drugs and carriers for drugs. Administration can be either by oral administration using tablets, pills, capsules, granules, powders, or liquid and the like, or by a non-oral administration using intra-articular, intravenous, or intramuscular injections, suppositories, eyedrops, eye ointments, transdermal liquids, ointments, transdermal patches, transmucosal liquids, transmucosal patches, and inhalants and the like. A solid composition for oral administration can be used in tablet, powder, and granule drugs. In these solid compositions, one or more type of active ingredient is blended with at least one type of inactive excipient. The composition may contain inert additives such as lubricating agents, disintegrating agents, stabilizers, and solubilizing agents, in accordance with standard methods. Tablets and pills can be coated with a sugar coating or with a gastric dissolving or enteric film, if necessary. Liquid compositions for oral administration include pharmacologically acceptable emulsions, solutions, suspensions, syrups or elixirs and the like, and contain commonly used inert diluents such as purified water or ethanol. These liquid compositions can also contain sweeteners, flavorings, fragrances, preservatives, and adjuvants such as wetting agents and suspending agents, in addition to the inert diluent. Injections for non-oral administration contain sterile aqueous or non-aqueous solutions, suspending agents, or emulsifiers. Examples of aqueous solutions include distilled water for injection and physiological saline solutions. Examples of nonaqueous solvents include alcohols such as ethanol. These compositions can also contain preservatives, wetting agents, emulsifiers, dispersing agents, stabilizers, or solubilizing agents. These agents are sterilized for example by filtering through a bacteria retaining filter, blending with a sterilizing agent, or sterilizing by irradiation. Furthermore, these compositions can be used by preparing a sterile solid composition, and dissolving or suspending in sterile water or a sterile solvent for injection prior to use. For the case of normal oral administration, a daily dose is between approximately 0.001 and 50 mg/kg of body weight, preferably between 0.1 and 10 mg/kg, and this dose is administered at one time or is divided between 2 to 4 times. For the case of intravenous administration, a daily dose of approximately 0.0001 to 1 mg/kg is suitable, and this dose is administered one time per day, or is divided over several administrations. The dose can be appropriately determined based on individual cases, considering the symptoms, age, sex and the like. The pharmaceutical composition according to aspect (i) of the present invention contains 0.1 to 100 weight %, or in another aspect, 0.01 to 50 weight %, of compound X maleic acid salt C type crystal ({drug1b}) which is the active ingredient, but this can vary depending on administration route, drug form, administration site, and type of excipients or additives. The compound X maleic acid salt C type crystals ({drug1b}) can be used in combination with other types of treatments or preventative agents for the aforementioned conditions that compound X maleic acid salt C type crystals ({drug1b}) are thought to be effective towards. This combined use can be administered simultaneously, or administered separately but continuously, or administered with a predetermined time interval. Simultaneously administered drugs can be manufactured as a mixed or fixed dose, or can be manufactured separately.

### Example

The compound X maleic acid salt C type crystal ({drug1b}) manufacturing method is described in WO2013/018362 and reiterated below in detail based on examples. The manufacturing method of the compound X maleic acid salt C type crystals ({drug1b}) is not restricted to only the manufacturing method specifically shown in the following examples, and manufacturing can also be performed by alternate methods of the manufacturing method shown below, or by methods that are obvious to one skilled in the art. For convenience, concentration mol/L is expressed as M. For example, a 1 M sodium hydroxide aqueous solution refers to a 1 mol/L aqueous solution of sodium hydroxide. The differential scanning calorimeter analysis was conducted on a test sample of approximately 5 mg using a TA Instruments TA 5000 (2910 AutoDSC) between room temperature and 300 °C or between room temperature and 400 °C (temperature increase rate: 10 °C/minute), under N₂ (flow rate: 50 mL/minute) using an aluminum sample pan. Furthermore, powder x-ray crystal diffraction was measured using a MXP18TAHF22 under conditions of tube: Cu, tube current: 40 mA, tube voltage: 40 kV, sampling width: 0.020 degree, scanning rate: 3 degree/minute, wavelength: 1.54056 angstrom, measurement diffraction angle range (2-theta): 3 to 40 degree or 5 to 40 degree.

Preparation Example 1: Manufacture of Compound X Maleic Acid Salt B Type Crystal: 310 mL of a 1 M aqueous solution of sodium hydroxide at room temperature was added to a mixture of 70.0 g of the ethyl ester of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid and 1.2 L of ethanol, the insoluble matter was filtered out, and then washed with 200 mL of ethanol. The reaction solution was stirred for 90 minutes at 60 °C. After cooling to room temperature, 1.4 L of an aqueous solution containing 24.11 g of maleic acid was added to the solution obtained, and then the precipitate was collected by filtering. The same operation was repeated and when combined with the previously obtained precipitate, 136.05 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid was obtained. 18.9 g of maleic acid and 2.1 L of 80% ethanol water were added to 88.90 g of the carboxylic acid obtained, and the solution was stirred for one hour at room temperature and for another hour at 100 °C. After cooling to room temperature and further cooling with ice, the precipitated solid was filtered out to obtain 87.79 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt as a crude product. 6.84 g of maleic acid was added to 231 g of the crude product containing the crude product obtained above and those manufactured in a similar manner, dissolved in 5.5 L of 80% ethanol water, and then the precipitated solid was collected by filtering to obtain 203 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt.

As shown in Preparation Example 1, when the preparation method of example 1 shown in patent literature was scaled up, the compound X maleic acid salt crystals exhibiting the differential scanning calorimeter analysis chart presented in FIG. 9 of WO2013/018362 were obtained, and were found to have different endothermic peaks when compared to the differential scanning calorimeter analysis chart of compound X maleic acid salt A type crystals presented in FIG. 7 of WO2013/018362. These results show that the compound X maleic acid salt A type crystals have a possibility that the crystal form will morph depending on the scale of production, and thus are clearly inappropriate as a drug substance for medicines which require constant quality and constant effects.
Example 1: Manufacture of Compound X Maleic Acid Salt C Type Crystals (1) ({drug1b}): 1.52 L of ethanol, 0.38 L of water, and 15.7 g of maleic acid were added to 78.59 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid, and heated while stirring. After cooling to room temperature and further cooling with ice, the precipitated solid was collected by filtering to obtain 71.60 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt as a crude product. 296 mg of maleic acid was added to 10.0 g of the crude product obtained, dissolved in 60 mL of acetone, 60 mL of DMSO, and 30 mL of water, and then the precipitated solids were collected to obtain 8.41 g of 1-(3-chlorc-5-{[4-(4-chlorothiophen-2-yl)-5-(4-cydohexylpiperazin-1-yl) thiazol-2-yl] carbamoyl} pyridin-2-yl) piperidine-4-carboxylic acid maleic acid salt.
Example 2: Manufacture of Compound X Maleic Acid Salt C Type Crystals (2) ({drug1b}): A mixture containing 80.1 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid, 580 mL of DMSO, 580 mL of acetone, 17.2 g of maleic acid, and 290 mL of water was stirred at 69 °C. The insoluble matter was filtered out, washed with a mixture of 32 mL of DMSO, 32 mL of acetone, and 16 mL of water, and then the filtrate was cooled and the precipitate was collected by filtering. Washing was successively performed using 150 mL of water, 80 mL of acetone, 650 mL of water, and 80 mL of acetone, followed by drying, to obtain 70.66 g of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt.
Example 3: Manufacture of Compound X Maleic Acid Salt C Type Crystals (3) ({drug1b}): A mixture containing 20 kg of 1-(3-chlorc-5-{[4-(4-chlorothiophen-2-yl)-5-(4-cyclohexyl piperazin-1-yl) thiazol-2-yl] carbamoyl} pyridin-2-yl) piperidine-4-carboxylic acid, 100 L of DMSO, 100 L of acetone, 4.29 kg of maleic acid, and 50 L of water is stirred at 65 °C, and then the insoluble matter is filtered out and washed with a mixture of 8 L of DMSO, 8 L of acetone, and 4 L of water, and then the filtrate is cooled, the precipitate is collected by filtering, successively washed using 40 L of acetone, 100 L of water, and 40 L of acetone, and then dried to obtain approximately 20 kg of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt.
Test Example 2: Mouse Oral Administration Test: 3 mg/kg of the test compound suspended in an aqueous solution of 0.5% methylcellulose was orally administered to male ICR mice. 2 hours after administration, blood was sampled from the inferior vena cava using 1/10 volume 3.8% sodium citrate solution as an anti-coagulating agent. The blood plasmas obtained by centrifugal separation at 12,000 RPM for 3 minutes were heated for 30 minutes at 56 °C and then added to the human c-mpl-Ba/F3 cell proliferation test system described in test example 1, to obtain 10%, 0.3% or 0.1% blood plasma concentration. The cell proliferation activity was measured and the cell proliferation activity (%) was determined based on the maximum cell proliferation activity of each test compound being 100%. The results are shown in Table 1. Note, "NT" indicates that a measurement was not performed.

**Table 1 Human c-mpl-Ba/F3 cell proliferation activity of blood platelets after orally administering test compounds:**

| Test Compound | Cell proliferation activity (%) | | |
|---|---|---|---|
| | 10% | 0.3% | 0.1% |
| Compound X hydrochloride | 34 | < 10 | NT |
| Compound X maleic acid salt A type crystal | >80 | 55 | NT |
| Compound X maleic acid salt B type crystal | NT | < 10 | NT |
| Compound X maleic acid salt C type crystal | NT | > 80 | 30 |

Compound X hydrochloride was manufactured in accordance with the method disclosed in patent literature. Furthermore, compound X maleic acid salt A type crystals were manufactured in accordance with the method disclosed in patent literature. From the foregoing results, it was made clear that of the three types of crystals of compound X maleic acid salt, compound X maleic acid salt A type crystals and compound X maleic acid salt C type crystal ({drug1b}) had excellent oral absorption, but compound X maleic acid B type crystals had inferior oral absorption. Therefore, the compound X maleic acid salt A type crystals had excellent oral absorption, but there is a possibility that the crystal form will morph depending on the scale of production, and therefore these crystals may be inappropriate as a drug substance for medicines which require constant quality and constant effects. Furthermore, the compound X maleic acid salt B type crystal can be stably supplied in mass production such as industrial production, but the oral absorption thereof was found to be inferior. On the other hand, compound X maleic acid salt C type crystals ({drug1b}) were the only crystal that had excellent oral absorbency and could be stably supplied in mass production such as industrial production.

All publications, patents and patent applications cited herein are incorporated herein by reference in their entirety.

Industrial Applicability: Compound X maleic acid salt C type crystals ({drug1b}) exhibit excellent oral absorption, ensure constant quality even during mass production such as industrial production and the like, and can be stably supplied. Furthermore, compound X maleic acid salt C type crystals ({drug1b}) can be used as an active ingredient for a pharmaceutical composition for treating thrombocytopenia.

Preferred embodiments of aspect (i) of the present invention are defined in the claims of WO2013018362 and are repeated as items 1 to 9 hereinafter:
[Item 1]: A crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt, wherein the endothermic peak top temperatures in differential scanning calorimeter analysis are between approximately 229 and 232 °C, and between approximately 300 and 303 °C. [Item 2]: A crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt, having peaks in powder x-ray crystal analysis using a Cu-K alpha beam at approximately 8.5 degree, approximately 9.5 degree, approximately 12.1 degree, approximately 20.4 degree, approximately 22.7 degree, and approximately 23.3 degree for a diffraction angle of 2-theta. [Item 3]: The crystal according to item 2, having endothermic peak top temperatures in differential scanning calorimeter analysis of approximately 229 to 232 °C, and approximately 300 to 303 °C. [Item 4]: A pharmaceutical composition comprising the crystal according to any one of items 1 to 3, and a pharmaceutically acceptable excipient. [Item 5]: The pharmaceutical composition according to item 4, which is a pharmaceutical composition for preventing or treating thrombocytopenia. [Item 6]: Use of the crystal according to any one of items I to 3, for producing a pharmaceutical composition for preventing or treating thrombocytopenia. [Item 7]: Use of the crystal according to any one of items 1 to 3, for preventing or treating thrombocytopenia. [Item 8]: The crystal according to any one of items 1 to 3, for preventing or treating thrombocytopenia. [Item 9]: A method of preventing or treating thrombocytopenia, comprising administering to a subject an effective dose of the crystal according to any one of items 1 to 3. Aspect (i) of the invention relates to an administration unit comprising crystals of 1-(3-chloro-5-{[4-(4-chlorothiophen-2-yl)-5-(4-cyclohexyl piperazin-1-yl) thiazol-2-yl] carbamoyl} pyridin-2-yl) piperidine-4-carboxylic acid maleic acid salt. The administration unit according to the invention is useful for treating various diseases and disorders which include but are not limited to thrombocytopenia.

### SPECIFIC INORMATION CONCERNING ASPECT (II) OF THE INVENTION

Aspect (ii) of the invention concerns forms of {drug2}, especially Crizotinib for treatment of cancer, namely to a solid form of Crizotinib for (use in the) treatment of cancer ({drug2a}), which is useful for treating cancer, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof.

Crizotinib for treatment of cancer ({drug2}) and / or solid form of Crizotinib for treatment of cancer ({drug2a}) is described in WO2013/017989, which is incorporated by reference. Crizotinib is represented by formula 1 (cf. WO2013017989): 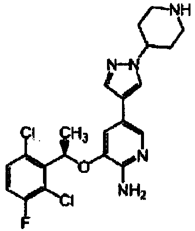 or a pharmaceutically acceptable salt thereof, wherein the cancer is mediated by at least one genetically altered ROS. The compound of formula 1 may be variously referred to herein by its generic name, Crizotinib, or by its chemical name, 3-[(R)-1-(2,6-dichloro-3-fluoro-phenyl)-ethoxy]-5-(1-piperidin-4-yl-1-*H* -pyrazol-4-yl)-pyridin-2-ylamine. Aspect (ii) of the invention relates to an administration unit comprising Crizotinib for (use in the) treatment of cancer ({drug2}) and / or a solid form of Crizotinib for (use in the) treatment of cancer ({drug2a}). The administration unit according to the invention is useful for treating various diseases and disorders which include but are not limited to cancer, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof.

Preferred embodiments of the administration unit are defined in the claims of WO2013017989 and are reiterated as items 1 to 15 hereinafter:
[Item 1]: A method of treating cancer in a mammal comprising administering to said mammal a therapeutically effective amount of 3-[(*R*)-1-(2,6-dichloro-3-fluoro-phenyl)-ethoxy]-5-(1-piperidin-4-yl-1-*H* -pyrazol-4-yl)-pyridin-2-ylamine or a pharmaceutically acceptable salt thereof, wherein the cancer is mediated by at least one genetically altered ROS. [Item 2]: The method of item 1, wherein the mammal is a human. [Item 3]: The method of item 1, wherein the at least one genetically altered ROS is a genetically altered ROS gene. [Item 4]: The method of item 3, wherein the genetically altered ROS gene is a ROS fusion gene. [Item 5]: The method of item 4, wherein the ROS fusion gene is the SLC34A2-ROS gene or the CD74-ROS gene. [Item 6]: The method of item 4, wherein the ROS fusion gene is the FIG-ROS gene. [Item 7]: The method of item 1, wherein the at least one genetically altered ROS is a ROS fusion protein. [Item 8]: The method of item 7, wherein the ROS fusion protein is SLC34A2-ROS kinase. [Item 9]: The method of item 7, wherein the ROS fusion protein is CD74-ROS kinase. [Item 10]: The method of item 7, wherein the ROS fusion protein is FIG-ROS kinase. [Item 11]: The method of any one of items 1-10, wherein the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. [Item 12]: The method of any one of items 1-10, wherein the cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. [Item 13]: The method of any one of items 1-10 wherein the cancer is non-small cell lung cancer (NSCLC). [Item 14]: The method of any one of items 1-10 wherein the cancer is glioblastoma. [Item 15]: The method of any one of the preceding items, wherein 3-[(R)-1-(2,6-dichloro-3-fluoro-phenyl)-ethoxy]-5-(1-piperidin4yl- H-pyrazol-4-yl)-pyridin-2-ylamine or a pharmaceutically acceptable salt thereof is administered as a pharmaceutical composition comprising 3-[(R)-1-(2,6-dichloro-3-fluoro-phenyl)-ethoxy]-5-(1-piperidin-4-yl-1 Hpyrazol-4-yl)-pyridin-2-ylamine or a pharmaceutically acceptable salt thereof and at least one pharmaceutically acceptable carrier.

Details and Synthesis are described in WO2013017989 and are reiterated here:

### Examples

*In-Vitro Assays - Materials and Methods - In-Vitro Methods - ROS-1 Enzymatic Assay:* Inhibition of ROS-1 enzyme was measured using a microfluidic mobility shift assay. The reactions were conducted in 50 µl_ volumes in 96-well plates, and contained 0.25 nM recombinant human ROS-1 catalytic domain (aa 1883-2347), GST-tagged (Invitrogen Inc), 1.5 µM phosphor-acceptor peptide, 5'FAM-KKSRGDYMTMQIG-CONH₂ (Caliper LifeSciences), test compound (1 1-dose 3-fold serial dilutions, 2% DMSO final) or DMSO only, 1 mM DTT, 0.002% Tween-20 and 5 mM MgCl₂ in 25 mM Hepes, pH 7.1, and were initiated by addition of ATP (56 µM final concentration, - Km level) following a 20-min pre-incubation. The reactions were incubated for 1 hour at room temperature, then stopped by the addition of 0.1 M EDTA (pH 8). The extent of reaction completion (-5% conversion with DMSO) was determined after electrophoretic separation of the fluorescently labeled peptide substrate and phosphorylated product on a LabChip EZ Reader II (Caliper LifeSciences). Ki values for each trial was calculated by fitting the % conversion to the equation for competitive inhibition using non-linear regression method (Graph Pad Prism, Graph Pad Software, San Diego, CA) and experimentally measured ATP Kₘ = 56 µM. A panel of four trials gave an average Ki value of 0.097 nM. *Cell lines:* HCC78 cells are a human non-small cell lung carcinoma cell line established from the pleural effusion of a 65-year-old man with adenocarcinoma of the lung, typed as non-small cell lung carcinoma. HCC78 cells were purchased from DSMZ cell bank (Braunschweig, Germany). U138 cells and NIH3T3 cells were purchased from American Tissue Culture Corporation TCC. *NIH3T3-ROS Fusion Cell Line Generation:* The NIH3T3 ROS fusion engineered cell lines were generated in house. ROS fusion variants SLC34A2-ROS (L), SLC34A2-ROS (S), CD74-ROS (L), FIG-ROS (L) and FIG-ROS (S) were cloned into the retroviral vector pMSCV puro (Clontech). The retroviruses carrying EML4-ALK genes were produced in 293T cells by co-transfection with the pMSCV vectors and the packaging plasmid pC10A1. The retroviral supernatants were used to transduce NIH3T3 cells and pooled populations were selected with 2µg/ml puromycin for 5 days and verified by DNA sequencing prior to use in subsequent experiments. *Cellular kinase phosphorylation assays:* Cellular assays (i.e., ELISA or immunoblot) used to directly determine the ability of Crizotinib to inhibit ligand-dependent or constitutive kinase phosphorylation were performed using a variety of serum-starved cells. *Cell-based Phospho-ROS ELISA Assay:* A panel of cell lines harboring various kinds of ROS fusion was used to determine the potency of Crizotinib on ROS phosphorylation. The cells were plated at a density of 20,000 cells/well in 100 µl of the growth media in 96-well plates. The ROS fusion negative cells wells were used as background. Plated cells were allowed to adhere overnight. The following day, growth media was removed and cells were cultured in serum-free media (with 0.04% BSA). Serial dilutions of Crizotinib were performed, appropriate controls or designated concentrations of Crizotinib were added to each well, and cells were incubated at 37°C for 1 hour. Cell lysates were generated and the total phospho-tyrosine levels of SLC34A2-ROS in HCC78 cells were determined by using the PathScan® Phospho-Ros (panTyr) Sandwich ELISA Kit (Cell Signaling, Cat#: 7093) as described in the manufacturer's protocol. The EC50 values were calculated by concentration-response curve fitting utilizing a four-parameter analytical method. *Cell-based Phospho-ROS ELISA Assay for SLC34A2-ROS:* HCC78 cells harboring SLC34A2-ROS fusion were used to determine the potency of Crizotinib on ROS phosphorylation. HCC78 cells were plated at a density of 20,000 cells/well in 100 µl of RPMI media with 10% FBS and penicillin/streptomycin in 96-well plates. The no cell wells were used as background. Plated cells were allowed to adhere overnight. The following day, growth media was removed and cells were cultured in serum-free media (with 0.04% BSA). Serial dilutions of Crizotinib were performed, appropriate controls or designated concentrations of Crizotinib were added to each well, and cells were incubated at 37°C for 1 hour. Cell lysates were generated and the total phospho-tyrosine levels of SLC34A2-ROS in HCC78 cells were determined by using the PathScan® Phospho-Ros (panTyr) Sandwich ELISA Kit (Cell Signaling, Cat#: 7093) as described in the manufacturer's protocol. The IC50 values were calculated by concentration-response curve fitting utilizing a four-parameter analytical method. The cell-based phospho-ROS ELISA assay provided a mean IC50 value of 45 nM (n=8). *Immunoblotting:* Immunoblotting methods were also used to determine relative kinase phosphorylation status and total protein levels in the HCC78 cells and 3T3-CD74-ROS tumor lysates for the protein of interest. For in vitro study, the HCC78 cells were treated with various dose levels of Crizotinib for three hours. The cells were lysed in the cold 1X Cell Lysis Buffer (Cell Signaling Technologies, Boston MT). For in vivo study, tumor bearing mice were treated with Crizotinib 75mg/kg PO BID for 10 days. At the end of study, tumors were resected after 7 hours following the last dose. The resected tumors were snap frozen on dry ice, pulverized using a liquid nitrogen-cooled cryomortar and pestle, and lysed in cold 1 X Cell Lysis Buffer (Cell Signaling Technologies, Boston MT). The Proteins were extracted from cell and tumor lysates and protein concentrations were determined using a BSA assay (Pierce, Rockford, 1L). Extracted protein samples from both cell and tumor lysates were separated by SDS-PAGE, transferred to nylon membranes, and immunoblotting hybridizations for the proteins of the interest were performed with the following antibodies. *Antibodies utilized in immunoblotting studies were all from Cell Signaling:* Technology (Danvers, Massachusetts, United States) and listed as follows: anti-total ROS (catalog*: 3266), anti-phospho ROS (catalog*: 3078), anti-phospho SHP2 (catalog*: 5431), anti-phospho STAT3 (catalog*: 9131), anti-total AKT (catalog*: 9272), anti-phospho-AKT S473 (catalog*: 4161), anti-total -MAPK44/42 (catalog*: 9102), anti phospho-MAPK44/42 (catalog*: 4370), cleaved Caspapse-3 (catalog*: 9661). *Cell viability, proliferation and survival assays:* Cell Viability Assay: Cultured HCC78 cells were adapted to RPM1 growth medium (Invitrogen, Carlsbad, CA) with 10% FBS and penicillin/streptomycin (Invitrogen) whenever possible to standardize screening. Some cells requiring special media were grown in vendor recommended media. Cells were trypsinized and seeded at a density of 3000-5000 cells/well into 96-well plates (Coming Costar #3904 plates, Kennebunk, ME) and allowed to adhere overnight. The following day, cells were treated with single agent drug administered in nine serial concentrations in duplicates (progressively decreasing from 10 µM to 152 pM by a 4-fold ratio yielding a full sigmoidal curve). After an additional 3-5 days incubation at 37°C (until cell confluency reached -70-80%), 1/5 of manufacturer's recommended volume of Cell Titer Glo (Promega, Madison, W1) was added to indirectly measure cell viability/proliferation using an Envision multi-reader (Perkin-Elmer, Waltham, MA). Baseline cell count readings were also taken from cell plates one-day after cell seeding and prior to drug treatment. Baseline count was subtracted from final cell count and plotted with PRISM (Graphpad, La Jolla, CA) or XLFIT (IDBS, Surrey, UK). The IC₅o value calculated for inhibition of HCC78 cell viability was approximately 59 nM. *Cell Proliferation*/*Survival Assay:* The cells were seeded in 96 well plates at low density in growth media (media supplemented with 2%, 5% or 10% fetal bovine serum-FBS) and cultured overnight at 37°C. The following day, serial dilutions of Crizotinib or appropriate controls were added to the designated wells, and cells were incubated at 37°C for 72 hours. A Cell Titer Glo assay (Promega, Madison, W1) was then performed to determine the relative cell numbers. EC₅o values were calculated by concentration-response curve fitting utilizing a four-parameter analytical method. *In Vivo Methods:* Subcutaneous Xenograft Models in Athymic Mice: Female nu/nu mice (5-8 weeks old) were obtained from Charles River (Wilmington, MA). Animals were maintained under clean room conditions in sterile filter top cages with Alpha-Dri/bed-o-cob comb bedding housed on HEPA-filtered ventilated racks. Animals received sterile rodent chow and water ad libitum. The designated cells for implantation into athymic mice were harvested and pelleted by centrifugation at 450Xg for 5-10 minutes. The cell pellets were washed once and re-suspended in serum-free medium. The cells were supplemented with 50% Matrigel (BD Biosciences, San Jose CA) to facilitate tumor take. Cells (5 x 106 in 100 µI_) were implanted SC into the hind flank region of the mouse and allowed to grow to the designated size prior to the administration of compound for each experiment. Tumor size was determined by measurement with electronic calipers and tumor volume was calculated as the product of its length x width² x 0.4.

### Data and Results:

Example 1: Inhibition of ROS1 kinase activity in biochemical enzyme assays by Crizotinib Crizotinib was evaluated for its effect on ROS catalytic activity in both enzyme and cell-based assays. Crizotinib was demonstrated to be a potent ATP-competitive inhibitor of recombinant, human ROS1 kinase (catalytic domain) with a mean Ki value of 0.097 nM (n=4).
Example 2: Kinase Activity of Crizotinib in Cell-based Assays: Crizotinib dose-dependently inhibited ROS phosphorylation with a mean IC₅o value of 41 nM (n=11) in the HCC78 cells that exhibit a 4p15, 6q22 chromosomal translocation event resulting in the expression of a constitutively active SLC34A2-ROS fusion protein (Rikova et al. (2007)) in these cells (Table 1, FIG. 1 of WO2013017989). Crizotinib also inhibited ROS phosphorylation with a mean IC50 value of 49 nM (n=2) in the U138MG human glioblastoma cells harboring FIG-ROS fusion (Charest, et al. (2003)) (Table 1, FIG. 1 of WO2013017989). In a panel of 3T3 cell lines that were engineered to express various ROS-fusion proteins, Crizotinib inhibited ROS phosphorylation with IC50 values ranging from 3.4 nM to 36 nM in these cells (Table 1). Table 1:

| **Cell-based ROS1 Kinase Phosphorylation Assays** | **IC₅₀ (nM) Mean + STD** | **n** |
|---|---|---|
| Endogenous SLC34A2-ROS phosphorylation in HCC78 human NSCLC cells | 41 ± 14 | 11 |
| Endogenous FIG-ROS phosphorylation in U138MG human glioma cells | 49 ± 18 | 2 |
| Engineered CD74-ROS phosphorylation in 3T3-CD74-ROS cells | 3.4 ± 2.4 | 4 |
| Engineered FIG-ROS(S) phosphorylation In 3T3-FIG-ROS(S) cells | 19 ± 11 | 4 |
| Engineered FIG-ROS(L) phosphorylation in 3T3-FIG-ROS(L) cells | 8.7 ± 0.6 | 3 |
| Engineered SLC34A2-ROS(S) phosphorylation In 3T3- SLC34A2-ROS(S) cells | 19 + 15 | 2 |
| Engineered SLC34A2-ROS(L) phosphorylation in 3T3- SLC34A2-ROS(L) cells | 36 | 1 |

Example 3: Inhibition of ROS mediated signal transduction and induction of cell apoptosis in the HCC78 human NSCLC cells in vitro: Crizotinib was evaluated for its ability to inhibit SLC34A2-ROS dependent signaling pathways in the HCC78 cells. As illustrated in the immunoblot in FIG. 3 of WO2013/017989, Crizotinib dose dependency inhibited ROS phosphorylation (activation loop), as well as the downstream adaptor or signaling molecules including SHP2, STAT3, AKT and ERK1/2 following 3 hours of drug treatment in the HCC78 cells in vitro. These data demonstrated a correlation between key signaling pathways and efficacious doses of Crizotinib. Crizotinib was evaluated for its dose-dependent modulation of the caspase-3 marker of apoptosis utilizing Western Blot analysis. Following 3-hour of drug treatment, a significant dose-dependent induction of activated caspase-3 levels was observed in the HCC78 NSCL cells (FIG. 4 of WO2013017989) indicating that increased apoptosis also correlated with efficacious dose levels.
Example 4: Cytoreductive effect of Crizotinib following oral administration in a panel of oncogenic ROS fusion variants engineered xenograft tumor models in nude mice: The antitumor efficacy of Crizotinib was evaluated in a panel of ROS fusion engineered tumor xenograft models in the NIH3T3 cells representative of human cancer indications in which ROS chromosomal translocation is implicated, including CD74-ROS, two forms of SLC34A2-ROS that were identified in human NSCLC, and two forms of FIG-ROS that were identified in human NSCLC, glioblastoma and cholangiocarcinoma (Rimkunas et al. (2012) Clin Cancer Res. Jun 1. [Epub ahead of print]); Gu et al. (2011) PLoS One. 6(1):e 15640). Crizotinib demonstrated significant cytoreductive effects in all of the five 3T3-ROS engineered tumor models that harbor human oncogenic ROS fusion variants with a dosing regimen of 75/mg PO BID as shown in FIG. 5 of WO2013017989. The mice started receiving Crizotinib treatment when the tumor volume reached -200 mm³, and the tumors regressed rapidly to the size of 5 to 10 mm³ in about 4 to 5 days of drug treatment. The control tumors reached the size of 1500 mm³ in -7days after dosing start, and the average Crizotinib treatment time for this study was -10 days.
Example 5: Dose-dependent inhibition of ROS phosphorylation and tumor growth in the 3T3-CD74-ROS and the 3T3-SLC34A2-ROS(U xenograft models in nude mice by Crizotinib: To evaluate pharmacodynamic inhibition of ROS kinase activity and tumor growth by Crizotinib, 3T3-CD74-ROS tumor xenograft study in nude mice with oral BID dosing at multiple dose levels was conducted. The tumor volume was measured utilizing electronic Vernier calipers throughout the study and tumors samples were harvested at 7-hour following oral administration of Crizotinib for 10 days (steady-state). ROS phosphorylation status in tumors was quantitated by ELISA. Crizotinib demonstrated dose-dependent inhibition in tumor growth as shown in FIG. 6B of WO2013017989. Tumor regressions of 94% and 61 % were observed in the 160 mg/kg/day group (80 mg/kg BID) and the 80 mg/kg/day group (40 mg/kg BID) respectively, and 78% and 54% tumor growth inhibition were observed in the 40 mg/kg/day group (20 mg/kg BID) and the 20 mg/kg/day group (10 mg/kg BID), respectively. At 7 hours post last Crizotinib oral administration, significant inhibition of ROS phosphorylation in the 3T3-CD74-ROS tumors was observed across all the treatment groups (FIG. 6A of WO2013/017989). A similar degree of antitumor efficacy by Crizotinib in the 3T3-SLC34A2-ROS(L) model was also observed (FIG. 7 of WO2013/017989). Example 6: Synthesis of the compound of formula 1 (Crizotinib): PLE is an enzyme produced by Roche and sold through Biocatalytics Inc. as a crude esterase preparation from pig liver, commonly known as PLE-AS (purchased from Biocatalytics as ICR-123, sold as an ammonium sulfate suspension). The enzyme is classified in the CAS registry as a "carboxylic-ester hydrolase, CAS no. 9016-18-6". The corresponding enzyme classification number is EC 3.1.1.1. The enzyme is known to have broad substrate specificity towards the hydrolysis of a wide range of esters. The lipase activity is determined using a method based on hydrolysis of ethyl butyrate in a pH titrator. 1 LU (lipase unit) is the amount of enzyme which liberates I µηηoI titratable butyric acid per minute at 22°C, pH 8.2. The preparation reported herein (PLE-AS, as a suspension) is usually shipped as an opaque brown-green liquid with a declared activity of > 45 LU/mg (protein content around 40 mg/mL). (1S)-1-(2,6-dichloro-3-fluorophenylethanol: (1 S)-1-(2,6-dichloro-3-fluorophenyl)ethanol, shown as compound (S-1) in the schemes below, was prepared by a combination of enzymatic hydrolysis of racemic 1-(2,6-dichloro-3-fluorophenyl)ethyl acetate, esterification and chemical hydrolysis with inversion according to Scheme B. Racemic 1-(2,6-dichloro-3-fluorophenyl)ethyl acetate (compound A2) was prepared according to Scheme A. Scheme A: 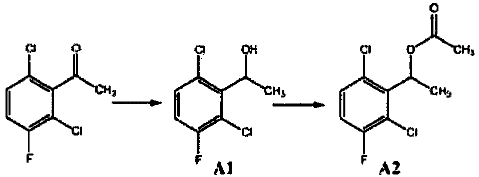 1-(2,6-dichloro-3-fluorophenyl)ethanol (A1): Sodium borohydride (90 mg, 2.4 mmol) was added to a solution of 2',6'-dichloro-3'-fluoro-acetophenone (Aldrich, catalog # 52,294-5) (207 mg, 1 mmol) in 2 ml. of anhydrous CH₃OH. The reaction mixture was stirred at room temperature for 1 h then was evaporated to give a colorless oil residue. The residue was purified by flash chromatography (eluting with 0->10% EtOAc in hexanes) to give compound A1 as a colorless oil (180 mg; 0.88 mmol; 86.5% yield); MS (APCI) (M-H)" 208; 1 H NMR (400 MHz, chloroform-D) δ ppm 1.64 (d, J=6.82 Hz, 3 H) 3.02 (d, J=9.85 Hz, 1 H) 6.97 - 7.07 (m, 1 H) 7.19 - 7.33 (m, 1 H).
1-(2,6-dichloro-3-fluorophenyl)ethyl acetate (A2): Acetic anhydride (1.42 ml, 15 mmol) and pyridine (1.7 ml, 21 mmol) were added sequentially to a solution of compound A1 (2.2 g, 10.5 mmol) in 20 ml. of CH2Cl2. The reaction mixture was stirred at room temperature for 12h and then evaporated to give a yellowish oil residue. The residue was purified by flash chromatography (eluting with 7->9% EtOAc in hexanes) to give compound A2 as a colorless oil (2.26 g; 9.0 mmol; 85.6% yield); 1 H NMR (400 MHz, chloroform-D) δ ppm 1.88 (d, J=6.82 Hz, 3 H) 2.31 (s, 3 H) 6.62 (q, J=6.82 Hz, 1 H) 7.25 (t, J=8.46 Hz, 1 H) 7.49 (dd, J=8.84, 5.05 Hz, 1 H).Scheme B:

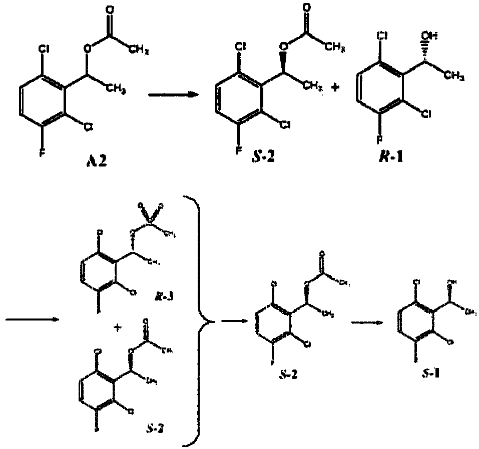

To a 50 mL jacketed flask equipped with a pH electrode, an overhead stirrer and a base addition line (1 M NaOH), was added 1.2 mL of 100 mM potassium phosphate buffer pH 7.0 and 0.13 mL of PLE AS suspension. Then, compound A2 (0.13 g, 0.5 mmol, 1.00 eq) was added dropwise and the resulting mixture was stirred at room temperature for 20 h, maintaining the pH of the reaction constant at 7.0 using 1 M NaOH. Both the conversion and enantiomeric excesses (ee's) of the reaction were monitored by RP-HPLC, and stopped after 50% starting material was consumed (approximately 17 hours under these conditions). The mixture was then extracted three times with 10 mL of ethyl acetate to recover both ester and alcohol as a mixture of R-1 and S-2. Methanesulfonyl chloride (0.06 mL, 0.6 mmol) was added to a solution of a mixture of R-1 and S-2 (0.48 mmol) in 4 mL of pyridine under nitrogen atmosphere. The reaction mixture was stirred at room temperature for 3 h then evaporated to obtain an oil. Water (20 mL) was added to the mixture and then EtOAc (20 mL x 2) was added to extract the aqueous solution. The organic layers were combined, dried, filtered, and evaporated to give a mixture of R-3 and S-2. This mixture was used in the next step reaction without further purification. ¹H NMR (400 MHz, chloroform-D) δ ppm 1.66 (d, =7.1 Hz, 3 H) 1.84 (d, =7.1 Hz, 3 H) 2.09 (s, 3 H) 2.92 (s, 3 H) 6.39 (q, J=7.0 Hz, 1 H) 6.46 (q, J=6.8 Hz, 1 H) 6.98 - 7.07 (m, 1 H) 7.07 - 7.17 (m, 1 H) 7.23 - 7.30 (m, 1 H) 7.34 (dd, J=8.8, 4.80 Hz, 1 H). Potassium acetate (0.027 g, 0.26 mmol) was added to a mixture of R-3 and S-2 (0.48 mmol) in 4 mL of DMF under nitrogen atmosphere. The reaction mixture was heated to 100°C for 12 h. Water (20 mL) was added to the reaction mixture and EtOAc (20 mL x 2) was added to extract the aqueous solution. The combined organic layer was dried, filtered, and evaporated to give an oil of S-2 (72 mg, 61 % yield in two steps). Chirality ee: 97.6%. ¹H NMR (400 MHz, chloroform-D) δ ppm 1.66 (d, J=7.1 Hz, 3 H) 2.09 (s, 3 H) 6.39 (q, J=6.8 Hz, 1 H) 7.02 (t, =8.5 Hz, I H) 7.22 - 7.30 (m, 1 H). Sodium methoxide (19 mmol; 0.5 M in methanol) was added slowly to compound S-2 (4.64 g, 18.8 mmol) under a nitrogen atmosphere at 0°C. The resulting mixture was stirred at room temperature for 4 hours. The solvent was evaporated and H₂O (100 mL) was added. The cooled reaction mixture was neutralized with sodium acetate-acetic acid buffer solution to pH 7. Ethyl acetate (100 mL x 2) was added to extract the aqueous solution. The combined organic layers were dried over Na₂SO₄, filtered, and evaporated to obtain S-1 as a white solid (4.36 g, 94.9% yield); SFC-MS: 97%ee. ¹H NMR (400 MHz, chloroform-D) δ ppm 1.65 (d, J=6.8 Hz, 3 H) 5.58 (q, J=6.9 Hz, 1 H) 6.96 - 7.10 (m, 1 H) 7.22 - 7.36 (m, 1 H).

5-bromo-3-[1-(2,6-dichloro-3-fluoro-phenyl)-ethoxyl-pyridin-2-ylamine (racemate): 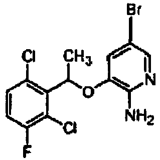
1.) 2,6-Dichloro-3-fluoroacetophenone (15 g, 0.072 mol) was stirred in THF (150 mL, 0.5M) at 0°C using an ice bath for 10 min. Lithium aluminum hydride (2.75 g, 0.072mol) was slowly added. The reaction was stirred at ambient temperature for 3 hr. The reaction was cooled in ice bath, and water (3 mL) was added drop wisely followed by adding 15% NaOH (3 mL) slowly. The mixture was stirred at ambient temperature for 30 min. 15% NaOH (9 mL), MgSO₄ were added and the mixture filtered to remove solids. The solids were washed with THF (50 mL) and the filtrate was concentrated to give 1-(2,6-dichloro-3-fluoro-phenyl)-ethanol (14.8 gm, 95% yield) as a yellow oil. ¹H NMR (400 MHz, DMSO-d₆) δ 1.45 (d, 3H), 5.42 (m, 2H), 7.32 (m, 1 H), 7.42 (m, I H). 2.) To a stirred solution of triphenyl phosphine (8.2 g, 0.03 mol) and DEAD (13.65 mL of a 40% solution in toluene) in THF (200 mL) at 0°C was added a solution of 1-(2,6-dichloro-3-fluoro-phenyl)-ethanol (4.55 g, 0.021 mol) and 3-hydroxy-nitropyridine (3.35 g, 0.023 mol) in THF (200 mL). The resulting bright orange solution was stirred under a nitrogen atmosphere at ambient temperature for 4 hours at which point all starting materials had been consumed. The solvent was removed, and the crude material was dry loaded onto silica gel, and eluted with ethyl acetate-hexanes (20:80) to yield 3-(2,6-dichloro-3-fluoro-benzyloxy)-2-nitro-pyridine (6.21 g, 0.021 mol, 98%) as a pink solid. ¹H NMR (CDCl₃, 300 MHz) 51.8-1.85 (d, 3H), 6.0-6.15 (q, 1 H), 7.0-7.1 (t, 1 H), 7.2-7.21 (d, 1 H), 7.25-7.5 (m, 2H), 8.0-8.05 (d, 1 H). 3.) To a stirred mixture of AcOH (650 mL) and EtOH (500 mL) was suspended 3-(2,6-dichloro-3-fluoro-benzyloxy)-2-nitro-pyridine (9.43 g, 0.028 mol) and iron chips (15.7 g, 0.28 mol). The reaction was heated slowly to reflux and allowed to stir for 1 hr. The reaction was cooled to room temperature then diethyl ether (500 mL) and water (500 mL) was added. The solution was carefully neutralized by the addition of sodium carbonate. The combined organic extracts were washed with sat'd NaHCO₃ (2 x 100 mL), H₂O (2 x 100 mL) and brine (1 x 100 mL) then dried (Na₂SO₄), filtered and concentrated to dryness under vacuum to yield 3-(2,6-dichloro-3-fluoro-benzyloxy)-pyridin-2-ylamine (9.04 g, 0.027 mol, 99%) as a light pink solid. ¹H NMR (CDCl₃, 300 MHz) 51.8-1.85 (d, 3H), 4.9-5.2 (brs, 2H), 6.7-6.84 (q, 1 H), 7.0-7.1 (m, 1 H), 7.2-7.3 (m, 1 H), 7.6-7.7 (m, 1 H). 4.) A stirring solution of 3-(2,6-dichloro-3-fluoro-benzyloxy)-pyridin-2-ylamine (9.07 g, 0.03 mol) in acetonitrile was cooled to 0°C using an ice bath. To this solution was added N-bromosuccinimide (NBS) (5.33 g, 0.03 mol) portionwise. The reaction was stirred at 0°C for 15 min. The reaction was concentrated to dryness under vacuum. The resulting dark oil was dissolved in EtOAc (500 mL), and purified via silica gel chromatography. The solvents were then removed in vacuo to yield 5-bromo-3-(2,6-dichloro-3-fluoro-benzyloxy)-pyridin-2-ylamine (5.8 g, 0.015 mol, 51 %) as a white crystalline solid. ¹H NMR (CDCl₃, 300 MHz) 51.85-1.95 (d, 3H), 4.7-5.0 (brs, 2H), 5.9-6.01 (q, 1 H), 6.8-6.95 (d, 1 H), 7.01-7.2 (t, 1 H), 7.4-7.45 (m, 1 H), 7.8-7.85 (d, 1 H). 5-bromo-3-[1-(R)-(2,6-dichloro-3-fluoro-phenyl)-ethoxyl-pyridin-2-ylamine:

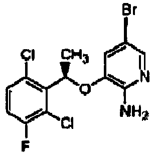

The enantiomerically pure R isomer was prepared as described above for the racemate, but using the enantiomerically pure starting materials described above. ¹H NMR (400 MHz, DMSO-d₆) δ 1.74 (d, 3H), 6.40 (m, 1 H), 6.52 (br s, 2H), 7.30 (m, I H), 7.48 (m, 1 H), 7.56 (s, 1 H); MS m/z 382 (M+1).

4-methanesulfonyloxy-piperidine-1-carboxylic acid tert-butyl ester (2):

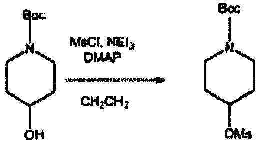

To a stirred solution of 4-hydroxy-piperidine-1-carboxylic acid tert-butyl ester (7.94 g, 39.45 mmol) in CH₂Cl₂ (100 mL), cooled to 0°C, was slowly added NEt₃ (5.54 mL, 39.45 mmol) followed by methane sulfonyl chloride (3.06 mL, 39.45 mmol) and DMAP (48 mg, 0.39 mmol). The mixture was stirred at room temperature overnight. To the mixture was added water (30 mL). Extraction with CH₂Cl₂ (3 x 30 mL) followed by drying (Na₂SO₄) and removal of the solvent in vacuo afforded 4-methanesulfonyloxy-piperidine-1-carboxylic acid tert-butyl ester as a white solid (1 1.00 g, >99% yield). ¹H NMR (CDCI3, 400 MHz) δ 4.89 (m, 1 H), 3.69 (m, 2H), 3.31 (m, 2H), 3.04 (s, 3H), 1.95 (m, 2H), 1.83 (m, 2H), 1.46 (s, 9H).

tert-butyl-4-[4-(4,4,5,5-tetramethyl-1,3,2-dioxaborolan-2-yl)-1 H-pyrazol-1-yllpiperidine-1-carboxylate:

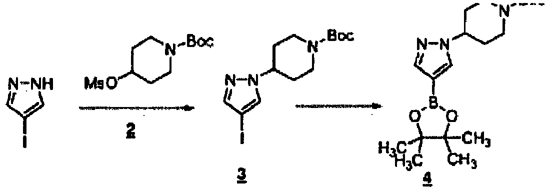

tert-butyl 4-(4-iodo-1 H-pyrazol-1-yl)piperidine-1-carboxylate (3): NaH (1.2 eq., 0.68 mmol) was added portionwise to a stirred solution of 4-iodopyrazole (0.57 mmol) in DMF (2 L) at 4°C. The resulting mixture was stirred for 1 hour at 4°C and 4-methanesulfonyloxy-piperidine-1-carboxylic acid tert-butyl ester, compound 2 (1.1 eq., 0.63 mmol) was then added. The resulting mixture was heated to 100°C for 12 h. The reaction was quenched with H₂O and extracted with EtOAc several times. The combined organic layers were dried, filtered, and concentrated to afford an orange oil. The residue was purified by silica gel chromatography (eluting with 5% EtOAc in pentane) to give compound 3 as a white solid (140 g, 66%).

tert-butyl-4-[4-(4,4,5,5-tetramethyl-1,3,2-dioxaborolan-2-yl)-1 H-pyrazol-1-yllpiperidine-1-carboxylate (4): Bis(pinacolato)-diboron (1.4 eq., 134 g, 0.52 mol) and potassium acetate (4 eq., 145 g, 1.48 mol) were added sequentially to a solution of compound 3 (140 g, 0.37 mol) in 1. 5 L of DMSO. The mixture was purged with nitrogen several times and dichlorobis(triphenylphosphino) palladium (II) (0.05 eq., 12.9 g, 0.018 mol) was then added. The resulting mixture was heated at 80°C for 2 h. The reaction mixture was cooled to room temperature and filtered through a bed of Celite® and washed with EtOAc. The filtrate was washed with saturated NaCl (500 ml. x 2), dried over Na₂SO₄, filtered and concentrated. The residue was purified by silica gel chromatography (eluting with 5% EtOAc in hexanes) to give compound 4 as a white solid (55 g, 40%).

3-[(R)-1-(2,6-dichloro-3-fluoro-phenyl)-ethoxyl-5-(1-piperidin-4-yl-1H-pyrazol-4-yl)-pyridin-2-ylamine (1):

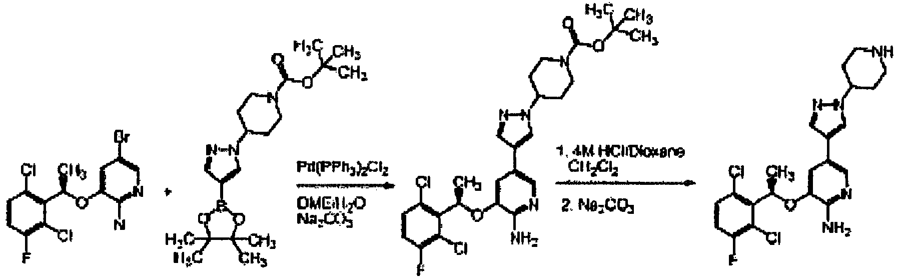

To a stirred solution of 3-[(R)-1-(2,6-dichloro-3-fluoro-phenyl)-ethoxy]-5-(4,4,5,5-tetramethyl-[1,3,2]dioxaborolan-2-yl)-pyridin-2-ylamine (15.22 g, 35.64 mmol) and 4-(4-bromo-pyrazol-1-yl)-piperidine-1-carboxylic acid tert-butyl ester (14.12 g, 42.77 mmol) in DME (143 ml.) was added a solution of Na₂CO₃ (1 1.33 g, 10692 mmol) in water (36 ml_). The solution was degassed and charged with nitrogen three times. To the solution was added Pd(PPh₃)₂Cl2 (1.25 mg, 1.782 mmol). The reaction solution was degassed and charged with nitrogen again three times. The reaction solution was stirred at 87°C oil bath for about 16 hours (or until consumption of the borane pinacol ester), cooled to ambient temperature and diluted with EtOAc (600 ml). The reaction mixture was filtered through a pad of Celite® and washed with EtOAc. The EtOAc solution was washed with brine, dried over Na₂SO₄, and concentrated. The crude product was purified on a silica gel column eluting with EtOAc/Hexane system (Biotage 90+ Column: equilibrium 600 ml. 100% Hexanes, segment 1: 2250 ml. 50% EtOAc/Hexanes Linear, segment 2: 4500 ml. 75% EtOAc/Hexanes Linear, segment 3: 4500 mL 100% EtOAc) to afford 4-(4-{6-amino-5-[(R)-1-(2,6-dichloro-3-fluoro-phenyl)-ethoxy]-pyridin-3-yl)-pyrazol-1-yl)-piperidine-1-carboxylic acid tert-butyl ester (11.8 g, 60% yield, -95% purity) with a Rf of 0.15 (50% EtOAc/Hexanes). MS m/e 550 (M+1)⁺. To a solution of 4-(4-{6-amino-5-[(R)-1-(2,6-dichloro-3-fluoro-phenyl)-ethoxy]-pyridin-3-yl}-pyrazol-1-yl)-piperidine-1-carboxylic acid tert-butyl ester (11.8 g, 21.45 mmol) in CH₂Cl₂ (59 mL, 0.2M) was added 4N HCl/Dioxane (21 mL). The solution was stirred overnight forming a solid. The solid was crushed thoroughly with a glass rod and sonicated to release starting material trapped in the solid. Additional 4N HCl/Dioxane (21 mL) was added and stirred for another 2 hours at room temperature in which LCMS showed no starting material. The suspension was filtered in a Buchner funnel lined with filter paper. The mother liquor was saved because it contained <5% of product. The solid was transferred to a 500 mL beaker and HPLC water was added until the solid dissolved completely. The pH was adjusted to 10 with the addition of solid Na₂CO3. The water solution was extracted with CH₂Cl₂ (5 x 200 mL) or until LCMS showed no product in the aqueous layer. The CH₂Cl₂ solution was dried over Na₂SO₄ and concentrated. The crude product, re-dissolved in CH₂Cl₂ (10 mL) and MeOH (1 mL), was purified on a silica gel column eluting with CH₂Cl₂/MeOH/NEt₃ system (Biotage 40+ Column: equilibrium 600 mL CH₂Cl₂ 100% giving byproduct, segment 1: 1200 mL 10% MeOH/CH₂Cl₂ linear, segment 2: 2400 mL 10% MeOH/CH₂Cl₂ step, segment 3: 2400 mL 9% MeOH/1 % NEt₃/CH₂Cl₂). The desired fractions were collected to provide 3-[{R)-1-(2,6-dichloro-3-fluoro-phenyl)-ethoxy]-5-(1-piperidin4-yl-1 H-pyrazol-4-yl)-pyridin-2-ylamine (7.19 g, 75% combined yield, white solid). MS m/e 450 (M+1)⁺. ¹H NMR (DMSO-de, 400 MHz) δ 7.92 (s, 1 H), 7.76 (s, 1 H), 7.58 (m, 1 H), 7.53 (s, 1 H), 7.45 (m, 1 H), 6.90 (s, 1 H), 6.10 (m, 1 H), 5.55 (bs, 2H), 4.14 (m, 1 H), 3.05 (m, 2H), 2.58 (m, 2H), 1.94 (m, 2H), 1.80 (d, 3H), 1.76 (m, 2H). The solid product 3-[(R)-1-(2,6-dichloro-3-fluoro-phenyl)-ethoxy]-5-(1-piperidin4-yl-1 H-pyrazol-4-yl)-pyridin-2-ylamine was dissolved in dichloromethane, and the solvent was evaporated slowly to generate fine crystalline solid. After high vacuum dry, the sample was confirmed to be a single crystalline polymorph form A with a melting point of 194°C.

Preferred embodiments are described in WO2013/017989 and are reiterated here:

In one aspect the present invention provides as method of treating cancer in a human in need of such treatment comprising, administering to said human a therapeutically effective amount of a f the formula 1: 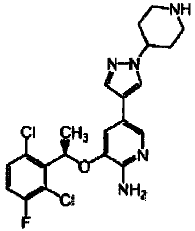 or a pharmaceutically acceptable salt thereof, wherein the cancer is mediated by at least one genetically altered ROS. The compound of formula 1 may be variously referred to herein by its generic name, Crizotinib, or by its chemical name, 3-[(R)-1-(2,6-dichloro-3-fluoro-phenyl)-ethoxy]-5-(1-piperidin4-yl-1H-pyrazol-4-yl)-pyridin-2-ylamine. In one embodiment of this aspect of the invention, the at least one genetically altered ROS is a fusion gene of ROS. In another embodiment of this aspect, the fusion gene of ROS is SLC34A2-ROS gene or CD74-ROS gene. In another embodiment of this aspect, the at least one genetically altered ROS is a genetic deletion involving ROS kinase. In another embodiment of this aspect, the genetic deletion is FIG-ROS gene. In another embodiment of this aspect, the at least one genetically altered ROS is a genetically altered ROS kinase. In another embodiment of this aspect, the genetically altered ROS kinase is a ROS fusion. In another embodiment of this aspect, the ROS fusion is SLC34A2-ROS kinase or CD74-ROS kinase. In another embodiment of this aspect, the at least one genetically altered ROS is a deletion protein involving ROS kinase. In another embodiment of this aspect, the deletion protein is FIG-ROS kinase. In another embodiment of this aspect, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In another embodiment of this aspect, the cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In another embodiment of this aspect, the cancer is non-small cell lung cancer (NSCLC). In another embodiment of this aspect, the cancer is glioblastoma. In another embodiment of this aspect, the compound of the formula is administered as a pharmaceutical composition comprising the compound of the formula 1 and at least one pharmaceutically acceptable carrier. In another embodiment of this aspect, the compound of the formula 1 or a pharmaceutically acceptable salt thereof is administered as a pharmaceutical composition comprising the compound of formula 1 or a pharmaceutically acceptable salt thereof and at least one pharmaceutically acceptable carrier. In another aspect the present invention provides a method comprising administering to a mammal having an abnormal cell growth mediated by ROS kinase a therapeutically effective amount of a ROS kinase inhibitor. In one embodiment of this aspect of the invention, the abnormal cell growth is mediated by at least one genetically altered ROS kinase. In another embodiment, the abnormal cell growth is mediated by a fusion gene of ROS kinase. In another embodiment, the abnormal cell growth is mediated by a genetic deletion involving ROS kinase. In another embodiment, the fusion gene is SLC34A2-ROS or CD74-ROS. In another embodiment, the genetic deletion is FIG-ROS. In another embodiment, the abnormal cell growth is mediated by a fusion protein of ROS kinase. In another embodiment, the abnormal cell growth is mediated by a deletion protein involving ROS kinase. In another embodiment, the fusion protein is SLC34A2-ROS or CD74-ROS. In another embodiment, the deletion protein is FIG-ROS. In some such embodiments of this aspect, the method comprises administering to said mammal having an abnormal cell growth mediated by ROS kinase a therapeutically effective amount of a ROS kinase inhibitor, thereby treating said abnormal cell growth. In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 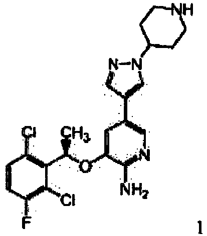 or a pharmaceutically acceptable salt thereof. In another embodiment of this aspect of the invention, the abnormal cell growth is cancer. In another embodiment of each of the preceding aspects of the invention, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone- refractory prostate cancer, papillary renal cell carcinoma, colorectal adenocarcinoma, neuroblastomas, anaplastic large cell lymphoma (ALCL) and gastric cancer. In yet another embodiment of each of the preceding aspects of the invention, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula 1 and at least one pharmaceutically acceptable carrier. In another embodiment of this aspect, the compound of the formula 1 or a pharmaceutically acceptable salt thereof is administered as a pharmaceutical composition comprising the compound of formula 1 or a pharmaceutically acceptable salt thereof and at least one pharmaceutically acceptable carrier. In another aspect the present invention provides a method comprising administering to a mammal having cancer mediated by ROS kinase a therapeutically effective amount of a ROS kinase inhibitor. In one embodiment of this aspect of the invention, the cancer is mediated by at least one genetically altered ROS kinase. In another embodiment, the cancer is mediated by a fusion gene of ROS kinase. In another embodiment, the abnormal cell growth is mediated by a genetic deletion involving ROS kinase. In another embodiment, the fusion gene is SLC34A2-ROS or CD74-ROS. In another embodiment, the genetic deletion is FIG-ROS. In another embodiment, the abnormal cell growth is mediated by a fusion protein of ROS kinase. In another embodiment, the abnormal cell growth is mediated by a deletion protein involving ROS kinase. In another embodiment, the fusion protein is SLC34A2-ROS or CD74-ROS. In another embodiment, the deletion protein is FIG-ROS. In some such embodiments of this aspect, the method comprises administering to said mammal having cancer mediated by ROS kinase a therapeutically effective amount of a ROS kinase inhibitor, thereby treating said cancer. In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 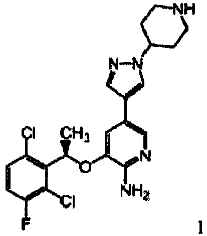 or a pharmaceutically acceptable salt thereof. In another embodiment, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone- refractory prostate cancer, papillary renal cell carcinoma, colorectal adenocarcinoma, neuroblastomas, anaplastic large cell lymphoma (ALCL) and gastric cancer. In yet another embodiment, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula and at least one pharmaceutically acceptable carrier. In another embodiment of this aspect, the compound of the formula I or a pharmaceutically acceptable salt thereof is administered as a pharmaceutical composition comprising the compound of formula 1 or a pharmaceutically acceptable salt thereof and at least one pharmaceutically acceptable carrier. In another aspect the present invention provides a method comprising treating cancer mediated by at least one ROS kinase in a mammal in need of such treatment by administering a therapeutically effective amount of a ROS kinase inhibitor. In one embodiment of this aspect of the invention, the cancer is mediated by at least one genetically altered ROS kinase. In another embodiment, the cancer is mediated by a fusion gene of ROS kinase. In another embodiment, the cancer is mediated by a genetic deletion involving ROS kinase. In another embodiment, the fusion gene is SLC34A2-ROS or CD74-ROS. In another embodiment, the genetic deletion is FIG-ROS. In another embodiment, the abnormal cell growth is mediated by a fusion protein of ROS kinase. In another embodiment, the abnormal cell growth is mediated by a deletion protein involving ROS kinase. In another embodiment, the fusion protein is SLC34A2-ROS or CD74-ROS. In another embodiment, the deletion protein is FIG-ROS. In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 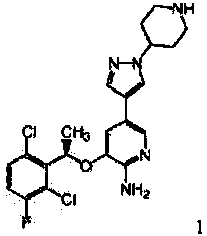 or a pharmaceutically acceptable salt thereof. In another embodiment, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone- refractory prostate cancer, papillary renal cell carcinoma, colorectal adenocarcinoma, neuroblastomas, anaplastic large cell lymphoma (ALCL) and gastric cancer. In yet another embodiment, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula 1 and at least one pharmaceutically acceptable carrier. In another embodiment of this aspect, the compound of the formula or a pharmaceutically acceptable salt thereof is administered as a pharmaceutical composition comprising the compound of formula 1 or a pharmaceutically acceptable salt thereof and at least one pharmaceutically acceptable carrier. In another aspect the present invention provides a method of treating abnormal cell growth in a mammal in need of such treatment comprising administering to said mammal a therapeutically effective amount of a ROS kinase inhibitor. In one embodiment of this aspect of the invention, the abnormal cell growth is mediated by at least one genetically altered ROS kinase. In another embodiment, the abnormal cell growth is mediated by a fusion gene of ROS kinase. In another embodiment, the abnormal cell growth is mediated by a genetic deletion involving ROS kinase. In another embodiment, the fusion gene is SLC34A2-ROS or CD74-ROS. In another embodiment, the genetic deletion is FIG-ROS. In another embodiment, the abnormal cell growth is mediated by a fusion protein of ROS kinase. In another embodiment, the abnormal cell growth is mediated by a deletion protein involving ROS kinase. In another embodiment, the fusion protein is CD74-ROS. In another embodiment, the fusion protein is SLC34A2-ROS. In another embodiment, the deletion protein is FIG-ROS. In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 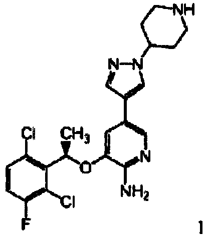 or a pharmaceutically acceptable salt thereof. In another embodiment of this aspect of the invention, the abnormal cell growth is cancer. In another embodiment of each of the preceding aspects of the invention, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone- refractory prostate cancer, papillary renal cell carcinoma, colorectal adenocarcinoma, neuroblastomas, anaplastic large cell lymphoma (ALCL) and gastric cancer. In yet another embodiment of each of the preceding aspects of the invention, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula 1 and at least one pharmaceutically acceptable carrier. In another embodiment of this aspect, the compound of the formula or a pharmaceutically acceptable salt thereof is administered as a pharmaceutical composition comprising the compound of formula 1 or a pharmaceutically acceptable salt thereof and at least one pharmaceutically acceptable carrier.

In one embodiment of each of the preceding aspects of the invention, the mammal is a human. In another embodiment of each of the preceding aspects of the invention, the mammal is a dog. In another aspect the present invention provides a method of treating cancer shown to be positive for at least one genetically altered ROS kinase in a mammal in need of such treatment comprising administering to said mammal a therapeutically effective amount of a ROS kinase inhibitor. In one embodiment of this aspect of the invention, the genetically altered ROS kinase is a fusion gene of ROS. In another embodiment, the fusion gene is SLC34A2-ROS or CD74-ROS. In another embodiment, the fusion gene is SLC34A2-ROS. In another embodiment, the fusion gene is CD74-ROS. In another embodiment, the genetically altered ROS kinase is a genetic deletion involving ROS kinase. In another embodiment, the genetic deletion is FIG-ROS. In another embodiment, the genetically altered ROS kinase is a fusion protein of ROS kinase. In another embodiment, the genetically altered ROS kinase is a deletion protein involving ROS kinase. In another embodiment, the fusion protein is SLC34A2-ROS or CD74-ROS. In another embodiment, the fusion protein is CD74-ROS. In another embodiment, the fusion protein is SLC34A2-ROS. In another embodiment, the deletion protein is FIG-ROS. In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 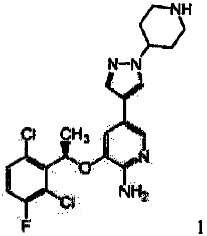 or a pharmaceutically acceptable salt thereof. In another embodiment, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In yet another embodiment, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula 1 and at least one pharmaceutically acceptable carrier. In another embodiment of this aspect, the compound of the formula 1 or a pharmaceutically acceptable salt thereof is administered as a pharmaceutical composition comprising the compound of formula 1 or a pharmaceutically acceptable salt thereof and at least one pharmaceutically acceptable carrier. In another aspect the present invention provides a method of treating ROS positive cancer comprising administering to a mammal in need of such treatment a therapeutically effective amount of a ROS kinase inhibitor. In one embodiment of this aspect of the invention, the ROS positive cancer is mediated by a fusion gene of ROS. In another embodiment, the fusion gene is SLC34A2-ROS or CD74-ROS. In another embodiment, the fusion gene is SLC34A2-ROS. In another embodiment, the fusion gene is CD74-ROS. In another embodiment, ROS positive cancer is mediated by a genetic deletion involving ROS kinase. In another embodiment, the genetic deletion is FIG-ROS. In another embodiment, the ROS positive cancer is mediated by a fusion protein of ROS kinase. In another embodiment, the ROS positive cancer is mediated by a deletion protein involving ROS kinase. In another embodiment, the fusion protein of ROS kinase is SLC34A2-ROS or CD74-ROS. In another embodiment, the fusion protein of ROS kinase is CD74-ROS. In another embodiment, the fusion protein of ROS kinase is SLC34A2-ROS. In another embodiment, the deletion protein of ROS kinase is FIG-ROS.

In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 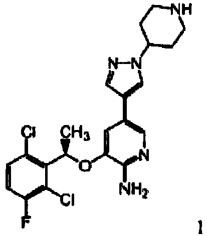 or a pharmaceutically acceptable salt thereof. In another embodiment, the ROS positive cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the ROS positive cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In yet another embodiment, the ROS positive cancer is non-small cell lung cancer (NSCLC). In yet another embodiment, the ROS positive cancer is glioblastoma. In some embodiments of this aspect, the compound of the formula 1 or a pharmaceutically acceptable salt thereof is administered as a pharmaceutical composition comprising the compound of formula 1 or a pharmaceutically acceptable salt thereof and at least one pharmaceutically acceptable carrier. In another aspect the present invention provides a method comprising administering to a mammal having abnormal cell growth mediated by ROS kinase a therapeutically effective amount of a ROS kinase inhibitor. In one embodiment of this aspect of the invention, the abnormal cell growth is mediated by at least one genetically altered ROS kinase. In another embodiment, the abnormal cell growth is mediated by a fusion gene of ROS kinase. In another embodiment, the abnormal cell growth is mediated by a genetic deletion involving ROS kinase. In another embodiment, the fusion gene is SLC34A2-ROS or CD74-ROS. In another embodiment, the genetic deletion is FIG-ROS. In another embodiment, the abnormal cell growth is mediated by a fusion protein of ROS kinase. In another embodiment, the abnormal cell growth is mediated by a deletion protein involving ROS kinase. In another embodiment, the fusion protein is SLC34A2-ROS or CD74-ROS. In another embodiment, the deletion protein is FIG-ROS.

In another embodiment of this aspect, the ROS kinase inhibitor is a small molecule inhibitor of ROS kinase. In another embodiment, the ROS kinase inhibitor is an amino-pyridine compound or an amino-pyrazine compound. In another embodiment of each of the preceding aspects of the invention, the ROS kinase inhibitor is a compound of the formula 1: 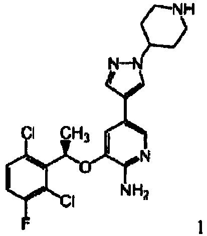 or a pharmaceutically acceptable salt thereof. In another embodiment of this aspect of the invention, the abnormal cell growth is cancer. In another embodiment, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone- refractory prostate cancer, papillary renal cell carcinoma, colorectal adenocarcinoma, neuroblastomas, anaplastic large cell lymphoma (ALCL) and gastric cancer. In yet another embodiment, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula 1 and at least one pharmaceutically acceptable carrier. In another embodiment of this aspect, the compound of the formula 1 or a pharmaceutically acceptable salt thereof is administered as a pharmaceutical composition comprising the compound of formula 1 or a pharmaceutically acceptable salt thereof and at least one pharmaceutically acceptable carrier. In yet another embodiment of this aspect of the invention, the compound or pharmaceutically acceptable salt thereof is administered as a pharmaceutical composition comprising the compound of the formula and at least one pharmaceutically acceptable carrier. In another embodiment of this aspect, the compound of the formula 1 or a pharmaceutically acceptable salt thereof is administered as a pharmaceutical composition comprising the compound of formula 1 or a pharmaceutically acceptable salt thereof and at least one pharmaceutically acceptable carrier. In another aspect, the invention provides for a method comprising administering a therapeutically effective amount of a ROS kinase inhibitor to a patient that is known to be ROS positive. In one embodiment the patient has cancer that is mediated by at least one genetically altered ROS kinase. In another embodiment, the cancer is mediated by a fusion gene of ROS kinase. In another embodiment, the cancer is mediated by a genetic deletion involving ROS kinase. In another embodiment, the fusion gene is SLC34A2-ROS or CD74-ROS. In another embodiment, the genetic deletion is FIG-ROS. In another embodiment, the cancer is mediated by a fusion protein of ROS kinase. In another embodiment, the cancer is mediated by a deletion protein involving ROS kinase. In another embodiment, the fusion protein is SLC34A2-ROS or CD74-ROS. In another embodiment, the deletion protein is FIG-ROS. In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 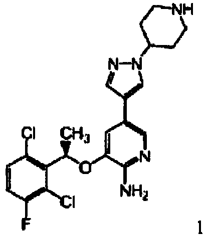 or a pharmaceutically acceptable salt thereof. In another embodiment, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In yet another embodiment of each of the preceding aspects of the invention, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula 1 and at least one pharmaceutically acceptable carrier. In yet another embodiment of this aspect of the invention, the compound or pharmaceutically acceptable salt thereof is administered as a pharmaceutical composition comprising the compound of the formula and at least one pharmaceutically acceptable carrier. In another embodiment of this aspect, the compound of the formula 1 or a pharmaceutically acceptable salt thereof is administered as a pharmaceutical composition comprising the compound of formula 1 or a pharmaceutically acceptable salt thereof and at least one pharmaceutically acceptable carrier.

In another aspect, the present invention provides a method comprising, i. identifying a patient having a cancer shown to be positive for at least one genetically altered ROS kinase; and ii. administering to said patient a therapeutically effective amount of a ROS kinase inhibitor. In one embodiment of this aspect of the invention, said genetically altered ROS kinase is a fusion gene of ROS. In another embodiment, the fusion gene is SLC34A2-ROS or CD74-ROS. In another embodiment, the fusion gene is SLC34A2-ROS. In another embodiment, the fusion gene is CD74-ROS. In another embodiment, said genetically altered ROS kinase is a genetic deletion involving ROS kinase. In another embodiment, the genetic deletion is FIG-ROS. In another embodiment, said genetically altered ROS kinase is a fusion protein of ROS kinase. In another embodiment, said genetically altered ROS kinase is a deletion protein involving ROS kinase. In another embodiment, the fusion protein is SLC34A2-ROS or CD74-ROS. In another embodiment, the fusion protein is SLC34A2-ROS. In another embodiment, the fusion protein is CD74-ROS. In another embodiment, the deletion protein is FIG-ROS. In some such embodiments of this aspect, the method comprises (i) identifying a patient having a cancer shown to be positive for at least one genetically altered ROS kinase; and (ii) administering to said patient a therapeutically effective amount of a ROS kinase inhibitor, thereby treating said cancer. In some embodiments of this aspect, said treating results in reversing or inhibiting the progression of cancer. In another embodiment of this aspect, the ROS kinase inhibitor is a small molecule inhibitor of ROS kinase. In another embodiment, the ROS kinase inhibitor is an amino-pyridine compound or an amino-pyrazine compound.

In another embodiment of each of the preceding aspects of the invention, the ROS kinase inhibitor is a compound of the formula 1: 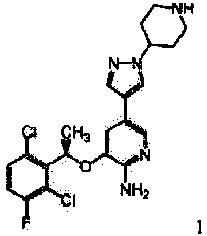 or a pharmaceutically acceptable salt thereof. In another embodiment of this aspect of the invention, the abnormal cell growth is cancer. In another embodiment of each of the preceding aspects of the invention, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In yet another embodiment of this aspect of the invention, the cancer is selected from the group consisting of non-small cell lung cancer (NSCLC), glioblastoma, squamous cell carcinoma, hormone- refractory prostate cancer, papillary renal cell carcinoma, colorectal adenocarcinoma, neuroblastomas, anaplastic large cell lymphoma (ALCL) and gastric cancer. In yet another embodiment of each of the preceding aspects of the invention, the cancer is non-small cell lung cancer (NSCLC). In yet another embodiment, the cancer is glioblastoma. In yet another embodiment of this aspect of the invention, the compound or pharmaceutically acceptable salt thereof is administered as a pharmaceutical composition comprising the compound of the formula 1 and at least one pharmaceutically acceptable carrier. In another embodiment of this aspect, the compound of the formula or a pharmaceutically acceptable salt thereof is administered as a pharmaceutical composition comprising the compound of formula 1 or a pharmaceutically acceptable salt thereof and at least one pharmaceutically acceptable carrier.

In one aspect the present invention provides a use of a ROS kinase inhibitor for the preparation of a medicament useful in the treatment of cancer in a human in need of such treatment comprising, administering to said mammal a therapeutically effective amount of a ROS kinase inhibitor of the formula 1: 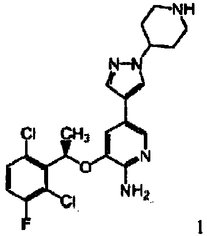 or a pharmaceutically acceptable salt thereof, wherein the cancer is mediated by at least one genetically altered ROS. In one embodiment of this aspect of the invention, the cancer is mediated by a fusion gene of ROS. In another embodiment of this aspect, the fusion gene of ROS is SLC34A2-ROS gene or CD74-ROS gene. In another embodiment of this aspect, the cancer is mediated by a genetic deletion involving ROS kinase. In another embodiment of this aspect, the genetic deletion is FIG-ROS gene. In another embodiment of this aspect, the cancer is mediated by a genetically altered ROS kinase. In another embodiment of this aspect, the genetically altered ROS kinase is a ROS fusion. In another embodiment of this aspect, the ROS fusion is SLC34A2-ROS kinase or CD74-ROS kinase. In another embodiment of this aspect, the cancer is mediated by a deletion protein involving ROS kinase. In another embodiment of this aspect, the deletion protein is FIG-ROS kinase. In another embodiment of this aspect, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In another embodiment of this aspect, the cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In another embodiment of this aspect, the cancer is non-small cell lung cancer (NSCLC). In another embodiment of this aspect, the cancer is glioblastoma. In another embodiment of this aspect, the compound of the formula 1 is administered as a pharmaceutical composition comprising the compound of the formula 1 and at least one pharmaceutically acceptable carrier. In another aspect, the present invention provides a use of a ROS kinase inhibitor for the preparation of a medicament useful in the treatment of a cancer mediated by at least one genetically altered ROS kinase. In one embodiment, ROS kinase inhibitor is a small molecule inhibitor of ROS kinase. In another embodiment, the ROS kinase inhibitor is an amino-pyridine compound or an amino-pyrazine compound.

In another embodiment, the ROS kinase inhibitor is a compound of the formula 1: 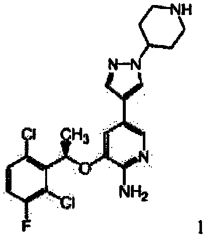 or a pharmaceutically acceptable salt thereof. In one embodiment of this aspect of the invention, said genetically altered ROS kinase is a fusion gene of ROS. In another embodiment, the fusion gene is SLC34A2-ROS or CD74-ROS. In another embodiment, the fusion gene is SLC34A2-ROS. In another embodiment, the fusion gene is CD74-ROS. In another embodiment, said genetically altered ROS kinase is a genetic deletion involving ROS kinase. In another embodiment, the genetic deletion is FIG-ROS. In another embodiment, said genetically altered ROS kinase is a fusion protein of ROS kinase. In another embodiment, said genetically altered ROS kinase is a deletion protein involving ROS kinase. In another embodiment, the fusion protein is SLC34A2-ROS or CD74-ROS. In another embodiment, the fusion protein is SLC34A2-ROS. In another embodiment, the fusion protein is CD74-ROS. In another embodiment, the deletion protein is FIG-ROS. In yet another embodiment of this aspect of the invention, the ROS positive cancer is non-small cell lung cancer (NSCLC). In yet another embodiment, the ROS positive cancer is glioblastoma. In another aspect, the present invention provides a use of a ROS kinase inhibitor for the preparation of a medicament useful in the treatment of a ROS positive cancer. In one embodiment, ROS kinase inhibitor is a small molecule inhibitor of ROS kinase. In another embodiment, the ROS kinase inhibitor is an amino-pyridine compound or an amino-pyrazine compound.

In another embodiment, the ROS kinase inhibitor is a compound of the formula 1: 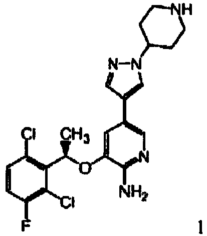 or a pharmaceutically acceptable salt thereof. In one embodiment of this aspect of the invention, said genetically altered ROS kinase is a fusion gene of ROS. In another embodiment, the fusion gene is SLC34A2-ROS or CD74-ROS. In another embodiment, the fusion gene is SLC34A2-ROS. In another embodiment, the fusion gene is CD74-ROS. In another embodiment, said genetically altered ROS kinase is a genetic deletion involving ROS kinase. In another embodiment, the genetic deletion is FIG-ROS. In another embodiment, said genetically altered ROS kinase is a fusion protein of ROS kinase. In another embodiment, said genetically altered ROS kinase is a deletion protein involving ROS kinase. In another embodiment, the fusion protein is SLC34A2-ROS or CD74-ROS. In another embodiment, the fusion protein is SLC34A2-ROS. In another embodiment, the fusion protein is CD74-ROS. In another embodiment, the deletion protein is FIG-ROS. In yet another embodiment of this aspect of the invention, the ROS positive cancer is non-small cell lung cancer (NSCLC). In yet another embodiment, the ROS positive cancer is glioblastoma. In another aspect, the present invention provides a kit comprising a pharmaceutical composition of a ROS kinase inhibitor and a set of instructions for administering said pharmaceutical composition to a patient having a ROS positive cancer. In one embodiment of this aspect of the invention, the ROS positive cancer is selected from the group consisting of non-small cell lung cancer (NSCLC), glioblastoma, squamous cell carcinoma, hormone- refractory prostate cancer, papillary renal cell carcinoma, colorectal adenocarcinoma, neuroblastomas, anaplastic large cell lymphoma (ALCL) and gastric cancer. In yet another embodiment of this aspect of the invention, the ROS positive cancer is non-small cell lung cancer (NSCLC). In yet another embodiment, the ROS positive cancer is glioblastoma. In another aspect, the present invention provides a kit comprising a pharmaceutical composition of a ROS kinase inhibitor and a set of instructions for administering said pharmaceutical composition to a patient having a ROS positive cancer. In one embodiment, said ROS positive cancer is mediated by at least one genetically altered ROS kinase. In another embodiment, said genetically altered ROS kinase is a fusion gene of ROS. In another embodiment, the fusion gene is SLC34A2-ROS or CD74-ROS. In another embodiment, the fusion gene is SLC34A2-ROS. In another embodiment, the fusion gene is CD74-ROS. In another embodiment, said genetically altered ROS kinase is a genetic deletion involving ROS kinase. In another embodiment, the genetic deletion is FIG-ROS. In another embodiment, said genetically altered ROS kinase is a fusion protein of ROS kinase. In another embodiment, said genetically altered ROS kinase is a deletion protein involving ROS kinase. In another embodiment, the fusion protein is SLC34A2-ROS or CD74-ROS. In another embodiment, the fusion protein is SLC34A2-ROS. In another embodiment, the fusion protein is CD74-ROS. In another embodiment, the deletion protein is FIG-ROS. In one embodiment of this aspect of the invention, the ROS positive cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In yet another embodiment of this aspect of the invention, the ROS positive cancer is non-small cell lung cancer (NSCLC). In yet another embodiment, the ROS positive cancer is glioblastoma. In another aspect, the present invention provides a kit comprising a pharmaceutical composition of Crizotinib and a set of instructions for administering said pharmaceutical composition to a patient having a ROS positive cancer. In one embodiment, said ROS positive cancer is mediated by at least one genetically altered ROS kinase. In another embodiment, said genetically altered ROS kinase is a fusion gene of ROS. In another embodiment, the fusion gene is SLC34A2-ROS or CD74-ROS. In another embodiment, the fusion gene is SLC34A2-ROS. In another embodiment, the fusion gene is CD74-ROS. In another embodiment, said genetically altered ROS kinase is a genetic deletion involving ROS kinase. In another embodiment, the genetic deletion is

FIG-ROS. In another embodiment, said genetically altered ROS kinase is a fusion protein of ROS kinase. In another embodiment, said genetically altered ROS kinase is a deletion protein involving ROS kinase. In another embodiment, the fusion protein is SLC34A2-ROS or CD74-ROS. In another embodiment, the fusion protein is SLC34A2-ROS. In another embodiment, the fusion protein is CD74-ROS. In one embodiment of this aspect of the invention, the ROS positive cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In yet another embodiment of this aspect of the invention, the ROS positive cancer is non-small cell lung cancer (NSCLC). In yet another embodiment, the ROS positive cancer is glioblastoma.

In yet another aspect, the present invention provides a method of inhibiting ROS kinase activity in a cell by administering a compound of the formula 1: 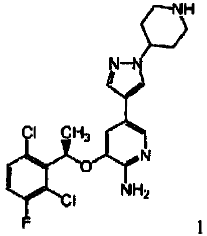 or a pharmaceutically acceptable salt thereof. In another aspect, the invention provides a method of treating cancer in a mammal comprising administering to said mammal a therapeutically effective amount of 3-[(R)-1-(2,6-dichloro-3-fluorophenyl)-ethoxy]-5-(1-piperidin-4-yl-1H-pyrazol-4-yl)-pyridin-2-ylamine or a pharmaceutically acceptable salt thereof, wherein the cancer is mediated by at least one genetically altered ROS. In some such embodiments, the at least one genetically altered ROS is a genetically altered ROS gene or a genetically altered ROS protein. In some embodiments of this aspect, said treating results in reversing or inhibiting the progression of cancer. In frequent embodiments of this aspect, the mammal is a human. In frequent embodiments of this aspect, the at least one genetically altered ROS is a genetically altered ROS gene, such as a ROS fusion gene. In some such embodiments, the ROS fusion gene is the SLC34A2-ROS gene or the CD74-ROS gene. In other such embodiments, the ROS fusion gene is the FIG-ROS gene. In frequent embodiments of this aspect, the at least one genetically altered ROS is a genetically altered ROS protein, such as a ROS fusion protein. In some such embodiments, the ROS fusion protein is the SLC34A2-ROS kinase or the CD74-ROS kinase. In other such embodiments, the ROS fusion protein is the FIG-ROS kinase. In some embodiments of this aspect, the invention provides a method of reversing or inhibiting the progression of cancer in a mammal comprising administering to said mammal a therapeutically effective amount of 3-[(R)-1-(2,6-dichloro-3-fluoro-phenyl)-ethoxy]-5-(1-piperidin-4-yl-1H-pyrazol-4-yl)-pyridin-2-ylamine or a pharmaceutically acceptable salt thereof, wherein the cancer is mediated by a ROS fusion gene. In some such embodiments, the ROS fusion gene is the SLC34A2-ROS gene. In other such embodiments, the ROS fusion gene is CD74-ROS gene. In still other such embodiments, the ROS fusion gene is the FIG-ROS gene. In certain embodiments, the ROS fusion gene is selected from the group consisting of the SLC34A2-ROS gene, the CD74-ROS gene and the FIG-ROS gene. In other embodiments of this aspect, the invention provides a method of reversing or inhibiting the progression of cancer in a mammal comprising administering to said mammal a therapeutically effective amount of 3-[(R)-1-(2,6-dichloro-3-fluoro-phenyl)-ethoxy]-5-(1-piperidin-4-yl-1H-pyrazol-4-yl)-pyridin-2-ylamine or a pharmaceutically acceptable salt thereof, wherein the cancer is mediated by a ROS fusion protein. In some such embodiments, the ROS fusion protein is the SLC34A2-ROS kinase. In other such embodiments, the ROS fusion protein is the CD74-ROS kinase. In still other such embodiments, the ROS fusion protein is the FIG-ROS kinase. In certain embodiments, the ROS fusion protein is selected from the group consisting of the SLC34A2-ROS kinase, the CD74-ROS kinase and the FIG-ROS kinase. In some embodiments of this aspect, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In other embodiments of this aspect, the cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In some embodiments of this aspect, the cancer is non-small cell lung cancer (NSCLC). In other embodiments of this aspect, the cancer is glioblastoma. In frequent embodiments of this aspect, 3-[(R)-1-(2,6-dichloro-3-fluoro-phenyl)-ethoxy]-5-(1-piperidin-4-yl-1H-pyrazol-4-yl)-pyridin-2-ylamine or a pharmaceutically acceptable salt thereof is administered as a pharmaceutical composition comprising 3-[(R)-1-(2,6-dichloro-3-fluoro-phenyl)-ethoxy]-5-(1-piperidin-4-yl-1H-pyrazol-4-yl)-pyridin-2-ylamine or a pharmaceutically acceptable salt thereof and at least one pharmaceutically acceptable carrier. In some embodiments of this aspect, the method further comprises a step of identifying a mammal having a cancer characterized by at least one genetically altered ROS, such as a genetically altered ROS gene or a genetically altered ROS protein, prior to said administering step. In some such embodiments, the cancer is characterized as having a genetically altered ROS polynucleotide and/or a genetically altered ROS polypeptide. In yet another aspect, the invention provides a method of treating cancer in a mammal comprising: (i) identifying a mammal having a cancer characterized by at least one genetically altered ROS; and (ii) administering to said mammal a therapeutically effective amount of 3-[(R)-1-(2,6-dichloro3-fluoro-phenyl)-ethoxy]-5-(1-piperidin-4-yl-1H-pyrazol-4-yl)-pyridin-2-ylamine or a pharmaceutically acceptable salt thereof. In some such embodiments, the at least one genetically altered ROS is a genetically altered ROS gene or a genetically altered ROS protein. In some embodiments of this aspect, said treating results in reversing or inhibiting the progression of cancer. In frequent embodiments of this aspect, the mammal is a human. In some embodiments of this aspect, the at least one genetically altered ROS is a genetically altered ROS gene, for example a ROS fusion gene. In some such embodiments, the ROS fusion gene is the SLC34A2-ROS gene or the CD74-ROS gene. In other such embodiments, the ROS fusion gene is the FIG-ROS gene. In some embodiments of this aspect, the at least one genetically altered ROS is a genetically altered ROS protein, for example a ROS fusion protein. In some such embodiments, the ROS fusion protein is the SLC34A2-ROS kinase or the CD74-ROS kinase. In other such embodiments, the ROS fusion protein is the FIG-ROS kinase. In some embodiments of this aspect, the invention provides a method of reversing or inhibiting the progression of cancer in a mammal comprising (i) identifying a mammal having a cancer characterized by at least one ROS fusion gene; and (ii) administering to said mammal a therapeutically effective amount of 3-[(R)-1-(2,6-dichloro-3-fluoro-phenyl)-ethoxy]-5-(1-piperidin-4-yl-1H-pyrazol-4-yl)-pyridin-2-ylamine or a pharmaceutically acceptable salt thereof. In some such embodiments, the ROS fusion gene is the SLC34A2-ROS gene. In other such embodiments, the ROS fusion gene is CD74-ROS gene. In still other such embodiments, the ROS fusion gene is the FIG-ROS gene. In certain embodiments, the ROS fusion gene is selected from the group consisting of the SLC34A2-ROS gene, the CD74-ROS gene and the FIG-ROS gene. In some embodiments of this aspect, the invention provides a method of reversing or inhibiting the progression of cancer in a mammal comprising (i) identifying a mammal having a cancer characterized by at least one ROS fusion protein; and (ii) administering to said mammal a therapeutically effective amount of 3-[(R)-1-(2,6-dichloro-3-fluoro-phenyl)-ethoxy]-5-(1-piperidin-4-yl-1 H-pyrazol-4-yl)-pyridin-2-ylamine or a pharmaceutically acceptable salt thereof. In some such embodiments, the ROS fusion protein is the SLC34A2-ROS kinase. In other such embodiments, the ROS fusion protein is the CD74-ROS kinase. In still other such embodiments, the ROS fusion protein is the FIG-ROS kinase. In certain embodiments, the ROS fusion protein is selected from the group consisting of the SLC34A2-ROS kinase, the CD74-ROS kinase and the FIG-ROS kinase. In some embodiments of this aspect, the cancer is characterized as having a genetically altered ROS polynucleotide and/or a genetically altered ROS polypeptide. In some embodiments of this aspect, the cancer is selected from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, and combinations thereof. In other embodiments of this aspect, the cancer is selected from the group consisting of non-small cell lung cancer (NSCLC), glioblastoma, squamous cell carcinoma, hormone-refractory prostate cancer, papillary renal cell carcinoma, colorectal adenocarcinoma, neuroblastomas, anaplastic large cell lymphoma (ALCL) and gastric cancer. In some embodiments of this aspect, the cancer is non-small cell lung cancer (NSCLC). In other embodiments of this aspect, the cancer is glioblastoma. In frequent embodiments of this aspect, 3-[(R)-1-(2,6-dichloro-3-fluoro-phenyl)-ethoxy]-5-(1-piperidin-4-yl-1H-pyrazol-4-yl)-pyridin-2-ylamine or a pharmaceutically acceptable salt thereof is administered as a pharmaceutical composition comprising 3-[(R)-1-(2,6-dichloro-3-fluoro-phenyl)-ethoxy]-5-(1-piperidin-4-yl-1H-pyrazol-4-yl)-pyridin-2-ylamine or a pharmaceutically acceptable salt thereof and at least one pharmaceutically acceptable carrier.

### SPECIFIC INORMATION CONCERNING ASPECT (III) OF THE INVENTION

Aspect (iii) of the invention concerns forms of {drug3}, especially a crystalline form of 2-chloro-N-{2-[3-(2-{[(4'-hydroxybiphenyl-3-yl)methyl]amino}-2-oxoethyl)phenyl]-1,1-dimethylethyl}-acetamide. Aspect (iii) of the invention relates to a solid form of 2-chloro-N-{2-[3-(2-{[(4'-hydroxybiphenyl-3-yl)methyl]amino}-2-oxoethyl)phenyl]-1,1-dimethylethyl}-acetamide, namely to a crystalline form of 2-chloro-N-{2-[3-(2-{[(4'-hydroxybiphenyl-3-yl)methyl]amino}-2-oxoethyl)phenyl]-1,1-dimethylethyl}-acetamide, which is useful for treating respiratory diseases such as asthma or COPD diseases. Crystalline forms of 2-chloro-N-{2-[3-(2-{[(4'-hydroxybiphenyl-3-yl)methyl]amino}-2-oxoethyl)phenyl]-1,1-dimethylethyl}-acetamide are described in WO2013/021309, which is incorporated by reference. 2-chloro-N-{2-[3-(2-{[(4'-hydroxybiphenyl-3-yl)methyl]amino}-2-oxoethyl)phenyl]-1,1-dimethylethyl}-acetamide can be described by formula (1):

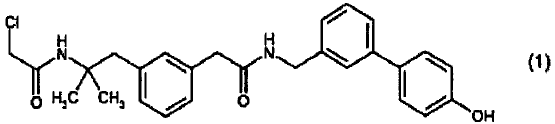

As used herein, crystalline form of 2-chloro-N-{2-[3-(2-{[(4'-hydroxybiphenyl-3-yl)methyl]amino}-2-oxoethyl)phenyl]-1,1-dimethylethyl}-acetamide is a crystalline form of 2-chloro-N-{2-[3-(2-{[(4'-hydroxybiphenyl-3-yl)methyl]amino}-2-oxoethyl)phenyl]-1,1-dimethylethyl}-acetamide which - as described in WO2013/021309 - can be prepared by and characterized by the methods reiterated hereinafter:

The isolation and use of a substantially crystalline form of 2-chloro-N-{2-[3-(2-{[(4'-hydroxybiphenyl-3-yl)methyl]amino}-2-oxoethyl)phenyl]-1,1-dimethylethyl}-acetamide of formula (1): 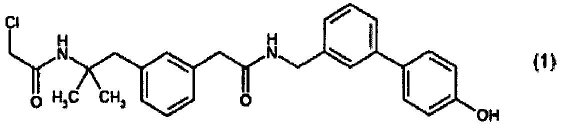 in the preparation of β2 agonist provides purge of critical impurities and their fate products as well as problematic residual reagents which were previously difficult to purge so that the final product is obtained with an acceptable higher purity. The intermediate 2-chloro-N-{2-[3-(2-{[(4'-hydroxybiphenyl-3-yl)methyl]amino}-2-oxoethyl)phenyl]-1,1-dimethylethyl}acetamide of form ula (1) is already known from WO2007/010356. However, the process described in WO2007/010356 does not involve the isolation of 2-chloro-N-{2-[3-(2-{[(4'-hydroxybiphenyl-3-yl)methyl]amino}-2-oxoethyl)-phenyl]-1,1-di methylethyl}acetamide which was only prepared as an uncrystallized solution. As a consequence, purging impurities and problematic residual reagents at this stage of the process was difficult and resulted in an unacceptably high impurity burden being carried out into the latter steps in the preparation of the above mentioned β2 agonist. A first object of asüect (iii) of the present invention is thus a substantially crystalline form of 2-chloro-N-{2-[3-(2-{[(4'-hydroxybiphenyl-3-yl)methyl]amino}-2-oxoethyl)phenyl]-1,1-dimethylethyl}acetamide (also referred as the "intermediate of formula (1)"). The intermediate of formula (1) according to aspect (iii) of the present invention has an X-ray diffraction pattern characterized by the following 3 unique X-ray diffraction pattern peaks expressed in terms of 2-theta angle (± 0.1 ° 2Θ) when measured using Cu Kal radiation (Wavelength = 1.5406 A):

| **Angle (°2θ)** | **Intensity (%)** |
|---|---|
| 9.1 | 7.7 |
| 10.5 | 18.8 |
| 11.4 | 6.7 |

According to another embodiment, the intermediate of formula (1) according to aspect (iii) of the present invention may also be characterized by a Fourier Transform Infra-red (FT-IR) pattern having the main characteristic peaks at 1535 (s), 1179 (s), 834 (s) and 769 (s) cm-1. According to another embodiment, the intermediate of formula (1) according to aspect (iii) of the present invention may also be characterized by a Fourier Transform Raman pattern having the main characteristic peaks at 1294 (s), 1001 (vs), and 772 (w) cm-1. According to another embodiment, the intermediate of formula (1) according to aspect (iii) of the present invention may also be characterized by a solid state ¹³C NMR pattern having the following principal carbon chemical shifts referenced to external sample of solid phase adamantane at 29.5 ppm:

| ¹³C Chemical Shifts [ppm ± 0.2 ppm] | Intensity*^{(a)}* |
|---|---|
| 173.2 | 42.2 |
| 158.5 | 50.2 |
| 55.5 | 60.4 |

| | |
|---|---|
| ^{(a)} Defined as peak heights. | |

Intensities can vary depending on the actual setup of the CPMAS experimental parameters and the thermal history of the sample. CPMAS intensities are not necessarily quantitative. For avoidance of doubt, "crystalline" or "substantially crystalline" according to the present invention means that the intermediate of formula (1) according to the present invention is at least 50% crystalline, preferably at least 70% crystalline, more preferably at least 80% crystalline, still more preferably at least 85% crystalline, still more preferably at least 90% crystalline and even more preferably at least 95% crystalline. The intermediate of formula (1) according to the present invention may be obtained by reaction of a compound of formula (2): 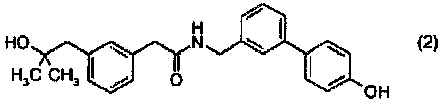 with chloroacetonitrile in the presence of an acid catalyst such as acetic acid, sulfuric acid, trifluoroacetic acid or mixtures thereof in a suitable solvent such as acetic acid, trifluoroacetic acid, chloroacetonitrile, toluene, or dichloromethane at temperatures of 0°C to 110°C followed by removal of the acid and volatile solvents during the reaction work-up and the addition an antisolvent such as 2-butanol, n-butanol, cyclohexane, heptanes, toluene or mixtures thereof, to crystallize the compound of formula (1) which is isolated using standard techniques. Typical conditions comprise treating 1.0 equivalents of the compound of formula (2) with 1 to 1 0 equivalents, preferably 5 to 7 equivalents, of chloroacetonitrile and 1 to 25 equivalents, preferably 14 to 16 equivalents, of trifluoroacetic acid at 20°C to 70°C, preferably 45°C to 55°C, for I to 24 hours then crystallizing the compound of formula (1) by removing the trifluoroacetic acid by distillation, then adding 10 to 25 equivalents, preferably 15 to 20 equivalents of 2-butanol and 0.005 to 0.1 equivalents, preferably 0.01 to 0.05 equivalents of seed crystals of the compound of formula (1) at 35°C to 70°C, preferably 55°C to 65°C, followed by 0 to 20 equivalents, preferably 9 to 11 equivalents of cyclohexane then cooling the mixture to 0°C to 30°C, preferably 15°C to 25°C over 0.5 to 3 hours. Preferably, said compound of formula (2) is prepared by reaction of a compound of formula 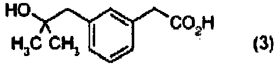 with a compound of formula (4): 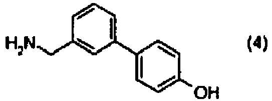 in the presence of a suitable amide coupling agent such as 1-(3-dimethylaminopropyl)-3-ethylcarbodiimide hydrochloride, dicyclohexylcarbodiimide, 1,1'-carbonyldiimidazole, 2,4,6-tripropyl-1,3,5,2,4,6-trioxatriphosphinane 2,4,5-trioxide (T3P®), pivaloyl chloride or isobutyl chloroformate in a suitable solvent such as tetrahydrofuran, 2-methyl tetrahydrofuran, ethyl acetate, isopropyl acetate, n-butyl acetate, dichloromethane, toluene, acetonitrile, propionitrile, pyridine or dimethylformamide, optionally in the presence of a base such as triethylamine, 4-methylmorpholine, or diisopropylethylamine, also optionally in the presence of a suitable additive such as 1-hydroxybenzotriazole, 2-hydroxypyridine or N-hydroxysuccinimide. Typical conditions comprise reacting 1.0 equivalent of the compound of formula (3) with 1.0 equivalents of 1,1'-carbonyldiimidazole in ethyl acetate at 15°C to 30°C for 0.25 to 4 hours followed by reaction with 1.1 equivalents of the amine of formula (4) at 20°C to 60°C for 1 to 24 hours. The compound of formula (4) may be prepared as described in WO 2007/010356. Alternatively, the com ound of formula (4) may be prepared according to Scheme 1 below:

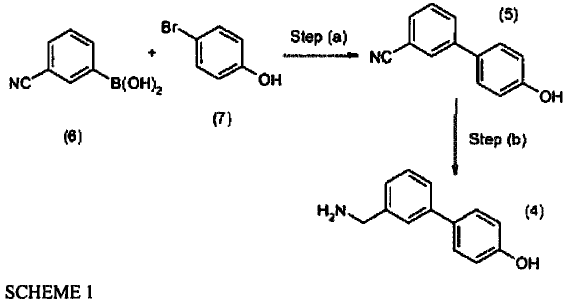

In Step (a), (3-cyanophenyl)boronic acid is reacted with 4-bromophenol in the presence of a suitable palladium catalyst such as pal ladium(II) acetate, (dibenzylideneacetone)dipalladium(O), palladium(II) trifluoroacetate, a suitable catalyst ligand such as triphenyl phosphine, tri-o-tolyl phosphine, tri-te/t-butyl phosphine, 1,1'-bis(diphenylphosphino)ferrocene or tris-te/t-butylphosphonium tetrafluoroborate, and a suitable base such as potassium carbonate, sodium carbonate, sodium hydrogen carbonate, potassium hydrogen carbonate, disodium hydrogen phosphate triethylamine, diisopropylethylamine, or N-methyl morpholine in a suitable solvent such as tetrahydrofuran, 2-methyltetrahydrofuran, ethyl acetate, dichloromethane, toluene, isopropyl acetate, 4-methyl-2-pentanone, butan-2-one, or acetone optionally in the presence of water. Typical conditions comprise reacting 1.0 equivalent of 4-bromophenol with 1.05 equivalents of (3-bromophenyl)boronic acid in the presence of 1 to 3 equivalents of potassium carbonate and a catalytic amount of a palladium catalyst such as 0.01 to 0.05 equivalents of palladium acetate or tris(dibenzylideneacetone)dipalladium(0) and a suitable ligand such as 0.01 to 0.05 equivalents of 1,1'-bis(diphenylphosphino)ferrocene or tris-te/f- butylphosphonium tetrafluoroborate in a suitable solvent such as aqueous 2-methyltetrahydrofuran or aqueous tetrahydrofuran under a nitrogen atmosphere at 20°C-70°C for 4 to 12 hours. In Step (b), the compound of formula (5) is reduced by catalytic hydrogenation using a suitable nitrile hydrogenation catalyst such as palladium, rhodium, platinum or nickel optionally on a suitable support such as carbon or alumina, in a suitable solvent such as water, methanol, ethanol, n-butanol, isopropanol, ethyl acetate, isopropyl acetate, tetrahydrofuran or 2-methyltetrahydrofuran optionally in the presence of either a base such a lithium hydroxide, sodium hydroxide, potassium hydroxide, sodium methoxide, sodium ethoxide or ammonia, or an acid such as sulfuric acid, hydrogen chloride, methanesulfonic acid, p-toluenesulfonic acid or camphorsulfonic acid. Typical conditions comprise either hydrogenating a solution of 1.0 equivalent of the compound of formula (5) in methanol containing 1 to 3 equivalents of sodium methoxide over a Raney Nickel catalyst at 20°C to 60°C for 2 to 24 hours, or hydrogenating a solution of 1 equivalent of the compound of formula (5) in a solution of methanol containing 1-3 equivalents of methanesulfonic acid over a palladium on carbon catalyst at 20°C to 60°C for 2 to 24 hours. The compound of formula (3) may be prepared as described in WO 2007/010356. Alternativel the compound of formula (3) may be prepared according to Scheme 2 below:

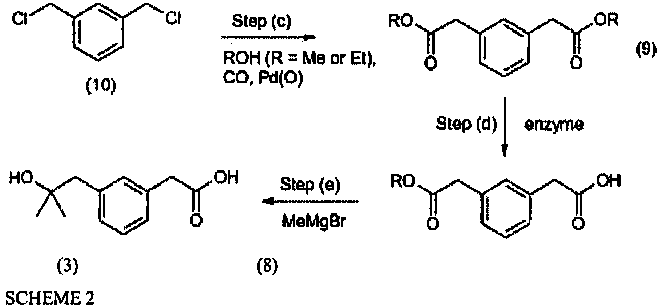

In step (c) a mixture of 1,3-bis(chloromethyl)benzene, a palladium catalyst and a base in methanol or ethanol is reacted with carbon monoxide at elevated pressure and elevated temperature. Typical conditions comprise combining a solution of 1.0 equivalents of 1,3-bis(chloromethyl)benzene in methanol or ethanol with 1 to 6 equivalents of N,N-diisopropylethylamine and a 0.005 to 0.05 equivalents of a palladium catalyst such as tetrakis(triphenylphosphine)palladium(0) and then heating the mixture to 30°C to 100°C for 4-24 hours under 20 to 500 psi pressure of carbon monoxide to give dimethyl 2,2'-(1,3-phenylene)diacetate. In step (d) one of the ester groups in the compound of formula (9), such as one of the methyl ester groups in dimethyl 2,2'-(1,3-phenylene)diacetate, is selectively hydrolysed to the compound of formula (8), such as [3-(2-methoxy-2-oxoethyl)phenyl]acetic acid, in the presence of a suitable enzyme known in the art, such as a lipase, esterase or protease, preferably a lipase. Preferred enzymes are Mucor Miehei esterase, Rhizomucor Miehei lipase, Thermomuces Languinosus lipase, Penicllin acylase. More preferably, the reaction is carried out with Lipase® (Thermomuces Languinosus lipase, (EC No 3.1.1.3)) at a pH between 5 and 9 and a temperature between 10°C and 40°C in water in the presence of a suitable buffering agent such as calcium acetate, dipotassium hydrogenphosphate or triethanolamine, and optionally in the presence of a suitable base such as sodium hydroxide, potassium hydroxide or lithium hydroxide. Typical conditions comprise 1.0 equivalents of dimethyl 2,2'-benzene-1,3-diyldiacetate reacting with 5 to 200 ml of Lipolase® (liquid formulation) in a calcium acetate buffer solution at temperatures between 20°C and 40°C, maintaining the pH between 5.5 and 6.8 by the addition of a base such as sodium hydroxide or potassium hydroxide. In step (e) the compound of formula (8), such as [3-(2-methoxy-2-oxoethyl)phenyl]acetic acid, is reacted with an "activated" methyl reagent (organometallic methyl such as CH₃MgCl, CH₃MgBr, CH₃Li) to give the compound of formula (3). Typical conditions comprise treating a solution of 1.0 equivalent of [3-(2-methoxy-2-oxoethyl)phenyl]acetic acid in a suitable solvent such as tetrahydrofuran or a m ixture of toluene and tetrahydrofuran with a solution of 2 to 5 equivalents of methylmagnesium bromide or methylmagnesium chloride in tetrahydrofuran at -20°C to 20°C. The intermediate of formula (1) according to the present invention is useful in the preparation of the β2 agonist N-[(4'-hydroxybiphenyl-3-yl)methyl]-2-(3-{2-[((2R)-2-hydroxy-2-{4-hydroxy-3-[(methylsulfonyl)amino]phenyl}ethyl)amino]-2-methylpropyl} henyl)acetamide of formula (14):

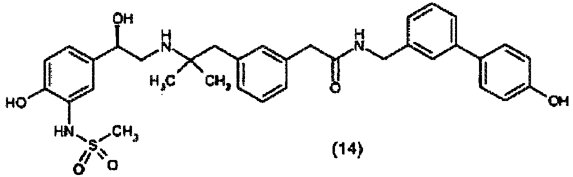

Another object of the present invention is thus a process for preparing the β2 agonist N-[(4'-hydroxybiphenyl-3-yl)methyl]-2-(3-{2-[((2R)-2-hydroxy-2-{4-hydroxy-3-[(methylsulfonyl)amino]phenyl}ethyl)-amino]-2-methylpropyl}phenyl)acetamide of formula (14) involving use of the compound of formula (1) according to the present invention. More precisely, the process for preparing the β2 agonist N-[(4'-hydroxybiphenyl-3-yl)methyl]-2-(3-{2-[((2R)-2-hydroxy-2-{4-hydroxy-3-[(methyl-sulfonyl)amino]phenyl}-ethyl)amino]-2-methylpropyl}phenyl)acetamide of formula (14) comprises the step of deprotecting the intermediate of formula (1) according to the present invention in order to obtain a compound of formula 11):

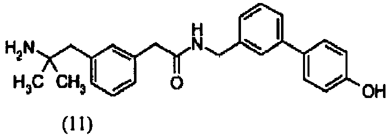

in the presence of thiourea and an acid such as hydrochloric acid, acetic acid or trifluoroacetic acid in a suitable solvent such as 2-butanol, acetic acid, isopropanol, ethyl acetate, isopropyl acetate, water or a mixture thereof. Typical conditions comprise reacting a mixture of 1.0 equivalent of the compound of formula (1) with 1 to 2 equivalents of thiourea and 1 to 4 equivalents of acetic acid in a mixture of water and 2-butanol and heating at reflux for 2 to 24 hours.

The compound of formula (11) is then reacted with a compound of formula (12): 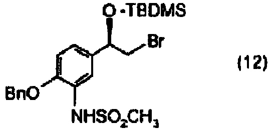 wherein Bn means benzyl and TBDMS means te/f-butyldimethylsilyl, to initially give the compound of formula 13): 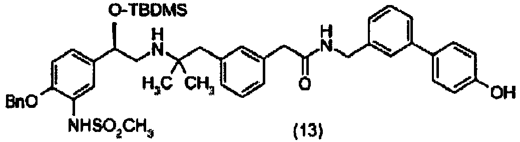 which is then followed by deprotection steps to obtain the final β2 agonist of formula (14). More precisely, the compound of formula (1 1) is reacted with the compound of formula (12) in the presence of a suitable base such as lithium carbonate, sodium hydrogen carbonate, sodium carbonate, potassium hydrogen carbonate, potassium carbonate or dipotassium hydrogenphosphate in a suitable solvent such as toluene, 4-methyl-2-pentanone, 2-methyl-2-butanol, 3-methyl-1-butanol, n-butyl acetate, n-propyl acetate, isopropyl acetate or propionitrile. Typical conditions comprise treating 1.0 equivalents of the compound of formula (11) with 0.5 to 2.0 equivalents of the compound of formula (12) and 1.0 to 4.0 equivalents of sodium hydrogen carbonate in n-butyl acetate at temperatures up to and including reflux for 10 to 48 hours. Preferably the compound of formula (13) thus obtained is not isolated and the first deprotection step is carried out directly. Preferably, two deprotection steps are carried out to remove the TBDMS and benzyl protecting groups and obtain the β2 agonist of formula (14). Preferably, a first deprotection step is carried out to remove the TBDMS protecting group to obtain a compound of formula (13a): 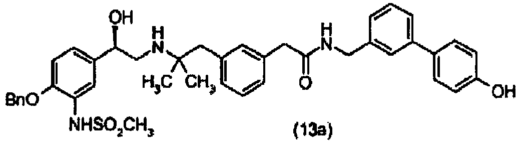 or a salt thereof, using a fluoride-containing reagent such as tetrabutylammonium fluoride, tetraethylammonium fluoride, tetramethylammonium fluoride, triethylamine trihydrofluoride or pyridine hydrofluoride i n a suitable solvent such as tetrahydrofuran, 2-methyl tetrahydrofuran, methanol, ethanol, 2-butanol, n-butyl acetate, ethyl acetate, isopropyl acetate, water or mixtures thereof and optionally in the presence of an acid such as acetic acid, trifluoroacetic acid, benzoic acid, fumaric acid or citric acid. Typical conditions comprise treating the compound of formula (13) with 1 to 2 equivale nts of tetraethylammonium fluoride and 1 to 4 equivalents of acetic acid in a mixture of n-butyl acetate, ethyl acetate and methanol at 20° to 60°C for 2 to 24 hours. Preferably a salt of compound of formula (13a) is then prepared and used in the next deprotection step. Possible salts of the compound of formula (13a) include L-tartrate, fumarate, L-ascorbate or xinafoate. A preferred salt of compound of formula (13a) is the hemifumarate salt (13b):

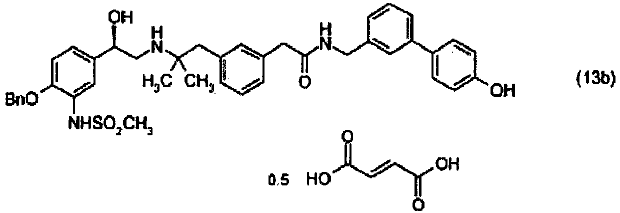

Said salt may be prepared by treating a solution of the compound of formula (13a) as a free base in a suitable solvent such as methanol, ethanol, isopropanol, n-butyl acetate, ethyl acetate, water, 4-methyl-2-pentanone, butan-2-one, tetrahydrofuran, 2-methyl tetrahydrofuran or mixtures thereof with fumaric acid at 20°C to reflux temperature. Typical conditions comprise treating 1.0 equivalents of the compound of formula (13a) with 0.4 to 0.6 equivalents of fumaric acid and seed crystals of the hemifumarate salt (13b) in a mixture of n-butyl acetate and ethanol at 50°C to 75°C for 0.25 to 2 hours then cooling the mixture to 10°C to 30°C for 1 to 10 hours. Preferably, the hemifumarate salt (13b) is prepared by combining a solution of 1.0 equivalent of the compound of formula (13a) as a free base in a suitable solvent such as ethanol or a mixture of ethanol and n-butyl acetate with a solution of 0.5 equivalents of fumaric acid in ethanol at 60°C to 70°C together with 0.5 to 5% w/w of seed crystals of the hemifumarate salt (13b) for 0.5 to 10 hours. Preferably a second deprotection step is then carried out to remove benzyl protecting group and obtain the β2 agonist of formula (14). Practically, the hemifumarate salt (13b) is treated with a base such as sodium carbonate, potassium carbonate, sodium hydrogen carbonate, potassium hydrogen carbonate, sodium hydroxide, potassium hydroxide in a mixture of water and a suitable solvent such as isopropyl acetate, ethyl acetate, n-butyl acetate, dichloromethane, tetrahydrofuran, or 2-methyl tetrahydrofuran to give the free base of the compound of formula (13a) which is then debenzylated using standard methodology as described in "Protective Groups in Organic Synthesis" by T. W. Greene and P. Wutz to provide the compound of formula (14). Typical conditions comprise treating 1.0 equivalents of the hemifumarate salt (13b) in tetrahydrofuran with an excess of an aqueous solution of potassium carbonate then washing the solution with aqueous sodium chloride solution to give a solution of the free base of compound of formula (13a) in a mixture of tetrahydrofuran and water which is reacted with an excess of hydrogen under 40 to 80 psi pressure in the presence of a suitable catalyst such as 5% palladium on carbon at 15 to 30°C for 2 to 48 hours. After removing the catalyst from the reaction mixture, the β2 agonist of formula (14) is crystallized by exchanging the solvent into an antisolvent which may be selected from ethyl acetate, butyl acetate, isopropyl acetate, acetonitrile, toluene, butan-2-one, 4-methyl-2-pentanone, methanol, ethanol, or 2-methyltetrahydrofuran using a distillation process and is then isolated. Preferably the antisolvent is acetonitrile. The β2 agonist of formula (14) thus obtained may then be purified by slurrying the compound of formula (14) in a solvent which may be selected from ethanol, methanol, isopropanol, acetonitrile, water, tetrahydrofuran, 2-methyl tetrahydrofuran, toluene, acetone, butan-2-on e, 4-methyl-2-pentanone, ethyl acetate, butyl acetate, isopropyl acetate or solvent mixture thereof. Preferably, the β2 agonist of formula (14) is purified by slurrying in a mixture of 10%v/v to 30%v/v water in methanol at 10 to 60°C for 1 to 24 hours. The β2 agonist of formula (14) thus obtained may be re-crystallized by dissolving the compound of formula (14) obtained according to the steps described above in a suitable solvent such as a mixture of water and tetrahydrofuran and then exchanging the solvent with an antisolvent which may be selected from ethyl acetate, butyl acetate, isopropyl acetate, acetonitrile, toluene, butan-2-one, 4-methyl-2-pentanone, methanol, ethanol, or 2-methyltetrahydrofuran. Preferably, the β2 agonist of formula (14) is re-crystallized by dissolving the compound of formula (14) in a mixture of 10%v/v to 30%v/v water in tetrahydrofuran and exchanging the solvent into mainly acetonitrile using a distillation process during which the β2 agonist of formula (14) crystallizes. The β2 agonist of formula (14) thus obtained may then be purified by slurrying the compound of formula (14) in a solvent which may be selected from ethanol, methanol, isopropanol, acetonitrile, water, tetrahydrofuran, 2-methyl tetrahydrofuran, toluene, acetone, butan-2-one, 4-methyl-2-pentanone, ethyl acetate, butyl acetate, isopropyl acetate or solvent mixture thereof. Preferably, the β2 agonist of formula (14) is purified by slurrying in a mixture of 10% v/v to 30% v/v water in methanol at 10 to 60°C for 1 to 24 hours.

The compound of formula (12) may be prepared as described in WO 2007/010356. Alternatively, the compound of formula (12) may be prepared as disclosed in scheme 3:

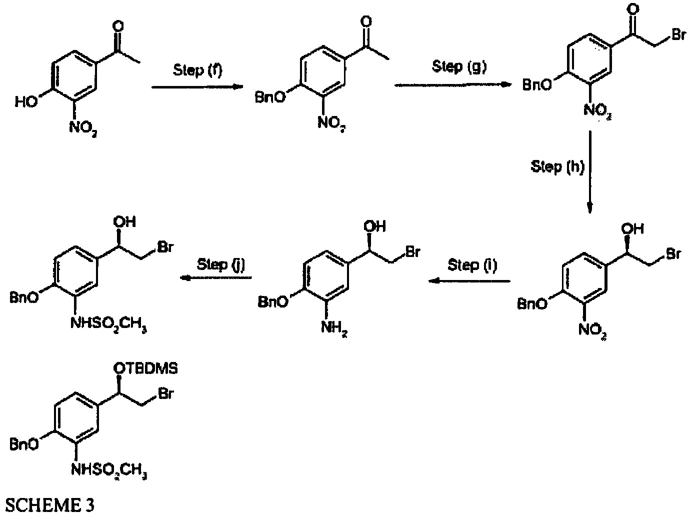

Steps (f) and (g) are performed according to the conditions well known from the literature (Step (f): Greene, T. W.; Wuts, P. G. M. (1999). Protective Groups in Organic Synthesis (3rd ed.), Wiley-Interscience, p. 266, ISBN 0-471-16019-9.; Step (g): Furniss, B. S.; Hannaford, A. J.; Smith, P. W. G.; Tatchell, A. R. (1989). Vogel's Textbook of Practical Organic Chemistry (5th ed.), Harlow:Longman, p. 1052, ISBN 0-582-46236-3). The starting materials are all commercially available. Steps (h) to (k) are similar to those described in WO 2007/010356. The process for preparing the β2 agonist N-[(4'-hydroxybiphenyl-3-yl)methyl]-2-(3-{2-[((2R)-2-hydroxy-2-{4-hydroxy-3-[(methylsulfonyl)amino]phenyl}ethyl)amino]-2-methylpropyl}phenyl)acetamide of formula (14) is further summarized below in Scheme 4:

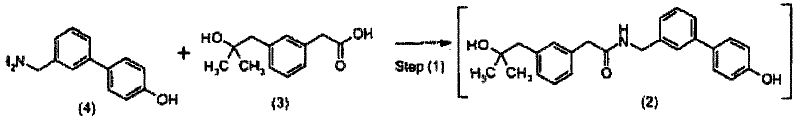

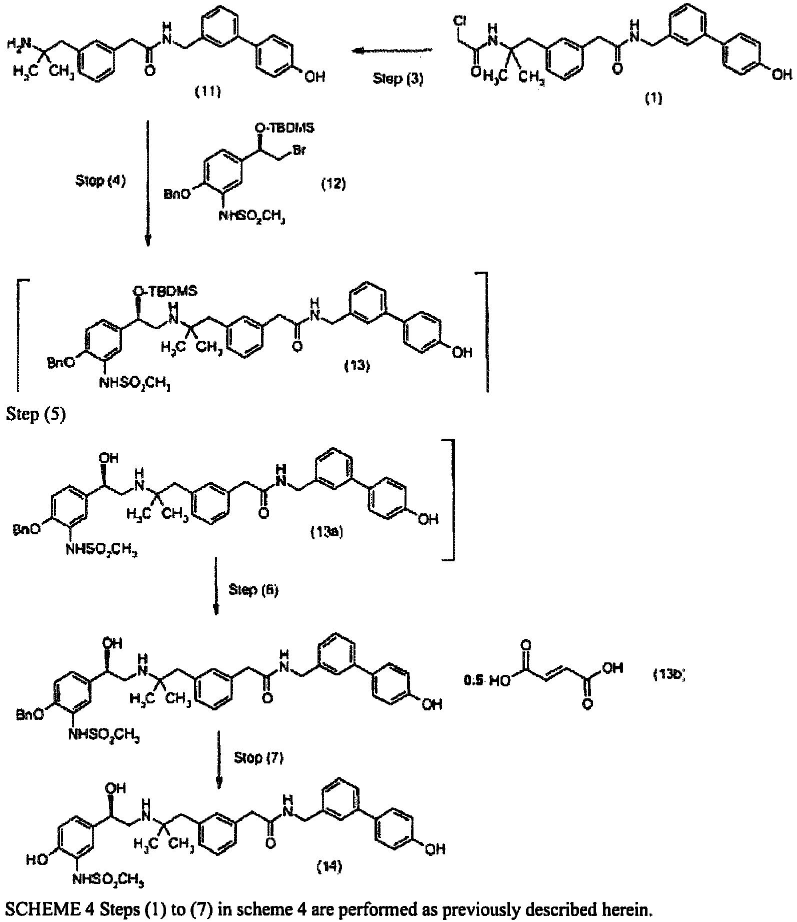

Aspect (iii) of the present invention is further illustrated by the examples below.

The figures of WO2013/021309 show the following: Figure 1 of WO2013/021309: DSC trace of the compound of example 1; Figure 2 of WO2013/021309: PXRD pattern of the compound of example I; Figure 3 of WO2013/021309: Fourier Transform Infra-red (FT-IR) spectroscopy pattern of example 1; Figure 4 of WO2013/021309: Expanded form of the fingerprint region of the FT-IR spectroscopy pattern of example 1; Figure 5 of WO2013/021309: Fourier Transform Raman spectroscopy pattern of example 1; Figure 6 of WO2013/021309: Expanded form of the fingerprint region of the Fourier Transform Raman spectroscopy pattern of example 1; Figure 7 of WO2013/021309: CPMAS spectrum of example 1;

Figure 8 of WO2013/021309: DSC trace of the compound of preparation 6; Figure 9 of WO2013/021309: PXRD pattern of the compound of preparation 6; Figure 10 of WO2013/021309: Fourier Transform Infra-red (FT-IR) spectroscopy pattern of preparation 6; Figure 11 of WO2013/021309: Expanded form of the fingerprint region of the FT-IR spectroscopy pattern of preparation 6; Figure 12 of WO2013/021309: Fourier Transform Raman spectroscopy pattern of preparation 6; Figure 13 of WO2013/021309: Expanded form of the fingerprint region of the Fourier Transform Raman spectroscopy pattern of preparation 6; Figure 14 of WO2013/021309: CPMAS spectrum of preparation 6; Figure 15 of WO2013/021309: PXRD pattern for the compound of example 2; Figure 16 of WO2013/021309: Fourier Transform Infra-red (FT-IR) spectroscopy pattern of example 2; Figure 17 of WO2013/021309: Expanded form of the fingerprint region of the FT-IR spectroscopy pattern of example 2; Figure 18 of WO2013/021309: Fourier Transform Raman spectroscopy pattern of example 2; Figure 19 of WO2013/021309: Expanded form of the fingerprint region of the Fourier Transform Raman spectroscopy pattern of example 2; Figure 20 of WO2013/021309: CPMAS spectrum of example 2; Figure 21 of WO2013/021309: ¹H NMR of chloroacetonitrile (performed in DMSO with water present); Figure 22 of WO2013/021309: ¹H NMR of a solution of 2-Chloro-N-{2-[3-(2-{[(4'-hydroxybiphenyl-3-yl)methyl]amino}-2-oxoethyl)phenyl]-1,1-dimethyl ethyl }acetamide as obtained in WO2007/010356; Figure 23 of WO2013/021309: ¹H NMR of the isolated crystalline 2-Chloro-N-{2-[3-(2-{[(4'-hydroxybiphenyl-3-yl)methyl]amino}-2-oxoethyl)phenyl]-1,1-dimethylethyl}-acetamide according to example 1.

EXAMPLES as also described in WO2013/021309:

### Example 1: Preparation of the crystalline form of 2-Chloro-N-{2-[3-(2-{[(4'-hydroxybiphenyl-3-yl)methyllamino)-2-oxoethyl)phenyl1-1,1-dimethylethyl)-acetamide of formula (1)

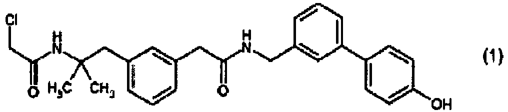

### a) Preparation 1: 2-[3-(2-Hydroxy-2-methylpropyl)phenyl]acetic acid of formula (3)

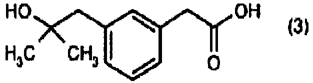

2-[3-(2-Hydroxy-2-methylpropyl)phenyl]acetic acid may be prepared as described in WO 2007/010356. Alternatively, it may be prepared as follows: Preparation 1 a): Dimethyl 2,2'-(1,3-phenylene)diacetate of formula (9) (wherein R is methyl) (9, R = methyl):

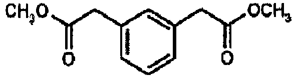

A solution of 1,3-bis(chloromethyl)benzene (15g, 85.6 mmol) and N,N-diisopropylethylamine (60 ml, 344 mmol) in methanol (107 ml) was charged to a bomb pressure reactor, which contained a magnetic stirrer bar, tetrakis(triphenylphosphine) palladium(O) (0.99g, 0.85 mmol) was added and the apparatus was purged with carbon monoxide. The apparatus was pressurized to 300psi of carbon monoxide and heated to 80°C and stirred at this temperature for 8 hours. The reaction was allowed to cool to room temperature and the mixture was evaporated under vacuum. The residue was dissolved in toluene (400ml) and washed with aqueous hydrochloric acid {2N, 2 x 300ml). The toluene layer was washed with saturated aqueous sodium hydrogen carbonate solution (300ml) and saturated brine (100ml) then dried (MgSO₄) and evaporated under vacuum to give dimethyl 2,2'-(1,3-phenylene)diacetate a yellow mobile oil with a few solids present (17.2g, 90%) that can be used directly in the next step. ¹H NMR: when analysed by conventional proton NMR (400MHz, d₆-DMSO), the compound of preparation 1a) gives the following spectrum: δ 3.62 (s, 6H), 3.67 (s, 4H), 7.16-7.17 (m, 3H) and 7.26-7.28 (m, 1 H). ¹³C NMR: when analysed by conventional carbon NMR (100MHz, d₆-DMSO), the compound of preparation 1a) gives the following spectrum: δ 40.0, 51.7, 127.8, 128.4, 130.2, 134.4 and 171.5. MS: when analysed by mass spectrometry, using positive electrospray ionisation technique, the compound of preparation 1a) gave a mass of 223.0966 (Ci₂Hi₅0), calculated 223.0965.

### Preparation 1b): [3-(2-methoxy-2-oxoethyl)phenyl]acetic acid of formula (8) (wherein R is methyl) (8, R = methyl):

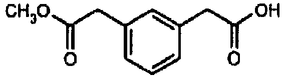

Lipolase® (Thermomyces lanuginsus lipase solution, 1.9 g) was added to a solution of calcium acetate in water (23.0 g of a 0.2M solution) and the resultant mixture was stirred at room temperature for 30 minutes. A solution of dimethyl 2,2'-(1,3-phenylene)diacetate (5.0 g) in toluene (5 ml) was added and the mixture was stirred at room temperature. The pH of the mixture was maintained at approximately pH 6.5 by the addition of aqueous 1 M sodium hydroxide controlled by a pH stat. The reaction was complete after 27 hours. The pH of the mixture was adjusted to pH 3.6 using 1 M aqueous hydrochloric acid, and ethyl acetate (25 ml) was added. After stirring for 1 hour, the mixture was filtered through filter aid and the solids were washed with ethyl acetate (100 ml). The filtrates were combined and the phases were separated. The aqueous phase was extracted with ethyl acetate (2 x 25 ml), and the combined organic phases were extracted with sodium hydrogen carbonate solution (3 x 30 ml, 10% aqueous solution). The combined aqueous extracts were acidified to pH 2 using 5M hydrochloric acid, and were extracted with toluene (2 x 50 ml). The combined organic layers were dried (MgSO₄) and concentrated in vacuo to give the title compound (2.81 g) as a mobile oil that can be used directly in the next step. ¹H NMR: when analysed by conventional proton NMR (400MHz, d₆-DMSO), the compound of preparation 1b) gives the following spectrum: δ 3.55 (s, 2H), 3.62 (s, 3H), 3.67 (s, 2H), 7.14-7.17 (m, 3H), 7.25-7.28 (m, 1 H) and 12.34 (bs, 1 H). ¹³C NMR: when analysed by conventional carbon NMR (100MHz, d₆-DMSO), the compound of preparation 1b) gives the following spectrum: δ 40.5, 40.6, 51.7, 127.6, 127.8, 128.3, 130.2, 134.3, 135.0, 171.5 and 172.5. MS: when analysed by mass spectrometry, using positive electrospray ionisation technique, the compound of preparation 1b) gave a mass of 209.0806 (CnHi₃O), calculated 209.0808.

### Preparation 1c): [3-(2-Hydroxy-2-methylpropyl)phenyl]acetic acid of formula (3)

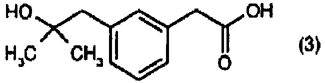

A solution of crude [3-(2-methoxy-2-oxoethyl)phenyl]acetic acid (20g, net 18.9g; 0.09mole; HPLC purity 94.6% including toluene peak) in dry tetrahydrofuran (170ml) and dry toluene (170ml) was cooled to -15 to -5°C under N₂ purge. A solution of 3M methylmagnesium chloride in tetrahydrofuran (106ml; 0.32 mole, 3.5eq) was added dropwise keeping the temperature below 0°C. On completion of the addition, the cooling was removed and the grey suspension was allowed to warm to ambient temperature. Once the reaction was complete, the slurry was cooled to 0 to 5°C and quenched by the slow addition of water (100ml). The pH of the mixture was then adjusted to between pH 1 and pH 2.5 by adding concentrated hydrochloric acid. The mixture was extracted with isopropyl acetate (100ml) and the isopropyl acetate extracts back washed with water (3 x 50ml). The organic phase was dried (MgSO₄), and the solvent was exchanged into approximately 100 ml of toluene using vacuum distillation. The resultant mixture was heated to 55°C, then cooled to 20°C over 4 hours and stirred for 2 hours. The resultant solid was collected by filtration, washed with toluene (20 ml) and dried at 50°C in vacuo to give the title compound as a yellowish solid (14.2 g). If necessary [3-(2-hydroxy-2-methylpropyl)phenyl]acetic acid can be purified using the following procedure. A mixture of 3-(2-hydroxy-2-methylpropyl)phenyl]acetic acid (14.2g) and ethyl acetate (55ml) and heptane (52ml) was heated to 50°C to give a clear solution. The solution was cooled to 35°C over 1 hour, and then seed crystals were added and the mixture was stirred at 35°C for 1 hour. The slurry was cooled to 30°C over 1 hour and cooled further to -5°C over 4 hours. The slurry was agitated for 4 hours. The resultant solid was collected by filtration, washed the with a mixture of ethyl acetate (6ml) and heptane (23ml) and dried at 50°C in vacuo to give purified [3-(2-hydroxy-2-methylpropyl)phenyl]acetic acid as a white solid (11.4g). Characterisation data for [3-(2-hydroxy-2-methylpropyl)phenyl]acetic synthesised using preparation 1c) is identical to that reported in WO 2007/010356. Alternatively, 2-[3-(2-hydroxy-2-methylpropyl)phenyl]acetic acid may be prepared through the intermediate diethyl 2,2'-(1,3-phenylene)diacetate as follows:

### Preparation 1d): diethyl 2,2'-1,3-phenylene)diacetate:

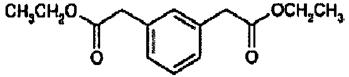

A solution of 1,3-bis(chloromethyl)benzene (15g, 85.6 mmol) and N,N-diisopropylethylamine (60 ml, 344 mmol) in ethanol (107 ml) was charged to a bomb pressure reactor, which contained a magnetic stirrer bar, Tetrakis(triphenylphosphine) palladium(O) (0.99g, 0.85 mmol) was added and the apparatus was purged with carbon monoxide. The apparatus was pressurized to 300psi of carbon monoxide and heated to 80°C and stirred at this temperature for 12 hours. The reaction was allowed to cool to room temperature. The mixture was evaporated under vacuum. The residue was dissolved in toluene (400ml) and washed with HCl (2N, 2 x 300ml). The organic phase was washed with saturated sodium hydrogen carbonate solution (300ml) and saturated brine (100ml), dried (MSO₄) and evaporated under vacuum to give the title compound as a yellow mobile oil with a few solids present (21.4g). ¹H NMR: when analysed by conventional proton NMR (400MHz, d₆-DMSO), the compound of preparation 1 d) gives the following spectrum: δ 1.19 (t, J 8.0Hz, 6H), 3.64 (s, 4H), 4.08 (q, J 8.0Hz, 4H), 7.16-7.18 (m, 3H) and 7.26-7.28 (m, 1 H). ¹³C NMR: when analysed by conventional carbon NMR (100MHz, d₆-DMSO), the compound of preparation 1 d) gives the following spectrum: δ 14.0, 40.2, 60.2, 127.8, 128.3, 130.1, 134.5 and 171.0. MS: when analysed by mass spectrometry, using positive electrospray ionisation technique, the compound of preparation 1 d) gave a mass of 251.1277 (C14H19O4), calculated 251.1278. Subsequent conversion to 2-[3-(2-hydroxy-2-methylpropyl)phenyl]acetic acid are then similar to those described in preparation 1 b) and 1 c), and in WO 2007/010356.

b) Preparation 2: 3'-(aminomethyl)biphenyl-4-ol of formula (4): 3'-(Aminomethyl)biphenyl-4-ol of formula (4) may be prepared using the method described in WO2008/010853. Alternatively, 3'-(aminomethyl)biphenyl-4-ol may be prepared as described below: Preparation 2a): 4-Hydroxybiphenyl-3-carbonitrile of formula (5):

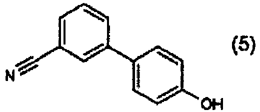

To a solution of (3-cyanophenyl)boronic acid (25.0 g, 0.170 mol) in tetrahydrofuran (125 ml) under a nitrogen atmosphere was added 4-bromophenol (28.0 g, 0.162 mol) followed by a second portion of tetrahydrofuran (100 ml). To this mixture was added a solution of sodium carbonate (20.0 g, 0.238 mol) in water (125 ml) and the resultant mixture was stirred at room temperature for 10 minutes whilst being sparged with nitrogen, To the mixture was added tris(dibenzylideneacetone)dipalladium(0) (3.16 g, 0.00324 mol), tris-te/t-butylphosphonium tetrafluoroborate (1.88 g, 0.00648 mol) and tetrahydrofuran (25 ml). The reaction was stirred at room temperature under an atmosphere of nitrogen for 4 hours and filtered. The solids were washed with tetrahydrofuran (50 ml), and the combined filtrates were extracted with te/f-butyl methyl ether (2 x 100 ml). The combined organic phases were filtered through a pad of filter aid, and the solvent in the filtrate was exchanged into 2-propanol (100ml) using distillation. The solution was cooled to 4 to 5°C over 3 hours and the resultant slurry was stirred at 4 to 5°C for 2 hours. The solid was collected by filtration, and was washed with cold 2-propanol (3 x 50 ml) and dried in vacuo to give 4-hydroxybiphenyl-3-carbonitrile (25.4 g) as an off-white solid. ¹H NMR: when analysed by conventional proton NMR (400MHz, de-DMSO), the compound of preparation 2a) gives the following spectrum: δ 6.88 (d, J 8.0Hz, 2H), 7.57-7.63 (m, 3H), 7.73 (d, J 8.0Hz, 1 H), 7.93 (d, J 8.0Hz, 1 H), 8.05 (s, 1 H) and 9.72 (bs, 1 H). ¹³C NMR: when analysed by conventional carbon NMR (100MHz, d₆-DMSO), the compound of preparation 2a) gives the following spectrum: δ 1 12.0, 1 15.9, 1 18.9, 128.1, 128.6, 129.3, 129.9, 130.0, 130.6, 141.3 and 157.9. MS: when analysed by mass spectrometry, using positive electrospray ionisation technique, the compound of preparation 2a) gave a mass of 196.0758 (Ci₃Hi₀NO), calculated 196.0757. Alternative conditions are provided as follows: A solution of 2-methyltetrahydrofuran (790.5 kg) and purified water (430.9 kg) was heated to reflux under a nitrogen atmosphere for two hours then cooled to 15-25°C. To this solution at 30-30°C was added (3-cyanophenyl)boronic acid (93.0 kg, 632.9 mol) and 4-bromophenol (1 15.0 kg, 664.7 mol). Anhydrous potassium carbonate (174.7 kg, 1264 mol) was then added in 10-12 kg portions every 5-8 minutes. After purging the reactor with nitrogen, 1,1'-bis(diphenylphosphino)ferrocene (7.02 kg, 12.7 mol) was added and the mixture was stirred for 10 minutes, and then palladium(11) acetate (2.84 kg, 12.7 mol) was added. The resultant mixture was heated at 65-70°C under nitrogen with stirring for 8 hours. The mixture was cooled to 15-25°C, and n-hexane (613.8 kg) was added. The resultant slurry was filtered, and the filter cake was washed with 2-methyltetrahydrofuran (79.0 kg). The filtrate and wash were collected and the lower aqueous phase was removed. To the organic phase was added n-hexane (202.6 kg) and the mixture was stirred for 3.5 hours then filtered through a pad of silica gel (60 kg) held in a filter. The filter cake was washed with a solution of 2-methyltetrahydrofuran (79 kg) and n-hexane (82 kg). The combined filtrate and wash were concentrated by distillation at atmospheric pressure until the volume of the residue was 250-300 L, and then distillation was continued at reduced pressure (<0.08MPa) until the volume of the residue was 150-160 L. The mixture was then cooled to 30-35°C, n-hexane (429.6 kg) was added, and the mixture was cooled to 15-25°C. After stirring for 2 hours, the solid was collected by filtration and, washed with n-hexane (61.4 kg) and then dried at 50°C in vacuo to give 4-hydroxybiphenyl-3-carbonitrile (1 13.4 kg) as a pale yellow solid.

### Preparation 2b): 3'-(Aminomethyl)biphenyl-4-ol of formula (4):

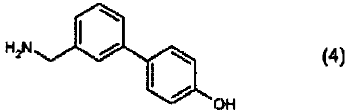

3'-(Aminomethyl)biphenyl-4-ol may be prepared using the method described i n WO 2008/010853. Alternatively, 3'-(aminomethyl)biphenyl-4-ol may be prepared as described below: To a solution of 4-hydroxybiphenyl-3-carbonitrile (18.0 g, 0.0922 mol) in methanol (270 ml) was added methanesulfonic acid (17.7 g, 0.184 mol) and 10% palladium on carbon (1.8 g). The resultant mixture was hydrogenated under 145 psi pressure of hydrogen at 40°C for 4 hours. The reaction mixture was filtered through filter aid, and the solvent in the filtrate was exchanged into predominantly water by distillation to give an aqueous solution having a total volume of approximately 180 ml. The solution was cooled to room temperature, and the pH was adjusted to between pH 9.3 and 9.7 by the addition of 2M aqueous sodium hydroxide. The resultant slurry was filtered. The solid was washed with water (2 x 50 ml) and dried at 45°C in vacuo to give the title compound (16.5 g) as a colourless solid. If necessary, 3'-(aminomethyl)biphenyl-4-ol may be purified as follows. A suspension of 3'-(aminomethyl)biphenyl-4-ol (1.0 g) and absolute ethanol (20 ml) was heated to reflux for 2 hours then cooled to room temperature over 2 hours and stirred at this temperature for 4 hours. The solid was collected by filtration, washed with ethanol (2 x 5 ml) and dried at 45°C in vacuo to give purified 3'-(aminomethyl)biphenyl-4-ol (0.85 g) as an off-white solid. ¹H NMR: when analysed by conventional proton NMR (400MHz, d₆-DMSO), the compound of preparation 2b) gives the following spectrum: δ 3.75 (s, 2H), 6.85 (d, J 8.0Hz, 2H), 7.23 (d, J 8.0Hz, 1 H), 7.33 (t, J 8.0Hz, 1 H), 7.40 (t, J 8.0Hz, 1 H), 7.48 (d, J 8.0Hz, 2H) and 7.55 (s, 1 H). ¹³C NMR: when analysed by conventional carbon NMR (100MHz, d₆-DMSO), the compound of preparation 2b) gives the following spectrum: δ 45.7, 1 15.6, 123.8, 124.7, 125.1, 127.7, 128.5, 131.1, 140.0, 144.8 and 157.0. MS: when analysed by mass spectrometry, using positive electrospray ionisation technique, the compound of preparation 2B) gave a mass of 200.1072 (C-|₃H-|₄N0), calculated 200.1070.

Alternative conditions for the synthesis of 3'-(aminomethyl)biphenyl-4-ol are provided as follows: To a suspension of freshly prepared Raney Nickel (prepared from nickel-aluminium alloy (86 kg)) in methanol (925.2 kg) was added 4-hydroxybiphenyl-3-carbonitrile (88.0 kg, 450.8 mol). To this mixture was added a solution of sodium methoxide in methanol (162.8 kg of a 30% solution, 904.4 mol) over 1 hour. After purging the reactor with nitrogen, it was purged and pressurised to 0.3MPa with hydrogen and then heated to 38-45°C and hydrogenated at this temperature for 6 hours. The hydrogen was vented and the catalyst was removed by filtration. The spent catalyst was washed with purified water (88 kg), and the combined filtrates were concentrated at a temperature of 55°C and pressure of <-0.08MPa until no further distillate was produced. Purified water (1936 kg) was added to the residue and the mixture was concentrated under the same conditions until no further distillate was produced. The mixture was cooled to 20-30°C, activated carbon (17.6 kg) was added and the suspension was stirred at this temperature for 4-6 hours after which time it was filtered through celite (40 kg). The filter cake was washed with purified water (88 kg) and the combined filtrates were neutralised to pH 6-7 with acetic acid (80 kg) at 15-25°C. The mixture was washed with a mixture of isopropyl acetate (224.4 kg) and tetrahydrofuran (78.4 kg) and then twice with isopropyl acetate (224.4 kg each wash). To the product-containing aqueous phase was added activated carbon (17.6 kg). The suspension was stirred at 20-30°C for 2-4 hours, and then filtered. The filter cake was washed with purified water (88 kg) and the combined filtrates were treated with 20% aqueous sodium hydroxide (84 kg) until the pH of the mixture was pH 9-10. The mixture was stirred at 15-25°C for 1 hour, and the solids were collected by filtration then washed with water (470.4 kg) until the pH of the filtrate was pH 7-8. To a suspension of the crude solid in purified water (1080 kg) was added acetic acid (18 kg) until the pH was 6-7. The mixture was filtered to remove particulate material, and the pH of the filtrate was adjusted to pH 9-10 by the addition of 20% aqueous sodium hydroxide. After stirring for 1 hour, the solid was collected by filtration and washed with purified water (600 kg). The damp cake was combined with 95% ethanol (800 kg) and purified water (50 kg) and mixture was heated at reflux for 8 hours after which time it cooled to 15-20°C, filtered and washed with 95 % ethanol (60 kg). The damp cake was dried at 50 to 60 ° C to give 3'-(aminomethyl)biphenyl-4-ol (47.2 kg) as a colourless solid. If necessary, further purification may be carried out using one or both of the procedures described as follows: - *Purification method 1:* Purified water (360 kg) and 3'-(aminomethyl)biphenyl-4-ol (34.2 kg) were combined and a solution of sodium hydroxide (43 kg of a 20% solution) was added until the mixture became a clear solution. The solution was filtered through a bed of celite (4 kg) and the cake was washed with purified water (10 kg). The pH of the filtrate was adjusted to pH 6-7 using acetic acid (22 kg), and purified water (100 kg) was then added. The mixture was then filtered. The pH of the filtrate was adjusted to pH 9-10 using 20% aqueous sodium hydroxide to give a slurry that was stirred for 1 hour then filtered. The filter cake was washed with purified water (140 kg). The damp cake was suspended in 95% ethanol (320 kg) and the slurry was heated at reflux for 3 hours. The mixture was the cooled to 25-30°C and stirred for 30 mins before the solid was collected by filtration. The filter cake was washed with 95% ethanol (40 kg), and then dried to give purified 3'-(aminomethyl)biphenyl-4-ol (17.0 kg). - *Purification method* 2: Purified water (680 kg) and 3'-(aminomethyl)biphenyl-4-ol (32.4 kg) were combined and acetic acid (1 1.3 kg) was added at 15-25°C to adjust the pH to pH 6-7. The mixture was filtered through a pad of celite (204 kg) and the cake was washed twice with purified water (68.4 kg and 157.3 kg) then with methanol (326.4 kg) then three times with purified water (306 kg each wash). The combined filtrates were basified to pH 9-10 using 20% aqueous sodium hydroxide (51 kg), and the mixture was stirred at 15-25°C for 1 hour. The solid was collected by filtration, and the filter cake was washed with purified water (681 kg then three times using 100 kg each wash). The damp cake was suspended in a mixture of 95% ethanol (438.3 kg) and purified water (28.3 kg) and the mixture was heated at reflux for 3 hours. After cooling to 15-20°C, the suspension was stirred for 2 hours and the solid was collected by filtration. The filter cake was washed with 95% ethanol (26.9 kg) and was then dried to give purified 3'-(aminomethyl)biphenyl-4-ol (29.4 kg).

### c) Preparation of the crystalline form of 2-chloro-N-(2-[3-(2-([(4'-hydroxybiphenyl-3-yl)methyl1amino}-2-oxoethyl)phenyl]-1,1-dimethylethyl}-acetamide of formula (1): [3-(2-Hydroxy-2-methylpropyl)phenyl]acetic acid according to preparation 1

(30 kg, 144 mol) was dissolved in ethyl acetate (150 L) at 25°C. 1,1'-Carbonyldiimidazole (24.1 Kg, 144 mol) was added to the sol ution and the reaction left to proceed for 1 hour. 3'-(Aminomethyl)biphenyl-4-ol according to preparation 2 (31.6 Kg, 159 mol) was added and the resulting slurry was heated to 50°C. After 3 hours the reaction mixture was cooled 20°C and was washed with 2M aqueous citric acid (158 L) and I M aqueous sodium bicarbonate solution (150 L). The organic layer was diluted with chloroacetonitrile (56.1 L) and the solution distilled at atmospheric pressure, the end point was when the internal temperature reached 110°C. The chloroacetonitrile solution was cooled to 50°C and trifluoroacetic acid (168 L) was charged over 90 minutes. The reaction was allowed to proceed at this temperature for a further 2 hours. The solution was subjected to a reduced pressure (-0.950 barg) to allow the distillation of trifluoroacetic acid maintaining a temperature of 50°C. Upon completion of the distillation 2-butanol (225 L) was added to the reaction and the solution was warmed to 60°C. Seed crystals of 2-chloro-N-{2-[3-(2-{[(4'-hydroxybiphenyl-3-yl)methyl]amino}-2-oxoethyl)phenyl]-1,1-dimethylethyl}acetamide (0.67 kg, 1 %w/w based on 100% conversion) were added and the slurry was held at 60°C for i hour. Cyclohexane (150 L) was added whilst maintaining the 60°C temperature. The slurry was allowed to cool from 60°C to 20°C at a rate of 0.5°C/min) and stirred at 20°C for 4 hours. The solid was collected by filtration, washed with cyclohexane (2 x 90 L), and dried in vacuo at 45°C to give 2-chloro-N-{2-[3-(2-{[(4'-hydroxybiphenyl-3-yl)methyl]amino}-2-oxoethyl)phenyl]-1,1-dimethylethyl}-acetamide (58.7kg, 87.6% yield) a colourless solid. Seed crystals of 2-chloro-N-{2-[3-(2-{[(4'-hydroxybiphenyl-3-yl)methyl]amino}-2-oxoethyl)phenyl]-1,1-dimethylethyl}acetamide were prepared as follows: [3-(2-Hydroxy-2-methylpropyl)phenyl]acetic acid according to preparation 1 (40 g, 0.192 mol) was dissolved in ethyl acetate (200 ml) at 25°C. 1,1'-Carbonyldiimidazole (33.02g, 0.192mol) was added to the solution and the reaction left to proceed for 1 hour. 3'-(Aminomethyl)biphenyl-4-ol according to preparation 2 (42.1 g, 0.21 1 mol) was added and the resulting slurry was heated to 50°C. After 3 hours the reaction mixture was cooled to 20°C and was washed with 2M aqueous citric acid (211 ml) and 1 M aqueous sodium bicarbonate solution (200 ml). The organic layer was diluted with chloroacetonitrile (74.8 ml) and the solution was distilled at atmospheric pressure, the end point was when the internal temperature reached 110°C. The chloroacetonitrile solution was cooled to 50°C and trifluoroacetic acid (224 ml) was added over 90 minutes. The reaction was allowed to proceed at this temperature for a further 2.5 hours. The solution was subjected to a reduced pressure (1 13 mbar) to allow the distillation of trifluoroacetic acid maintaining a temperature of 50°C. Upon completion of the distillation, 2-butanol (300 ml) was added to the reaction and the solution was warmed to 60°C and held under a positive pressure of nitrogen overnight. The solution was transferred to a larger reactor also maintained at 60°C whereupon crystallisation occurred within 10 minutes. Cyclohexane (748 ml) was added whilst maintaining the 60°C temperature. The slurry was allowed to cool from 60°C to 20°C and stirred at 20°C for 3 hours. The solid was collected by filtration and was dried in vacuo at 45 °C to give 2-chloro-N-{2-[3-(2-{[(4'-hydroxybiphenyl-3-yl)methyl]amino}-2-oxoethyl)phenyl]-1,1-dimethylethyl}acetamide (81. Og, 90.7% yield) as an off-white solid. ¹H NMR: When analysed by conventional proton NMR (600MHz, d6-DMSO), the material obtained in example 1 gave the following spectrum: δ 1.22 (s, 6H), 2.96 (s, 2H), 3.48 (s, 2H), 4.00 (s, 2H), 4.34 (d, J 5.9 Hz, 2H), 6.87 (m, 2H), 7.00 (dt, J 7.5 & 1.5 Hz, 1 H), 7.06 (t, J 1.5 Hz, 1 H), 7.15 (d, J 7.7 Hz, 3H), 7.17 (d, J 7.5 Hz, 1 H), 7.21 (t, J 7.5 Hz, 1 H), 7.34 (t, J 7.7 Hz, 1 H), 7.42 (m, 4H), 7.60 (s, I H), 8.58 (t, J 5.9 Hz, 1 H) and 9.56 (br s, 1 H). DSC Data: The melting point of the material obtained in example 1 was determined by Differential Scanning Calorimetry (DSC) using a TA instruments Q1000 differential scanning calorimeter. The sample was heated at 20°C/minute, from 20°C to 300°C, in a closed aluminium pan under nitrogen purge gas. 1.635 mg of the material obtained in example 1 shows a sharp endothermic melt with onset of 157°C ± 2°C and peak at 159°C. ± 2°C. The DSC trace obtained is shown in Figure 1 of WO2013/021309. Elemental analysis for the material obtained in example 1: Found: C, 69.64%; H, 6.31 % and N, 5.99%. C27H29CIN2O3 requires C, 69.74%; H, 6.29% and N, 6.02%. Powder X-Rav Diffraction Data: The powder X-ray diffraction pattern of the material obtained in example I was determined using a Bruker-AXS Ltd. D4 EN DEAVOR powder X-ray diffractometer fitted with an automatic sample changer, a theta-theta goniometer geometry, automatic beam divergence slit and a PSD Vantec-1 detector. The sample was prepared for analysis by mounting onto a low background silicon wafer specimen mount with a 0.5 mm cavity. The specimen was rotated whilst being irradiated with copper KCH X-rays (wavelength = 1.5406 Angstroms) with the X-ray tube operated at 35kV/40mA. The analysis was in continuous mode set for data acquisition at 0.2 second count per 0.018° step size over a two theta range of 2° to 55° at room temperature. The instrument calibration was verified using a corundum reference standard (NIST: SRM 1976 XRD flat plate intensity standard). The peaks obtained were aligned against a silicon reference standard. Peak search was carried out using the threshold and width parameters set to 1 and 0.15, respectively, within the Eva software released by Bruker-AXS. Characteristic diffraction angles and their intensities are given in Table 1 and Figure 2 of WO2013/021309 shows the experimentally measured pattern. The unique diffraction peaks of this material are given in Table 2. Table 1: Characteristic diffraction peaks of example 1 (± 0.1 ° 2Θ):

| **Angle (°2θ)** | **Intensity %** | **Angle (°2θ)** | **Intensity %** |
|---|---|---|---|
| 9.1 | 7.7 | 22.9 | 25.9 |
| 10.5 | 18.8 | 23.3 | 88.7 |
| 11.4 | 6.7 | 24.0 | 79.9 |
| 13.2 | 7.1 | 25.7 | 37.2 |
| 13.4 | 4.6 | 26.7 | 25.9 |
| 14.1 | 12.9 | 27.0 | 45.7 |
| 15.5 | 40.2 | 27.4 | 17.3 |
| 16.1 | 63.0 | 28.3 | 16.7 |
| 17.0 | 59.0 | 29.8 | 18.5 |
| 17.4 | 32.6 | 30.3 | 24.1 |
| 18.0 | 33.5 | 30.6 | 16.9 |
| 18.5 | 7.7 | 31.4 | 14.5 |
| 19.8 | 100.0 | 32.1 | 24.4 |
| 20.2 | 24.5 | 32.4 | 21.3 |
| 21.1 | 31.3 | 32.8 | 19.3 |
| 21.4 | 68.6 | 33.4 | 14.6 |
| 22.4 | 84.0 | 34.5 | 15.8 |
| 9.1 | 7.7 | | |
| 10.5 | 18.8 | | |
| 11.4 | 6.7 | | |

Table 2: Unique characteristic diffraction peaks of example 1 (± 0.1 ° 2Θ)

As will be appreciated by the skilled person, the relative intensities of the various peaks within Tables 1 and 2 may vary due to a number of factors such as for example orientation effects of crystals in the X-ray beam or the purity of the material being analysed or the degree of crystallinity of the sample. The peak positions may also shift for variations in sample height but the peak positions will remain substantially as defined in given Tables 1 and 2. The skilled person will also appreciate that measurements using a different wavelength will result in different shifts according to the Bragg equation- ηλ=2d sinB. Such alternative PXRD patterns generated by use of alternative wavelengths are nevertheless representations of the same material. Fourier Transform Infra-red (FT-IR) spectrum: When characterised by Fourier Transform Infra-red (FT-IR) spectroscopy, the compound of example 1 gives the pattern shown in Figure 3 of WO2013/021309. The fingerprint region is shown in expanded form in Figure 4 of WO2013/021309. The characteristic peaks are given in Table 3 below (w = weak, s = strong, m = medium). The main characteristic peaks are 1535 (s), 1 179 (s), 834 (s), and 769 (s) cm-1. Table 3: characteristic FT-IR peaks of example 1 (cm-1):

| **Wavenumber (cm⁻¹)** | | | |
|---|---|---|---|
| 3367m | 1643s | 1350m | 947w |
| 3332m | 1615m | 1316m | 929w |
| 3157m | 1603s | 1292m | 906w |
| 3053w | 1596s | 1268s | 886w |
| 3017w | **1535s** | 1240s | **834s** |
| 3002w | 1522s | 1207m | 817m |
| 2986w | 1481s | **1179s** | 805m |
| 2972m | 1466m | 1145m | 787s |
| 2945w | 1444m | 1109w | 781s |
| 2912w | 1431s | 1094m | **769s** |
| 2851w | 1422s | 1064w | 732s |
| 2703w | 1412m | 1025m | 704s |
| 2617w | 1391 m | 1000m | 688s |
| 2482w | 1367m | 972w | 663s |

The IR spectrum was acquired using a ThermoNicolet Nexus FTIR spectrometer equipped with a 'DurasampllR' single reflection ATR accessory (diamond surface on zinc selenide substrate) and d-TGS KBr detector. The spectrum was collected at 2 cm-1 resolution and a co-addition of 512 scans. Happ-Genzel apodization was used. Because the FT-IR spectrum was recorded using single reflection ATR, no sample preparation was required. Using ATR FT-IR will cause the relative intensities of infrared bands to differ from those seen in a transmission FT-IR spectrum using KBr disc or nujol mull sample preparations. Due to the nature of ATR FT-IR, the bands at lower wavenumber are more intense than those at higher wavenumber. Experimental error, unless otherwise noted, was ± 2 cm-1. Peaks were picked using ThermoNicolet Omnic 6.1 a software. Intensity assignments are relative to the major band in the spectrum so they are not based on absolute values measured from the baseline. Fourier Transform Raman spectrum: When characterised by Fourier Transform Raman spectroscopy, the compound of example 1 gives the pattern shown in Figure 5 of WO2013/021309. The fingerprint region is shown in greater detail in Figure 6 of WO2013/021309. The characteristic peaks are given in Table 4 below (w = weak, s = strong, m = medium, vs = very strong). The main characteristic peaks are 1294 (s), 1001 (vs), and 772 (w) cm-1. Table 4: characteristic Raman peaks of example Kcm-1):

| **Wavenumber (cm⁻¹)** | | | |
|---|---|---|---|
| 3370w | 2600w | 1090w | 440w |
| 3336w | 1644w | 1027m | 418w |
| 3168w | 1616s | **1001vs** | 373w |
| 3055m | 1606vs | 888w | 315w |
| 3035w | 1523w | 870w | 295w |
| 3019w | 1460w | 817m | 263m |
| 3003w | 1443w | 806w | 240w |
| 2988w | 1423w | 789w | 220m |
| | | | |
| 2974w | 1352w | **772w** | 182w |
| 2941m | 1308s | 736w | 108vs |
| 2925w | **1294s** | 705w | 92vs |
| 2914w | 1263m | 644w | 61s |
| 2859w | 1242w | 604w | |
| 2772w | 1209w | 540w | |
| 2721w | 1181m | 516w | |

The Raman spectra were collected using a Bruker Vertex70 FT-IR spectrometer with Ramll Raman module equipped with a 1064nm NdYAG laser and LN-Germanium detector. All spectra were recorded using 2cm-1 resolution and Blackman-Harris 4-term apodization. The laser power was 250mW and 1024 scans for PF-3653478 or 1300 scans for PF2434029-42 were co-added. Each sample was placed in a glass vial and exposed to the laser radiation. The data is presented as intensity as a function of Raman shift. Experimental error, unless otherwise noted, was ± 2 cm-1. Peaks were picked using ThermoNicolet Omnic 6.1 a software. Intensity assignments are relative to the major band in the spectrum so they are not based on absolute values measured from the baseline. Solid state NMR data for the material obtained in example 1: Approximately 15 mg of sample were tightly packed into a 2.5 mm Zr02 rotor. Spectra were collected at ambient temperature and pressure on a Bruker-Biospin 2.5 mm CPMAS probe positioned into a wide-bore Bruker-Biospin Avance 600 MHz (¹H frequency) NMR spectrometer. The packed rotor was oriented at the magic angle and spun at 15.0 kHz. The ¹³C solid state spectra were collected using a proton decoupled cross-polarization magic angle spinning (CPMAS) experiment. The cross-polarization contact time was set to 2.0 ms. A proton decoupling field of approximately 100 kHz was applied during acquisition. The carbon spectrum of the compound of example 1 was acquired for 16,384 scans with a 3.5 second recycle delay. Carbon spectra were referenced using an external standard of crystalline adamantane, setting its upfield resonance to 29.5 ppm. Automatic peak picking was performed using Bruker-BioSpin TopSpin version 3.0 software. The output of the automated peak picking was visually checked to ensure validity and adjustments manually made if necessary. The carbon chemical shifts observed for example 1 are as follows in Table 5:

| **¹³C Chemical Shifts [ppm ± 0.2 ppm]*^{(a)}*** | **Intensity*^{(b)}*** | **¹³C Chemical Shifts [ppm ± 0.2 ppm]*^{(a)}*** | **Intensity***^{(b)}* |
|---|---|---|---|
| 173.2 | 42.2 | 127.8 | 76.5 |
| 169.5 | 37.5 | 126.3 | 31.0 |
| 158.5 | 50.2 | 124.7 | 56.4 |
| 143.7 | 41.4 | 119.1 | 32.1 |
| 139.8 | 46.0 | 115.3 | 33.0 |
| 138.7 | 46.5 | 55.5 | 60.4 |
| 135.3 | 47.0 | 45.8 | 45.0 |
| 131.5 | 34.3 | 43.6 | 82.6 |
| 130.7 | 100.0 | 31.0 | 65.3 |
| 128.2 | 45.7 | 29.4 | 57.8 |

| | | | |
|---|---|---|---|
| ^{(a>} Referenced to external sample of solid phase adamantane at 29.5 ppm. ^{<b)} Defined as peak heights. Intensities can vary depending on the actual setup of the CPMAS experimental parameters and the thermal history of the sample. CPMAS intensities are not necessarily quantitative. The unique carbon chemical shifts observed for example 1 are as follows in Table 6: | | | |

| **¹³C Chemical Shifts [ppm ± 0.2 ppm]*^{(a)}*** | **Intensity*^{(b)}*** |
|---|---|
| 173.2 | 42.2 |
| 158.5 | 50.2 |
| 55.5 | 60.4 |

| | |
|---|---|
| ^{<a)} Referenced to external sample of solid phase adamantane at 29.5 ppm. ^{<b)} Defined as peak heights. | |

Intensities can vary depending on the actual setup of the CPMAS experimental parameters and the thermal history of the sample. CPMAS intensities are not necessarily quantitative. The corresponding carbon CPMAS spectrum for example 1 is illustrated in FIG. 7 of WO2013/021309.

### Example 2: preparation of the β2 agonist N-[(4'-Hydroxybiphenyl-3-yl)methyl]-2-(3-{2-[((2R)-2-hydroxy-2-(4-hydroxy-3-[(methylsulfonyl)amino]phenyl}ethyl)amino]-2-methylpropyl}phenyl)acetamide of formula (14).

### a) Preparation 3: A -(2-(Benzyloxy)-5-[(1R)-2-bromo-1-hydroxyethyl] phenyl) methane sulfonamide:

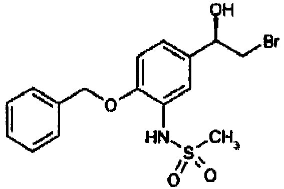

N-{2-(Benzyloxy)-5-[(1R)-2-bromo-1-hydroxyethyl] phenyl} methane sulfonamide is prepared according to the methods described in WO 2008/010356.

### b) Preparation 4: N-[2-(Benzyloxy)-5-((1R)-2-bromo-1-{[tert-butyl(dimethyl)-silyl]oxy} ethyl)phenyl]methanesulfonamide of formula (12):

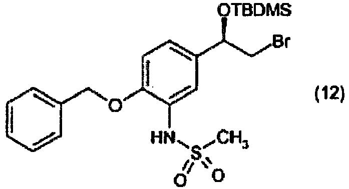

N-[2-(Benryloxy)-5-((1R)-2-bromo-1-{[tert-butyl(dimethyl)silyl]oxy} ethyl)phenyl] methane sulfonamide may be prepared the methods described in WO 2008/010356. Alternatively, the following conditions may be used: To a solution of N-{2-(benzyloxy)-5-[(1R)-2-bromo-1-hydroxyethyl] phenyl} methane sulfonamide according to Preparation 3 (1.0 kg, 2.50 mol) in dichloromethane (2.0 L), was added imidazole (0.255 kg, 3.74 mol) followed by te/t-butyldimethylsilyl chloride (0.527 kg, 3.50 mol). The resultant mixture was heated to reflux for 1-2 hours. The reaction was cooled to 20-25°C and was washed with demineralised water (3 x 5.0 L). The organic layer was dried (Na₂SO₄) then filtered into a reactor. The solvent was then exchanged into ethanol by distillation under vacuum at <30°C to give a slurry in approximately 2 L of ethanol. The solid was collected by filtration, washed with ethanol (2.0 L) and dried at 40-45°C in vacuo to give the title compound (1.0 to 1.15 kg typically obtained) as a colourless solid. If necessary, N-{2-(benzyloxy)-5-[(1R)-2-bromo-1-hydroxyethyl] phenyl} methane sulfonamide may be purified as follows:

A stirred suspension of N-{2-(benzyloxy)-5-[(1R)-2-bromo-1-hydroxyethyl] phenyl} methane sulfonamide (84.6 g) in ethanol (846 ml) under an atmosphere of nitrogen was heated at reflux until a clear solution was formed. The mixture was then slowly cooled to 25°C, and the suspension is stirred for 18 hours. The solid was collected by filtration, washed with ethanol (170 ml) and dried at 40°C in vacuo to give purified N-{2-(benzyloxy)-5-[(1R)-2-bromo-1-hydroxyethyl] phenyl} methane sulfonamide (72.5 g) as an off-white solid.

### c) Preparation 5: 2-[3-(2-Amino-2-methylpropyl)-phenyl]-N-[(4'-hydroxybiphenyl-3-yl)methyl]acetamide of formula (11)

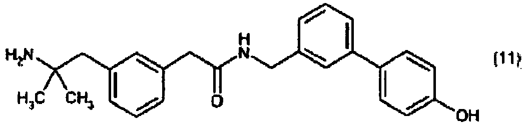

The compound of example 1 (55.0 kg, 118mol)) was added to a stirred solution of 2-butanol (248 L) and water (275 ml) at 20°C forming a slurry. Acetic acid (14.9 L) and thiourea (10.8 g, 142 mol) were added and the reaction was heated at reflux.

After 5 hours the solution was cooled to 40°C. The pH was adjusted to 10.8 ± 0.2 (at 25°C) using 2M NaOH and the layers were then separated. The organic layer was diluted with additional 2-butanol (165 L) before washing with water (2 x 248 L) and brine (248 L). The organic layer was distilled down to a volume of 143 L at atmospheric pressure. Acetonitrile (242 L) was then added and the solution was distilled down to a volume of 180 L. Acetonitrile (198 L) was then added. The solution was cooled to 70°C at which point seed crystals of the title compound were added (0.4 Kg, 1 % based on 100% yield of the title compound) and the resulting slurry was stirred at 70°C for 1 hour. The slurry was diluted with additional acetonitrile (275 L) before cooling to 20°C at 0.3°C/min where it was left to stir for 4 hours. The slurry was diluted by the addition of acetonitrile (121 L). The solid was collected by filtration, washed with acetonitrile (2 x 121 L) and dried in vacuo at 50 °C to give the title compound (39.8 kg, 86.7% yield) as a colourless solid.

### d) Preparation 6: (R)-2-(3-{2-[2-(4-Benzyloxy-3-methanesulfonamidophenyl)-2-hydroxy-ethylamino]-2-methylpropyl}phenyl)-N-[(4'-hydroxybiphenyl-3-yl)methyl-acetamide hemifumarate of formula (13b)

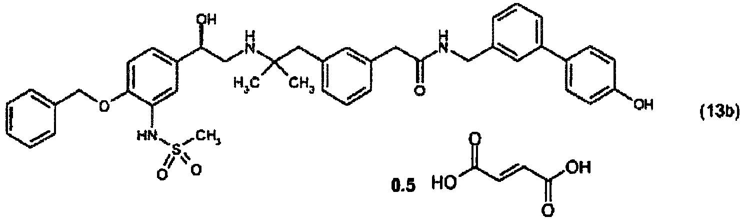

(2-[3-(2-Amino-2-methylpropyl)phenyl]-N-[(4'-hydroxybiphenyl-3-yl)methyl]acetamide according to preparation 5 (50.0 kg, 129 mol), ((R)-N-{2-benzyloxy-5-[2-bromo-1-(tert-butyldimethylsiloxy)ethyl]phenyl}methanesulfonamide according to preparation 4 (69.5 kg, 135 mol) and sodium bicarbonate (21.7 kg, 258 mol) were added to a reactor containing n-butyl acetate (500 L). The resulting slurry was heated to reflux temperature and n-butyl acetate (350 L) was removed from the mixture by distillation at atmospheric pressure. The concentrated reaction mixture was heated at reflux for 35 hours. The reaction was cooled to 20-25°C, diluted with ethyl acetate (500 L) and washed with water (500 L). The n-butyl acetate/ethyl acetate solution was diluted with methanol (150 L). Acetic acid (14.8 L, 258 mol) was added in one portion at 22°C followed by tetraethylammonium fluoride hydrate (28.8 kg, 142 mol). The reaction mixture was heated to 50°C and was maintained at this temperature for 5 hours. The reaction was cooled to 20°C, quenched by the addition of aqueous 1 M potassium carbonate solution (450 L) at 20°C and the resulting biphasic mixture was stirred for 15 min. The phases were separated and the organic phase was washed with water (200 L). To the organic phase was added absolute ethanol (200 L) and the solution was distilled under reduced pressure to a volume of approximately 400 L. The concentrated solution was diluted with absolute ethanol (350 L). The solution was concentrated under reduced pressure to a volume of approximately 250 L. The concentrate was diluted with absolute ethanol (450 L) and the mixture was heated to 65°C. Seed crystals of the title compound (0.5 kg, 1 % w/w) were added to the mixture and a solution of fumaric acid (7.5 kg, 65 mol) in absolute ethanol (250 L) was added whilst maintaining a temperature of 65°C. After 1 hour the resulting slurry was cooled to 20°C at 0.23°C/min and stirring at this temperature was continued for 3 hours. The slurry was filtered and the solid was washed with absolute ethanol (300 L). The wet filter-cake was suspended in ethyl acetate (600 L), and the slurry was heated to 50°C and agitated for 1 hour. The slurry was cooled to 20°C and the solid was collected by filtration, washed with ethyl acetate (50 L) and dried at 45°C under vacuum to obtain the title compound as a colourless solid (82.3 kg, 83.5% yield) which contained 0.79% w/w water by Karl-Fischer analysis (equivalent to 0.34 moles of water per mole of title compound). ¹H NMR: When analysed by conventional proton NMR (600MHz, d₆-DMSO), the compound of preparation 6 gave the following spectrum: δ 1.03 (s, 6H), 2.73 (s, 2H), 2.78 (dd, J 1 1.5 & 9.3 Hz, 1 H), 2.89 (m, 4H), 3.46 (m, 2H), 4.31 (d, J 6 Hz, 2H), 4.68 (dd, J 9.3 & 3.1 Hz, 1 H), 5.17 (s, 2H), 6.51 (s, 1 H), 6.84 (m, 2H), 7.04 (d, J 7.7 Hz, 1 H), 7.11 (m, 3H), 7.17 (d, J 7.6 Hz, 1 H), 7.21 (m, 2H), 7.32 (m, 3H), 7.39 (m, 6H), 7.54 (d, J 7.2 Hz, 2H) and 8.56 (t, J 6.0 Hz, 1 H). Elemental Analysis for the compound of preparation 6: Found: C, 66.85%; H, 6.23% and N, 5.42%. C^H^NsOsS. 0.34 H₂0 requires C, 66.90%; H, 6.22% and N, 5.44%. Differential Scanning Calorimetry: Differential Scanning Calorimetry was undertaken using a DSC Q1000 instrument to heat 1.974mg of the title compound from 25 to 250°C at 20°C per minute in a closed aluminium pan under nitrogen purge gas. The material obtained from preparation 6 shows a sharp endothermic melt with onset of 157°C ± 2°C and peak at 159°C ± 2°C. The DSC trace is shown in Figure 8 of WO2013/021309. Powder X-Rav Diffraction: The powder X-ray diffraction pattern of the compound of preparation 6 was determined using a Bruker-AXS Ltd. D4 EN DEAVOR powder X-ray diffractometer fitted with an automatic sample changer, a theta-theta goniometer geometry, automatic beam divergence slit and a PSD Vantec-1 detector. The sample was prepared for analysis by mounting onto a low background silicon wafer specimen mount with a 0.5mm cavity. The specimen was rotated whilst being irradiated with copper KCH X-rays (wavelength = 1.5406 Angstroms) with the X-ray tube operated at 35kV/40mA. The analysis was in continuous mode set for data acquisition at 0.2 second count per 0.018° step size over a two theta range of 2° to 55° at room temperature. The instrument calibration was verified using a corundum reference standard (NIST: SRM 1976 XRD flat plate intensity standard). The peaks obtained were aligned against a silicon reference standard. Peak search was carried out using the threshold and width parameters set to 1 and 0.3, respectively, within the Eva software released by Bruker-AXS. Characteristic diffraction angles and their intensities are given in Table 7 and Figure 9 of WO2013/021309 shows the experimentally measured pattern. The unique diffraction peaks of this material are given in Table 8. Table 7: Characteristic diffraction peaks of compound of preparation 6 (± 0.1 ° 2Θ)

| **Angle (°2θ)** | **Intensity %** | **Angle (°2θ)** | **Intensity %** |
|---|---|---|---|
| 5.8 | 5.1 | 22.6 | 75.6 |
| 6.7 | 5.9 | 23.1 | 86.7 |
| 9.9 | 23.1 | 23.9 | 50.0 |
| 11.5 | 37.2 | 24.4 | 31.5 |
| 13.3 | 19.6 | 25.4 | 32.0 |
| 15.0 | 34.5 | 26.0 | 69.0 |
| 16.1 | 24.2 | 26.7 | 31.7 |
| 16.4 | 21.3 | 27.8 | 34.3 |
| 17.5 | 94.1 | 28.6 | 27.2 |
| | | | |
| 18.1 | 39.6 | 29.4 | 45.5 |
| 19.3 | 100.0 | 30.3 | 25.9 |
| 20.3 | 87.7 | 30.8 | 26.9 |
| 21.1 | 76.3 | 31.0 | 28.7 |
| 21.7 | 76.3 | 31.2 | 26.0 |
| 21.9 | 75.5 | 31.8 | 21.7 |
| 22.3 | 97.2 | 32.3 | 26.9 |

| **Angle (°2θ)** | **Intensity %** |
|---|---|
| 5.8 | 5.1 |
| 6.7 | 5.9 |
| 9.9 | 23.1 |
| 11.5 | 37.2 |
| 17.5 | 94.1 |

Table 8: Unique characteristic diffraction peaks of the compound of preparation 6 (± 0.1 ° 2Θ)

Fourier Transform Infra-red (FT-IR) spectrum: When characterised by Fourier Transform Infra-red (FT-IR) spectroscopy, the compound of preparation 6 gives the pattern shown in Figure 10 of WO2013/021309. The fingerprint region is shown in expanded form in Figure 11 of WO2013/021309. The characteristic peaks are given in Table 9 below (w = weak, s = strong, m = medium). The main characteristic peaks are 1118 (s), 837 (m), and 766 (m). Table 9: characteristic FT-IR peaks of the compound of preparation 6 (cm-1):

| **Wavenumber (cm⁻¹)** | | | |
|---|---|---|---|
| 3029m | 1396m | 1005m | 785m |
| 1627m | 1363s | 977m | **766m** |
| 1608s | 1329s | 961m | 754m |
| 1547s | 1266s | 934w | 731m |
| 1517s | 1202m | 906w | 705s |
| 1508s | 1151s | 885w | 697s |
| 1481m | **1118s** | 859w | 669s |
| 1470m | 1083m | **837m** | |
| 1449m | 1045w | 818m | |
| 1417m | 1020w | 793m | |

The IR spectrum was acquired using a ThermoNicolet Nexus FT-IR spectrometer equipped with a 'DurasampllR' single reflection ATR accessory (diamond surface on zinc selenide substrate) and d-TGS KBr detector. The spectrum was collected at 2cm-1 resolution and a co-addition of 512 scans. Happ-Genzel apodization was used. Because the FT-IR spectrum was recorded using single reflection ATR, no sample preparation was required. Using ATR FT-IR will cause the relative intensities of infrared bands to differ from those seen in a transmission FT-IR spectrum using KBr disc or nujol mull sample preparations. Due to the nature of ATR FT-IR, the bands at lower wavenumber are more intense than those at higher wavenumber. Experimental error, unless otherwise noted, was ± 2 cm-1. Peaks were picked using ThermoNicolet Omnic 6.1 a software. Intensity assignments are relative to the major band in the spectrum so they are not based on absolute values measured from the baseline. Fourier Transform Raman spectrum: When characterised by Fourier Transform Raman spectroscopy, the compound of preparation 6 gives the pattern shown in Figure 12 of WO2013/021309. The fingerprint region is shown in greater detail in Figure 13 of WO2013/021309. The characteristic peaks are given in Table 10 below (w = weak, s = strong, m = medium, vs = very strong). The main characteristic peaks are 1298 (s), 1002 (vs), and 819 (m). Table 10: characteristic FT-Raman peaks of the compound of preparation 6:

| **Wavenumber (cm⁻¹)** | | | |
|---|---|---|---|
| 3064m | 1589m | 1094w | 621w |
| | | | |
| 3043m | 1519w | 1047w | 591w |
| 3019w | 1472w | 1028w | 523w |
| 3007w | 1445w | **1002vs** | 431w |
| 2969w | 1408m | 904w | 369w |
| 2951m | 1333w | 859w | 304w |
| 2928m | **1298s** | **819m** | 259w |
| 2887w | 1272m | 802w | 226m |
| 2718w | 1221w | 749w | 205m |
| 2594w | 1174w | 729w | 96vs |
| 1657m | 1157w | 699w | 78vs |
| 1610vs | 1120w | 644w | |

The Raman spectra were collected using a Bruker Vertex70 FT-IR spectrometer with Ramll Raman module equipped with a 1064nm NdYAG laser and LN-Germanium detector. All spectra were recorded using 2cm-1 resolution and Blackman-Harris 4-term apodization. The laser power was 250mW and 1024 scans for the compound of example 1 or 1300 scans for the title compound were co-added. Each sample was placed in a glass vial and exposed to the laser radiation. The data is presented as intensity as a function of Raman shift. Experimental error, unless otherwise noted, was ± 2 cm-1. Peaks were picked using ThermoNicolet Omnic 6.1 a software. Intensity assignments are relative to the major band in the spectrum so they are not based on absolute values measured from the baseline. Solid state NMR data for the compound of preparation 6: Approximately 15 mg of sample were tightly packed into a 2.5 mm Zr02 rotor. Spectra were collected at ambient temperature and pressure on a Bruker-Biospin 2.5 mm CPMAS probe positioned into a wide-bore Bruker-Biospin Avance 600 MHz (¹H frequency) NMR spectrometer. The packed rotor was oriented at the magic angle and spun at 15.0 kHz. The ¹³C solid state spectra were collected using a proton decoupled cross-polarization magic angle spinning (CPMAS) experiment. The cross-polarization contact time was set to 2.0 ms. A proton decoupling field of approximately 100 kHz was applied during acquisition. The carbon spectrum of the title compound was acquired for 16,384 scans with a 2.5 second recycle delay and is shown in Figure 14 of WO2013/021309. Carbon spectra were referenced using an external standard of crystalline adamantane, setting its upfield resonance to 29.5 ppm. Automatic peak picking was performed using Bruker-BioSpin TopSpin version 3.0 software. The output of the automated peak picking was visually checked to ensure validity and adjustments manually made if necessary. The carbon chemical shifts observed are as follows in Table 11 and the characteristic peaks are as follows in Table 12. Table 11: ¹³C chemical shifts for the compound of preparation 6 expressed in parts per million (± 0.2 ppm):

| **¹³C Chemical Shifts [ppm]*^{(a)}*** | **Relative Intensity*^{(b)}*** | **¹³C Chemical Shifts [ppm]*^{(a)}*** | **Relative Intensity*^{(b)}*** |
|---|---|---|---|
| 174.8 | 27.3 | 117.1 | 28.5 |
| 172.2 | 35.8 | 115.5 | 24.9 |
| 155.3 | 45.7 | 113.1 | 15.1 |
| 145.9 | 1.1 | 112.0 | 15.4 |
| 141.8 | 45.9 | 74.7 | 32.2 |
| 140.6 | 24.0 | 70.0 | 17.5 |
| 138.8 | 18.5 | 69.0 | 15.2 |
| 137.2 | 50.2 | 61.7 | 34.1 |
| 136.5 | 62.8 | 53.2 | 11.0 |
| 133.7 | 83.5 | 48.7 | 9.0 |
| 132.9 | 69.2 | 45.2 | 29.0 |
| 130.2 | 95.3 | 42.3 | 29.1 |
| 129.3 | 82.7 | 41.0 | 59.4 |
| 128.1 | 78.6 | 25.5 | 40.8 |
| 126.7 | 100.0 | 24.1 | 26.7 |
| 125.3 | 34.6 | | |

| | | | |
|---|---|---|---|
| Referenced to external sample of solid phase adamantane at 29.5 ppm. ^{<b)} Defined as peak heights. Intensities can vary depending on the actual setup of the CPMAS experimental parameters and the thermal history of the sample. CPMAS intensities are not necessarily quantitative. | | | |

Table 12: ¹³C characteristic chemical shifts for the compound of preparation 6 expressed in parts per million (± 0.2 ppm)

| **¹³C Chemical Shifts [ppm]*^{(a)}*** | **Relative Intensity*^{(b)}*** |
|---|---|
| 172.2 | 35.8 |
| 155.3 | 45.7 |
| 25.5 | 40.8 |

| | |
|---|---|
| ^{(a>} Referenced to external sample of solid phase adamantane at 29.5 ppm. ^{<b)} Defined as peak heights. Intensities can vary depending on the actual setup of the CPMAS experimental parameters and the thermal history of the sample. CPMAS intensities are not necessarily quantitative. e) Preparation of the B2 agonist N-[(4'-Hydroxybiphenyl-3-yl)methyl]-2-(3-{2-[((2R)-2-hydroxy-2-(4-hydroxy-3-[(methylsulfonyl)amino]phenyl}ethyl)amino]-2-methylpropyl}phenyl)acetamide of formula (14): | |

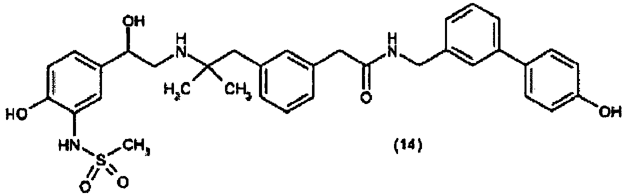

To a suspension of (R)-2-(3-{2-[2-(4-benzyloxy-3-methanesulfonamidophenyl)-2-hydroxyethylamino]-2-methylpropyl}-phenyl)-N-[(4'-hydroxybiphenyl-3-yl)methyl]-acetamide hemifumarate according to preparation 6 (10.0 kg, 13.0 mol) in tetrahydrofuran (89.6 kg) under an atmosphere of nitrogen was added as solution of potassium carbonate (4.1 kg, 30 mol) in water (30 L) and the mixture was stirred at 20°C for 35 minutes. The phases were separated, and the organic phase was washed with a solution of sodium chloride (5.0 kg, 85.6 mol) in water (30 L). The phases were separated and the organic phase was diluted with tetrahydrofuran (67.2 kg) and water (12 L). To the solution was added 5% palladium on carbon (1.0 kg) followed by a rinse of a mixture of tetrahydrofuran (16 kg) and water (2 L) and the mixture was hydrogenated under 50 psi pressure of hydrogen at 20-25°C for 13 hours. The catalyst was removed by filtration and solid washed with a mixture of tetrahydrofuran (77 kg) and water (10 L). To the combined filtrates was added Quadrapure TU® resin (2.5 kg) and the suspension was stirred at 20°C for 9.5 hours. The mixture was filtered, and the solid was washed with a mixture of tetrahydrofuran (20 kg) and water (4 L). The combined filtrates were concentrated by removal 233 L of solvent by distillation under vacuum. To the concentrated solution was added acetonitrile (39 kg) and the solution was concentrated by removing 55 L of solvent by distillation under vacuum. To the concentrated solution was added acetonitrile (40 kg) and the solution was concentrated by removing approximately 50 L of solvent by distillation at atmospheric pressure. The concentrated solution was diluted with acetonitrile (39 kg). Concentration of the mixture by removal of approximately 50 L of solvent by distillation at atmospheric pressure followed by dilution with approximately 39 kg of acetonitrile was repeated 6 times until the solvent composition was predominantly acetonitrile and the temperature of the mixture reached 81 °C. The resultant slurry was cooled to 20 to 25°C over 2.5 hours and stirred at this temperature for 5.5 hours. The solid was collected by filtration, washed with acetonitrile (2 x 39 kg) and dried at 50°C under vacuum to give a solid (6.80 kg). A suspension of this solid (6.80 kg) in a mixture of methanol (61 L) and water (7 L) under an atmosphere of nitrogen was heated at 50°C for 1 hour, then cooled to 20°C and stirred for 0.5 hours at this temperature. The solid was collected by filtration, washed with a mixture of methanol (31 L) and water (3 L), then with methanol (34 L) and dried at 50°C in vacuo to give the compound of formula (14) (6.40 kg) as a colourless solid. The title compound of formula (14) so prepared may be recrystallised using the following procedure. A stirred suspension of the compound of formula 14 (9.42 kg) in a mixture of tetrahydrofuran (81.4 kg) and water (20.3 L) under an atmosphere of nitrogen was heated to reflux to give a clear solution. The mixture was then distilled at a rate of approximately 27 L/hour atmospheric pressure for 13 hours using a fixed temperature difference between the reactor contents and the reactor's heated jacket (for the reactor used, this temperature difference was 30°C). Over the whole course of the distillation, acetonitrile (a total of approximately 350 L) was continuously added to the mixture at a rate of approximately 27 L/hour. The resultant slurry was cooled to 20°C, stirred at this temperature for 4 hours and the solid was collected by filtration. The filter cake was washed with acetonitrile (2 x 37 kg) and dried at 40°C under vacuum to give a solid (8.86 kg). A stirred suspension of this solid (8.86 kg) in a mixture of methanol (80 L) and water (9 L) under an atmosphere of nitrogen was heated at 50°C for 70 min. The mixture was cooled to 20°C and stirred for 140 min. The solid was collected by filtration, washed firstly with a mixture of methanol (41 L) and water (4 L), secondly with methanol (45 L) then dried at 50°C under vacuum to give a solid (8.38 kg) that was milled on a hammer mill to give the compound of formula (14) as a colourless solid (8.20 kg) which contained 0.48%w/w water by Karl-Fischer analysis (equivalent to 0.17 moles of water per mole of the compound of formula (14)). Characterisation data for the compound of formula (14) so prepared is provided below. ¹H NMR: When analysed by conventional proton NMR (600MHz, d6-DMSO), the compound of formula (14) gives the following spectrum: δ 0.89 (s, 3H), 0.91 (s, 3H), 2.54 (s, 2H), 2.59-2.68 (m, 2H), 2.89 (s, 3H), 3.44 (s, 2H), 4.31 (d, 2H), 4.42 (dd, 1 H), 6.80-8.83 (m, 3H), 6.97-7.02 (m, 2H), 7.08-7.12 (m, 3H), 7.16 (t, 1 H), 7.19 (d, 1 H), 7.30 (t, 1 H), 7.37-7.41 (m, 4H), and 8.50 (t, 1 H). ¹³C NMR: When analysed by conventional carbon NMR (151 MHz, d6-DMSO), the compound of formula (14) gives the following spectrum: δ 26.39, 26.64, 39.85, 42.22, 42.48, 46.36, 50.15, 52.71, 72.00, 1 15.14, 1 15.70, 1 15.70, 123.76, 124.02, 124.29, 124.38, 124.76, 125.23, 126.49, 127.57, 127.63, 128.40, 128.71, 130.82, 131.13, 135.38, 135.73, 138.43, 139.94, 140.20, 149.76, 157.1 1 and 170.28. Elemental Analysis for the compound of formula (14): Found: C, 65.70%; H, 6.33%, N, 6.71% and S, 5.24%. C34H39N3O6S.0.17 H₂O requires C, 65.78%; H, 6.39%, N, 6.77% and S, 5.16%. Powder X-Rav Diffraction: The powder X-ray diffraction pattern of the compound of formula (14) was determined using a Bruker-AXS Ltd. D4 ENDEAVOR powder X-ray diffracto meter fitted with an automatic sample changer, a theta-theta goniometer geometry, automatic beam divergence slit and a PSD Vantec-1 detector. The sample was prepared for analysis by mounting onto a low background silicon wafer specimen mount with a 0.5mm cavity. The specimen was rotated whilst being irradiated with copper Kcd X-rays (wavelength = 1.5406 Angstroms) with the X-ray tube operated at 35kV/40mA. The analysis was in continuous mode set for data acquisition at 0.2 second count per 0.018° step size over a two theta range of 2° to 55° at room temperature. The instrument calibration was verified using a corundum reference standard (NIST: SRM 1976 XRD flat plate intensity standard). The peaks obtained were aligned against a silicon reference standard. The peaks obtained were aligned against a silicon reference standard. Peak search was carried out using the threshold and width parameters set to 1 and 0.3, respectively, within the Eva software released by Bruket-AXS (2009). Characteristic diffraction angles and their intensities are given in Table 13 and Figure 15 of WO2013/021309 shows the experimentally measured pattern for the compound of formula (14). The unique diffraction peaks of this material are given in Table 14. Table 13: Characteristic diffraction peaks of the compound of formula (14) (± 0.1 ° 2Θ):

| **Angle (°2θ)** | **Intensity %** | **Angle (°2θ)** | **Intensity %** |
|---|---|---|---|
| 4.2 | 8.3 | 23.5 | 19.2 |
| 8.4 | 7.0 | 23.9 | 18.7 |
| 10.3 | 3.1 | 24.7 | 8.3 |
| 10.9 | 7.6 | 25.2 | 10.5 |
| 11.3 | 6.0 | 25.8 | 17.9 |
| 12.9 | 14.9 | 26.4 | 7.6 |
| 13.6 | 3.7 | 26.6 | 7.9 |
| 14.4 | 2.5 | 27.1 | 6.1 |
| 15.0 | 19.5 | 27.4 | 6.2 |
| 15.6 | 28.0 | 28.6 | 6.5 |
| 16.7 | 20.0 | 29.4 | 14.4 |
| | | | |
| 17.2 | 27.6 | 30.5 | 7.3 |
| 18.0 | 25.2 | 31.2 | 7.7 |
| 19.1 | 4.9 | 31.8 | 7.0 |
| 19.6 | 14.3 | 32.2 | 7.5 |
| 20.2 | 8.0 | 32.8 | 6.1 |
| 20.8 | 24.2 | 33.9 | 8.0 |
| 21.3 | 9.7 | 34.7 | 6.3 |
| 21.8 | 14.6 | 36.0 | 7.2 |
| 22.8 | 100.0 | 36.7 | 6.0 |

| **Angle (°2θ)** | **Intensity %** | | |
|---|---|---|---|
| 4.2 | 8.3 | | |
| 8.4 | 7.0 | | |
| 22.8 | 100.0 | | |

Table 14: Unique characteristic diffraction peaks of the compound of formula (14) (± 0.1 °2Θ):

As will be appreciated by the skilled person, the relative intensities of the various peaks within Tables 13 and 14 may vary due to a number of factors such as for example orientation effects of crystals in the X-ray beam or the purity of the material being analysed or the degree of crystallinity of the sample. The peak positions may also shift for variations in sample height but the peak positions will remain substantially as defined in given Tables 13 and 14. The skilled person will also appreciate that measurements using a different wavelength will result in different shifts according to the Bragg equation - ηλ = 2d sinB.

Such alternative PXRD patterns generated by use of alternative wavelengths are nevertheless representations of the same material. Fourier Transform Infra-red (FT-IR) spectrum: When characterised by Fourier Transform Infra-red (FT-IR) spectroscopy, the compound of formula (14) gives the pattern shown in Figure 16 of WO2013/021309. The fingerprint region is shown in expanded form in Figure 17 of WO2013/021309. The characteristic peaks are given in Table 15 below (w = weak, s = strong, m = medium). The main characteristic peaks are 1498 (m), 1284 (s), 913 (s) and 801 (s). Table 15: characteristic FT-IR peaks of the compound of formula (14) (cm-1):

| **Wavenumber (cm⁻¹)** | | | |
|---|---|---|---|
| 3443w* | 1517s | 1234m | **913s** |
| 3357w* | **1498m** | 1196s | 889m |
| 3261w* | 1483m | 1173s | 875w |
| 3083w | 1451m | 1145m | 839s |
| 3033w | 1416m | 1124s | **801s** |
| 3002w | 1395w | 1114s | 781s |
| 2971w | 1380w | 1087s | 768m |
| 2930w | 1356w | 1055m | 743m |
| 2895w | 1315w | 1015m | 728s |
| 1661m | **1284s** | 992w | 704s |
| 1608w | 1271m | 966w | 696m |
| 1592m | 1262s | 949w | |

The IR spectrum was acquired using a ThermoNicolet Nexus FT-IR spectrometer equipped with a 'DurasampllR' single reflection ATR accessory (diamond surface on zinc selenide substrate) and d-TGS KBr detector. The spectrum was collected at 2cm-1 resolution and a co-addition of 512 scans. Happ-Genzel apodization was used. Because the FT-IR spectrum was recorded using single reflection ATR, no sample preparation was required. Using ATR FT-IR will cause the relative intensities of infrared bands to differ from those seen in a transmission FT-IR spectrum using KBr disc or nujol mull sample preparations. Due to the nature of ATR FT-IR, the bands at lower wavenumber are more intense than those at higher wavenumber. Experimental error, unless otherwise noted, was ± 2 cm-1 (* error may be larger). Peaks were picked using ThermoNicolet Omnic 6.1 a software. Intensity assignments are relative to the major band in the spectrum so they are not based on absolute values measured from the baseline. Fourier Transform Raman spectrum: When characterised by Fourier Transform Raman spectroscopy, the compound of formula (14) gives the pattern shown in Figure 18 of WO2013/021309.

The fingerprint region is shown in greater detail in Figure 19 of WO2013/021309. The characteristic peaks are given in Table 16 below (w = weak, s = strong, m = medium, vs = very strong). The main characteristic peaks are 1297 (vs), 1000 (vs), 819 (m) and 731 (m). Table 16: characteristic FT-Raman peaks of the compound of formula (14):

| **Wavenumber (cm⁻¹)** | | | |
|---|---|---|---|
| 3065s | 1346w | 803w | 347m |
| 3049m | **1297vs** | 784w | 314w |
| 2996m | 1271m | 747m | 275w |
| 2961m | 1242w | **731m** | 259m |
| 2921s | 1187m | 706w | 225m |
| 2848w | 1178m | 644w | 201s |
| 2819w | 1089w | 607w | 121vs |
| 1608vs | 1017m | 565w | 71vs |
| 1594vs | **1000vs** | 526w | 60vs |
| 1521w | 969w | 463m | |
| 1450w | 910w | 415m | |
| 1430w | **819m** | 369w | |

The Raman spectra were collected using a Bruker Vertex70 FT-IR spectrometer with Ramll Raman module equipped with a 1064nm NdYAG laser and LN-Germanium detector. All spectra were recorded using 2cm-1 resolution and Blackman-Harris 4-term apodization. The laser power was 250mW and 1024 scans for the compound of example 1 or 1300 scans for the title compound were co-added. Each sample was placed in a glass vial and exposed to the laser radiation. The data is presented as intensity as a function of Raman shift. Experimental error, unless otherwise noted, was ± 2 cm-1. Peaks were picked using ThermoNicolet Omnic 6.1 a software. Intensity assignments are relative to the major band in the spectrum so they are not based on absolute values measured from the baseline. Solid state NMR data for the compound of formula (14):

Approximately 80 mg of sample were tightly packed into a 4.0 mm Zr02 rotor. Spectra were collected at ambient temperature and pressure on a Bruker-Biospin 4.0 mm CPMAS probe positioned into a wide-bore Bruker-Biospin Avance III 500 MHz (¹H frequency) NMR spectrometer. The packed rotor was oriented at the magic angle and spun at 15.0 kHz. The ¹³C solid state spectra were collected using a proton decoupled cross-polarization magic angle spinning (CPMAS) experiment. The cross-polarization contact time was set to 2.0 ms. A proton decoupling field of approximately 100 kHz was applied during acquisition. The carbon spectrum of the compound of formula (14) was acquired for 8,192 scans with a 7.0 second recycle delay and is shown in Figure 20 of WO2013/021309. Carbon spectra were referenced using an external standard of crystalline adamantane, setting its upfield resonance to 29.5 ppm. Automatic peak picking was performed using Bruker-BioSpin TopSpin version 3.0 software. The output of the automated peak picking was visualy checked to ensure validity and adjustments manually made if necessary. The carbon chemical shifts observed are as follows in Table 17 and the characteristic peaks of the compound of formula (14) are as follows in Table 18. Table 17: ¹³C chemical shifts for the compound of formula (14) expressed in parts per million (± 0.2 ppm):

| **¹³C Chemical Shifts [ppm]*^{(a)}*** | **Relative Intensity*^{(b)}*** | **¹³C Chemical Shifts [ppm]*^{(a)}*** | **Relative Intensity*^{(b)}*** |
|---|---|---|---|
| 172.6 | 36 | 126.4 | 45 |
| 158.5 | 49 | 124.6 | 40 |
| 151.1 | 53 | 122.7 | 41 |
| 142.6 | 35 | 120.0 | 43 |
| 139.3 | 48 | 119.2 | 48 |
| 136.1 | 74 | 117.6 | 53 |
| 133.8 | 40 | 116.3 | 46 |
| 132.9 | 60 | 113.2 | 39 |
| 132.0 | 78 | 71.2 | 67 |
| 131.7 | 67 | 59.9 | 66 |
| 131.2 | 61 | 50.2 | 53 |
| 130.5 | 72 | 45.2 | 46 |
| 130.0 | 60 | 43.2 | 100 |
| 129.4 | 71 | 37.9 | 67 |
| 129.1 | 85 | 23.6 | 72 |
| 128.5 | 62 | 21.1 | 62 |

| | | | |
|---|---|---|---|
| ^{(a>} Referenced to external sample of solid phase adamantane at 29.5 ppm. ^{<b)} Defined as peak heights. Intensities can vary depending on the actual setup of the CPMAS experimental parameters and the thermal history of the sample. CPMAS intensities are not necessarily quantitative. | | | |

Table 18: ¹³C characteristic chemical shifts of the compound of formula (14) expressed in parts per million (± 0.2 ppm):

| **¹³C Chemical Shifts [ppm]*^{(a)}*** | **Relative Intensity*^{(b)}*** |
|---|---|
| 43.2 | 100 |
| 136.1 | 74 |
| 158.5 | 49 |

| | |
|---|---|
| ^{(a>} Referenced to external sample of solid phase adamantane at 29.5 ppm. ^{<b)} Defined as peak heights. Intensities can vary depending on the actual setup of the CPMAS experimental parameters and the thermal history of the sample. CPMAS intensities are not necessarily quantitative. | |

Example 3: Absence of residual reagents in the compound of example 1: The data provided below show that the use of a substantially crystalline form of 2-chloro-N-{2-[3-(2-{[(4'-hydroxybiphenyl-3-yl)methyl]amino}-2-oxoethyl)phenyl]-1,1-dimethylethyl}acetamide according to example 1 allows purging problematic residual reagents which were previously difficult to purge as compared with the same intermediate previously used in solution, i.e. in a non-crystalline non-isolated form. Figure 21 of WO2013/021309 represents the ¹H NMR of chloroacetonitrile (performed in DMSO with water present). Figure 22 of WO2013/021309 represents the ¹H NMR of solution of 2-chloro-N-{2-[3-(2-{[(4'-hydroxybiphenyl-3-yl)methyl]amino}-2-oxoethyl)phenyl]-1,1-dimethylethyl}acetamide as obtained in WO 2007/010356. As can be seen from this pattern, chloroacetonitrile and also residual ethyl acetate (see peaks at δ 1.21 (t, 3H), 2.02 (s, 3H) and 4.08 (q, 2H)) are present in the solution. On the contrary no chloroacetonitrile is detected in the ¹H NMR of isolated crystalline 2-chloro-N-{2-[3-(2-{[(4'-hydroxybiphenyl-3-yl)methyl]amino}-2-oxoethyl)phenyl]-1,1-dimethylethyl}-acetamide according to exa m ple 1 which is represented in Figure 23 of WO2013/021309. The absence of residual chloroacetonitrile is particularly advantageous since it would otherwise react with the thiourea used in the next step of the route to the β2 agonist *N*-[(4'-Hydroxybiphenyl-3-yl)methyl]-2-(3-{2-[((2R)-2-hydroxy-2-{4-hydroxy-3-[(methylsulfonyl)-amino]phenyl}ethyl)amino]-2-methylpropyl} phenyl)acetamide to give 1,3-thiazole-2,4-diamine, which is problematic to remove. Because of chloroacetonitrile consuming part of the thiourea, it was also previously necessary to use large amounts of thiourea (about 4 to 5 equivalents) which is toxic and potentially carcinogenic. The absence of residual chloroacetonitrile now allows using a lower amount of thiourea (about 1.2 equivalents).

Example 4: Absence of impurities in the compound of preparation 5: Low level impurities structurally related to the starting materials can be present in the starting materials used in the first steps of the process for preparing the β2 agonist N-[(4'-hydroxybiphenyl-3-yl)methyl]-2-(3-{2-[((2R)-2-hydroxy-2-{4-hydroxy-3-[(methylsulfonyl)amino]phenyl}ethyl)amino]-2-methyl propyl} phenyl)acetamide. Also, additional impurities can be generated during the reactions involved in these first steps. These impurities can be transformed into new impurities in the downstream synthesis, and some can be difficult to purge. The use of the compound according to the present invention, i.e. in isolated crystalline form, offers a solution to these issues as demonstrated by the data provided below. To quantify the impurity purge provided by the crystalline compound of example 1, a direct comparison of the process where by 2-chloro-N-{2-[3-(2-{[(4'-hydroxybiphenyl-3-yl)methyl]amino}-2-oxoethyl)phenyl]-1,1-dimethylethyl}-acetamide is not isolated as a solid but is carried through as a solution and the process involving the isolation was performed. The compound of preparation 5 was synthesised using the same starting material batch responsible for the introduction of impurities which fate to the final β2 agonist. First, the intermediate of preparation 5 was prepared in a pilot plant trial with 2-chloro-N-{2-[3-(2-{[(4'-hydroxybiphenyl-3-yl)methyl]amino}-2-oxoethyl)phenyl]-1,1-dimethylethyl}acetamide not isolated as a solid form. To that effect, in the process followed to prepare the compound of example 1, the trifluoroacetic acid was removed by distillation and replaced by 2-butanol, however, rather than crystallising the compound according to the present invention, the deprotection reagents used in Preparation 5 were added to the solution to synthesise the compound of Preparation 5. Additionally, in a laboratory trial, the compound according to the present invention was crystallised as the form identified in example 1 following the standard protocol as described in example 1 and this isolated material was then converted to the compound of preparation 5 as previously described in example 2. Both processes involved the crystallisation of the compound of preparation 5 from the same solvent system of acetonitrile and 2-butanol. The purity data shown in Table 19 below is that of the compound of preparation 5 and illustrates the impact of the crystallisation of 2-chloro-N-{2-[3-(2-{[(4'-hydroxybiphenyl-3-yl)methyl]amino}-2-oxoethyl)phenyl]-1,1-dimethylethyl}-acetamide according to the present invention on the level of impurity in the downstream synthesis of the next intermediate. Table 19: Comparison of the HPLC purity of the intermediate of Preparation 5 where 2-Chloro-N-{2-[3-(2-{[(4'-hydroxybiphenyl-3-yl)methyl]amino)-2-oxoethyl)phenyl]-1,1-dimethylethyl}acetamide has not been isolated as a solid form (pilot plant trial) and where it has been isolated as a solid form (lab trial).

| **Impurity reference** | **Using 2-Chloro-*N*-{2-[3-(2-{[(4'-hydroxybiphenyl-3-yl)methyl]amino)-2-oxoethyl)phenyl]-1,1-dimethylethyl}acetamide not isolated as a solid form Impurity level (%)** | **Using 2-Chloro-*N*-{2-[3-(2-{[(4'-hydroxybiphenyl-3-yl)methyl]amino}-2-oxoethyl)phenyl]-1,1-dimethylethyl}acetamide isolated as a solid form Impurity level (%)** |
|---|---|---|
| RRT*⁽¹⁾* 0.60 | 0.08 | ND*⁽²⁾* |
| RRT 0.75 | 0.18 | ND |
| RRT 1.13 | 0.26 | NMT*⁽³⁾* 0.05 |
| RRT 1.45 | 0.17 | ND |
| RRT 1.49 | 0.09 | ND |
| RRT 1.52 | 0.07 | ND |
| RRT 1.56 | NMT 0.05 | ND |
| RRT 1.58 | 0.08 | ND |
| RRT 1.59 | 0.36 | ND |
| RRT 1.62 | NMT 0.05 | ND |
| RRT 1.70 | 0.15 | ND |
| RRT 1.75 | NMT 0.05 | ND |
| RRT 1.76 | 0.13 | 0.08 |
| **Total** | **1.57** | **0.08** |

| | | |
|---|---|---|
| RRT = Relative Retention Time (by HPLC) ^{(2>} ND = Not Detected ^{<3)} NMT = Not More Than | | |

The above data clearly demonstrates that the compound of preparation 5 is essentially free of most of the impurities previously generated. The isolation of the compound according to the present invention thus provides a significant purge point for a number of impurities so that the next downstream isolated intermediate according to preparation 5 in the route to the final β2 agonist is of vastly improved purity. Moreover, these two samples of the compound of preparation 5 were separately converted to the β2 agonist of formula (14) using the protocols described in example 2 and the purity of the two samples were compared by HPLC. Specifically, the levels of the impurities in the β2 agonist of formula (14) that were derived from impurities that were present in the solutions of 2-chloro-N-{2-[3-(2-{[(4'-hydroxybiphenyl-3-yl)methyl]amino}-2-oxoethyl)phenyl]-1,1-dimethylethyl}acetamide prior to isolation were measured. The results are shown in Table 20, and they demonstrate that the sample prepared with isolation of a solid form of 2-chloro-N-{2-[3-(2-{[(4'-hydroxybiphenyl-3-yl)methyl]amino}-2-oxoethyl)phenyl]-1,1-dimethylethyl}acetamide has superior purity compared to the sample that was derived from 2-chloro-N-{2-[3-(2-{[(4'-hydroxybiphenyl-3-yl)methyl]amino}-2-oxoethyl)phenyl]-1,1-dimethylethyl}-acetamide that was not isolated as a solid form. It will be appreciated by those skilled in the art that prevention of impurities such as these from being present in pharmaceutical substances is critically important to ensure regulatory acceptability and patient safety. For pharmaceutical substances that can be formulated as dry powders for inhalation, such as the β2 agonist of formula (14), it is additionally important to limit the presence of impurities such as those in Table 20 from the stage that the pharmaceutical substance is crystallised since such impurities have the potential to adversely affect the critical physical properties of the pharmaceutical substance by changing the growth and agglomeration of the pharmaceutical substance crystals during the crystallisation process. Hence such impurities, if not limited prior to the stage that the pharmaceutical substance is crystallised, have the potential to ultimately impact the performance and safety of the drug product.
Table 20: Comparison of the HPLC purities of the compound of formula (14) where 2-Chloro-N-{2-[3-(2-{[(4'-hydroxybiphenyl-3-yl)methyl]amino)-2-oxoethyl)phenyl]-1,1-dimethylethyl}acetamide has not been isolated as a solid form (pilot plant trial) and where it has been isolated as a solid form (lab trial):

| **Impurity reference** | **Using 2-Chloro-*N*-{2-[3-(2-{[(4'-hydroxybiphenyl-3-yl}methyl]amino}-2-oxoethyl)phenyl]-1,1-dimethylethyl}acetamide not isolated as a solid form Impurity level (%)** | **Using 2-Chloro-*N*-{2-[3-(2-{[(4'-hydroxybiphenyl-3-yl)methyl]amino}-2-oxoethyl)phenyl]-1,1-dimethylethyl}acetamide isolated as a solid form Impurity level (%)** |
|---|---|---|
| RRT*⁽¹⁾* 1.08 | 0.19 | NMT*⁽²⁾* 0.05 |
| RRT 1.71 | 0.08 | NMT 0.05 |
| RRT 1.81 | 0.08 | 0.06 |
| RRT 2.45 | 0.11 | 0.06 |

| | | |
|---|---|---|
| RRT = Relative Retention Time (by HPLC) ^{<2)} NMT = Not More Than | | |

Thus, aspect (iii) of the invention relates to an administration unit comprising crystalline form of 2-chloro-N-{2-[3-(2-{[(4'-hydroxybiphenyl-3-yl)methyl]amino}-2-oxoethyl)phenyl]-1,1-dimethylethyl}-acetamide.

### SPECIFIC INORMATION CONCERNING ASPECT (IV) OF THE INVENTION

Aspect (iv) of the invention concerns forms of {drug4}, especially a crystalline and/or hydrated form of compound A, which is useful for treating HCV in a patient. Crystalline and/or hydrated form of compound A is described in WO2013/028465, which is incorporated by reference. Compound A has the following structure:

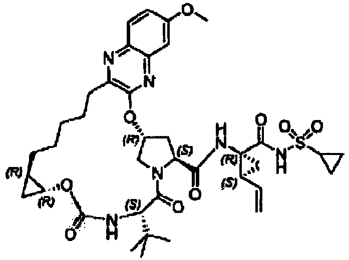

Preferred embodiments of aspect (iv) of the invention for crystalline and/or hydrated form of compound A are defined in the claims of WO2013/028465 and are reiteratred as items 1 to 6 hereinafter:
[Item 1]: Compound A having a form selected from the group consisting of:
   a) Hydrate III of compound A {drug4b}, wherein Hydrate III is preferably characterized by either (i) an X-ray powder diffraction pattern obtained using copper Kₐ radiation which comprises 2Θ values in degrees of about 20.5, 5.0, and 18.2; or (ii) a solid state carbon-13 CPMAS NMR comprising peaks at about 5.14, 6.31, 12.49, 18.35, 26.81, 28.03, 30.33, 31.27, 34.95, 35.99, 38.68, 42.01, 54.93, 56.39, 60.14, 74.20, 107.02, 120.11, 121.60, 129.73, 134.35, 135.95, 142.89, 148.47, 155.37, 157.32, 160.90, 168.32, 172.17, and 175.53 ppm;
   b) Hydrate II of compound A {drug4c}, wherein Hydrate II is preferably characterized by an X-ray powder diffraction pattern obtained using copper Kₐ radiation which comprises 2Θ values in degrees of about 11.7, 16.6, and 11.2;
   c) a crystalline Na salt of compound A {drug4d} which is preferably characterized by an X-ray powder diffraction pattern obtained using copper Kₐ radiation which comprises 2Θ values in degrees of about 18.4, 9.1, and 9.8;
   d) a crystalline K salt of compound A {drug4e} which is preferably characterized by an X-ray powder diffraction pattern obtained using copper Kₐ radiation which comprises 2Θ values in degrees of about 18.2, 8.9, and 20.3;
   e) Hydrate I of compound A {drug4f}, wherein Hydrate I is preferably characterized by either (i) an X-ray powder diffraction pattern obtained using copper Kₐ radiation which comprises 2Θ values in degrees of about 8.6, 20.6, and 26.6; or (ii) a solid state carbon-13 CPMAS NMR comprising peaks at about 4.22, 7.23, 11.45, 17.79, 24.04, 26.95, 28.29, 31.15, 32.47, 32.47, 33.46, 34.03, 35.74, 42.32, 53.50, 56.05, 56.96, 77.49, 108.95, 1 19.65, 122.55, 131.05, 133.13, 135.38, 142.28, 150.78, 156.03, 157.99, 161.36, 171.40, 173.42, 174.30 ppm;
   f) Hydrate IV of compound A {drug4g}, wherein Hydrate IV is preferably characterized by either (i) an X-ray powder diffraction pattern obtained using copper Kₐ radiation which comprises 20 values in degrees of about 14.7, 11.5, and 7.1; or (ii) a solid state carbon-13 CPMAS NMR comprising peaks at about 3.90, 5.30, 6.99, 10.49, 13.13, 17.81, 24.73, 27.52, 28.14, 29.42, 31.02, 32.80, 36.08, 39.22, 42.45, 53.62, 55.93, 59.14, 60.76, 74.77, 109.22, 111.19, 11.38, 120.24, 122.50, 133.96, 139.74, 147.2, 148.90, 154.65, 158.25, 159.53, 160.12, 170.14, 171.05, 172.08, 173.47, and 174.46 ppm;
   g) Hydrate V of compound A {drug4h}, wherein Hydrate V is preferably characterized by either (i) an X-ray powder diffraction pattern obtained using copper Kₐ radiation which comprises 2Θ values in degrees of about 9.1, 18.3, and 19.8; or (ii) a solid state carbon-13 CPMAS NMR comprising peaks at about 7.86, 8.92, 13.10, 18.31, 23.72, 27.44, 28.47, 30.77, 35.79, 36.25, 37.15, 37.15, 42.95, 53.13, 55.67, 57.31, 60.47, 62.06, 75.09, 110.59, 1 12.24, 118.32, 132.18, 134.05, 135.83, 139.88, 148.30, 155.19, 157.97, 159.41, 170.31 and 175.20; and
   h) Hydrate VI of compound A {drug4i}, wherein Hydrate VI is preferably characterized by either (i) an X-ray powder diffraction pattern obtained using copper Kₐ radiation which comprises 20 values in degrees of about 20.5, 12.8, and 19.4; or (ii) a solid state carbon-13 CPMAS NMR comprising peaks at about 4.87, 6.24, 11.70, 12.85, 18.36, 26.55, 28.31m 31.51, 34.98, 38.47, 42.09, 54.27, 56.12, 60.10, 73.49, 73.97, 105.91, 108.04, 118.39, 1 19.75, 121.33, 129.96, 133.87, 136.13, 142.26, 142.97, 146.85, 148.36, 154.97, 157.32, 160.71, 168.23, 172.21 and 175.34 ppm.
[Item 2]: A pharmaceutical composition comprising a therapeutically effect amount of a compound of item 1 and a pharmaceutically acceptable carrier. [Item 3]: A method of treating HC V in a patient comprising the step of administering to the patient a therapeutically effective amount of a compound of item 1. [Item 4]: The preparation of a medicament of use in treating HCV in a patient comprising an effective amount of a compound of item 1. [Item 5]: The compound of item 1 for use in the treatment of HCV. [Item 6]: A method of making a compound of item 1, where said Compound A is Hydrate III, made by a process comprising crystallising Compound A using an acetone/water ratio of 80:20 v/v acetone to water to 0: 100 v/v acetone to water and drying.

The crystalline and/or hydrated forms of compound A according to aspect (iv) of the invention can be characterized by / obtained by methods as described in WO2013/028465 and reiterated hereinafter:

Macrolactam compounds able to inhibit HCV activity have different uses including inhibiting HCV activity in vivo, inhibiting HCV activity in vitro, and inhibiting HCV NS3 enzymatic activity. In vivo inhibition of HCV activity can be used for therapeutic applications. Inhibiting HCV activity in vitro has different applications including being used to obtain HCV resistant mutants, further characterizing the ability of a functional group to inhibit HCV replicon or enzymatic activity, and studying HCV replication or protease activity. Compound A is described in Harper et al., WO201001 1566. Harper et al, WO201001 1566 includes data illustrating the ability of Compound A to inhibit HCV replicon activity and NS3/4A. Compound A Forms: Six different Compound A hydrates were identified (Forms I, II, III, IV, and V). Hydrate III was the most stable hydrate form identified.

In a first embodiment directed to the Hydrate II, the hydrate is characterized by an X-ray powder diffraction pattern obtained using copper Kₐ radiation {i.e., the radiation source is a combination of Cu Kₐ1 and Kₐ2 radiation), which comprises three or more characteristic peaks. Characteristic peeks are illustrated in Figure 1 of WO2013/028465. In a second embodiment, the hydrate is characterized by an X-ray powder diffraction pattern obtained using copper Kₐ radiation which comprises 2Θ values in degrees of about 2Θ values (i.e., reflections at 2Θ values) in degrees of about 11.7, 16.6, and 11.2. Reference to "about" with respect to 2Θ values provided herein indicates + 0.1. In this embodiment and analogous embodiments which follow the term "about" is understood to modify each of the 2Θ values; e.g., the expression "about 11.7, 16.6, and 1 1.2" is short-hand for "about 11.7, about 16.6, and about 11.2". In a third embodiment directed to the Hydrate II, the hydrate is characterized by an X-ray powder diffraction pattern obtained using copper Kₐ radiation which comprises 2Θ values in degrees of about 1 1.7, 16.6, 11.2, 15.1, and 16.1. In a fourth embodiment directed to the Hydrate II, the hydrate is characterized by an X-ray powder diffraction pattern obtained using copper Kₐ radiation which comprises 2Θ values in degrees of about 11.7, 16.6, 11.2, 15.1, 16.1, 23.0, 20.9, 8.0, 23.9, 25.0, 16.8, 17.8, 19.8, 22.5, and 8.3. In another embodiment, Hydrate II is substantially pure. Reference to "substantially pure" means the particular form makes up at least 50% of the compound present. In different embodiments, Hydrate II makes up at least 75%, at least 85%, at least 90%, at least 95%, or about 94%-98% of Compound A present.

In a first embodiment directed to Hydrate III, the hydrate is characterized by an X-ray powder diffraction pattern obtained using copper Kₐ radiation which comprises three or more characteristic peaks. Characteristic peeks are illustrated in Figure 2 of WO2013/028465. In a second embodiment directed to Hydrate III, the hydrate is characterized by an X-ray powder diffraction pattern obtained using copper Kₐ radiation which comprises 2Θ values in degrees of about 20.5, 5.0, and 18.2. In a third embodiment directed to Hydrate III, the hydrate is characterized by an X-ray powder diffraction pattern obtained using copper Kₐ radiation which comprises 2Θ values in degrees of about 20.5, 5.0, 18.2, 20.1, and 20.7. In a fourth embodiment directed to Hydrate III, the hydrate is characterized by an X-ray powder diffraction pattern obtained using copper Kₐ radiation which comprises 2Θ values in degrees of about 20.5, 5.0, 18.2, 20.1, 20.7, 14.1, 23.7, 13.1, 18.9, 15.3, 20.9, 11.4, 18.4, 14.7, and 13.8. In a fifth embodiment directed to Hydrate III, the hydrate is characterized by a thermogravimetric analysis as provided in Figure 3 of WO2013/028465. In a sixth embodiment directed to Hydrate III, the hydrate is characterized by a differential scanning calorimetry curve as provided in Figure 4 of WO2013/028465. A seventh embodiment is directed to Hydrate III, where the hydrate is characterized by the solid state carbon- 13 CPMAS NMR spectrum provided in Figure 5 of WO2013/028465. A eighth embodiment is directed to Hydrate III, where the hydrate is characterized by a solid state carbon-13 CPMAS NMR comprising peaks at about 5.14, 6.31, 12.49, 18.35, 26.81, 28.03, 30.33, 31.27, 34.95, 35.99, 38.68, 42.01, 54.93, 56.39, 60.14, 74.20, 107.02, 120.1 1, 121.60, 129.73, 134.35, 135.95, 142.89, 148.47, 155.37, 157.32, 160.90, 168.32, 172.17, and 175.53 ppm. Reference to "about" with respect to the solid state carbon- 13 CPMAS NMR 2Θ values provided herein indicates + 0.1. In another embodiment, Hydrate III is substantially pure. Reference to "substantially pure" means the particular form makes up at least 50% of the compound present. In different embodiments, Hydrate III makes up at least 75%, at least 85%, at least 90%, at least 95%, or about 94%-98% of Compound A present.

In additional aspects the Compound A is provided as a K or Na crystalline salt. These salts are hydrates.

In a first embodiment directed to the Compound A crystalline Na salt, the compound is characterized by an X-ray powder diffraction pattern obtained using copper Kₐ radiation which comprises three or more characteristic peaks. Characteristic peeks are illustrated in Figure 6 of WO2013/028465. In a second embodiment directed to the Compound A crystalline Na salt, the compound is characterized by an X-ray powder diffraction pattern obtained using copper Kₐ radiation which comprises 2Θ values in degrees of about 18.4, 9.1, and 9.8. In a third embodiment directed to the Compound A crystalline Na salt, the compound is characterized by an X-ray powder diffraction pattern obtained using copper Kₐ radiation which comprises 2Θ values in degrees of about 18.4, 9.1, 9.8, 9.6, 19.3, 15.3 and 16.5. In a fourth embodiment directed to the Compound A crystalline Na salt, the compound is characterized by an X-ray powder diffraction pattern obtained using copper Kₐ radiation which comprises 2Θ values in degrees of about 18.4, 9.1, 9.8, 9.6, 19.3, 15.3, 16.5, 22.5, 17.4, 20.2, 8.4, 21.3, 26.9, 4.8, and 26.2. In another embodiment, Compound A crystalline Na salt is substantially pure. Reference to "substantially pure" means the particular form makes up at least 50% of the compound present. In different embodiments, Compound A crystalline Na salt makes up at least 75%, at least 85%, at least 90%, at least 95%, or about 94%-98% of Compound A present.

In a first embodiment directed to the Compound A crystalline K salt, the compound is characterized by an X-ray powder diffraction pattern obtained using copper Kₐ radiation which comprises three or more characteristic peaks. Characteristic peeks are illustrated in Figure 7 of WO2013/028465. In a second embodiment directed to the Compound A crystalline K salt, the compound is characterized by an X-ray powder diffraction pattern obtained using copper a radiation which comprises 2Θ values in degrees of about 18.2, 8.9, and 20.3. In a third embodiment directed to the Compound A crystalline K salt, the compound is characterized by an X-ray powder diffraction pattern obtained using copper Kₐ radiation which comprises 2Θ values in degrees of about 18.2, 8.9, 20.3, 18.7 and 22.5. In a fourth embodiment directed to the Compound A crystalline K salt, the compound is characterized by an X-ray powder diffraction pattern obtained using copper Kₐ radiation which comprises 2Θ values in degrees of about 18.2, 8.9, 20.3, 18.7, 22.5, 8.4, 19.6, 16.7, 27.1, 10.3, 21.9, 9.4, 21.2, 25.9, and 12.5. In another embodiment, Compound A crystalline K salt is substantially pure. Reference to "substantially pure" means the particular form makes up at least 50% of the compound present. In different embodiments, Compound A crystalline K salt makes up at least 75%, at least 85%, at least 90%, at least 95%, or about 94%-98% of Compound A present.

In a first embodiment directed to Hydrate I, the hydrate is characterized by an X-ray powder diffraction pattern obtained using copper Kₐ radiation which comprises three or more characteristic peaks. Characteristic peeks are illustrated in Figure 8 of WO2013/028465. In a second embodiment directed to Hydrate I, the hydrate is characterized by an X-ray powder diffraction pattern obtained using copper Kₐ radiation which comprises 2Θ values in degrees of about 8.6, 20.6, and 26.6.

In a third embodiment directed to Hydrate I, the hydrate is characterized by an X-ray powder diffraction pattern obtained using copper Kₐ radiation which comprises 2Θ values in degrees of about 8.6, 20.6, 26.6, 17.4, and 16.6. In a fourth embodiment directed to Hydrate I, the hydrate is characterized by an X-ray powder diffraction pattern obtained using copper Kₐ radiation which comprises 2Θ values in degrees of about 8.6, 20.6, 26.6, 17.4, 16.6, 12.2, 21.2, 18.8, 15.0, 23.0, 14.1 and 16.9. A seventh embodiment is directed to Hydrate I, where the hydrate is characterized by the solid state carbon-13 CPMAS NMR spectrum provided in Figure 9 of WO2013/028465. A eighth embodiment is directed to Hydrate I, where the hydrate is characterized by a solid state carbon-13 CPMAS NMR comprising peaks at about 4.22, 7.23, 11.45, 17.79, 24.04, 26.95, 28.29, 31.15, 32.47, 32.47, 33.46, 34.03, 35.74, 42.32, 53.50, 56.05, 56.96, 77.49, 108.95, 119.65, 122.55, 131.05, 133.13, 135.38, 142.28, 150.78, 156.03, 157.99, 161.36, 171.40, 173.42, 174.30 ppm. In another embodiment, Hydrate I is substantially pure. Reference to "substantially pure" means the particular form makes up at least 50% of the compound present. In different embodiments, Hydrate I makes up at least 75%, at least 85%, at least 90%, at least 95%, or about 94%-98% of Compound A present.

In a first embodiment directed to Hydrate IV, the hydrate is characterized by an X-ray powder diffraction pattern obtained using copper Kₐ radiation which comprises three or more characteristic peaks. Characteristic peaks are illustrated in Figure 10 of WO2013/028465. In a second embodiment directed to Hydrate IV, the hydrate is characterized by an X-ray powder diffraction pattern obtained using copper Kₐ radiation which comprises 2Θ values in degrees of about 14.7, 11.5, and 7.1.

In a third embodiment directed to Hydrate IV, the hydrate is characterized by an X-ray powder diffraction pattern obtained using copper Kₐ radiation which comprises 2Θ values in degrees of about 14.7, 11.5, 7.1, 9.3, and 15.6. In a fourth embodiment directed to Hydrate IV, the hydrate is characterized by an X-ray powder diffraction pattern obtained using copper Kₐ radiation which comprises 2Θ values in degrees of about 14.7, 11.5, 7.1, 9.3, 15.6, 7.7, and 8.0. A seventh embodiment is directed to Hydrate IV, where the hydrate is characterized by the solid state carbon-13 CPMAS NMR spectrum provided in Figure 11 of WO2013/028465. A eighth embodiment is directed to Hydrate IV, where the hydrate is characterized by a solid state carbon-13 CPMAS NMR comprising peaks at about 3.90, 5.30, 6.99, 10.49, 13.13, 17.81, 24.73, 27.52, 28.14, 29.42, 31.02, 32.80, 36.08, 39.22, 42.45, 53.62, 55.93, 59.14, 60.76, 74.77, 109.22, 111.19, 11.38, 120.24, 122.50, 133.96, 139.74, 147.2, 148.90, 154.65, 158.25, 159.53, 160.12, 170.14, 171.05, 172.08, 173.47, and 174.46 ppm. In another embodiment, Hydrate IV is substantially pure. Reference to "substantially pur e" means the particular form makes up at least 50% of the compound present. In different embodiments, Hydrate IV makes up at least 75%, at least 85%, at least 90%, at least 95%, or about 94%-98% of Compound A present.

In a first embodiment directed to Hydrate V, the hydrate is characterized by an X-ray powder diffraction pattern obtained using copper Kₐ radiation which comprises three or more characteristic peaks. Characteristic peeks are illustrated in Figure 12 of WO2013/028465. In a second embodiment directed to Hydrate V, the hydrate is characterized by an X-ray powder diffraction pattern obtained using copper Kₐ radiation which comprises 2Θ values in degrees of about 9.1, 18.3, and 19.8. In a third embodiment directed to Hydrate V, the hydrate is characterized by an X-ray powder diffraction pattern obtained using copper Kₐ radiation which comprises 2Θ values in degrees of about 9.1, 18.3, 19.8, 15.2, and 23.2. In a fourth embodiment directed to Hydrate V, the hydrate is characterized by an X-ray powder diffraction pattern obtained using copper Kₐ radiation which comprises 2Θ values in degrees of about 9.1, 18.3, 19.8, 15.2, 23.2, 10.9, 17.6 and 23.9. A seventh embodiment is directed to Hydrate V, where the hydrate is characterized by the solid state carbon- 13 CPMAS NMR spectrum provided in Figure 13 of WO2013/028465. A eighth embodiment is directed to Hydrate V, where the hydrate is characterized by a solid state carbon-13 CPMAS NMR comprising peaks at about 7.86, 8.92, 13.10, 18.31, 23.72, 27.44, 28.47, 30.77, 35.79, 36.25, 37.15, 37.15, 42.95, 53.13, 55.67, 57.31, 60.47, 62.06, 75.09, 110.59, 112.24, 118.32, 132.18, 134.05, 135.83, 139.88, 148.30, 155.19, 157.97, 159.41, 170.31 and 175.20 ppm. In another embodiment, Hydrate V is substantially pure. Reference to "substantially pure" means the particular form makes up at least 50% of the compound present. In different embodiments, Hydrate V makes up at least 75%, at least 85%, at least 90%, at least 95%, or about 94%-98% of Compound A present.

In a first embodiment directed to Hydrate VI, the hydrate is characterized by an X-ray powder diffraction pattern obtained using copper Kₐ radiation which comprises three or more characteristic peaks. Characteristic peeks are illustrated in Figure 14 of WO2013/028465. In a second embodiment directed to Hydrate VI, the hydrate is characterized by an X-ray powder diffraction pattern obtained using copper Kₐ radiation which comprises 2Θ values in degrees of about 20.5, 12.8, and 19.4.

In a third embodiment directed to Hydrate VI, the hydrate is characterized by an X-ray powder diffraction pattern obtained using copper Kₐ radiation which comprises 2Θ values in degrees of about 20.5, 12.8, 19.4, 21.2, and 16.8. In a fourth embodiment directed to Hydrate VI, the hydrate is characterized by an X-ray powder diffraction pattern obtained using copper Kₐ radiation which comprises 2Θ values in degrees of about 20.5, 12.8, 19.4, 21.2, 16.8, 13.9, 5.0, 18.5, 23.7, and 26.8. A seventh embodiment is directed to Hydrate VI, where the hydrate is characterized by the solid state carbon- 13 CPMAS NMR spectrum provided in Figure 15 of WO2013/028465. A eighth embodiment is directed to Hydrate VI, where the hydrate is characterized by a solid state carbon-13 CPMAS NMR comprising peaks at about 4.87, 6.24, 11.70, 12.85, 18.36, 26.55, 28.31m 31.51, 34.98, 38.47, 42.09, 54.27, 56.12, 60.10, 73.49, 73.97, 105.91, 108.04, 118.39, 119.75, 121.33, 129.96, 133.87, 136.13, 142.26, 142.97, 146.85, 148.36, 154.97, 157.32, 160.71, 168.23, 172.21 and 175.34 ppm. In another embodiment, Hydrate VI is substantially pure. Reference to "substantially pure" means the particular form makes up at least 50% of the compound present. In different embodiments, Hydrate VI makes up at least 75%, at least 85%>, at least 90%, at least 95%, or about 94%-98% of Compound A present.

Another aspect is directed to a method of making Hydrate III from Compound A involving the use of acetone/water and drying. Different ratios of acetone/water can be employed. In different embodiments, the acetone to water ratio is between 80:20 v/v acetone to water and 0: 100 v/v acetone to water. In additional embodiments, the acetone to water ratio is 65:35, 50:50, about 65:35, or about 50:50; and/or Hydrate II or Hydrate IV of Compound A is used as the starting point to make Hydrate III; and/or the compound is initially dissolved in acetone. Reference to about with respect to acetone:water ratio indicates a range of +_10 for each component. For example, "about" for the ratio 65:35, indicates acetone can vary from 55-75. In an embodiment, crude (Hydrate II) is dissolved in acetone. Water is added to the process to bring the solvent composition to 85:15 acetone: water. The process is then seeded to start crystallization, and water is then added to improve the yield and the final solvent composition is 50:50 acetone:water.

*Isotopic Enrichment:* The atoms in a compound described herein may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds described herein. For example, different isotopic forms of hydrogen (H) include protium (iH) and deuterium (2H). Protium is the predominant hydrogen isotope found in nature. Enriching for deuterium may afford certain therapeutic advantages, such as increasing in vivo half-life or reducing dosage requirements, or may provide a compound useful as a standard for characterization of biological samples. Isotopically-enriched compounds described herein can be prepared without undue experimentation by conventional techniques well known to those skilled in the art or by processes analogous to those described in the Schemes and Examples provided herein using appropriate isotopically-enriched reagents and/or intermediates.

*Administration and Compositions:* Pharmaceutically acceptable salts are suitable for administration to a patient, preferably, a human. Suitable salts include acid addition salts which may, for example, be formed by mixing a solution of a compound with a solution of a pharmaceutically acceptable acid such as hydrochloric acid, sulfuric acid, acetic acid, trifluoroacetic acid, or benzoic acid. Compounds carrying an acidic moiety can be mixed with suitable pharmaceutically acceptable salts to provide, for example, alkali metal salts (e.g., sodium or potassium salts), alkaline earth metal salts (e.g., calcium or magnesium salts), and salts formed with suitable organic ligands such as quaternary ammonium salts. Also, in the case of an acid (-COOH) or alcohol group being present, pharmaceutically acceptable esters can be employed to modify the solubility or hydrolysis characteristics of the compound. Compound A can be administered to a patient infected with HCV. The term "administration" and variants thereof (e.g., "administering" a compound) means providing the compound to the individual in need of treatment. When a compound is provided in combination with one or more other active agents (e.g., antiviral agents useful for treating HCV infection), "administration" and its variants are each understood to include concurrent and sequential provision of the compound or salt and other agents. As used herein, the term "composition" is intended to encompass a product comprising the specified ingredients, as well as any product which results, directly or indirectly, from combining the specified ingredients. By "pharmaceutically acceptable" is meant the ingredients of the pharmaceutical composition are compatible with each other and are suitable to the recipient thereof. The term "subject" (alternatively referred to herein as "patient") as used herein refers to an animal, preferably a mammal, most preferably a human, who is the object of treatment, observation or experiment. The term "effective amount" indicates a sufficient amount to exert a therapeutic or prophylactic effect. For a patient infected with HCV, an effective amount is sufficient to achieve one or more of the following effects: reduce the ability of HCV to replicate, reduce HCV load, and increase viral clearance. For a patient not infected with HCV, an effective amount is sufficient to achieve one or more of the following: a reduced susceptibility to HCV infection, and a reduced ability of the infecting virus to establish persistent infection for chronic disease. For the purpose of inhibiting HCV NS3 protease and treating HCV infection and/or reducing the likelihood or severity of symptoms of HCV infection, the compounds, optionally in the form of a salt, can be administered by means that produces contact of the active agent with the agent's site of action. They can be administered by conventional means available for use in conjunction with pharmaceuticals, either as individual therapeutic agents or in a combination of therapeutic agents. They can be administered alone, but typically are administered with a pharmaceutical carrier selected on the basis of the chosen route of administration and standard pharmaceutical practice. Compounds can, for example, be administered by one or more of the following routes: orally, parenterally (including subcutaneous injections, intravenous, intramuscular, intrasternal injection or infusion techniques), by inhalation (such as in a spray form), or rectally, in the form of a unit dosage of a pharmaceutical composition containing an effective amount of the compound and pharmaceutically-acceptable carrier (e.g., a carrier suitable for administration to a human patient), adjuvants and vehicles. Liquid preparations suitable for oral administration (e.g., suspensions, syrups, elixirs and the like) can employ media such as water, glycols, oils, alcohols and the like. Solid preparations suitable for oral administration (e.g., powders, pills, capsules and tablets) can employ solid excipients as such starches, sugars, kaolin, lubricants, binders, disintegrating agents and the like. Parenteral compositions typically employ sterile water as a carrier and optionally other ingredients, such as solubility aids. Injectable solutions can be prepared, for example, using a carrier comprising a saline solution, a glucose solution or a solution containing a mixture of saline and glucose. Further guidance for methods suitable for use in preparing pharmaceutical compositions is provided in Remington:

The Science and Practice of Pharmacy, 21th edition (Lippincott Williams & Wilkins, 2006). Therapeutic compounds can be administered orally in a dosage range of 0.001 to 1000 mg/kg of mammal (e.g., human) body weight per day in a single dose or in divided doses. One dosage range is 0.01 to 500 mg/kg body weight per day orally in a single dose or in divided doses.

Another dosage range is 0.1 to 100 mg/kg body weight per day orally in single or divided doses. For oral administration, the compositions can be provided in the form of tablets or capsules containing 1.0 to 500 mg of the active ingredient, particularly 1, 5, 10, 15, 20, 25, 50, 75, 100, 150, 200, 250, 300, 400, 500, and 750 mg of the active ingredient for the symptomatic adjustment of the dosage to the patient to be treated. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Other embodiments of the present invention include the following: (a) A pharmaceutical composition comprising an effective amount of Compound A and a pharmaceutically acceptable carrier. (b) The pharmaceutical composition of (a), further comprising a second therapeutic agent selected from the group consisting of HCV antiviral agents, immunomodulators, and anti-infective agents. (c) The pharmaceutical composition of (b), wherein the HCV antiviral agent is an antiviral selected from the group consisting of HCV protease inhibitors, NS5A inhibitors, and HCV NS5B polymerase inhibitors. (d) A pharmaceutical combination that is (i) Compound A and (ii) a second therapeutic agent selected from the group consisting of HCV antiviral agents, immunomodulators, and anti-infective agents; wherein Compound A and the second therapeutic agent are each employed in an amount that renders the combination effective for inhibiting HCV replication, for treating HCV infection and/or reducing the likelihood or severity of symptoms of HCV infection. (e) The combination of (d), wherein the HCV antiviral agent is an antiviral selected from the group consisting of HCV protease inhibitors, NS5A inhibitors, and HCV NS5B polymerase inhibitors. (f) A method of inhibiting HCV replication in a subject in need thereof which comprises administering to the subject an effective amount of Compound A. (g) A method of treating HCV infection and/or reducing the likelihood or severity of symptoms of HCV infection in a subject in need thereof which comprises administering to the subject an effective amount of Compound A. (h) The method of (g), wherein the compound is administered in combination with an effective amount of at least one second therapeutic agent selected from the group consisting of HCV antiviral agents, immunomodulators, and anti-infective agents. (i) The method of (h), wherein the HCV antiviral agent is an antiviral selected from the group consisting of HCV protease inhibitors, NS5A inhibitors and HCV NS5B polymerase inhibitors. (j) A method of inhibiting HCV replication in a subject in need thereof which comprises administering to the subject the pharmaceutical composition of (a), (b) or (c) or the combination of (d) or (e). (k) A method of treating HCV infection and/or reducing the likelihood or severity of symptoms of HCV infection in a subject in need thereof which comprises administering to the subject the pharmaceutical composition of (a), (b) or (c) or the combination of (d) or (e).

Aspect (iv) of the present invention also includes a compound of the present invention for use in medicine; or for use in (i) a medicament, (ii) in the preparation of a medicament, for (a) inhibiting HCV replication or (b) treating HCV infection and/or reducing the likelihood or severity of symptoms of HCV infection. In these uses, the compounds of the present invention can optionally be employed in combination with one or more second therapeutic agents selected from HCV antiviral agents, anti-infective agents, and immunomodulators.

*HCV Inhibitory Activity:* The ability of a compound to inhibit HCV NS3 activity, HCV replicon activity, and HCV replication activity can be evaluated using techniques well-known in the art. (See, for example, Carroll et al, J. Biol. Chem. 278: 1 1979-1 1984, 2003.). One such assay is a HCV NS3 protease time-resolved fluorescence (TRF) assay as described below and in Mao et al, Anal. Biochem. 373:1-8, 2008 and Mao et al, WO 2006/102087. A NS3 protease assay can be performed, for example, in a final volume of 100 µî assay buffer containing 50 mM HEPES, pH 7.5, 150 mM NaCl, 15 % glycerol, 0.15 % TRITON X- 100, 10 mM DTT, and 0.1 % PEG 8000. NS3 and NS4A are pre-incubated with various concentrations of inhibitors in DMSO for 30 minutes. The reaction is initiated by adding the TRF peptide substrate (final concentration 100 nM). NS3 mediated hydrolysis of the substrate is quenched after I hour at room temperature with 100 µl of 500 mM MES, pH 5.5. Product fluorescence is detected using either a VICTOR V2 or FUSION fluorophotometer (Perkin Elmer Life and Analytical Sciences) with excitation at 340 nm and emission at 615 nm with a 400 µ8 delay. Testing concentrations of different enzyme forms are selected to result in a signal to background ratio (S/B) of 10-30. IC₅₀ values are derived using a standard four-parameter fit to the data. K; values are derived from IC50 values using the following formula, IC₅₀ = Ki (1 + [S] / K_{M}), Eqn (1), where [S] is the concentration of substrate peptide in the reaction and KM is the Michael is constant. See P. Gallinari et al, 38 BIOCHEM. 5620-32(1999); P. Gallinari et al, 72 J. VIROL. 6758-69 (1998); M. Taliani et al, 240 ANAL. BIOCHEM. 60-67 (1996); and Mao et al, Analytical Biochemistry 373: 1-8, (2008).

Abbreviations BOC: 7-Butoxycarbonyl; Cbz: Benzyloxycarbonyl; CDI: 1,1 '-Carbony Idiimidazol; CIP: 2-Chluoro- 1-methylpyridinium iodide; CPME: Cyclopentyl methyl ether; DABO: 1,4-Diazabicyclo [2.2.2.] octane; DBA saltdibenzylamine; DBU: 1,8-Diazobicyclo[5.4.0]undec-7-ene; DCC: N,7V-Dicyclohexylcarbodiimide; DIC: N,N'-diisopropylcarbodiimide; DIPEA: Diisopropylethylamine; DM Ac: N,N-Dimethylacetamide; DMF:;N,N'-Dimethylformamide; DMPU: N,N-dimethylpropyleneurea; DMSO: Dimethylsulfoxide; EDC: 1-Ethyl-3-(3-dimethylaminopropyl) carbodiimide; EtOAc: Ethyl acetate; Fmoc: 9-Fluorenylmethyloxycarbonyl; H ATU: 2-(1 H-7-Azabenzotriazole- 1-yl)- 1,1,3,3 -tetramethyluronium hexafluorophosphate; HO AT: 1-Hydroxy-7-azabenzotriazole; HOBT: 1-Hydroxybenzotriazole; HOPO: 2-Hydroxypyridine-N-oxide; HOSu: N-hydroxysuccinimide; IPA: Isopropanol; IP Ac: Isopropyl acetate; MTBE: t-butyl methyl ether; MsOH and MSA: CH₃SO₃H or methanesulfonic acid; Moz: p-Methoxybenzyloxycarbonyl; Msz: 4-Methylsulfinylbenzyloxycarbonyl; NMP: N-Methylpyrrolidone; PFP: pentafluorophenol; T3P: propylphosphonic anhydride; TBA: t-butyl amine; TEA: Triethylamine; THF: Tetrahydrofuran; pTSA and TsOH are each abbreviations for p-toluenesulfonic acid.

The following examples of WO2013/028465 are reiterated hereinafter:

The examples provided below are intended to illustrate the invention and its practice. Unless otherwise provided in the claims, the examples are not to be construed as limitations on the scope or spirit of the invention.

### Example 1: Preparation of 2-[2-(3-Chloro-propyl)cyclopropyl]-4,4,5,5-tetramethyl-[1.3.2]dioxaborolane (Compound 3):

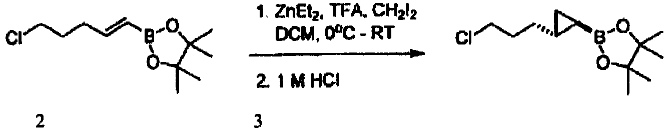

Compound 2 can be prepared as described by Shirakawa et al. Synthesis 77:1814- 1820, 2004.) Compound 3 was produced as follows: To a 5 L flask equipped with a nitrogen inlet, mechanical stirrer, dropping funnel and thermocouple under N₂ was added 800 mL dichloromethane and 800 mL of a 1 M diethylzinc solution in heptane (0.8 mol, 1.07 equiv). The solution was cooled with an ice bath to an internal temperature of 3 °C. To the flask was then added from the dropping funnel a solution of 57.6 mL trifluoroacetic acid (0.748 mol, 1.0 equiv) in 200 mL dichloromethane over 1 hour, keeping the internal temperature below 10 °C. The resulting suspension was stirred for 30 min at 3 °C. To the flask was then added 72.4 mL diiodomethane (0.897 mol, 1.2 equiv) in a single portion. After stirring at 3 °C for 30 min, 172 mL of 2 (0.748 mol, 1.0 equiv) was added to the solution in a single portion. The flask was then allowed to warm to room temperature and a white precipitate began to form. After 3 hours, GC analysis indicated the reaction was at 90% conversion. The suspension was aged for an additional 17 hours or until complete consumption of 2 is observed. At that point, 800 mL of 1 M HCl (0.8 mol, 1.07 equiv) was added and a +5 °C exotherm was observed. The biphasic mixture was stirred for 30 min to dissolve the precipitated solids and the organic layer was separated. Extraction of the aqueous layer with 200 mL dichloromethane, washing of the combined organic layers with 500 mL brine and concentration in vacuo gave 194 g of 3 as a yellow oil (74 wt % in DCM). 'H NMR (400 MHz, CDCl₃) δ 3.59 (t, 2H, J = 6.7 Hz), 1.90 (pent, 2H, J= 7.1 Hz), 1.49 (sext, 1 H, J= 7.0 Hz), 1.36 (sext, 1H, J = 7.0 Hz), 1.23 (s, 12H), 0.93 (m, 1H), 0.71 (m, 1H), 0.44 (m, 1H), -0.35 (dt, 1H, J= 9.4, 5.7 Hz); ¹³C NMR (100 MHz, CDCl₃) 6 82.82, 44.74, 32.67, 32.22, 24.64, 17.22, 1 1.24, 0.5 (bs); GC: HPI (30 m x 0.32 mm; 0.25 µm), 25 psi, 200 °C front inlet. 5 min at 50 °C, ramp 25 °C / min to 250 °C then hold for 4 min, tᵣ(2)=9.78 min, tᵣ(3)=10.08 min.

### Example 2: Preparation of 2-(3-Chloro-propyl)-cyclopropanol (Compound 4)

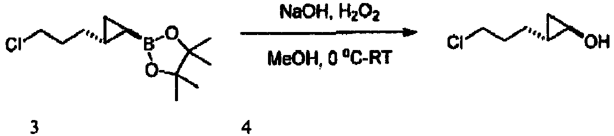

To a 3 L flask equipped with a nitrogen inlet, mechanical stirrer, dropping funnel and thermocouple was added 143 g of 3 (0.585 mol, 1.0 equiv) in 1 L methanol. To the flask was then added from the dropping funnel 58.5 mL of 10 M sodium hydroxide (0.585 mol, 1.0 equiv) over 30 min, while the internal temperature was maintained below 10 °C with external cooling. After stirring for 30 min, 120 mL of 30 wt% hydrogen peroxide solution (1.17 mol, 2 equiv) was slowly added from the dropping funnel over 1 hour, keeping the internal temperature below 10 °C. Upon completion of the addition, the resulting colorless slurry was then stirred at ambient temperature for 30 min or until complete consumption of 3 was observed by GC. 2 M HCl (375 mL) was added from the dropping funnel over 30 min, keeping the internal temperature below 10 °C. To this clear yellow solution was then slowly added 500 mL of a 1 M solution of Na₂SO₃ from the dropping funnel, keeping the internal temperature below 10 °C. The resulting suspension was then filtered and extracted 3 x 200 mL MTBE. Concentration followed by silica gel column chromatography (6:4 hexane:ethyl acetate), to remove pinacol, gave 60.6 g of product 4 as a clear oil (90 wt %). 1H NMR (400 MHz, CDCl₃) δ 3.62 (t, 2H, J= 6.6 Hz), 3.27 (dt, 1H, J= 6.3, 2.6 Hz), 1.89 (pent, 2H, J = 6.8 Hz), 1.85 (bs, OH), 1.43 (sext, 1H, J= 7.0 Hz), 1.28 (sext, 1H, J= 7.0 Hz), 0.94 (m, 1H), 0.75 (m, 1H), 0.38 (q, 1H, J = 6.0 Hz); ¹³C NMR (100 MHz, CDCl₃) δ 52.21, 44.69, 31.91, 28.69, 19.69, 14.15; GC: HPI (30 m x 0.32 mm; 0.25 µm), 25 psi, 200 °C front inlet. 5 min at 50 °C, ramp 25 °C / min to 250 °C then hold for 4 min, tᵣ(3)=10.08 min, tᵣ(4)=7.15 min.

### Example 3: Preparation of 2-Pent-4-ynyl-cyclopropanol (rac-Compound 5)

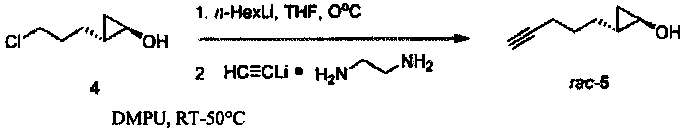

To a 2-neck 15-mL flask equipped with a temperature probe, N₂ inlet, and septum was added 1 g of 4 (7.28 mmol, 1.0 equiv) and 3.0 mL THF. The solution was cooled to an internal temperature of 0 °C with an ice bath. To this solution was added 2.95 mL of 33 wt% n- Hexyllithium (7.28 mmol, 1.0 equiv) slowly via syringe pump over 1 hour. Internal temperature rose to 6.8 °C and solution became yellow. In a separate 3-neck 100-mL flask equipped with a temperature probe, N₂ inlet, and septum 0.82 g of lithium acetylide-ethylenediamine complex (8.01 mmol, 1.1 equiv) was slurried in 5.0 mL of DMPU at room temperature. To this room temperature slurry, the cold solution of the deprotonated cyclopropanol was transferred via cannula over 5 min. After the addition, the brown mixture was heated to an internal temperature of 52 °C with a heating mantle for 3 hours or until greater than 98% conversion was observed by GC. The brown mixture was cooled with an ice bath to 3 °C and then the ice bath was removed to prevent freezing. To this was slowly added 17.5 mL of 0.5 N HCl and an ice bath was applied to maintain an internal temperature below 21 °C. The mixture was then diluted with 10 mL MTBE and 5 mL of water before transfer to a separatory funnel and removal of the aqueous layer. The aqueous layer was extracted once with 15 mL MTBE and then the combined organic layers were washed with 20 mL water followed by 20 mL brine. The organic layer was then concentrated in vacuo to afford 1.27 g of rac-5 as a yellow oil (72 wt%). 1H NMR (400 MHz, CDCl₃) δ 3.24 (dt, 1H, J= 2.6, 5.3 Hz), 2.25 (dt, 2H, J= 2.6, 7.6 Hz), 1.96 (t, 1H, J= 2.6 Hz), 1.92 (s, 1H, OH), 1.64 (pent, 2H, J= 7.3 Hz), 1.38 (sext, 1H, J= 6.9 Hz), 1.24 (sext, 1H, J= 6.9 Hz), 0.93 (m, 1H), 0.72 (m, 1H), 0.35 (q, 1H, J = 6.0 Hz); ¹³C NMR (100 MHz, CDCl₃) δ 84.49, 68.37, 52.45, 30.50, 27.74, 20.17, 18.01, 14.25; GC: HPI (30 m x 0.32 mm; 0.25 µm), 25 psi, 200 °C front inlet. 5 min at 50 °C, ramp 25 °C / min to 250 °C then hold for 4 min, tᵣ(4) = 7.15 min, tᵣ(rac-5) = 6.72 min.

### Examples 4: Preparation of Acetic Acid racemic trans-2-pent-4-vnyl-cyclopropyl ester (rac-Compound 6):

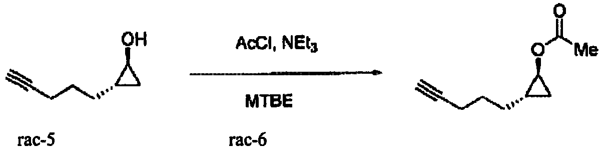

To a 5L flask equipped with a nitrogen inlet, mechanical stirrer, dropping funnel and thermocouple under N₂ was added 31.2 g of rac-5 (251 mmol, 1.0 equiv), 350 mL of MTBE and 45.5 mL of triethylamine (327 mmol, 1.3 equiv) prior to cooling the solution in an acetone/ice bath to an internal temp of < 5 °C. To the solution was added from the dropping funnel 23.7 mL acetyl chloride (301 mmol, 1.1 equiv) over a 30 min period while maintaining the internal temp <10 °C. The resulting slurry was then wanned to room temperature and aged for 2 hours. The reaction mixture was then diluted with 200 mL of water. The organic layer was washed with 200 mL of 2 N HCl and then with 300 mL of sat. NaHCO₃ prior to drying over MgSO₄. The solvent was removed in vacuo to give 41.8 g of rac-6. 1H NMR (400 MHz, CDCl₃) δ 3.84 (dt, 1H, J= 6.7, 2.9 Hz), 2.25 (dt, 2H, J= 2.7, 7.0 Hz), 2.03 (s, 3H), 1.95 (t, 1H, J= 2.6 Hz), 1.67 (m, 2H), 1.39 (m, 2H), 1.01 (m, 1H), 0.89 (m, 1H), 0.57 (q, 1H, J= 6.5 Hz); ¹³C NMR (100 MHz, CDCl₃) δ 171.60, 84.15, 68.47, 54.20, 30.12, 27.40, 20.85, 17.92, 17.83, 11.81; GC: Restek RT-Bdex SA (30 m x 0.25mm x 0.25 µm), 60 cm/s linear velocity, 20:1 split, 120°C isothermal, tᵣ(5) = 25.0, 29.6 min, tᵣ(6) = 17.1, 17.5 min.

### Example 5: Preparation of (1R, 2R)-2-Pent-4-ynyl-cyclopropanol (ent-Compound-5):

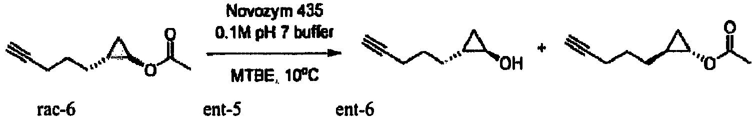

To a 1-L flask equipped with an overhead stirrer and temperature probe was added a 60 wt% solution of rac-6 in MTBE (44.8 g, 0.27 mol) and an additional 730 ml of MTBE that had been saturated with aqueous 0.1 M pH 7 phosphate buffer, giving a final solution concentration of rac-6 of 60 g/l. The flask was placed in an ice bath to maintain an internal temperature of approximately 10 °C throughout the hydrolysis reaction, which was initiated by the addition of 730 mg Novozym 435. The reaction was aged at 10 °C for approximately 4 hours until conversion had reached 41%, at which point the ee of ent-5 was 96%. The reaction mixture was then filtered through a 150-ml medium-pore glass filter funnel and the solid immobilized enzyme was washed three times with 80 ml MTBE. The resulting MTBE solution was then solvent switched to heptane. The mixture in heptane (39.2 kg, approximately 50 L) was applied to a Biotage Flash 400 L cartridge (40 x 60 cm, 40 kg silica gel, 60 angstrom, 40-63 um) and eluted sequentially with 165 L of 2.5:97.5, 75 L of 10:90, and 330 L of 25:75 EtOAc/heptane (v/v). After the mixture was applied to the column, 18 L fractions were taken. The rich cut fractions of the alcohol ent-5 were located by TLC (silica, 20% EtO Ac/heptane) and then analyzed by GC (HP-1, 30 m x 320 um x 0.25 um film, 9.14 psi constant He pressure, 15: 1 split, 50 °C for 5 min then 25 deg/min to 275 °C and hold 5 min, RT of alcohol 8.8 min). Fractions 15-21 were concentrated to give 3.48 kg (80 wt%, 92 %ee) of the desired ent-5 (Compound 7). GC: Restek RT-Bdex SA (30 m x 0.25mm x 0.25 µm), 60 cm/s linear velocity, 20: 1 split, 120°C isothermal, tᵣ(5) = 25.0, 29.6 min, tᵣ(6) = 17.1, 17.5 min.

### Example 6: Preparation of (S)-3,3-Dimethyl-2-((1R,2R)-2-pent-4-vnyl-cyclopropoxycarbonylamino)-butyric acid (Compound 8):

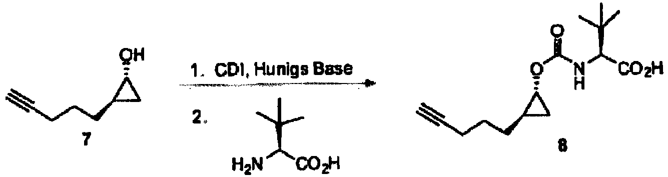

To a 50L round bottom flask equipped with a mechanical stirrer, thermocouple and reflux condenser was added Compound 7 (3.477 kg at 81 wt% by NMR, 92 % ee) and 14.1 L (5 L/kg) of Hunigs base. To the resulting homogeneous solution was added CDI portion wise as a solid while maintaining the internal temperature between 21-25 °C. The resulting slurry was aged at room temperature for 1 hour. To the slurry was added L-tert-leucine as a solid and the reaction mixture was heated to an internal temperature of 95 °C for 2.5 hours. The reaction mixture was cooled to room temperature and diluted with 17 L of water. The mixture was aged for 30 min to dissolve all the solids and then transferred to a 100 L cylindrical extractor. The aqueous layer was then washed with 12 L of MTBE. The aqueous layer was washed with 8 L of MTBE. The resulting aqueous layer was pH adjusted with concentrated HCl to a final pH of 1.5-2.0. The biphasic mixture was extracted with MTBE (2 X 12 L) and the combined organic phase was washed with 6 L of water followed by 5 L of brine. The MTBE layer was then transferred via vacuum into a 50 L round bottom flask equipped with a mechanical stirrer, thermocouple, and batch concentrator and the solvent was removed under reduced pressure keeping the internal temperature of the batch < 20 °C during the distillation. The solvent was then switched to cyclopentyl methyl ether (CPME) by flushing with - 5 L of CPME and then diluted to a final volume of - 20 L. This material was used in the next reaction without further purification. An analytical sample was obtained by silica gel chromatography as a colorless oil: 1H NMR (CDCl₃, 400 MHz) δ 0.54 (q, 1H, J= 6.4 Hz), 0.83 (m, 1H), 0.99 (m, 1H), 1.01 (s, 9H), 1.40 (m, 2H), 1.67 (m, 2H), 1.94 (t, 1H, J= 2.6 Hz), 2.23 (m, 2H), 3.77 (br m, 1H), 4.20 (torn, 1H), 5.28 (br m, 1H), 9.40 (br s, 1 H);, ³C NMR (CDCl₃, 100 MHz) δ 1 1.8, 18.0, 26.5, 27.4, 30.1, 34.6, 55.0, 62.0, 68.4, 84.2, 156.7, 175.8.

### Example 7: Preparation of 6-Methoxy-quinoxaline-2,3-diol (Compound 10):

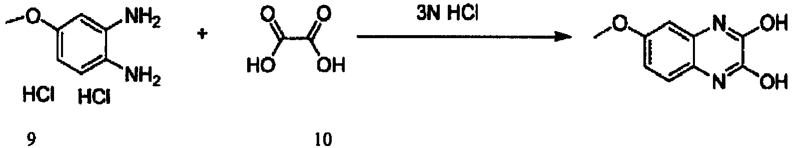

In a 50 L flask equipped with a mechanical stirrer, thermocouple and condenser was added 4-methoxy-1,2-phenylenediamine dihydrochloride salt (Compound 9) (2.65 kg at 98 wt%, 12.30 mol), oxalic acid (1.582 kg at 98 wt.%, 17.22 mol) and 3 N HCl _{(aq)} (17.8 L) under nitrogen. The grey heterogeneous slurry was heated to 90 °C with steam for 7.25 hours. The reaction was monitored by HPLC. The resulting grey slurry was then cooled to an internal temperature of 20 °C overnight. The slurry was filtered, water (1.0-1.5 L/Kg) was used to help with the transfer. The light grey solids were washed with 2 cake volumes water (5.0-5.5 L/Kg). The solids were dried under vacuum/N₂ sweep for 24 hours, at which time the solids were still very wet. The product was then slurry washed with methanol, and dried over 48 hours at 40-45 °C in a vacuum oven to give Compound 10 as an off-white product of 99.95% purity by HPLC assay. There was no methanol by NMR and the KF= 0.05 wt. % water. HPLC Conditions: Zorbax Eclipse Plus CI 8 50 x 4.6 mm, 1.8 urn. 1.5 mL/min, 210 nm, 25 °C, Eluents: Water 0.1% H₃PO₄ (A), Acetonitrile (B). 90% A 0 min, 5% A 5 min, 5% A 6 min
Compound 9 (diamine HCl salt) 0.394 min
Compound 10 1.55 min (sometimes two peaks)

### Example 8: Preparation of 2,3-Dichloro-6-methoxyquinoxaline (Compound 11)

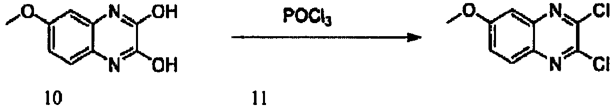

In a 22 L round bottomed flask equipped with a mechanical stirrer, thermocouple and condenser was added to 2,3-dichloro-6-methoxyquinoxalone Compound 10 (3.8 kg). Charged slowly at room temperature was POCl₃ (5.92 L at 99%). The grey slurry was heated to 98 °C for 20 hours. After 2-3 hours the slurry turned from grey to green, then to yellow and finally turned homogeneous red. As the slurry became homogenous in POCl₃, significant amounts of HCl off-gassing were produced. The dark red, homogenous solution was allowed to cool slowly to below 80 °C. At this point, 19 L of acetonitrile (5.0 L/Kg) was charged which produced a dark brown slurry. The reaction was cooled to 10-15 °C in an ice bath and reverse quenched into 45.6 L of cold water (12.0 L/Kg) in a 100 L cylindrical vessel. This exothermic quench was kept below 27 °C. MeCN (- 4L) was used to aide in slurry transfer. The brown slurry was filtered and 5 L of water was used to wash the flask. The solids were washed with 1 cake volume of water (∼5 L). The pH of the filtrate was acidic. The solids were next displacement washed with 2 cake volumes of 5% sodium bicarbonate (-20.00 L). The pH was between 8-9. A slurry wash was performed with 2 cake volumes of water (20 L total). The pH did not change. The solids were dried for 72 hours under reduced pressure and nitrogen flow to give tan product Compound 1 1 of 99.5% purity by HPLC assay with KF= 0.5 wt. % water. HPLC Conditions: Zorbax Eclipse Plus C18 50 x 4.6 mm, 1.8 urn, 1.5 mL/min, 210 nm, 25 °C; Eluents: Water 0.1% H₃PO₄ (A), Acetonitrile (B). 90% A 0 min, 5% A 5 min, 5% A 6 min.
Compound 10 1.55 min (sometimes two peaks)
Compound 1 1 4.55 min

An analytical sample was obtained by silica gel chromatography and as a colorless foam: 'H NMR (CDCl₃, 400 MHz) δ 0.50 (q, 1H, J = 6.3 Hz), 1.04 (br s, 1 1 H), 1.20 (br s, 3H), 1.45 (br s, 13 H), 1.72 (m, 2H), 2.40 (m, 1H), 2.63 (m, I H), 2.93 9m, 2H), 3.68-3.94 (m, 9H), 4.15 (br m, 1 H), 4.46 and 4.60 (t, due to rotamers, 1H, J = 7.8 Hz), 5.27 (br m, 1H), 5.78 (br m, 1H), 7.18 (m, 1H), 7.20 (m, 1H), 7.85 (m, 1H); ¹³C NMR (CDCl₃, 100 MHz) δ 11.9, 18.5, 26.6, 27.0, 28.1, 28.3, 28.4, 29.1, 30.9, 32.9, 34.1, 35.7, 36.6, 49.4, 52.1, 52.2, 52.4, 55.1, 55.7, 57.7, 58.2, 62.3, 73.5, 74.1, 80.7, 106.0, 118.8, 128.5, 133.7, 141.1, 148.2, 153.9, 154.5, 155.3, 157.1, 160.4, 173.2, 173.3, 174.4.

### Example 9: Preparation of (2S,4R)-4-(3-chloro-7-methoxyquinoxalin-2-yloxy)-2-(methoxycarbonyl)pyrrolidinium methane-sulfonate (14):

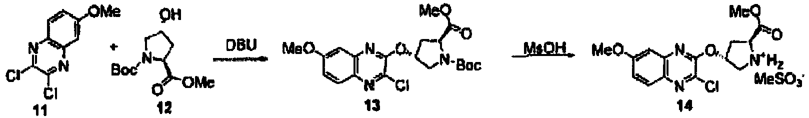

To a slurry of 2,3-dichloroquinoxaline 11 (100 g, 0.437 mol) and N-Boc-4-trans-hydroxy-L-proline methyl ester (12, 1 18 g, 0.48 mol) in DMAc (500 ml, KF < 150) at ambient temperature was added DBU (86 g, 0.568 mol). The slurry was agitated at 40-45 °C for -35 hours. The batch was then cooled to 15 °C. Ethyl acetate (1.2 L) followed by citric acid (10%, 504 mL, 162 mmol) was added while the internal temperature was maintained < 25 °C. The organic phase was washed with a solution of 10% citric acid (200 mL) and water (200 mL) followed by water (400 mL x 2). The organic phase was azeotropically dried and solvent switched to MeCN at a final volume of -880 mL. MeSO₃H (36 mL, 0.555 mol) was added and the reaction mixture was aged at 40 °C for -16 hours. To the reaction slurry was added MTBE (1.05 L) dropwise over 2 hours at 35 °C. Then, the batch was further cooled to 0-5 °C and aged for 2-3 hours before filtration. The wet cake was displacement washed with 30% MeCN in MTBE (600 mL x 2), and vacuum oven dried at 40 °C to give the product 14. ¹H NMR (400 MHz, </₆-DMSO) δ 9.74 (s, br, 2 H), 7.86 (d, J = 9.2 Hz, 1 H), 7.34 (dd, J = 9.2, 2.8 Hz, 1 H), 7.26 (d, J = 2.8 Hz, 1 H), 5.77 (m 1 H), 4.69 (dd, J = 10.6, 7.6 Hz, 1 H), 3.92 (s, 3 H), 3.89 (dd, J = 13.2, 5.2 Hz, 1 H), 3.81 (s, 3 H), 3.63 (m, 1 H), 2.71 (m, 1 H), 2.60 (m, 1 H), 2.35 (s, 3 H). ¹³C NMR (100 MHz, i/₆-DMSO) δ 168.3, 161.0, 151.8, 140.4, 135.4, 133.3, 128.6, 119.8, 106.0, 75.6, 58.0, 56.0, 53.2, 50.5, 39.6, 33.9. HPLC conditions: Hypersil Gold PFP column, 150 x 4.6 mm, 3.0 um; Column temperature of 40 °C; Flow rate of 1.8 mL/min; and Wavelength of 215 nm.

| Gradient: | min | CH₃CN | 0.1% H₃PO₄ |
|---|---|---|---|
| | 0 | 25 | 75 |
| | 12 | 70 | 30 |
| | 12.1 | 25 | 75 |
| | 14 | 25 | 75 |
| | | | |
| Retention times: | | min. | |
| Dichloroquinoxaline **11** | | **7.8** | |
| Proline quinoxaline **13** | | **9.8** | |
| De-Boc quinoxaline **14** | | **3.6** | |

### Example 10: Preparation of (S)-2-(((1R.2R)-2-(5-(6-methoxy 3-((3R,5S)-5-(methoxycarbonyl)pyrrolidin-3-yloxy)quinoxalin-2-yl)pent-4-ynyl)cyclopropoxy)carbonylamino)-3,3-dimethylbutanoic acid (16) and alkyne macrocyclic ester(17)

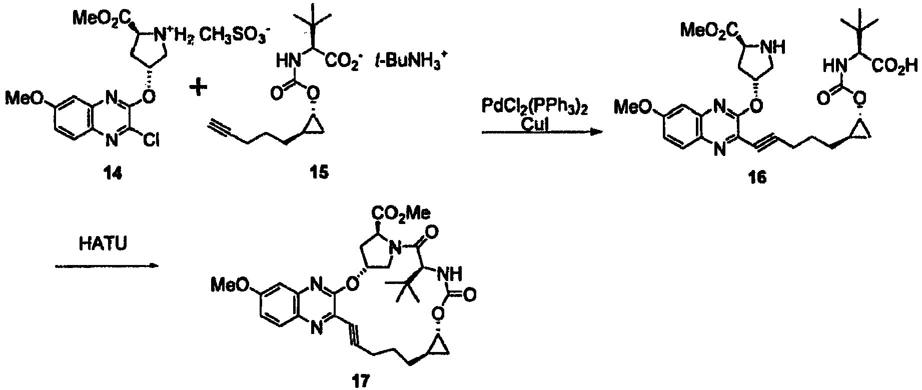

To a three-neck flask were added copper(I) iodide (0.219 g, 1.152 mmol), chloroquinoxaline MsOH salt 14 (50 g, 115 mmol), alkyne acid TBA salt 15 (49.3 g, 121 mmol), and bis(triphenylphosphine)palladium(II) dichloride (0.404 g, 0.573 mmol). The flask was vacuumed degassed with N₂. MeOH (500 ml) was added and the reaction mixture was vacuum degassed again with N₂. Triethylamine (32.1 ml, 230 mmol) was added. The reaction solution was aged at 35 °C for 3-5 hours. The batch was then concentrated to a volume of-100 mL in vacuum. THF (250 mL) and EtOAc (250 mL) were added. The reaction mixture was cooled to below 5 °C. HCl solution (1 N, -180 mL) was added slowly at below 5 °C until the reaction solution was pH adjusted to -2. NaCl aq. solution (10%, 350 mL) was added. The separated aqueous phase was back-extracted with a solution of THF (250 mL) and EtOAc (250 mL). The combined organic phase was washed with 10% NaCl aq. solution (500 mL). The organic phase was azeotropically concentrated in vacuum with THF at below 20 °C until the KF of the solution was less than 500 ppm. Then, the reaction solvent was switched to DMAc (650 mL) in vacuum at below 20 °C. A solution of HATU (55.1 g, 145 mmol) in DMAc (650 mL) at ambient temperature was vacuumed degassed with N₂. The solution was then cooled to 0 °C and DIPEA (58.5 mL, 335 mmol) was added dropwise at below 0-5 °C. Then, the above solution of alkyne quinoxaline acid 16 (65 g assay, 1 12 mmol) in DMAc was added dropwsie over 10 hours, while maintaining the internal temperature at 0 °C. After addition, the batch was agitated at 0 °C for additional 2 hours. EtOAc (750 mL) was added at below 5 °C. A solution of 10% NaCl aq. solution (400 mL), water (125 mL) and 1 N HCl solution (100 mL) was slowly added while maintaining the batch temperature at below 5 °C. The solution was then adjusted to pH = 2 with 1 N HCl (∼25 mL). The separated aqueous phase was backextracted with EtOAc (500 mL). The combined organic phase was washed with 10% NaCl aq. solution (500 mL). After 10% NaCl aq. solution (500 mL) was added to the combined organic phase, the mixed solution was cooled to 0-5 °C. I N NaOH aq. solution (-25 mL) was added to adjust the pH = ∼7. The separated organic phase was filtered through Celite and solvent switched to IP A at a final volume of 300 mL. Acetic acid (5.0 mL) was added, and the batch was then heated up to reflux for 30 min. The slurry was cooled to 60 °C and water (250 mL) was added dropwise over 1 hour. After addition, the batch was aged for additional 30 min before slowly cooling to ambient temperature in about 2 hours. After aging at least 1 hour, the batch was filtered. The wet cake was displacement washed with 50% aq IPA (100 mL). Suction dry at ambient temperature afforded 56 g of macrocyclic alkyne ester 17.'H NMR (400 MHz, CDCl₃) δ 7.80 (d, J = 9.2 Hz, I H), 7.17 (dd, J = 9.2, 2.8 Hz, 1 H), 7.04 (d, J = 2.8 Hz, 1 H), 5.82 (t, J = 4.2 Hz, 1 H), 5.26 (d, J = 9.9 Hz, 1 H), 4.62 (dd, J = 10.3, 7.3 Hz, 1 H), 4.51 (d, J = 11.6 Hz, 1 H), 4.40 (d, J = 9.9 Hz, 1 H), 4.03 (dd, J = 11.6, 4.4 Hz, 1 H), 3.91 (s, 3 H), 3.87 (m, 1 H), 3.73 (s, 3 H), 2.85 (dt, J = 12.1, 4.2 Hz, 1 H), 2.76 (d, J = 14.4, 7.3 Hz, 1 H), 2.49 (dt, J = 12.2, 5.4 Hz, 1 H), 2.30 (ddd, J = 14.6, 10.1, 4.2 Hz, 1 H), 1.99 (m, 1 H), 1.82 (m, 1 H), 1.74 (m, 1 H), 1.08 (s, 9 H), 0.92 (m, 2 H), 0.76 (m, 1 H), 0.47 (m, 1 H). ¹³C NMR (100 MHz, CDCl₃) δ 172.3, 171.3, 161.2, 157.4, 156.3, 140.4, 134.3, 130.2, 129.5, 119.5, 105.7, 98.9, 75.5, 75.2, 59.4, 58.1, 55.7, 55.6, 54.1, 52.3, 35.3, 35.0, 29.9, 28.0, 26.3,18.7, 18.3, 10.3. IPC HPLC conditions: Ascentis Express CI 8 column, 100 x 4.6 mm, 2.7micron; Column temperature of 40 °C; Flow rate of 1.8 mL/min; and Wavelength of 215 nm.

| Gradient: | min | CH₃CN | 0.1% H₃PO₄ |
|---|---|---|---|
| | 0 | 10 | 90 |
| | 6 | 95 | 5 |
| | 9 | 95 | 5 |
| | 9.1 | 10 | 90 |
| | | | |
| **Retention times:** | | min. | |
| **De-Boc quinoxaline 14** | | 2.3 | |
| **Alkyne quinoxaline acid 16** | | 3.3 | |
| **Alkyne macrocyclic ester 17** | | 5.7 | |

### Example 11: Preparation of Macrocyclic Ester 18

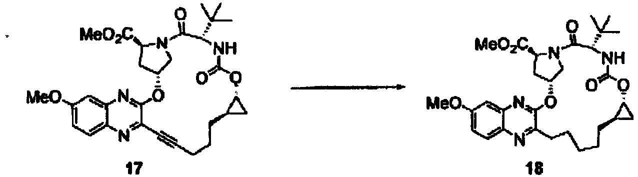

A mixture of alkyne macrocyclic ester 17 (10.0 g, 17.71 mmol) and 5% Pd/C 50%wet (3.5 g, 0.822 mmol) in THF (100 mL) was hydrogenated at ambient temperature under
40 psig of hydrogen for at least 10 hours. Upon reaction completion, the batch was filtered through Celite and the filtered catalyst was washed with THF (100 mL). The combined filtrate was solvent switched to IPA in vacuum at a final volume of-50 mL. The slurry was heated up to reflux for about 1 hour. The batch was then cooled to 50 °C and water (30 mL) was added dropwise over 1 hour. The batch was slowly cooled to below 0 °C over 2 hour and stirred at 0 °C for additional 1 hour before filtration. The wet cake was washed with a cold solution (0-5 °C) of 57% IPA in water (17.5 mL). Suction dry at ambient temperature gave 8.5 g of the desired macrocyclic ester 18.'H NMR (400 MHz, CDCl₃) δ 7.83 (d, J = 9.2 Hz, 1 H), 7.18 (dd, J - 9.2, 2.8 Hz, 1 H), 7.1 (d, J = 2.8 Hz, 1 H), 5.98 (t, J = 4.0 Hz, 1 H), 5.24 (d, J = 9.9 Hz, 1 H), 4.60 (dd, J = 10.7, 7.3 Hz, 1 H), 4.46 (d, J = 1 1.9 Hz, 1 H), 4.40 (d, J = 10.0 Hz,1 H), 4.01 (dd, J = 11.6, 4.0 Hz, 1 H), 3.93 (s, 3 H), 3.80 (m, 1 H), 3.75 (s, 3 H), 2.90 (ddd, J = 13.7, 1 1.5, 4.8 Hz, 1 H), 2.79 (ddd, J = 13.7, 12.1, 4.8 Hz, 1 H), 2.69 (dd, J = 14.2, 6.5 Hz, 1 H), 2.28 (ddd, J = 14.5, 10.7, 4.3 Hz, 1 H), 1.76 (m, 2 H), 1.66 (m, 2 H), 1.52 (m, 3 H), 1.09 (s, 9 H), 0.99 (m, 1 H), 092 (m, 1 H), 0.67 (m, 1 H), 0.46 (m, 1 H). ¹³C NMR (100 MHz, CDCl₃) δ 172.4, 171.5, 160.4, 157.5, 155.1, 148.7, 140.1, 134.6, 129.4, 118.7, 106.1, 74.4, 59.4, 58.2, 55.8, 55.5, 54.4, 52.5, 35.7, 35.2, 34.0, 30.9, 29.5, 28.6, 28.3, 26.5, 18.9, 11.2. IPC HPLC conditions: Ascentis Express CI 8 Column, 100 x 4.6 mm, 2.7micron; Column temperature or 40 °C; Flow rate or 1.8 mL/min; and Wavelength of 215 nm.

| Gradient: | min | CH₃CN | 0.1% H₃PO₄ |
|---|---|---|---|
| | 0 | 10 | 90 |
| | 6 | 95 | 5 |
| | 9 | 95 | 5 |
| | 9.1 | 10 | 90 |

| **Retention times:** | | | min. |
|---|---|---|---|
| **Alkyne macrocyclicester 17** | | | 5.7 |
| ***cis*-Alkene macrocyclic_ester (reaction intermediate)** | | | 6.0 |
| ***trans*-Alkene macrocyclic_ester (reaction intermediate)** | | | 6.1 |
| **Compound 18** | | | 6.2 |

### Example 12: Preparation of Macrocyclic Acid (19)

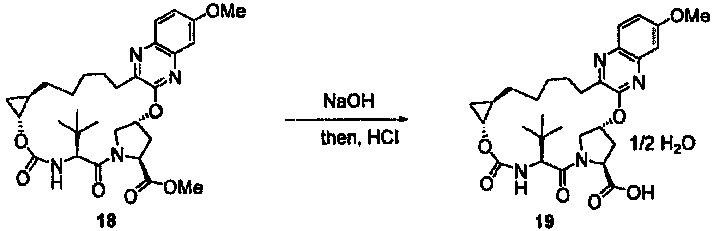

To a slurry of macrocyclic ester 18 (90 g, 158.3 mmol) in MeOH (720 mL) at ambient temperature was added 2 M NaOH (237.4 mL, 475 mmol) dropwise. The reaction mixture was aged at 50 °C for 2-3 hours. The reaction solution was cooled to 35-00 °C and 5 N HCl in 50% aq MeOH (70 mL) was added dropwise. The batch was seeded with free acid hemihydrate 19 (-100 mg) and aged for 30 min to 1 hour at 40 °C. Additional 5 N HCl in 50% aq MeOH (30 mL) was added dropwise over 2-4 hours at 40 °C. The slurry was aged additional 1 hour before cooling to ambient temperature. The slurry was aged for additional 1 hour before filtration. The wet cake was washed with 65% MeOH in water (3x 270 mL, displacement wash, slurry wash and displacement wash). Suction dry at ambient temperature or vacuum oven dry with dry N₂ sweep at 60-80 °C gave 85.6 g of macrocyclic acid hemihydrate 19 as an off- white solid. 'H NMR (400 MHz, CDCl₃) δ 7.85 (d, J = 9.0 Hz, 1 H), 7.19 (dd, J = 9.0, 2.8 Hz, 1 H), 7.13 (d, J = 2.8 Hz, 1 H), 5.99 (t, J = 3.9 Hz, 1 H), 5.45 (d, J = 9.9 Hz, 1 H), 4.80 (s, br, 2 H, COOH, hemihydrate H₂O), 4.64 (dd, J = 10.4, 7.4 Hz, 1 H), 4.49 (d, J = 11.6 Hz, 1 H), 4.44 (d, J= 10.0 Hz, 1 H), 3.99 (dd, J = 11.7, 4.0 Hz, 1 H), 3.94 (s, 3 H), 3.81 (m, 1 H), 2.90 (ddd, J = 13.8, 11.8, 4.8, 1 H), 2.80 (ddd, J = 13.8, 11.8, 4.8 Hz, 1 H), 2.71 (dd, J = 14.3, 7.3, 1 H), 2.42 (ddd, J = 14.4, 10.6, 4.2 Hz, 1 H), 1.76 (m, 2 H), 1.66 (m, 2 H), 1.52 (m, 3 H), 1.07 (s, 9 H), 0.96 (m, 2 H), 0.67 (m, 1 H), 0.47 (m, I H). ¹³C NMR (100 MHz, CDCl₃) δ 174.5, 172.1, 160.5, 157.6, 155.1, 148.6, 141.0, 134.3, 129.1, 118.9, 106.1, 74.3, 59.6, 58.3, 55.6, 54.6, 35.6, 35.3, 33.7, 30.8, 29.4, 28.6, 283, 26.5, 18.9, 11.2. IPC HPLC conditions: Hypersil Gold PFP Column, 150 x 4.6mm, 3.0µm, Column temperature of 40 °C; Flow rate of 1.8 mL/min; and Wavelength of 215 nm

| **Gradient:** | **min** | **CH₃CN** | **0.1% H₃PO₄** |
|---|---|---|---|
| | 0 | 25 | 75 |
| | 12 | 80 | 20 |
| | 12.1 | 25 | 75 |
| | 14 | 25 | 75 |

| Retention times: | | min. | |
|---|---|---|---|
| Compound **18** | | 6.78 | |
| Compound **19** | | 5.41 | |

### Example 13: Preparation of Compound A

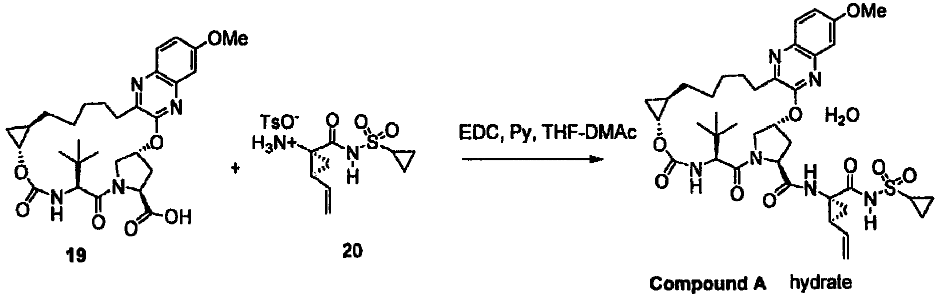

Macrocyclic acid hemihydrate 19 (10.16 g, 18.03 mmol) was dissolved in THF (50 - 90 mL). The solution was azetropically dried at a final volume of 100 mL. Sulfonamide pTSA salt 20 (7.98 g, 1.983 mmol) followed by DMAc (15 mL) was added at ambient temperature. The batch was cooled to 0-10 °C and pyridine (10 mL) was added dropwise. Then, EDC HCl (4.49 g, 23.44 mmol) was added in portions or one portion at 0-10 °C. The reaction mixture was aged at 0-10 °C for 1 hour, then warmed to 15-20 °C for 2-4 hours. MeOAc (100 mL) followed by 15wt% citirc acid in 5% NaCl in water (50 mL) was added, while the internal temperature was maintained to < 25 °C with external cooling. The separated organic phase was washed with 15wt% citirc acid in 5% NaCl in water (50 mL) followed by 5% NaCl (50 mL). The organic phase wassolvent switched to acetone at a final volume of -80 mL. Water (10 mL) was added dropwise at 35-40 °C. The batch was seeded with Compound A monohydrate form III (∼10 mg) and aged for 0.5 -1 hour at 35-40 °C. Additional water (22 mL) was added dropwise over 2-4 hours at 35-40 C. The slurry was aged at 20 °C for 2-4 hours before filtration. The wet cake was displacement washed with 60% acetone in water (40 mL x 2). Suction dry at ambient temperature gave Compound A monohydrate form III as a white solid. 1H NMR (400 MHz, CDCl₃) δ 9.95 (s, br, 1 H), 7.81 (d, J = 9.1 Hz, 1 H), 7.18 (dd, J = 9.1, 2.7 Hz, 1 H), 7.16 (s, br, 1 H), 7.13 (d, J - 2.7 Hz, 1 H), 5.96 (t, J = 3.8 Hz, 1 H), 5.72 (m, 1 H), 5.68 (d, J = 10.1 Hz, 1 H), 5.19 (d, J = 17.1 Hz, 1 H), 5.07 (d, J = 10.1 Hz, 1 H), 4.52 (d, J = 11.4 Hz, 1 H), 4.45 (d, J = 9.8 Hz, 1 H), 4.36 (d, J = 10.5, 6.9 Hz, 1 H), 4.05 (dd, J = 11.5, 3.9 Hz, 1 H), 3.93 (s, 3 H), 3.78 (m, 1 H), 2.90 (m, 1 H), 2.82 (tt, J = 8.0, 4.8 Hz, 1 H), 2.74 (dt, J = 13.2, 4.8 Hz, 1 H), 2.59 (dd, J = 14.0,6.7 Hz, 1 H), 2.40 (ddd, J =14.0, 10.6, 4.0 Hz, 1 H), 2.10 (dd, J = 17.7, 8.7 Hz, I H), 1.98 (2 H, mono hydrate H₂0), 1.88 (dd, J 8.2, 5.9 Hz, 1 HO, 1.74 (m, 3 H), 1.61 (m, 1 H), 1.50 (m, 3 H), 1.42 (dd, J = 9.6, 5.8 Hz, 1 H), 1.22 (m, 2 H), 1.07 (s, 9 H), 0.95 (m, 4 H), 0.69 (m, 1 H), 0.47 (m, 1 H). ¹³C NMR (100 MHz, CDCl₃) δ 173.5, 172.1, 169.1, 160.4, 157.7, 154.9, 148.4, 141.0, 134.3, 132.7, 129.1, 118.8, 118.7, 106.5, 74.4, 59.6, 59.4, 55.8, 55.5, 54.9, 41.8, 35.4, 35.3, 35.2, 34.3, 31.2, 30.7, 29.5, 28.6, 28.2, 26.6, 22.6, 18.7, 11.2, 6.31, 6.17. HPLC conditions: Ascentis Express Column, 10 cm x 4.6mm x 2.7µm; Column temperature of 40 °C; Flow rate of 1.8 mL/min; and Wavelength of 215 nm

| **Gradient:** | **min** | **CH₃CN** | **0.1% H₂PO₄** |
|---|---|---|---|
| | 0 | 20 | 80 |
| | 5 | 55 | 45 |
| | 15 | 55 | 45 |
| | 25 | 95 | 5 |
| | 27 | 95 | 5 |
| | 27.1 | 20 | 80 |
| | 32 | 20 | 80 |
| | | | |

| Retention times: | | min. | |
|---|---|---|---|
| Compound A | | 14.50 | |

### Example 14: Alternative Procedure for Making Compound A

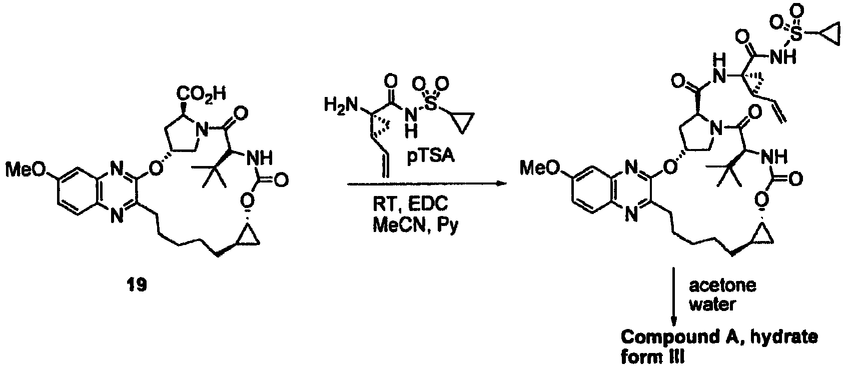

To a 50 L flask equipped with overhead stirring was added macrocyclic acid 19 (1.06 kg crude, 100eq), amine-pTSA (862 g crude, 1.12q) and MeCN 7.42 L at 19 °C. The slurry was cooled in a water bath, pyridine (2.12 L, 13.8eq) was added, aged 15 minutes, and then added EDC (586 g, 1.60eq) in one portion, aged 1.5 hours while it turned into a clear homogeneous solution. The solution cooled in a water bath, then quenched with 2 N HCl (1.7 L), seeded (9.2 g), aged 15 minutes, and the rest of the aqueous HCl was added over 2.5 hours. A yellow slurry was formed. The slurry was aged overnight at RT, filtered, washed with MeCN/water (1:1v/v) 8 L, to obtain Compound A (Hydrate II). Compound A was dissolved in acetone 4 L at RT, filtered and transferred to a 12 L RBF with overhead stirring, rinsed with extra acetone 1 L, heated to 50 °C, water 0.9 L was added, seeded 10 g, aged 15 minutes, then added water 0.8 L over 2.5 hours, extra water 3.3v over 2.5 hours was added, stopped heating, cooled to RT, aged at RT overnight, filtered, washed with water/acetone (1: 1 v/v) 4 L, and dried in air under vacuum. Compound A Hydrate III, 670 g, was obtained as an off-white solid.

### Example 15: Alternative Preparation of Macrocyclic Ester (18)

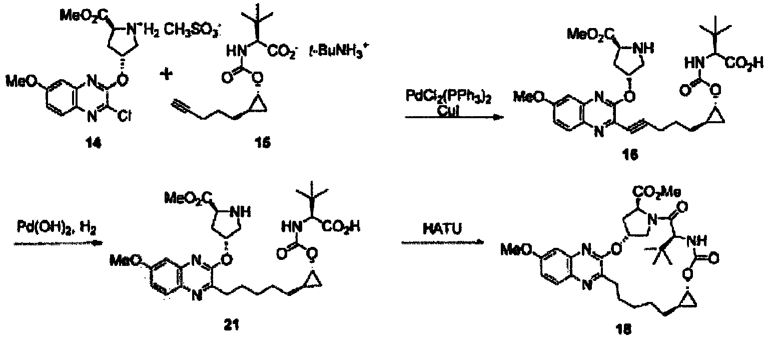

To a three-neck flask were added copper(I) iodide (0.020 g, 0.104 mmol), chloroquinoxaline MsOH salt 14 (4.5 g, 10.5 mmol), alkyne acid TBA salt 15 (4.4 g, 10.9 mmol), and bis(triphenylphosphine)palladium(II) dichloride (0.036 g, 0.052 mmol). The flask was vacuumed degassed with N₂. MeOH (45 ml) was added and the reaction mixture was vacuum degassed again with N₂. Triethylamine (2.89 ml, 20.7 mmol) was added. The reaction solution was aged at 35 °C for 3-5 hours. The batch was then concentrated to a volume of ∼9 mL in vacuum. THF (23 mL) and EtOAc (23 mL) were added. The reaction mixture was cooled to below 5 °C. HCl solution (1 N, ∼16 mL) was added slowly at below 5 °C until the reaction solution was pH adjusted to -2. NaCl aq. solution (10%, 32 mL) was added. The separated aqueous phase was back-extracted with a solution of THF (23 mL) and EtOAc (23 mL). The combined organic phase was washed with 10% NaCl aq. solution (45 mL). The solvent was switched to MeOH (75 mL) in vacuum at below 20 °C. To the reaction mixture was added DARCO KB-B (1.0 g), and the resulting suspension was stirred at 20 °C for I hour followed by filtration through Celite. The wet cake was washed with MeOH (25 mL). The combined filtrate was hydrogenated in the presence of Pearlman's catalyst (1.2 g, 20% Pd(OH)₂ on carbon, 50% wet) under 1 atmosphere of hydrogen at ambient temperature for at least 5 hours. Upon reaction completion, the suspension was filtered through Celite and the filtrate containing acid 21 was solvent switched to DMAc (65 mL). A solution of HATU (5.05 g, 13.3 mmol) in DMAc (65 mL) at ambient temperature was vacuumed degassed with N₂. The solution was cooled to 0 °C and DIPEA (5.4 mL, 30.9 mmol) was added dropwise at 0-5 °C. Then, the above solution of acid 21 (5.98 g assay, 10.2 mmol) in DMAc was added dropwsie over 10 hours, while maintaining the internal temperature at 0 °C. After addition, the batch was agitated at 0 °C for additional 2 hours to afford macrocyclic ester 18. The workup procedure and isolation of macrocyclic ester 18 were the same as described in Example 11.

### Example 16: Compound A Hydrate II

Hydrate II was prepared by adding the Compound A free base to a solution containing acetonitrile: water ratios with a water activity equal to or greater than 75% RH (relative humidity) to form an acetonitrile solvate and then drying at an elevated temperature. Hydrate II were characterized by different methods including X-ray powder diffraction, a thermogravimetric analysis, and differential scanning calorimetry curve TG. The X-ray powder diffraction patterns were generated on a Philips Analytical X'Pert PRO X-ray Diffraction System with PW3040/60 console. A PW3373/00 ceramic Cu LEF X-ray tube K-Alpha radiation was used as the source. DSC data were acquired using TA Instruments DSC 2910 or equivalent. Between 2 and 6 mg sample is weighed into a pan and covered. This pan is then crimped and placed at the sample position in the calorimeter cell. An empty pan is placed at the reference position. The calorimeter cell is closed and a flow of nitrogen is passed through the cell. The heating program is set to heat the sample at a heating rate of 10 °C/min to a temperature of approximately 250 °C. The heating program is started. When the run is completed, the data are analyzed using the DSC analysis program contained in the system software. The thermal events are integrated between baseline temperature points that are above and below the temperature range over which the thermal event is observed. The data reported are the onset temperature, peak temperature and enthalpy. TG data were acquired using a Perkin Elmer model TGA 7. Experiments were performed under a flow of nitrogen and using a heating rate of 10 °C/min to a maximum temperature of approximately 250 °C. After automatically taring the balance, 5 to 20 mg of sample is added to the platinum pan, the furnace is raised, and the heating program started. Weight/temperature data are collected automatically by the instrument. Analysis of the results are carried out by selecting the Delta Y function within the instrument software and choosing the temperatures between which the weight loss is to be calculated. Weight losses are reported up to the onset of decomposition/evaporation. Figure 1 illustrates a characteristic X-ray diffraction pattern of the crystalline Hydrate II of Compound A. Hydrate II exhibited characteristic reflections corresponding to d-spacings of:

**Table 1**

| d-spacing [Å] | 2 theta | Relative Intensity |
|---|---|---|
| 7.594 | 11.654 | 100.00 |
| 5.348 | 16.577 | 94.28 |
| 7.898 | 11.204 | 70.04 |
| 5.874 | 15.082 | 69.31 |
| 5.521 | 16.054 | 59.34 |
| 3.865 | 23.014 | 47.39 |
| 4.257 | 20.867 | 47.22 |
| 11.071 | 7.986 | 40.56 |
| 3.725 | 23.890 | 39.30 |
| 3.561 | 25.006 | 37.79 |
| 5.273 | 16.813 | 35.33 |
| 4.985 | 17.795 | 33.23 |
| 4.493 | 19.759 | 30.78 |
| 3.954 | 22.487 | 24.45 |
| 10.639 | 8.311 | 24.44 |

### Example 16: Hydrate III

Hydrate III was characterized by different methods including X-ray powder diffraction, a thermogravimetric analysis, differential scanning calorimetry curve TG, and solid-state carbon- 13 nuclear magnetic resonance (NMR) spectra. The X-ray powder diffraction patterns were generated on a Philips Analytical X'Pert PRO X-ray Diffraction System with PW3040/60 console. A PW3373/00 ceramic Cu LEF X-ray tube K-Alpha radiation was used as the source. DSC data were acquired using TA Instruments DSC 2910 or equivalent. Between 2 and 6 mg sample is weighed into a pan and covered. This pan is then crimped and placed at the sample position in the calorimeter cell. An empty pan is placed at the reference position. The calorimeter cell is closed and a flow of nitrogen is passed through the cell. The heating program is set to heat the sample at a heating rate of 10 °C/min to a temperature of approximately 250 °C. The heating program is started. When the run is completed, the data are analyzed using the DSC analysis program contained in the system software. The thermal events are integrated between baseline temperature points that are above and below the temperature range over which the thermal event is observed. The data reported are the onset temperature, peak temperature and enthalpy. TG data were acquired using a Perkin Elmer model TGA 7. Experiments were performed under a flow of nitrogen and using a heating rate of 10 °C/min to a maximum temperature of approximately 250 °C. After automatically taring the balance, 5 to 20 mg of sample is added to the platinum pan, the furnace is raised, and the heating program started. Weight/temperature data are collected automatically by the instrument. Analysis of the results is carried out by selecting the Delta Y function within the instrument software and choosing the temperatures between which the weight loss is to be calculated. Weight losses are reported up to the onset of decomposition/evaporation. The carbon- 13 spectrum was recorded on a Bruker AV400 NMR spectrometer using a Bruker 4 mm H/F/X BB double resonance CPMAS probe. The spectrum were collected utilizing proton/carbon- 13 variable-amplitude cross-polarization (VACP) at 80 kHz, with a contact time of 3 ms. Other experimental parameters used for data acquisition were a proton 90-degree pulse of 100 kHz, SPINAL64 decoupling at 100 kHz, a pulse delay of 2 s, and signal averaging for 26824 scans. The magic-angle spinning (MAS) rate was set to 13 kHz. A Lorentzian line broadening of 10 Hz was applied to the spectrum before Fourier Transformation. Chemical shifts are reported on the TMS scale using the carbonyl carbon of glycine (176.70 ppm) as a secondary reference. Figure 2 of WO2013/028465 is a characteristic X-ray diffraction pattern of the crystalline Hydrate III. The Hydrate III exhibited characteristic reflections corresponding to d-spacings of:

| **d-spacing [Å]** | **2 theta** | **Relative Intensity** |
|---|---|---|
| 4.338 | 20.474 | 100.00 |
| 17.575 | 5.028 | 47.92 |
| 4.887 | 18.154 | 26.30 |
| 4.428 | 20.052 | 19.59 |
| 4.294 | 20.684 | 18.61 |
| 6.298 | 14.063 | 11.59 |
| 3.760 | 23.660 | 11.52 |
| 6.764 | 13.089 | 11.45 |
| 4.703 | 18.871 | 11.39 |
| 5.793 | 15.295 | 9.70 |
| 4.252 | 20.890 | 9.66 |
| 7.776 | 11.380 | 9.16 |
| 4.811 | 18.442 | 7.81 |
| 6.016 | 14.726 | 7.01 |
| 6.405 | 13.825 | 6.31 |

Figure 3 of WO2013/028465 illustrates a typical thermogravimetric analysis curve of the crystalline Hydrate III. Figure 4 of WO2013/028465 illustrates a Differential scanning calorimetry curve of the crystalline Hydrate III. Figure 5 of WO2013/028465 illustrates a solid state C-13 CPMAS NMR for Compound A Hydrate III. Characteristic peaks for Hydrate III are observed at 5.14, 6.31, 12.49, 18.35, 26.81, 28.03, 30.33, 31.27, 34.95, 35.99, 38.68, 42.01, 54.93, 56.39, 60.14, 7420, 107.02, 120.11, 121.60, 129.73, 134.35, 135.95, 142.89, 148.47, 155.37, 157.32, 160.90, 168.32, 172.17, and 175.53 ppm.

### Example 18: Additional Hydrates

Hydrates I, IV, V, and VI were characterized by X-ray powder diffraction and Carbon- 13 NMR. The X-ray powder diffraction patterns were generated on a Philips Analytical X'Pert PRO X-ray Diffraction System with PW3040/60 console. A PW3373/00 ceramic Cu LEF X-ray tube - Alpha radiation was used as the source. Carbon- 13 spectrum was recorded on a Bruker AV400 NMR spectrometer using a Biuker 4 mm H/F/X BB double resonance CPMAS probe. The spectrum were collected utilizing proton/carbon- 13 variable-amplitude cross-polarization (VACP) at 80 kHz with a proton 90-degree pulse of 100 kHz, SPINAL64 decoupling at 100 kHz. A Lorentzian line broadening of 30 Hz was applied to the spectrum before Fourier Transformation. Chemical shifts are reported on the TMS scale using the carbonyl carbon of glycine (176.70 ppm.) as a secondary reference. Other experimental parameters are outlined in each section below.

Hydrate I: Hydrate I was prepared by adding the free form to neat methanol, allowed to equilibrate for -24h at room temperature and air dried. Figure 8 of WO2013/028465 is a characteristic X-ray diffraction pattern of the crystalline Hydrate Form I of Compound I of the present invention. The hydrate Form I exhibited characteristic reflections corresponding to d-spacings of:

| **2 theta** | **d-spacing [Å]** |
|---|---|
| 8.6 | 10.27 |
| 20.6 | 4.30 |
| 26.6 | 3.35 |
| 17.4 | 5.09 |
| 16.6 | 5.34 |
| 12.2, | 7.25 |
| 21.2 | 4.20 |
| 18.8 | 4.71 |
| 15.0 | 5.92 |
| 23.0 | 3.86 |
| 14.1 | 6.28 |
| 16.9 | 5.26 |

Hydrate I was characterized based on its solid-state carbon- 13 nuclear magnetic resonance (NMR) spectrum. Other experimental parameters used for data acquisition were a pulse delay of 2 s, and signal averaging for 20480 scans. The magic-angle spinning (MAS) rate was set to 13 kHz and the temperature was set to 270 K. (Figure 19 of WO2013/028465) Characteristic peaks for Hydrate I were observed at 4.22, 7.23, 11.45, 17.79, 24.04, 26.95, 28.29, 31.15, 32.47, 32.47, 33.46, 34.03, 35.74, 42.32, 53.50, 56.05, 56.96, 77.49, 108.95, 119.65, 122.55, 131.05, 133.13, 135.38, 142.28, 150.78, 156.03, 157.99, 161.36, 171.40, 173.42, 174.30 ppm.

Hydrate IV: Hydrate IV was prepared by adding the potassium salt to a solution of 1: 1 Acetone:water with 1 equivalence of HCl and dried at RT. Figure 10 of WO2013/028465 is a characteristic X-ray diffraction pattern of the crystalline Hydrate IV. The Hydrate IV exhibited characteristic reflections corresponding to d-spacings of:

| **2 theta** | **d-spacing [Å]** |
|---|---|
| 14.7 | 6.04 |
| 11.5 | 7.66 |
| 7.1 | 12.38 |
| 9.3 | 9.46 |
| 15.6 | 5.68 |
| 7.7 | 11.41 |
| 8.0 | 10.99 |

Hydrate IV was characterized based on its solid-state carbon- 13 nuclear magnetic resonance (NMR) spectrum. Other experimental parameters used for data acquisition were a pulse delay of 2 s, and signal averaging for 1245 scans. The magic-angle spinning (MAS) rate was set to 13 kHz and the temperature was set to 275 K. (Figure 11 of WO2013/028465) Characteristic peaks for Hydrate IV are observed at 3.90, 5.30, 6.99, 10.49, 13.13, 17.81, 24.73, 27.52, 28.14, 29.42, 31.02, 32.80, 36.08, 39.22, 42.45, 53.62, 55.93, 59.14, 60.76, 74.77, 109.22, 111.19, 11.38, 120.24, 122.50, 133.96, 139.74, 1472, 148.90, 154.65, 158.25, 159.53, 160.12, 170.14, 171.05, 172.08, 173.47, and 174.46 ppm.

Hydrate V: Hydrate V was prepared by exposing Hydrate IV to relative humidity above 81%. Figure 12 of WO2013/028465 is a characteristic X-ray diffraction pattern of the crystalline Hydrate V. The Hydrate V exhibited characteristic reflections corresponding to d-spacings of:

| **2 theta** | **d-spacing [Å]** |
|---|---|
| 9.1 | 9.7 |
| 18.3 | 4.8 |
| 19.8 | 4.5 |
| 15.2 | 5.8 |
| 23.2 | 3.8 |
| 10.9 | 8.1 |
| 17.6 | 5.0 |
| 23.9 | 3.7 |

Hydrate V was characterized based on its solid-state carbon- 13 nuclear magnetic resonance (NMR) spectrum. (Figure 13 of WO2013/028465) The spectrum was collected utilizing proton/carbon-13 variable-amplitude cross-polarization (VACP) at 80 kHz, with a contact time of 2 ms. Other experimental parameters used for data acquisition a pulse delay of 3 s, and signal averaging for 3425 scans. The magic-angle spinning (MAS) rate was set to 13 kHz. Characteristic peaks for Hydrate V are observed at 7.86, 8.92, 13.10, 18.31, 23.72, 27.44, 28.47, 30.77, 35.79, 36.25, 37.15, 37.15, 42.95, 53.13, 55.67, 57.31, 60.47, 62.06, 75.09, 110.59, 112.24, 118.32, 132.18, 134.05, 135.83, 139.88, 148.30, 155.19, 157.97, 159.41, 170.31 and 175.20 ppm.

Hydrate VI: Hydrate VI was prepared by adding the free base to a 50/50 mixture of methanol/acetone and drying at room temperature. Figure 14 of WO2013/028465 is a characteristic X-ray diffraction pattern of the crystalline Hydrate VI. The Hydrate VI exhibited characteristic reflections corresponding to d-spacings of:

| **d-spacing [Å]** | **2 theta** |
|---|---|
| 4.3 | 20.5 |
| 6.9 | 12.8 |
| 4.6 | 19.4 |
| 4.2 | 21.2 |
| 5.3 | 16.8 |
| 6.4 | 13.9 |
| 17.9 | 5.0 |
| 4.8 | 18.5 |
| 3.8 | 23.7 |
| 3.3 | 26.8 |

Hydrate VI was characterized based on its solid-state carbon- 13 nuclear magnetic resonance (NMR) spectrum. (Figure 15 of WO2013/028465) The spectrum was collected utilizing proton/carbon-13 variable-amplitude cross-polarization (VACP) at 80 kHz, with a contact time of 3 ms. Other experimental parameters used for data acquisition were a pulse delay of 2 s, and signal averaging for 3425 scans. Characteristic peaks for Hydrate VI are observed at 4.87, 6.24, 11.70, 12.85, 18.36, 26.55, 28.31m 31.51, 34.98, 38.47, 42.09, 54.27, 56.12, 60.10, 73.49, 73.97, 105.91, 108.04, 118.39, 119.75, 121.33, 129.96, 133.87, 136.13, 142.26, 142.97, 146.85, 148.36, 154.97, 157.32, 160.71, 168.23, 172.21 and 175.34 ppm.

Relative stability in water of Hydrates I, II, and III showed predominantly Hydrate II after 12 hours and predominantly Hydrate III after 5 days. Relative stability of Hydrate II, HI and IV showed predominantly Hydrate III in acetone, water and mixtures thereof at 25 °C and 50 °C. The relative stability of the hydrate forms was determined by competitive slurry turnover experiments in Acetone:water at water activities ranging from 0.072 to 1 at room temperature. In all cases, the Hydrate form III was the resultant solids from these experiments indicating that this is the most stable of the hydrate forms in the solvents investigated.

### Example 19: Compound A K⁺ and Na⁺ Salt Production

Compound A K⁺ and Na⁺ salts were produced as follows:

### Preparation of Compound KNa-Salt

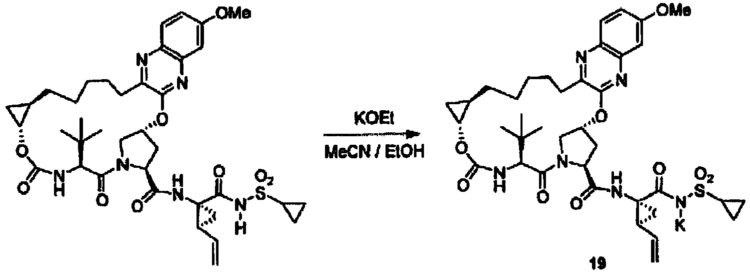

| **Compound A** | **Compound A potassium salt** | | | |
|---|---|---|---|---|
| **Materials** | **MW** | **Amount** | **Moles** | **Equiv** |
| Compound A free acid | 766.90 | 3.4 kg | 4.18 | 1.00 |
| KOEt in EtOH 24 wt % | 84.16 | 1.97 L | 5.02 | 1.20 |
| MeCN | | 30 L | | |
| EtOH | | 10 L | | |

To a 50 L jacketed cylindrical vessel equipped with a mechanical stirrer, thermocouple and nitrogen inlet containing 30 L 2: 1 MeCN:EtOH was added the 3.3 kg Compound A free acid. This was then transferred through an in-line filter to a 72 L RBF equipped with a mechanical stirrer, thermocouple and nitrogen inlet. To this solution was added over 1 hour the KOEt in EtOH. The solution was seeded with the form II after addition of 20 % of the KOEt in EtOH. The resulting slurry was stirred for 3 hour at ambient temperature and then filtered. The suspension was then filtered, washed with 2 x 10 L of MeCN and dried under N₂/vacuum sweep to yield 3.40 kg of Compound A potassium salt (98.3 wt%, 98.1 LCAP, 99 % yield). HPLC Conditions: Zorbax Eclipse Plus CI 8 50 x 4.6 mm, 1.8 µπl, A-0.1% phosphoric acid, C=Acetonitrile: 10% to 95% C, 5 min; 95% C, 6 min; 10 %C, 6.1 min; 2 min post, 1.5 mL/min, 230 nm, 25 °C. Compound A 5.41 min

### Preparation of Compound A Na-Salt:

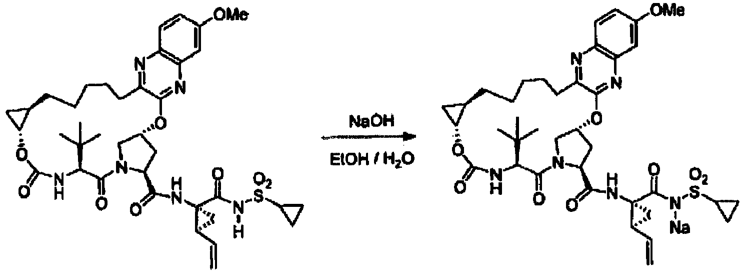

| **Materials** | **MW** | **Amount** | **mMol** | **Equiv** |
|---|---|---|---|---|
| Compound A free acid | 766.90 | 2 g | 2.61 | 1.00 |
| NaOH (2 M) | 40.00 | 1.30 mL | 2.61 | 1.00 |
| EtOH | | 40 mL | | |
| H₂O | | 0.2 mL | | |

In a 100 mL 2 necked round bottom flask under nitrogen, Compound A free acid was dissolved in 40 mL EtOH with 0.2 mL water added at 50 °C. To this was added slowly over 30 min the 1.30 mL 2 M sodium hydroxide solution, held at 50 °C until a solid was formed and then allowed to cool slowly to room temperature and age for 3 hours. The slurry was then cooled in an ice bath and the suspension was then filtered, washed with 2 x 10 mL of MeCN and dried under N₂/vacuum sweep to yield 2.01 g of Compound A sodium salt (98.3 LCAP, 99 % yield). HPLC Conditions: Zorbax Eclipse Plus CI 850 x 4.6 mm, 1.8 µηl, A=0.1% phosphoric acid, C=Acetonitrile: 10% to 95% C, 5 min; 95% C, 6 min; 10 %C, 6.1 min; 2 min post, 1.5 mL/min, 230 nm, 25 °C. Compound A 5.41 min

### Example 20: Compound A K⁺ and Na⁺ Salt Characterization

The X-ray powder diffraction patterns were generated on a Philips Analytical X'Pert PRO Xray Diffraction System with PW3040/60 console. A PW3373/00 ceramic Cu LEF X-ray tube K- Alpha radiation was used as the source. DSC data were acquired using TA Instruments DSC 2910 or equivalent. Between 2 and 6 mg sample is weighed into a pan and covered. This pan is then crimped and placed at the sample position in the calorimeter cell. An empty pan is placed at the reference position. The calorimeter cell is closed and a flow of nitrogen is passed through the cell. The heating program is set to heat the sample at a heating rate of 10 °C/min to a temperature greater than 300 °C. The heating program is started. When the run is completed, the data are analyzed using the DSC analysis program contained in the system software. The thermal events are integrated between baseline temperature points that are above and below the temperature range overwhich the thermal event is observed. The data reported are the onset temperature, peak temperature and enthalpy. TG data were acquired using a Perkin Elmer model TGA 7. Experiments were performed under a flow of nitrogen and using a heating rate of 10 °C/min to a maximum temperature greater than 300 °C. After automatically taring the balance, 5 to 20 mg of sample is added to the platinum pan, the furnace is raised, and the heating program started. Weight/temperature data are collected automatically by the instrument. Analysis of the results are carried out by selecting the Delta Y function within the instrument software and choosing the temperatures between which the weight loss is to be calculated. Weight losses are reported up to the onset of decomposition/evaporation.

Compound A Na-salt: Figure 6 of WO2013/028465 illustrates a characteristic X-ray diffraction pattern of the crystalline Na-salt of Compound A. The Na-salt exhibited characteristic reflections corresponding to d-spacings of:

| **d-spacing [Å]** | **Pos. [°2Th.]** | **Rcl. Int. [%]** |
|---|---|---|
| 4.8 | 18.4 | 82.2 |
| 9.7 | 9.1 | 59.5 |
| 9.1 | 9.8 | 49.0 |
| 9.3 | 9.6 | 25.1 |
| 4.6 | 19.3 | 25.1 |
| 5.8 | 15.3 | 23.6 |
| 5.4 | 16.5 | 20.2 |
| 4.0 | 22.5 | 18.5 |
| 5.1 | 17.4 | 17.9 |
| 4.4 | 20.2 | 16.6 |
| 10.6 | 8.4 | 16.4 |
| 4.2 | 21.3 | 16.0 |
| 3.3 | 26.9 | 15.0 |
| 18.3 | 4.8 | 14.8 |
| 3.4 | 26.2 | 14.7 |

Compound A K-salt: Figure 11 of WO2013/028465 illustrates a characteristic X-ray diffraction pattern of the crystalline K-salt of Compound A. The K-salt exhibited characteristic reflections corresponding to d-spacings of:

| **d-spacing [Å]** | **Pos. [°2Th.]** | **Rel. Int. [%]** |
|---|---|---|
| 4.9 | 18.2 | 100.0 |
| 10.0 | 8.9 | 49.1 |
| 4.4 | 20.3 | 32.3 |
| 4.7 | 18.7 | 27.9 |
| 4.0 | 22.5 | 27.2 |
| 10.5 | 8.4 | 24.7 |
| 4.5 | 19.6 | 22.6 |
| 5.3 | 16.7 | 20.6 |
| 3.3 | 27.1 | 19.2 |
| 8.6 | 10.3 | 17.9 |
| 4.1 | 21.9 | 17.9 |
| 9.4 | 9.4 | 16.4 |
| 4.2 | 21.2 | 15.6 |
| 3.4 | 25.9 | 15.5 |
| 7.1 | 12.5 | 15.1 |

Thus, aspect (iv) of the invention relates to an administration unit comprising crystalline and/or hydrated form of compound A. The administration unit according to the invention is useful for treating various diseases and disorders which include but are not limited to HCV in a patient.

### SPECIFIC INFORMATION CONCERNING ASPECT (V) OF THE INVENTION

Aspect (v) of the invention concerns forms of {drug5}, especially to crystalline form I of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) hydrochloride salt Aspect (v) of the invention relates to a solid form of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) and pharmaceutically acceptable salts thereof, and prodrugs thereof ({drug5}), namely to crystalline form I of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) hydrochloride salt ({drug5a}), which is useful for treating disorders of cell proliferation, including cancers, and inflammatory disorders, in particular, the inhibition of the activity of NEDD8-activating enzyme. Crystalline form I of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) hydrochloride salt is described in WO2013/028832, which is incorporated by reference. {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) and pharmaceutically acceptable salts thereof, and prodrugs thereof is described by formula (herein referred to as "I-216"):

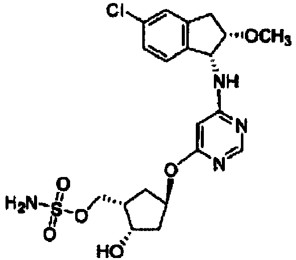

As used herein, crystalline form I of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) hydrochloride salt is a crystalline form of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclo-pentyl}methyl sulfamate (I-216) and pharmaceutically acceptable salts thereof, and prodrugs thereof characterized by / can be synthesized by methods as described in WO2013/028832 and reiterated hereinafter:

Unless otherwise specified, when a crystalline form of the hydrochloride salt is identified using one or more XRPD peaks given as angles 2Θ, each of the 2Θ values is understood to mean the given value ± 0.2 degrees. Throughout the specification and claims, when a crystalline form of the hydrochloride salt is identified using one or more temperatures from a DSC profile (e.g., onset of endothermic transition, melt, etc.), each of the temperature values is understood to mean the given value ± 2 °C.

SOLID STATE FORMS: Provided herein is an assortment of characterizing information to describe crystalline form 1 (Form 1) of the hydrochloride salt of 1-216. Figure 4 of WO2013/028832 shows an X-ray powder diffraction (XRPD) pattern of Form I of the hydrochloride salt of 1-216 obtained using CuKa radiation. Peaks identified in Figure 4 of WO2013/028832 include those listed in Table 4.

| **Angle 2-Theta °** | **Intensity %** |
|---|---|
| 4.491 | 63.1 |
| 7.506 | 58.9 |
| 8.89 | 24.7 |
| 9.847 | 41.6 |
| 13.274 | 29.2 |
| 14.418 | 59.1 |
| 14.613 | 58 |
| 15.176 | 100 |
| 15.874 | 58.1 |
| 17.012 | 16.7 |
| 17.205 | 42.1 |
| | |
| 17.847 | 28.4 |
| 18.241 | 32.2 |
| 18.49 | 21.7 |
| 19.177 | 33.9 |
| 19.454 | 53.6 |
| 20.045 | 35.4 |
| 21.31 | 67.6 |
| 21.771 | 83.5 |
| 22.206 | 34.2 |
| 22.35 | 55.6 |
| 22.707 | 58.9 |
| 23.045 | 27.4 |
| | |
| 23.528 | 34.4 |
| 24.032 | 69.3 |
| 24.803 | 53.6 |
| 25.654 | 41.1 |
| 26.407 | 40.9 |
| 26.694 | 34.5 |
| 26.932 | 33.1 |
| 27.978 | 17 |
| 28.36 | 16.7 |
| 29.066 | 21.4 |

In some embodiments, Form I is characterized by an XRPD pattern having peaks at 2Θ angles of 4.5°, 15.2°, 21.3°, 21.8° and 24.0°. In some embodiments, Form I is characterized by an XRPD pattern having peaks at 2Θ angles of 4.5°, 7.5°, 14.4°, 14.6°, 15.2°, 15.9°, 19.5°, 21.3°, 21.8°, 22.4°, 22.7°, 24.0° and 24.8°. In some embodiments, Form I is characterized by an XRPD pattern having peaks at 2Θ angles of 4.5°, 7.5°, 8.9°, 9.8°, 13.3°, 14.4°, 14.6°, 15.2°, 15.9°, 17.2°, 19.5°, 20.0°, 21.3°, 21.8°, 22.4°, 22.7°, 24.0°, 24.8°, 25.7° and 26.4°. In some embodiments, Form I is characterized by an XRPD pattern substantially as shown in Figure 4 of WO2013/028832. In some embodiments, Form I is characterized by an XRPD pattern having a reference peak with a 2Θ angle of 4.5 ± 0.3°, and having peaks at 2Θ angles of 10.7°, 16.8°, 17.3° and 19.5° relative to the reference peak. The term "reference peak" refers to a peak in the XRPD diffractogram that one skilled in the art considers as informing the polymorphic form of the material, i.e., differentiated from instrument noise. By "relative" it is meant that the observed 2Θ angle of each peak will be the sum of the 2Θ angle of the reference peak and the relative 2Θ angle of that peak. For example, if the reference peak has a 2Θ angle of 4.4°, the relative peaks will have 2Θ angles of 15.1°, 21.2°, 21.7° and 23.9°; if the reference peak has a 2Θ angle of 4.5°, the relative peaks will have 2Θ angles of 15.2°, 21.3°, 21.8° and 24.0°; if the reference peak has a 2Θ angle of 4.6°, the relative peaks will have 2Θ angles of 15.3°, 21.4°, 21.9° and 24.1°; etc. In some embodiments, Form I is characterized by an XRPD pattern having a reference peak with a 2Θ angle of 4.5 ± 0.3°, and having peaks at 2Θ angles of 3.0°, 9.9°, 10.1°, 10.7°, 11.4°, 15.0°, 16.8°, 1.3°, 17.9°, 18.2°, 19.5° and 20.3° relative to the reference peak. In some embodiments, Form I is characterized by an XRPD pattern having a reference peak with a 2Θ angle of 4.5 ± 0.3°, and having peaks at 2Θ angles of 3.0°, 4.4°, 5.3°, 8.8°, 9.9°, 10.1°, 10.7°, 11.4°, 12.7°, 15.0°, 15.5°, 16.8°, 17.3°, 17.9°, 18.2°, 19.5°, 20.3°, 21.2° and 21.9° relative to the reference peak. Any of the peaks that one skilled in the art considers as informing the polymorphic form of the material can serve as the reference peak and the relative peaks can then be calculated. For example, if the reference peak has a 2Θ angle of 24.0°, then the relative peaks will have 2Θ angles of -19.5°, -8.8°, -2.7° and -2.2° relative to the reference peak. Figure 5 of WO2013/028832 shows a differential scanning calorimetry (DSC) profile of Form **I.** The DSC thermogram plots the heat flow as a function of temperature from a sample, the temperature rate change being about 10 °C/min. In some embodiments, Form I is characterized by a DSC profile substantially as shown in Figure 5 of WO2013/028832. Figure 5 of WO2013/028832 shows an endotherm event with onset of about 129.8°C and peak at about 135.6°C corresponding to the loss of water coupled with melting. A broad endotherm with an onset of about 181.6°C and peak at about 195.5°C, and a sharp endotherm with an onset of about 275.3°C and peak at about 275.5°C are also observed. Figure 6 of WO2013/028832 shows a thermal gravimetric analysis (TGA) profile of Form I of the hydrochloride salt of 1-216. The TGA thermogram plots the percent loss of weight of the sample as a function of temperature, the temperature rate change being about 10 °C/min. Figure 6 of WO2013/028832 shows approximately 3.7 % weight loss between 100°C to 150°C, suggesting that 1-216 HCl Form I is a monohydrate. In some embodiments, 1-216 HCl Form I is characterized by a TGA profile substantially as shown in Figure 6 of WO2013/028832. Karl Fischer measurements show a water content of about 3.5 %, further suggesting that the loss of weight seen in the TGA profile is due to the loss of water, indicating Form I is a monohydrate.

Figure 10 of WO2013/028832 shows an X-ray powder diffraction (XRPD) pattern of Form II of the hydrochloride salt of 1-216 obtained using CuKa radiation. Peaks identified in Figure 10 of WO2013/028832 include those listed in Table 5.

| **Angle 2-Theta °** | **Intensity %** |
|---|---|
| 3.261 | 8.4 |
| 4.269 | 24.9 |
| 6.85 | 5.1 |
| 8.693 | 81.3 |
| 11.1 | 2.6 |
| 11.252 | 3.8 |
| 12.426 | 18.4 |
| 13.115 | 3.3 |
| 13.522 | 3.6 |
| 14.529 | 13 |
| 15.176 | 37.4 |
| 15.708 | 100 |
| 16.574 | 6.9 |
| 17.253 | 11.3 |
| 18.202 | 12 |
| 18.495 | 11.8 |
| 19.579 | 37.2 |
| 20.014 | 27.6 |
| 20.813 | 20.1 |
| 22.004 | 18.4 |
| 22.456 | 23 |
| 23.128 | 29 |
| 24.234 | 50.4 |
| 24.728 | 18 |
| 25.737 | 16.5 |
| 28.163 | 9.6 |
| 29.403 | 11 |

In some embodiments, Form II is characterized by an XRPD pattern having peaks at 2Θ angles of 8.7°, 15.2°, 15.7°, 19.6° and 24.2°. In some embodiments, Form II is characterized by an XRPD pattern having peaks at 2Θ angles of 4.3°, 8.7°, 15.2°, 15.7°, 19.6°, 20.0°, 20.8°, 22.5°, 23.1° and 24.2°. In some embodiments, Form II is characterized by an XRPD pattern having peaks at 2Θ angles of 4.3°, 8.7°, 12.4°, 14.5°, 15.2°, 15.7°, 17.3°, 18.2°, 18.5°, 19.6°, 20.0°, 20.8", 22.0°, 22.5°, 23.1°, 24.2°, 24.7°, 25.7°, 28.2° and 29.4°. In some embodiments, Form II is characterized by an XRPD pattern substantially as shown in Figure 10 of WO2013/028832. In some embodiments, Form II is characterized by an XRPD pattern having a reference peak with a 2Θ angle of 8.7 ± 0.3°, and having peaks at 2Θ angles of 6.5°, 7.0°, 10.9° and 15.5° relative to the reference peak. The terms "reference peak" and "relative" have the same meaning as previously described. In some embodiments, Form II is characterized by an XRPD pattern having a reference peak with a 2Θ angle of 8.7 ± 0.3°, and having peaks at 2Θ angles of -4.4°, 6.5°, 7.0°, 10.9°, 11.3°, 12.1°, 13.8°, 14.4°, and 15.5°, relative to the reference peak. In some embodiments, Form II is characterized by an XRPD pattern having a reference peak with a 2Θ angle of 8.7 ± 0.3°, and having peaks at 2Θ angles of -4.4°, 3.7°, 5.8°, 6.5°, 7.0°, 8.6°, 9.5°, 9.8°, 10.9°, 1 1.3°, 13.3°, 13.8°, 14.4°, 15.5°, 16.0°, 17.0°, 19.5° and 20.7° relative to the reference peak. Any of the peaks that one skilled in the art considers as informing the polymorphic form of the material can serve as the reference peak and the relative peaks can then be calculated. For example, if the reference peak has a 2Θ angle of 24.2°, then the relative peaks will have 2Θ angles of -15.5°, -9.0°, -8.5° and -4.6° relative to the reference peak.

SYNTHETIC METHODS: Compound 1-216 can be prepared by methods known to one skilled in the art and/or by reference to the schemes shown below and the examples that follow. Exemplary synthetic routes are disclosed of WO2013/028832 in in Schemes 1-4 below, and in the Examples below:

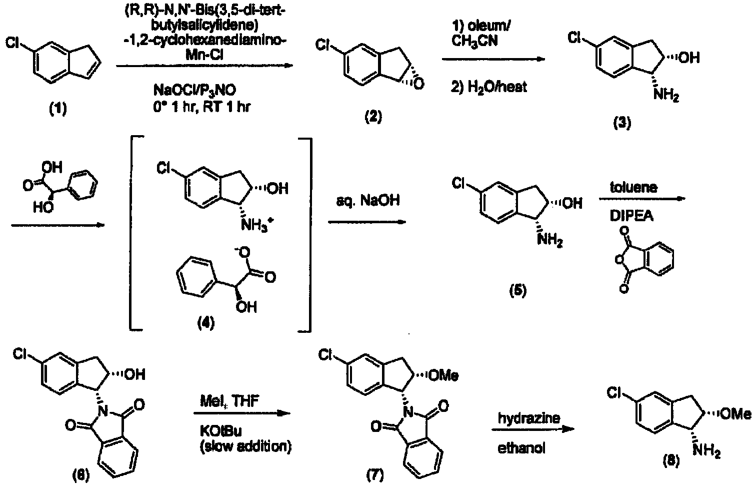

Scheme 1 describes the synthesis of (1R,2S)-5-chloro-2-methoxyindan-1-amine (8) which is further exemplified in Example 1 below. 6-chloro-1H-indene (1) was epoxidized using the Jacobsen catalyst to give oxirene (2) which was treated with fuming sulfuric acid in acetonitrile which led, after the addition of water and heating to rel-(1R,2S)-1-amino-5-chloroindan-2-ol (3). Rel-(1R,2S)-1-amino-5-chloroindan-2-ol (3) was chirally resolved using D-(-)-mandelic acid to give (1R,2S)-1-amino-5-chloroindan-2-ol (5) after removal of the chiral auxiliary. Protection of the primary amine in (5) was achieved using phthalic anhydride leading to compound (6). Methylation of the hydroxyl group with methyl iodide lead to compound (7), which was subsequently deprotected with hydrazine to give the desired (1R,2S)-5-chloro-2-methoxyindan-1-amine (8). 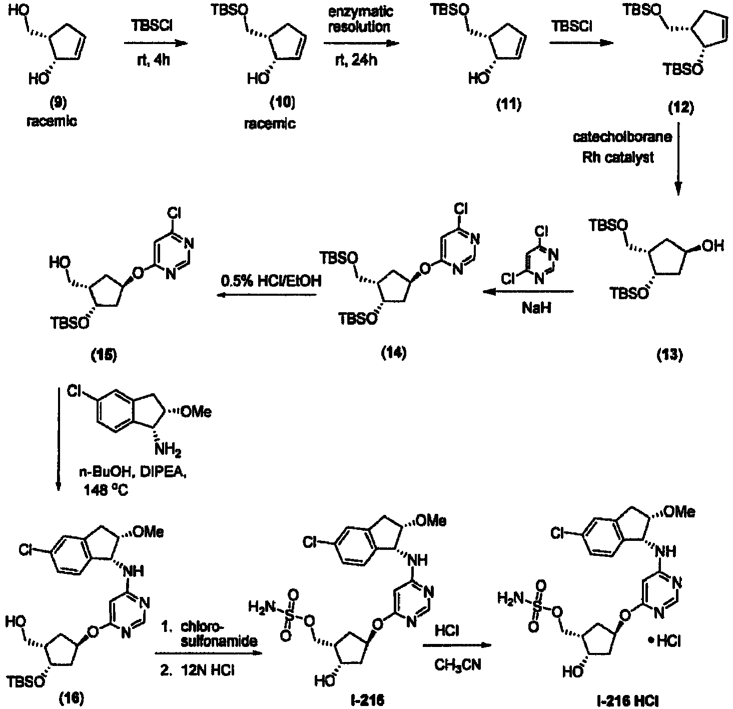 Scheme 2 shows the synthesis of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate HCl Form 1 which is further exemplified in Example 2 below. The primary alcohol of racemic-(9) was protected as the tert-butyldimethylsilyl ether to give compound (10) which was enzymatically resolved using Candida Antartica on acrylic resin to give compound (11) with an enantiomeric excess of greater than 99%. The secondary alcohol in (11) was then protected as its tert-butyldimethylsilyl ether to afford (12). Compound (12) was treated with catechol borane in the presence of Wilkinson's catalyst to afford (13) which was further reacted with 4,6- dichloropyrimidine to afford compound (14). The primary alcohol of (14) was selectively deprotected and then the indane portion of the molecule was installed by reaction of (15) with (1R,2S)-5-chloro-2-methoxyindan-1-amine (8) to afford compound (16). 1-216 was prepared by reacting compound (16) with chlorosulfonamide, followed by deprotection of the secondary alcohol under acidic conditions. 1-216 was treated with hydrochloric acid in acetonitrile to afford Form I of the hydrochloride salt of 1-216.

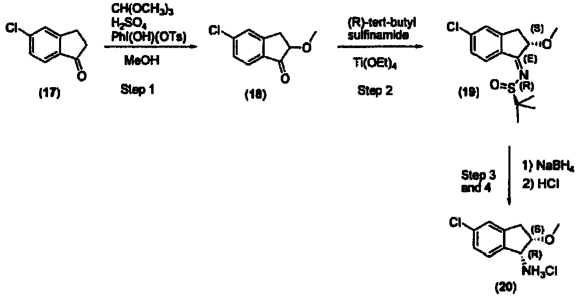

Scheme 3 describes the preparation of (1R,2S)-5-chloro-2-methoxy-2,3-dihydro-1H-inden-1-amine hydrochloride (20) which is further exemplified in Example 4 below. 5-chloro-2,3-dihydro-1H-inden-1-one (17) was reacted with trimethylorthoformate under acidic conditions, followed by treatment with Koser's reagent [Phl(OH)(OTs)] to give 5-chloro-2-methoxy-2,3-dihydro-1H-inden-1-one (18). The indenone was treated with (R)-tert-butyl sulfinamide in the presence of titanium tetraethoxide to afford the corresponding sulfinamide (19). The reaction was allowed to proceed until less than 5% of the undesired diastereoisomer could be detected by HPLC. The crude sulfinamide was reduced using NaBH₄ to afford the primary amine which was treated with hydrochloric acid to afford (1R,2S)-5-chloro-2-methoxy-2,3-dihydro-1H-inden-1-amine hydrochloride (20). 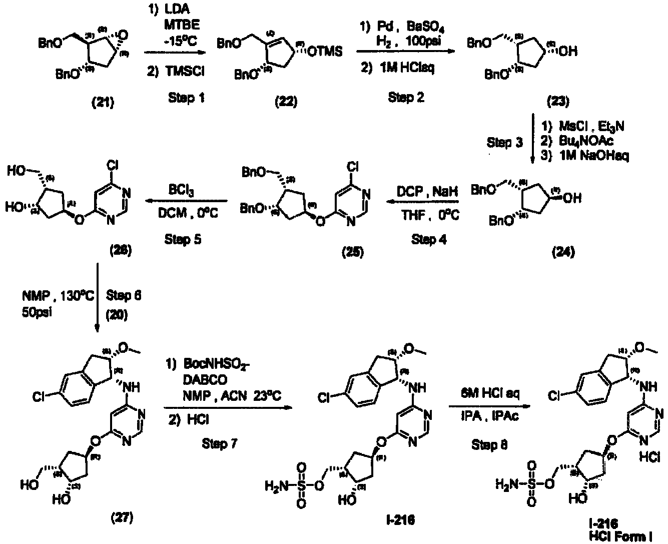 Scheme 4 shows a route for the preparation of 1-216 hydrochloride salt Form I which is further exemplified in Example 5 below. The epoxide in (1S,2R,3S,5R)-3-(benzyloxy)-2-(benzyloxymethyl)-6-oxabicyclo[3.1.0]hexane (21) was ring opened by treatment with lithium diisopropylamide and the resulting anion was trapped by treatment with trimethylsilylchloride to afford (22). The double bond was reduced using hydrogen and a Pd/BaSO₄ catalyst and the trimethylsilyl protecting group was removed to afford secondary alcohol (23). Secondary alcohol (23) was mesylated and then treated with tetrabutylammonium acetate followed by sodium hydroxide to afford (24) which was reacted with sodium hydride and 4,6-dichloropyrimidine to afford intermediate (25). Removal of the benzyl protecting groups using boron trichloride to afford (26) followed by reaction with (1R,2S)-5-chloro-2-methoxy-2,3-dihydro-1H-inden-1-amine hydrochloride (20) at 130 °C and 50 psi led to the formation of ((1S,2S,4R)-4-(6-((1R,2S)-5-chloro-2-methoxy-2,3-dihydro-1H-inden-1-ylamino)pyrimidin-4-yloxy)-2-hydroxycyclopentyl)methyl sulfamate (27). The primary alcohol in compound (27) was sulfamated to afford 1-216. Form I of the hydrochloride salt of 1-216 was generated by treatment of 1-216 in isopropyl alcohol with 6M hydrochloric acid followed by addition of isopropyl acetate as an anti-solvent.

*In vivo Tumor Pharmacodynamic Model:* HCT116 tumor cells (2x10<6>) (ATCC #CCL-247) in 100 µl phosphate buffered saline were aseptically injected into the subcutaneous space in the right dorsal flank of female Ncr nude mice (age 5-8 weeks, Charles River) using a 26-gauge needle. Beginning on day 7 after inoculation, tumors were measured twice weekly using a vernier caliper. Tumor volumes were calculated using standard procedures (0.5 x (length x width2)). When the tumors reached a volume of approximately 3-700mm3 mice were randomized into groups and injected subcutaneously with compound inhibitor (200 µl) at various doses. Tumors were harvested and crushed in Covaris bags and then transferred to glass tubes on dry ice for sonication in the Covaris E200. Mammalian protein extraction reagent (MPER) lysis buffer (Pierce, 78501) was supplemented with the following (final concentrations): 1x protease inhibitor cocktail set (Calbiochem, 539134), 5 mM o-phenanthroline in dimethyl sulfoxide (DMSO) (Sigma, #P1294 and Sigma DMSO #D2650), 10 mM iodoacetimide (Sigma), 2 mM sodium orthovanadate (Sigma, #56508), 25 mM sodium fluoride, and 25 mM [beta]-glycerophosphate. Cold lysis buffer (300-800 µl) was added to the tumors just before sonication. The sonication steps were: 10 seconds, 1 %500mV50, 20 seconds, 20%500mV50, 20 seconds, 10%500mV50. After sonication samples were placed on wet ice, poured into Eppendorf tubes and spun at 14000 rpm for 20 min at 4°C in a microfuge. Supernatants were transferred to new tubes and protein concentrations were determined using the Pierce bicinchoninic acid (BCA) reagents and protein standards. Tumor lysates were stored at -80°C. For quantitative analysis of neddylated cullins the procedure was as follows: 20 Mg of tumor lysate with lithium dodecyl sulfate (LDS) loading buffer and sample reducing agent (Invitrogen NP0007 and NP0004) was loaded onto 4-12% bis-tris gels, 1.5 mM, 10 well gels (Invitrogen NP0315Box). Gels were run at 150V in 2-(N-morpholino) ethane sulfonic acid (MES) running buffer (Invitrogen NP0002). Gels were cut at appropriate molecular weight marker and transferred to PVDF-FL (Millipore, IPFL00010) using a semi dry transfer apparatus (Amersham Biosciences, TE70). After transfer, membranes were blocked in Odyssey blocker (LI-COR Biosciences # 927-40000), then incubated with primary antibodies in Odyssey blocker + 0.1% Tween-20 (Sigma #P7949) overnight at 4 degrees. Membranes were washed three times in tris buffered saline with Tween-20 (TBST) and then incubated with Alexa Fluor 680 labeled goat anti-rabbit immunoglobulin G, heavy and light chain (IgG (H+L)) antibody (Molecular Probes Cat # A-21109). After 1 hour incubation with secondary antibody in the dark, membranes were washed 5 times with TBST and once with tris buffered saline (TBS), protected from light. Membranes were dried for at least one hour and then scanned with the Odyssey Infrared Imaging System (LI-COR Biosciences). The following primary antibody was used: Anti-Nedd-8 (IL10 clone 52-9-5, developed with Epitomics, dilution of 1 :4000). Secondary antibody was used at 1 :2000. Quantitation of signals on Western blots was performed with the Odyssey software.

Aspect (v) of the invention relates to an administration unit comprising crystalline form I of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) hydrochloride salt. The administration unit according to the invention is useful for treating various diseases and disorders which include but are not limited to disorders of cell proliferation, including cancers, and inflammatory disorders., in particular, the inhibition of the activity of NEDD8-activating enzyme.

### SPECIFIC INFORMATION CONCERNING ASPECT (VI) OF THE INVENTION

Aspect (vi) of the invention concerns forms of {drug6}, especially a solid form of rilapladib ({drug6}), namely to crystalline form of rilapladib ({drug6a}), which is useful for treating disorders mediated by Lp-PLA2 activity, therosclerosis, diabetes, hypertension, angina pectoris, rheumatoid arthritis, stroke, inflammatory conditions of the brain such as Alzheimer's Disease, myocardial infarction, ischaemia, reperfusion injury, sepsis. Crystalline form of rilapladib is described in WO2013/030374, which is incorporated by reference. Rilapladib can be described also as 2-[2-[(2,3-difluorophenyl)methylsulfanyl]-4-oxoquinolin-1-yl]-N-[1-(2-methoxyethyl)piperidin-4-yl]-N-[[4-[4-(trifluoromethyl)phenyl]phenyl]methyl]acetamide or N-[1-(2-methoxyethyl)-piperidin-4-yl]-2-[2-(2,3-difluorobenzylthio)-4-oxo-4H quinolin-1-yl]-N-(4'-trifluoromethylbiphenyl-4-yl-methyl)acetamide or by formula:

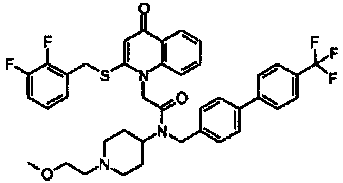

As used herein, crystalline form of rilapladib is a crystalline form of rilapladib characterized by I can be obtained by methods as described in WO2013030374 and reiterated hereinafter:

As one aspect, the present invention provides a crystalline form of rilapladib characterized by an X-ray powder diffraction ("XRPD") pattern substantially in accordance with Figure 1 of WO2013/030374. In another aspect, the present invention provides a crystalline form of rilapladib characterized by an XRPD pattern comprising diffraction angles (2 theta) at least at positions of about 6.2, 7.6, 9.1, 11.2, and 14.3 degrees. As another aspect, the present invention provides a crystalline form of rilapladib characterized by an XRPD pattern comprising diffraction angles (2 theta) at least at positions of about 6.2, 7.6, 9.1, 11.2, 11.7, 12.4, 13.1, 14.0, 14.3, 14.9, 15.3, 16.5, 16.8, 17.5, 17.8, 18.5, 18.9, 19.3, 20.0, 20.6, 21.1, and 22.1 degrees. As another aspect, the present invention provides a crystalline form of rilapladib characterized by a Raman spectrum substantially in accordance with Figure 2 of WO2013/030374. As another aspect, the present invention provides a crystalline form of rilapladib characterized by a Raman spectrum comprising peaks at least at positions of about 103, 276, 752, 1155, 1336, 1623, and 3075 cm-1. As another aspect, the present invention provides a crystalline form of rilapladib characterized by a Raman spectrum comprising peaks at least at positions of about 3075, 2952, 1623, 1611, 1576, 1528, 1467, 1336, 1288, 1179, 1155, 808, and 103 cm-1. As a another aspect, the present invention provides a crystalline form of rilapladib characterized by a Raman spectrum comprising peaks at least at positions of about 103, 159, 186, 276, 519, 524, 613, 628, 694, 736, 752, 766, 776, 808, 820, 1038, 1155, 1179, 1288, 1336, 1467, 1528, 1576, 1611, 1623, 2933, 2952, and 3075 cm-1.

In another aspect, the present invention provides a form of crystalline rilapladib characterized by a melting point of from about 165°C to about 185°C, e.g., from about 170°C to about 180°C. In another aspect, the present invention provides a form of crystalline rilapladib characterized by infrared (IR) absorption spectrum (e.g., attenuated total reflectance infrared or "ATR-IR") substantially in accordance with Figure 7 of WO2013/030374. As another aspect, the present invention provides a form of crystalline rilapladib characterized by an IR (e.g. ATR-IR) absorption spectrum, comprising peaks at least at about wavenumber 2931, 1652, 1621, 1595, 1528, 1494, 1478 1423, 1403, 1327, 1317, 1286, 1237, 1204, 1187, 1166, 1140, 1109, 1066, 1024, 992, 969, 932, 865, 859, 813, 795, 767, 751, 708, and 693. In another aspect, the present invention provides a form of crystalline rilapladib characterized by one or more of (i.e., at least one of) the aforementioned XRPD patterns and one or more of the aforementioned Raman spectra. In another aspect, the present invention provides a form of crystalline rilapladib characterized by one or more of the aforementioned XRPD patterns, one or more of the aforementioned Raman spectra, and one or more of the aforementioned melting points. In another aspect, the present invention provides a form of crystalline rilapladib characterized by one or more of the aforementioned XRPD patterns, one or more of the aforementioned Raman spectra, one or more of the aforementioned melting points, and one or more of the aforementioned IR spectra. In another aspect, the present invention provides a form of crystalline rilapladib characterized by at least two properties, selected from an XRPD pattern, a Raman spectrum, a melting point, and an IR spectrum, wherein those properties are as defined according to any one of the aforementioned embodiments. As another aspect, the present invention provides a pharmaceutical composition comprising one or more of the above mentioned crystalline forms of rilapladib, referred to herein as Form 3, and one or more pharmaceutically acceptable carriers. In some embodiments, the pharmaceutical composition further comprises another form of rilapladib. In other aspects, the present invention provides: a method for the treatment of a disease or disorder mediated by Lp-PLA₂ in a subject (e.g. a mammal, e.g. a human) comprising administering to the subject an effective amount of rilapladib Form 3; rilapladib Form 3 for use in therapy; and the use of rilapladib Form 3 in the preparation of a medicament for the treatment of a disease or disorder mediated by Lp-PLA₂.

Processes for preparing rilapladib Form 3 of the invention are also within the ambit of this invention. In some embodiments, a process for preparing rilapladib Form 3 comprises: (a) forming a mixture of rilapladib in a solvent, (b) crystallizing rilapladib from the mixture, where the crystallized material comprises Form 3 rilapladib, and (c) isolating the crystallized rilapladib comprising Form 3 rilapladib. In some embodiments, the process further comprises seeding the mixture of rilapladib and solvent with rilapladib Form 3. In some embodiments, the process further comprises the step of drying the isolated rilapladib comprising Form 3 rilapladib. In step (a), forming a mixture of rilapladib and solvent, in some embodiments the rilapladib is a form other than Form 3, e.g., Form 1 rilapladib. In some embodiments, the rilapladib is Form 3 (for example, in a recrystallization of Form 3 previously prepared, including from another form such as Form 1). Suitable solvents include acetonitrile, THF(tetrahydrofuran), ethyl acetate, toluene, heptane, acetone, aqueous ethanol, MIBK, DiMAC, and mixtures thereof. In some particular embodiments, the solvent is or comprises MIBK or a mixture of MIBK and DiMAC. In some embodiments, the solvent is 1,4-dioxane,1-butanol, 1-propanol, acetone, acetone:water (1%), acetone:water (50%), cyclohexane, cyclohexanone, DEGDME (diethylene glycol), dimethylether, dimethyl carbonate, dioxane:water (1%), DMA (dimethyl acetamide), DMSO, EtOAc (ethyl acetate), EtOAccyclohexane (1:2), EtOActoluene (1:2), heptane, IPA (isopropyl alcohol), IPA:iPrOAc (1:2), IPA:water (1%), isopropyl ether (IPE), isopropyl acetate (iPrOAc), MeCN (acetonitrile), MeCN:water (1%), MEK (methyl ethyl ketone), MeOAc (methyl acetate), MeOH (methanol), MeOH:water (1%), MeOH:water (20%), MeOH:water (50%), MIBK, MIBK saturated with water, MIBK:DMA (8:1), NMP (methyl pyrrolidone), PEG(polyethylene glycol) 400,TBME (t-butyl methyl ether),THF:Water (1%), toluene, water or a combination thereof. In some embodiments, the rilapladib is dissolved in the solvent. In other embodiments, the rilapladib/solvent mixture is a slurry. The solvent and/or mixture may be heated, e.g., to facilitate dissolution. In some embodiments, the solvent and/or mixture are at ambient temperature, e.g., about 25°C. In other embodiments, the solvent and/or mixture are cooled, e.g., to facilitate crystallization, e.g. to a temperature below ambient. Step (b) of crystallizing rilapladib from the mixture may optionally comprise cooling the mixture, e.g., cooling from above ambient temperature, or cooling from ambient temperature. The process may involve heating and/or cooling, or temperature cycling of the solvent and/or mixture such as known in the art of crystallization and recrystallization. In various embodiments, Form 3 is recrystallized from one or more of the above mentioned solvents by preparing a slurry of rilapladib Form 3 in the solvent and temperature cycling, e.g., from about 0°C to about 40°C or 50°C, e.g., for about 2-3 days (for instance 48-60 hours). In some embodiments, the crystallization step is carried out over a period of at least about 12 hours, particularly e.g., at least about 15, 24, 48, 60, or 72 hours (including about 12, 15, 24, 48, 60 and 72 hours). Step (c) of isolating the crystallized rilapladib may be accomplished by conventional methods such as filtration. Drying of the isolated material may be accomplished by conventional methods, such as vacuum drying with our without heat. Aspect (vi) of the present invention encompasses combinations of the aforementioned process embodiments. Rilapladib is an inhibitor of the enzyme lipoprotein associated phospholipase A2 (Lp- PLA2) and as such is expected to be of use in therapy. Therefore, in one aspect the present invention provides a method of inhibiting Lp-PLA₂ in a subject in need thereof, comprising administering to the subject an effective amount of rilapladib Form 3. Therefore, in a further aspect the present invention provides rilapladib Form 3 for use in therapy. Diseases or disorders for which rilapladib Form 3 may be used include those disclosed in WO02/30904A1, WO08/140449, WO2008/141176, WO2012/080497, WO2012/037782, WO2012/075917, WO2012/076435 or in US Patent Application Publication Nos. US 2008/0279846 (published Nov 13, 2008), US2010/0239565 (published Sep 23, 2010), or US2008/0280829 (published Nov 13, 2008), all incorporated herein by reference in their entirety. In some embodiments, rilapladib Form 3 is used for treating a disease or disorder selected from atherosclerosis, diabetes, hypertension, angina pectoris, rheumatoid arthritis, stroke, inflammatory conditions of the brain such as Alzheimer's Disease, myocardial infarction, ischaemia, reperfusion injury, sepsis, acute inflammation, chronic inflammation, psoriasis, diabetic retinopathy, diabetic macular edema, vascular dementia, multiple sclerosis, and skin ulcers.

Aspect (vi) of the invention relates to an administration unit comprising crystalline form of rilapladib. The administration unit according to the invention is useful for treating various diseases and disorders which include but are not limited to disorders mediated by Lp-PLA2 activity, therosclerosis, diabetes, hypertension, angina pectoris, rheumatoid arthritis, stroke, inflammatory conditions of the brain such as Alzheimer's Disease, myocardial infarction, ischaemia, reperfusion injury, sepsis.

### SPECIFIC INFORMATION CONCERNING ASPECT (VII) OF THE INVENTION

Aspect (vii) of the invention concerns forms of {drug7}, especially a solid form of 6-chloro-3-[2-(1-ethyl-propylamino)-3,4-dioxo-cyclobut-1-enylamino]-2-hydroxy-N-methoxy-N-methyl-benzenesulfonamide choline. The invention relates to a solid form of 6-chloro-3-[2-(1-ethyl-propylamino)-3,4-dioxo-cyclobut-1-enylamino]-2-hydroxy-N-methoxy-N-methyl-benzenesulfonamide choline, namely to solid form of 6-chloro-3-[2-(1-ethyl-propylamino)-3,4-dioxo-cyclobut-1-enylamino]-2-hydroxy-N-methoxy-N-methyl-benzenesulfonamide choline, which is useful for treating conditions and diseases mediated by CXCR2, for example inflammatory, obstructive or allergic conditions and diseases, particularly chronic obstructive pulmonary airways disease (COPD), including chronic bronchitis or dyspnea, emphysema, asthma, BOS. Solid form of 6-chloro-3-[2-(1-ethyl-propylamino)-3,4-dioxo-cyclobut-1-enylamino]-2-hydroxy-N-methoxy-N-methyl-benzenesulfonamide choline is described in WO2013/030803, which is incorporated by reference. Cholin salt of 6-chloro-3-[2-(1-ethyl-propylamino)-3,4-dioxo-cyclobut-1-enylamino]-2-hydroxy-N-methoxy-N-methyl-benzenesulfonamide, namely 6-chloro-3-[2-(1-ethyl-propylamino)-3,4-dioxo-cyclobut-1-enylamino]-2-hydroxy-N-methoxy-N-methyl-benzenesulfonamide cholinecan can be described by formula II:

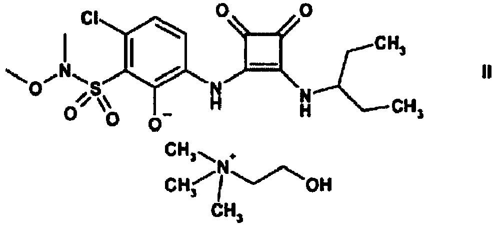

Preferred embodiments of aspect (vii) of the invention are defined in the claims of WO2013030803 and are reiterated hereinafter as items 1 to 23:
[Item 1]: Choline salt of 6-chloro-3-[2-(1-ethyl-propylamino)-3,4-dioxo-cyclobut-1-enylamino]-2-hydroxy-N-methoxy-N-methyl-benzenesulfonamide of formula II 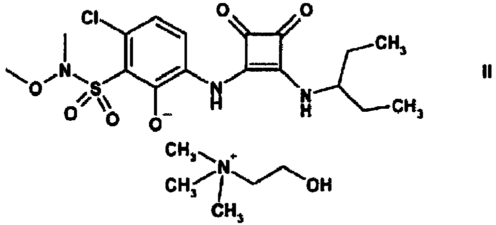
[Item 2]: A solid pharmaceutical composition comprising a) choline salt of 6-chloro-3-[2-(1-ethyl-propylamino)-3,4-dioxo-cyclobut-1-enylamino]-2-hydroxy-N-methoxy-N-methyl-benzenesulfonamide; b) an alkalizing agent; c) a precipitation inhibiting agent; and d) an additional pharmaceutically acceptable carrier. [Item 3]: The composition according to item 2, wherein the alkalizing agent is selected from magnesium oxide, calcium carbonate, calcium phosphate and combinations thereof. [Item 4]: The composition according to item 2 or 3, wherein the alkalizing agent is magnesium oxide. [Item 5]: The composition according to any one of items 2 to 4, wherein the precipitation inhibiting agent is selected from polyvinylpyrrolidone and hydroxypropyl methyl cellulose. [Item 6]: The composition according to any one of items 2 to 5, wherein precipitation inhibiting agent is polyvinylpyrrolidone. [Item 7]: The composition according to any one of items 2 to 6, wherein polyvinylpyrrolidone is selected from PVP K30, PVP K29/32 and PVPP XL, in particular PVP K30. [Item 8]: The composition according to any one of items 3 to 7, wherein the amount of alkalizing agent is in the range from about 5% to about 30% by weight of the total weight of solid pharmaceutical composition. [Item 9]: The composition according to any one of items 2 to 8, wherein the amount of alkalizing agent is in the range from about 10% to about 20% by weight of the total weight of solid pharmaceutical composition, in particular in the range of about 10% to about 15% by weight. [Item 10]: The composition according to any one of items 2 to 9, wherein the amount of precipitation inhibiting agent is in the range from about 8% to about 20% by weight of the total weight of solid pharmaceutical composition, in particular in the range of about 10% to about 15% by weight. [Item 11]: The composition according to any one of items 2 to 10, wherein the additional pharmaceutically acceptable carrier is selected from disintegrants, diluents, lubricants, glidants and binders. [Item 12]: The composition according to any one of items 2 to 1 1 comprising a) choline salt of 6-chloro-3-[2-(1-ethyl-propylamino)-3,4-dioxo-cyclobut-1-enylamino]-2-hydroxy-N-methoxy-N-methyl-benzenesulfonamide; b) magnesium oxide; c) polyvinylpyrrolidone; and d) a mixture of croscarmellose sodium and mannitol. [Item 13]: The composition according to item 12 further comprising microcrystalline cellulose, magnesium stearate, colloidal silicon dioxide, or a mixture of these. [Item 14]: An oral dosage form comprising the composition according to any one of items 2 to 13 and wherein the amount of the choline salt of 6-chloro-3-[2-(1-ethyl-propylamino)-3,4-dioxo-cyclobut-1-enylamino]-2-hydroxy-N-methoxy-N-methyl-benzenesulfonamide is from about 5 to about 100 mg on a dry basis calculated as the free acid. [Item 15]: An oral dosage form comprising the composition according to item 14 and wherein the amount of the choline salt of 6-chloro-3-[2-(1-ethyl-propylamino)-3,4-dioxo-cyclobut-1-enylamino]-2-hydroxy-N-methoxy-N-methyl-benzenesulfonami is about 75 mg on a dry basis calculated as the free acid. [Item 16]: An oral dosage form comprising the composition according to item 14 and wherein the amount of the choline salt of 6-chloro-3-[2-(1-ethyl-propylamino)-3,4-dioxo-cyclobut-1-enylamino]-2-hydroxy-N-methoxy-N-methyl-benzenesulfonamide is about 25 mg on a dry basis calculated as the free acid. [Item 17]: An oral dosage form comprising the composition according to item 14 and wherein the amount of the choline salt of 6-chloro-3-[2-(1-ethyl-propylamino)-3,4-dioxo-cyclobut-1-enylamino]-2-hydroxy-N-methoxy-N-methyl-benzenesulfonamide is about 5 mg on a dry basis calculated as the free acid. [Item 18]: The oral dosage form according to any one of items 14 to 17, wherein the dosage form is selected from a tablet, a capsule and a caplet. [Item 19]: The oral dosage form according to item 18, wherein the dosage from is a capsule, in particular a hard capsule. [Item 20]: The compound according to item 1, or the composition according to any one of items 2 to 13, or the oral dosage form according to any one of items 14 to 19, for use in the treatment of conditions and diseases mediated by CXCR2. [Item 21]: The compound, composition or oral dosage form according to item 20, for use in the treatment of inflammatory, obstructive or allergic conditions and diseases. [Item 22]: The compound, composition or oral dosage form according to item 20, for use in the treatment of chronic obstructive pulmonary airways disease (COPD), including chronic bronchitis or dyspnea associated therewith, emphysema, bronchiolitis obliterans syndrome and severe asthma. [Item 23]: A method of treating conditions and diseases mediated by CXCR2, for example inflammatory or allergic conditions and diseases, particularly chronic obstructive pulmonary airways disease (COPD), including chronic bronchitis or dyspnea associated therewith, emphysema, bronchiolitis obliterans syndrome and severe asthma, comprising administering to a person or mammal an effective amount of the compound according to item 1, or the composition according to any one of items 2 to 13, or the oral dosage form according to any one of items 14 to 19.

Characterization and Production of solid form of 6-chloro-3-[2-(1-ethyl-propylamino)-3,4-dioxo-cyclobut-1-enylamino]-2-hydroxy-N-methoxy-N-methyl-benzenesulfonamide choline is described in WO2013/030803 and reiterated hereinafter:

In a first aspect, the present invention relates to the choline salt of 6-chloro-3-[2-(1-ethyl-propylamino)-3,4-dioxo-cyclobut-1-enylamino]-2-hydroxy-N-methoxy-N-methyl-benzenesulfonamide of formula II

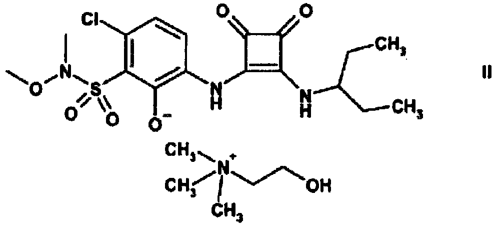

In this salt form 6-chloro-3-[2-(1-ethyl-propylamino)-3,4-dioxo-cyclobut-1-enylamino]-2-hydroxy-N-methoxy-N-methyl-benzenesulfonamide is surprisingly more soluble in simulated gastric fluid than the free acid disclosed in WO 2010/015613. In a second aspect, the invention relates to a solid pharmaceutical composition comprising a) choline salt of 6-chloro-3-[2-(1-ethyl-propylamino)-3,4-dioxo-cyclobut-1-enylamino]-2-hydroxy-N-methoxy-N-methyl-benzenesulfonamide; b) an alkalizing agent; c) a precipitation inhibiting agent; and d) an additional pharmaceutically acceptable carrier. As used herein, the term "alkalizing agent" is an agent capable of raising the pH of the micro-environment for 6-chloro-3-[2-(1-ethyl-propylamino)-3,4-dioxo-cyclobut-1-enylamino]-2-hydroxy-N-methoxy-N-methyl-benzenesulfonamide in the hydrated formulation to a pH greater than about its pKa (pKa = 5.4). One skilled in the art will appreciate that acidic agents can also be used to adjust the pH of the alkalizing agent as long as the alkalizing agent as a whole raises the pH of the micro-environment in the hydrated formulation to greater than about the pKa of 6-chloro-3-[2-(1-ethyl-propylamino)-3,4-dioxo-cyclobut-1-enylamino]-2-hydroxy-N-methoxy-N-methyl-benzenesulfonamide. Suitable alkalizing agents include but are not limited to organic and inorganic basic compounds of a wide range of aqueous solubilities and molecular weights and mixtures thereof. Examples of inorganic basic salts include ammonium hydroxide, alkali metal salts, alkaline earth metal salts such as magnesium oxide, magnesium hydroxide, calcium hydroxide, sodium hydroxide, potassium hydroxide, aluminium hydroxide, potassium carbonate, sodium bicarbonate and the like and mixtures thereof. In particular, an alkalizing agent is selected from magnesium oxide, calcium carbonate, calcium phosphate and combinations thereof. More particularly, the alkalizing agent is magnesium oxide. As used herein, the term "precipitation inhibiting agent" refers to salts, ions, carbohydrates, surfactants, amino acids, polymers and other compounds which, when present in solution, decrease the precipitation (crystallization) of 6-chloro-3-[2-(1-ethyl-propylamino)-3,4-dioxo-cyclobut-1-enylamino]-2-hydroxy-N-methoxy-N-methyl-benzenesulfonamide. Examples of precipitation inhibitors include but are not limited to polyvinyl pyrrolidone, for example those products known as PVP K30, PVP K29/32 and PVPP XL, and hydroxy propyl methyl cellulose (HPMC), for example those products known as PHARMACOAT® low viscous hypromellose-based water-soluble film coating agent. As used herein, the term "additional pharmaceutically acceptable carrier" refers to conventional pharmaceutical carriers, such as disintegrants, diluents, lubricants, glidants and binders which are known to a person skilled in the galenic art, and which are added to the composition in addition to the alkalizing agent(s) and the precipitation inhibiting agent(s). For example, known disintegrants such as croscarmellose sodium, sodium starch glycolate and crospovidone may be used as additional pharmaceutically acceptable carriers\. In particular, an additional pharmaceutically acceptable carrier is selected from disintegrants, diluents, lubricants, glidants and binders. More particularly, the pharmaceutically acceptable carrier is selected from croscarmellose sodium, mannitol, microcrystalline cellulose, magnesium stearate and colloidal silicon dioxide. As used herein, the term "oral dosage form" refers to physically discrete units suitable as unitary dosages for humans and other mammals, each unit containing a predetermined quantity of a therapeutic agent in association with one or more pharmaceutically acceptable carrier. Well-known oral dosage forms are for example tablets, capsules and caplets. Throughout this specification and in the claims that follow, unless the context requires otherwise, the word "comprise", or variations such as "comprises" or "comprising", should be understood to imply the inclusion of a stated integer or step or group of integers or steps but not the exclusion of any other integer or step or group of integers or steps. The entire disclosure of each [United States patent and] international patent application mentioned in this patent specification is fully incorporated by reference herein for all purposes.

Description of the drawing: Figure I of WO2013/030803: A is 6-chloro-3-[2-(1-ethyl-propylamino)-3,4-dioxo-cyclobut-1-enylamino]-2-hydroxy-N-methoxy-N-methyl-benzenesulfonamide; the concentration [ng/mL] relates to the mean concentrations of A (ng/mL, mean ± standard deviation) in plasma of male beagle dogs after adminstration of 63 mg/animal (calculated as free acid; n=5 dogs per treatment group) of A as capsules according to Example 3 (= Form 1), a solid dispersion formulation (= Form 2) and enteric coated beads (= Form 3).

In a first aspect the present invention (aspect (vii)) provides a novel salt of 6-chloro-3-[2-(1-ethyl-propylamino)-3,4-dioxo-cyclobut-1-enylamino]-2-hydroxy-N-methoxy-N-methyl-benzenesulfonamide, namely 6-chloro-3-[2-(1-ethyl-propylamino)-3,4-dioxo-cyclobut-1-enylamino]-2-hydroxy-N-methoxy-N-methyl-benzenesulfonamidecholine of formula II:

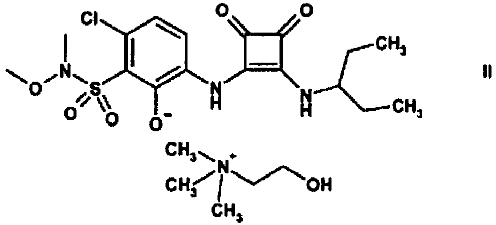

It was surprisingly found that the choline salt of 6-chloro-3-[2-(1-ethyl-propylamino)-3,4-dioxo-cyclobut-1-enylamino]-2-hydroxy-N-methoxy-N-methyl-benzenesulfonamide has superior solubility in simulated gastric fluid. Whereas the solubility of the free acid is 0.001 mg/ml in simulated gastric fluid at pH 1.2, the solubility of the choline salt is 8.018 mg/ml. Furthermore, the solubility of the choline salt in simulated gastric fluid is also significantly improved compared to other salts of 6-chloro-3-[2-(1-ethyl-propylamino)-3,4-dioxo-cyclobut-1-enylamino]-2-hydroxy-N-methoxy-N-methyl-benzenesulfonamide such as sodium, calcium, N,N-dimethyl-2-aminoethanol and N-(2-hydroxyethyl)pyrroiidine salts. Furthermore, the hygroscopicity of the choline salt is much reduced compared with the free acid. The amount of water absorbed by the free acid is 2.4% and 2.8% by weight after 1 day at 80% and 92% humidity, respectively, and the amount of water absorbed by the choline salt is 0.7% and 0.6% by weight after 1 day at 80% and 92% humidity, respectively. The choline salt has also a much improved dissolution rate at pH 1 and oral exposure when dosed at 10mg/kg to male SPRAGUE DAWLEY® rats compared to the free acid form. Although the choline salt of 6-chloro-3-[2-(1-ethyl-propylamino)-3,4-dioxo-cyclobut-1-enylamino]-2-hydroxy-N-methoxy-N-methyl-benzenesulfonamide has good solubility in gastric acidic conditions in the stomach, the choline salt of 6-chloro-3-[2-(1-ethyl-propylamino)-3,4-dioxo-cyclobut-1-enylamino]-2-hydroxy-N-methoxy-N-methyl-benzenesulfonamide readily converts in gastric acidic conditions into the free acid form which is poorly soluble. The result of this is poor bioavailability of 6-chloro-3-[2-(1-ethyl-propylamino)-3,4-dioxa-cyclobut-1-enylamino]-2-hydroxy-N-methoxy-N-methyl-benzenesulfonamide.

In a second aspect the present invention (aspect (vii)) provides a solid pharmaceutical composition of 6-chloro-3-[2-(1-ethyl-propylamino)-3,4-dioxo-cyclobut-1-enylamino]-2-hydroxy-N-methoxy-N-methyl-benzenesulfonamide choline. The solid pharmaceutical composition comprises a) choline salt of 6-chloro-3-[2-(1-ethyl-propylamino)-3,4-dioxo-cyclobut-1-enylamino]-2-hydroxy-N-methoxy-N-methyl-benzenesulfonamide; b) an alkalizing agent; c) a precipitation inhibiting agent; and d) an additional pharmaceutically acceptable carrier. It was found that this composition has an improved dissolution rate and an improved bioavailability as measured as AUC, mean Cₘₐx and lower inter-animal variability denoted by the %CV (see Example 7, Table 1, Figure 1 of WO2013/030803). The mean concentration of 6-chloro-3-[2-(1-ethyl-propylamino)-3,4-dioxo-cyclobut-1-enylamino]-2-hydroxy-N-methoxy-N-methyl-benzenesulfanamide in plasma of male beagle dogs after oral administration of similar doses is higher when a composition according to the second aspect is used than when special delivery formulations are used such as enteric coated beads formulation or solid dispersion formulation (see Example 7, Table 1, Figure 1 of WO2013/030803). In one embodiment (i) of the second aspect of the present invention, the alkalizing agent is selected from magnesium oxide, calcium carbonate, calcium phosphate and combinations thereof. Particularly, the alkalizing agent is magnesium oxide. In one embodiment (ii) of the second aspect or embodiment (i) of the second aspect, the precipitation inhibiting agent is selected from polyvinylpyrrolidone and hydroxypropyl methyl cellulose. Particularly, the precipitation inhibiting agent is polyvinylpyrrolidone. More particularly, polyvinylpyrrolidone is selected from products known as PVP K30, PVP K29/32 and PVPP XL, even more particularly PVP K30. In one embodiment (iii) of the second aspect or embodiment (i) or (ii) of the second aspect, the amount of alkalizing agent is in the range from about 5% to about 30% by weight of the total weight of solid pharmaceutical composition, particularly the amount of alkalizing agent is in the range from about 10% to about 20% by weight of the total weight of solid pharmaceutical composition, more particularly in the range from about 10% to about 15% by weight. In one embodiment (iv) of the second aspect or embodiments (i) to (iii) of the second aspect, the amount of precipitation inhibiting agent is in the range from about 8% to about 20% by weight of the total weight of solid pharmaceutical composition, particularly in the range from about 10% to about 15% by weight. In one embodiment (v) of the second aspect or embodiments (i) to (iv) of the second aspect, the additional pharmaceutically acceptable carrier is selected from disintegrants, diluents, lubricants, glidants and binders, or a mixture thereof. In one embodiment (vi) of the second aspect or embodiments (i) to (v) of the second aspect, the invention relates to a solid pharmaceutical composition comprising a) choline salt of 6-chloro-3-[2-(1-ethyl-propylamino)-3,4-dioxo-cyclobut-1-enylamino]-2-hydroxy-N-methoxy-N-methyl-benzenesulfonamide; b) magnesium oxide; c) polyvinylpyrrolidone; and d) a mixture of croscarmellose sodium and mannitol. In one particular embodiment (vii) of embodiment (vi) of the second aspect, the composition further comprises microcrystalline cellulose, magnesium stearate and colloidal silicon dioxide.

In a third aspect, the present invention (aspect (vii)) relates to an oral dosage form comprising the composition according to the second aspect or embodiments (i)-(vii) of the second aspect. In an embodiment (i) of the third aspect, the amount of the choline salt of 6-chloro-3-[2-(1-ethyl-propylamino)-3,4-dioxo-cyclobut-1-enylamino]-2-hydroxy-N-methoxy-N-methyl-benzenesulfonamide is from about 5 to about 100 mg on a dry basis calculated as the free acid. In an embodiment (ii) of the third aspect, the amount of the choline salt of 6-chloro-3-[2-(1-ethyl-propylamino)-3,4-dioxo-cyclobut-1-enylamino]-2-hydroxy-N-methoxy-N-methyl-benzenesulfonamide is about 75 mg on a dry basis calculated as the free acid. In another embodiment (iii) of the third aspect, the amount of the choline salt of 6-chloro-3-[2-(1-ethyl-propylamino)-3,4-dioxo-cyclobut-1-enylamino]-2-hydroxy-N-methoxy-N-methyl-benzenesulfonamide is about 25 mg on a dry basis calculated as the free acid. In another embodiment (iv) of the third aspect, the amount of the choline salt of 6-chloro-3-[2-(1-ethyl-propylamino)-3,4-dioxo-cyclobut-1-enylamino]-2-hydroxy-N-methoxy-N-methyl-benzenesulfonamide is about 5 mg on a dry basis calculated as the free acid. In a further embodiment (v) of the third aspect or embodiments (i)-(iv) of the third aspect, the dosage form is selected from a tablet, capsule and caplet, particularly a capsule. Such capsules may be prepared by a conventional blending process, for example in a diffusion blender and using appropriate sieving steps to enable a homogenous distribution of the ingredients. As a person skilled in the galenic art would know, the particle sizes of carriers should not differ too much from the particle size of the choline salt of 6-chloro-3-[2-(1-ethyl-propylamino)-3,4-dioxo-cyclobut-1-enylamino]-2-hydroxy-N-methoxy-N-methyl-benzenesulfonamide to avoid segregation during blending. The final blend can be filled manually or an automated capsule filling machine into hard gelatin, HPMC, starch or pullulan capsules.

In a fourth aspect, the present invention (aspect (vii)) relates to a composition or oral dosage form according to the second and third aspect, respectively, for use in medicine, particularly for use in the treatment of conditions and diseases mediated by CXCR2, for example inflammatory, obstructive or allergic conditions and diseases, particularly chronic obstructive pulmonary airways disease (COPD), including chronic bronchitis or dyspnea associated therewith, emphysema, bronchiolitis obliterans syndrome and severe asthma.

In a fifth aspect, the present invention (aspect (vii)) relates to a method of treating conditions and diseases mediated by CXCR2, for example inflammatory or allergic conditions and diseases, particularly chronic obstructive pulmonary airways disease (COPD), including chronic bronchitis or dyspnea associated therewith, emphysema, bronchiolitis obliterans syndrome and severe asthma, comprising administering to a person or mammal an effective amount of a composition or an oral dosage form according to the second and third aspect, respectively. Additional embodiments and features are set forth in part in the description that follows, and in part will become apparent to those skilled in the art upon examination of the specification or may be learned by the practice of the invention.

Aspect (vii) of the invention relates to an administration unit comprising solid form of 6-chloro-3-[2-(1-ethyl-propylamino)-3,4-dioxo-cyclobut-1-enylamino]-2-hydroxy-N-methoxy-N-methyl-benzenesulfonamide choline. The administration unit according to the invention is useful for treating various diseases and disorders which include but are not limited to conditions and diseases mediated by CXCR2, for example inflammatory, obstructive or allergic conditions and diseases, particularly chronic obstructive pulmonary airways disease (COPD), including chronic bronchitis or dyspnea, emphysema, asthma, BOS.

### SPECIFIC INORMATION CONCERNING ASPECT (VIII) OF THE INVENTION

Aspect (viii) of the invention concerns forms of {drug8}, especially an oral dosage form comprising: 1) a dispersion of a compound of formula (I) or a pharmaceutically acceptable salt thereof, in a hydrophilic polymer; and 2) a pharmaceutically acceptable carrier or excipient and / or solid form of an oral dosage form comprising: 1) a dispersion of a compound of formula (I) or a pharmaceutically acceptable salt thereof, in a hydrophilic polymer; and 2) a pharmaceutically acceptable carrier or excipient. The invention relates to a form of an oral dosage form comprising: 1) a dispersion of a compound of formula (I) or a pharmaceutically acceptable salt thereof, in a hydrophilic polymer; and 2) a pharmaceutically acceptable carrier or excipient, namely to solid form of an oral dosage form comprising: 1) a dispersion of a compound of formula (I) or a pharmaceutically acceptable salt thereof, in a hydrophilic polymer; and 2) a pharmaceutically acceptable carrier or excipient, which is useful for treating cancer, pain, Macular Degeneration, a skin disease, a pulmonary disorder, an asbestos-related disorder, a parasitic disease, an immunodeficiency disorder, a CNS disorder, CNS injury, atherosclerosis, a sleep disorder, hemoglobinopathy, anemia. An oral dosage form comprising: 1) a dispersion of a compound of formula (I) or a pharmaceutically acceptable salt thereof, in a hydrophilic polymer; and 2) a pharmaceutically acceptable carrier or excipient and / or solid form of an oral dosage form comprising: 1) a dispersion of a compound of formula (I) or a pharmaceutically acceptable salt thereof, in a hydrophilic polymer; and 2) a pharmaceutically acceptable carrier or excipient is described in WO2013/040120, which is incorporated by reference.

The invention relates to an administration unit comprising an oral dosage form comprising: 1) a dispersion of a compound of formula (I) or a pharmaceutically acceptable salt thereof, in a hydrophilic polymer; and 2) a pharmaceutically acceptable carrier or excipient and / or solid form of an oral dosage form comprising: 1) a dispersion of a compound of formula (I) or a pharmaceutically acceptable salt thereof, in a hydrophilic polymer; and 2) a pharmaceutically acceptable carrier or excipient. The administration unit according to the invention is useful for treating various diseases and disorders which include but are not limited to cancer, pain, Macular Degeneration, a skin disease, a pulmonary disorder, an asbestos-related disorder, a parasitic disease, an immunodeficiency disorder, a CNS disorder, CNS injury, atherosclerosis, a sleep disorder, hemoglobinopathy, anemia.

Preferred embodiments of the administration unit are described in WO2013/040120 and are reiterated hereinafter:

Provided herein are formulations and dosage forms of cyclopropanecarboxylic acid {2-[(1S)-1-(3-ethoxy-4-methoxy-phenyl)-2-methanesulfonyl-ethyl]-3-oxo-2,3-dihydro-1H-isoindol-4-yl}-amide. Methods of using the formulations and dosage forms are also provided herein.

Drug substances are usually administered as part of a formulation in combination with one or more other agents that serve varied and specialized pharmaceutical functions. Dosage forms of various types may be made through selective use of pharmaceutical excipients. As pharmaceutical excipients have various functions and contribute to the pharmaceutical formulations in many different ways, e.g., solubilization, dilution, thickening, stabilization, preservation, coloring, flavoring, etc. The properties that are commonly considered when formulating an active drug substance include bioavailability, ease of manufacture, ease of administration, and stability of the dosage form. Due to the varying properties of the active drug substance to be formulated, dosage forms typically require pharmaceutical excipients that are uniquely tailored to the active drug substance in order to achieve advantageous physical and pharmaceutical properties. Cyclopropanecarboxylic acid {2-[(1S)-1-(3-ethoxy-4-methoxy-phenyl)-2-methanesulfonyl-ethyl]-3-oxo-2,3-dihydro-1H-isoindol-4-yl}-amide ("Compound A") is a novel compound with anti-inflammatory activity in clinical development for the treatment of a variety of chronic inflammatory conditions. Pharmacologically, Compound A blocks the degradation of cyclic adenosine monophosphate (cAMP) via inhibition of the phosphodiesterase type IV (PDE4) enzyme, resulting in an increase in cAMP in PDE4-expressing cells including monocytes, T cells, and neutrophils. Enzyme assay data using purified PDE4 enzyme from U937 human monocytic cells indicate that Compound A has a PDE4 IC₅₀ of 100 nM (50 ng/mL). Compound A and methods for its synthesis are described, e.g., in U.S. Patent Publication No. 2010/0129363, the disclosure of which is hereby incorporated by reference in its entirety. Compound A may also be prepared according to the process described in U.S. Patent Publication No. 2010/0168475, the disclosure of which is hereby incorporated in its entirety.

Due to its diversified pharmacological properties, Compound A is useful in treating, preventing, and/or managing various diseases or disorders. However, Compound A is poorly soluble, thus, a need exists as to dosage forms of Compound A having advantageous physical and pharmaceutical properties. It has been demonstrated in monkeys that delivering an amorphous solid dispersion using a capsule gave approximately 3.5 times the exposure compared to the crystalline form. A tablet formulation can disintegrate quickly, increasing the timeline for absorption. Polymers can help maintain the supersaturation, further increasing the timeline for absorption. A tablet formulation with low friability may also help decrease chipping and loss of active pharmaceutical ingredient during film coating. See, e.g., Puri V., Dantuluri A.K. and Bansal A.K, "Investigation of Atypical Dissolution Behavior of an Encapsulated Amorphous Solid Dispersion," Journal of Pharmaceutical Sciences (2011) 100(6):2460-8.

Provided herein are pharmaceutical dosage forms of cyclopropanecarboxylic acid {2-[(1S)-1-(3-ethoxy-4-methoxy-phenyl)-2-methanesulfonyl-ethyl]-3-oxo-2,3-dihydro-1H-isoindol-4-yl}-amide ("Compound A"), or a pharmaceutically acceptable prodrug, salt, solvate, hydrate, or clathrate thereof. Also provided herein are methods of treating, managing, or preventing diseases and conditions such as, but not limited to, cancer, pain, Macular Degeneration, a skin disease, a pulmonary disorder, an asbestos-related disorder, a parasitic disease, an immunodeficiency disorder, a CNS disorder, CNS injury, atherosclerosis, a sleep disorder, hemoglobinopathy, anemia, an inflammatory disease, an autoimmune disease, a viral disease, a genetic disease, an allergic disease, a bacterial disease, an ocular neovascular disease, a choroidal neovascular disease, a retina neovascular disease, and rubeosis, using Compound A, or a pharmaceutically acceptable stereoisomer, prodrug, salt, solvate, hydrate, or clathrate thereof, in the dosage forms described herein.
Figure 1 of WO2013/040120 shows a plot of tablet formulation stability hardness for an oral dosage form of Compound A.
Figure 2 of WO2013/040120 shows the tablet formulation dissolution profile for an oral dosage form of Compound A prepared using AI/AI packaging. Figure 3 of WO2013/040120 shows the tablet formulation dissolution profile for an oral dosage form of Compound A prepared using no protective packaging. Figure 4 of WO2013/040120 shows the tablet formulation dissolution profile for an oral dosage form of Compound A prepared using AI/AI packaging at initial, 3 month and 12 month time points.

As used herein, the term "Compound A" refers to enantiomerically pure cyclopropanecarboxylic acid {2-[(1S)-1-(3-ethoxy-4-methoxy-phenyl)-2-methanesulfonyl-ethyl]-3-oxo-2,3-dihydro-1H-isoindol-4-yl}-amide. Without being limited by theory, Compound A is believed to be (5)-N-(2-(1-(3-ethoxy-4-methoxyphenyl)-2-(methylsulfonyl)ethyl)-3-oxoisoindolin-4-yl)cyclopropanecarboxamide, which has the following structure:

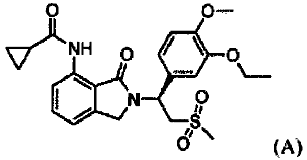

As used herein and unless otherwise indicated, a composition that is "substantially free" of a compound means that the composition contains less than about 20 percent by weight, more preferably less than about 10 percent by weight, even more preferably less than about 5 percent by weight, and most preferably less than about 3 percent by weight of the compound.

As used herein and unless otherwise indicated, the term "stereomerically pure" means a composition that comprises one stereoisomer of a compound and is substantially free of other stereoisomers of that compound. For example, a stereomerically pure composition of a compound having one chiral center will be substantially free of the opposite enantiomer of the compound. A stereomerically pure composition of a compound having two chiral centers will be substantially free of other diastereomers of the compound. A typical stereomerically pure compound comprises greater than about 80 percent by weight of one stereoisomer of the compound and less than about 20 percent by weight of other stereoisomers of the compound, more preferably greater than about 90 percent by weight of one stereoisomer of the compound and less than about 10 percent by weight of the other stereoisomers of the compound, even more preferably greater than about 95 percent by weight of one stereoisomer of the compound and less than about 5 percent by weight of the other stereoisomers of the compound, and most preferably greater than about 97 percent by weight of one stereoisomer of the compound and less than about 3 percent by weight of the other stereoisomers of the compound. As used herein and unless otherwise indicated, the term "enantiomerically pure" means a stereomerically pure composition of a compound having one chiral center. As used herein, unless otherwise specified, the term "pharmaceutically acceptable salt(s)," as used herein includes, but is not limited to, salts of acidic or basic moieties of thalidomide. Basic moieties are capable of forming a wide variety of salts with various inorganic and organic acids. The acids that can be used to prepare pharmaceutically acceptable acid addition salts of such basic compounds are those that form non-toxic acid addition salts, i.e., salts containing pharmacologically acceptable anions. Suitable organic acids include, but are not limited to, maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (i.e., 1,1'-methylene-bis-(2-hydroxy-3-naphthoate) acids. Suitable inorganic acids include, but are not limited to, hydrochloric, hydrobromic, hydroiodic, sulfuric, phosphoric, or nitric acids. Compounds that include an amine moiety can form pharmaceutically acceptable salts with various amino acids, in addition to the acids mentioned above. Chemical moieties that are acidic in nature are capable of forming base salts with various pharmacologically acceptable cations. Examples of such salts are alkali metal or alkaline earth metal salts and, particularly, calcium, magnesium, sodium, lithium, zinc, potassium, or iron salts. As used herein, and unless otherwise specified, the term "solvate" means a compound provided herein or a salt thereof, that further includes a stoichiometric or non-stoichiometric amount of solvent bound by non-covalent intermolecular forces. Where the solvent is water, the solvate is a hydrate. As used herein and unless otherwise indicated, the term "prodrug" means a derivative of a compound that can hydrolyze, oxidize, or otherwise react under biological conditions (in vitro or in vivo) to provide the compound. Examples of prodrugs include, but are not limited to, derivatives of thalidomide that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Other examples of prodrugs include derivatives of thalidomide that include -NO, -NO₂, -ONO, or -ONO₂ moieties. As used herein and unless otherwise indicated, the terms "biohydrolyzable carbamate," "biohydrolyzable carbonate," "biohydrolyzable ureide," "biohydrolyzable phosphate" mean a carbamate, carbonate, ureide, or phosphate, respectively, of a compound that either: 1) does not interfere with the biological activity of the compound but can confer upon that compound advantageous properties in vivo, such as uptake, duration of action, or onset of action; or 2) is biologically inactive but is converted in vivo to the biologically active compound. Examples of biohydrolyzable carbamates include, but are not limited to, lower alkylamines, substituted ethylenediamines, aminoacids, hydroxyalkylamines, heterocyclic and heteroaromatic amines, and polyether amines. As used herein and unless otherwise indicated, the term "biohydrolyzable ester" means an ester of a compound that either: 1) does not interfere with the biological activity of the compound but can confer upon that compound advantageous properties in vivo, such as uptake, duration of action, or onset of action; or 2) is biologically inactive but is converted in vivo to the biologically active compound. Examples of biohydrolyzable esters include, but are not limited to, lower alkyl esters, alkoxyacyloxy esters, alkyl acylamino alkyl esters, and choline esters. As used herein and unless otherwise indicated, the term "biohydrolyzable amide" means an amide of a compound that either: 1) does not interfere with the biological activity of the compound but can confer upon that compound advantageous properties in vivo, such as uptake, duration of action, or onset of action; or 2) is biologically inactive but is converted in vivo to the biologically active compound. Examples of biohydrolyzable amides include, but are not limited to, lower alkyl amides, a-amino acid amides, alkoxyacyl amides, and alkylaminoalkylcarbonyl amides. As used herein, and unless otherwise specified, the terms "treat," "treating" and "treatment" contemplate an action that occurs while a patient is suffering from the specified disease or disorder, which reduces the severity of the disease or disorder, or retards or slows the progression of the disease or disorder. As used herein, and unless otherwise specified, the terms "prevent," "preventing" and "prevention" refer to the prevention of the onset, recurrence or spread of a disease or disorder, or of one or more symptoms thereof. The terms "prevent," "preventing" and "prevention" contemplate an action that occurs before a patient begins to suffer from the specified disease or disorder, which inhibits or reduces the severity of the disease or disorder. As used herein, and unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder. As used herein, and unless otherwise specified, the term "about," when used in connection with doses, amounts, or weight percent of ingredients of a composition or a dosage form, means dose, amount, or weight percent that is recognized by those of ordinary skill in the art to provide a pharmacological effect equivalent to that obtained from the specified dose, amount, or weight percent is encompassed. Specifically, the term "about" contemplates a dose, amount, or weight percent within 30%, 25%, 20%, 15%, 10%), 5%o, 1%o, 0.5%), or 0.25%> of the specified dose, amount, or weight percent is encompassed. As used herein, and unless otherwise specified, the term "stable," when used in connection with a formulation or a dosage form, means that the active ingredient of the formulation or dosage form remains solubilized for a specified amount of time and does not significantly degrade or aggregate or become otherwise modified (e.g., as determined, for example, by HPLC).

Provided herein are pharmaceutical dosage forms of cyclopropanecarboxylic acid {2-[(1S)-1-(3-ethoxy-4-methoxy-phenyl)-2-methanesulfonyl-ethyl]-3-oxo-2,3-dihydro-1H-isoindol-4-yl}-amide ("Compound A"), or a pharmaceutically acceptable prodrug, salt, solvate, hydrate, or clathrate thereof. In some embodiments, the dosage forms are suitable for oral administration to a patient. In other embodiments, the dosage forms provided herein exhibit advantageous physical and/or pharmacological properties. Such properties include, but are not limited to, fast disintegration, low friability, ease of assay, content uniformity, flow properties for manufacture, dissolution and bioavailability, and/or stability. Also provided herein are kits comprising pharmaceutical compositions and dosage forms provided herein. Also provided herein are methods of treating, managing, and/or preventing a disease or condition, which comprises administering to a patient in need thereof a pharmaceutical composition or a dosage form provided herein.

Since it is contemplated in some embodiments that Compound A is delivered as an amorphous solid dispersion, increasing the timeframe for absorption is critical since the active pharmaceutical ingredient may become supersaturated in the gastrointestinal media. The tablet formulation can quickly disintegrate, decreasing the time it takes for the active pharmaceutical ingredient to go into solution and increasing the absorption time. Without being limited by a particular theory, combining a drug in a polymer with a higher glass transition temperature helps to stabilize the amorphous drug in a solid dispersion, which in turn helps to inhibit crystallization. However, a large concentration of polymer is often required to stabilize the amorphous drug. Therefore, tablets manufactured using amorphous solid dispersions are generally weak, have high friabilities, and may gel upon contact with an aqueous medium due to the high concentration of the polymer. Withouth being limited by a particular theory, the oral dosage forms provided herein can stabilize Compound A, which is a poorly soluble compound, in an amorphous solid dispersion using a hydrophilic polymer. In one embodiment, the oral dosage form is a tablet. In certain embodiments, the oral dosage forms provided herein comprise improved hardness and dissolution for tablets prepared using an amorphous solid dispersion. In certain embodiments, the oral dosage forms provided herein comprise tablets containing an amorphous solid dispersion of Compound A having fast disintegration and low friability. In certain embodiments, the amorphous solid dispersion comprises Compound A in a hydrophilic polymer such as hydroxypropyl methylcellulose (HMPC), polyvinylpyrrolidone (PVP), or hydroxypropyl cellulose (HPC), PVP VA64, hydroxypropyl methylcellulose acetate succinate, Eudragit polymers, polyvinylacetate, Polyox, Soluplus, or polyethylene glycol. In certain embodiments, the amorphous solid dispersion can be prepared by various methods such as lyophilization, spray drying, solvent casting, melt quenching, or hot melt extrusion. In certain embodiments, the oral dosage forms provided herein further comprise a diluent combination of different grades of microcrystalline cellulose and mannitol.

*Compositions and Dosage Forms:* Pharmaceutical compositions and formulations provided herein can be presented as discrete dosage forms, such as capsules (e.g., gelcaps), caplets, tablets, troches, lozenges, dispersions, and suppositories each containing a predetermined amount of an active ingredient as a powder or in granules, a solution, or a suspension in an aqueous or nonaqueous liquid, an oil-in- water emulsion, or a water-in-oil liquid emulsion. Because of their ease of administration, tablets, caplets, and capsules represent a preferred oral dosage unit forms. In some embodiments, the formulation is in the form of a tablet. In certain embodiments, provided herein is a solid dispersion of Compound A. In one embodiment, the dispersion comprises: 1) a dispersion of Compound A, or a pharmaceutically acceptable prodrug, salt, solvate, hydrate, or clathrate thereof; and 2) a hydrophilic polymer. In certain embodiments, the dispersion of the oral dosage form is an amorphous solid dispersion. In certain embodiments, the amorphous solid dispersion is prepared by hot melt extrusion, lyophilization, spray drying, solvent casting, or melt quenching. In some embodiments, the hydrophilic polymer of the dispersion is hydroxypropyl methylcellulose, polyvinylpyrrolidone, or hydroxypropyl cellulose. In one embodiment, the hydrophilic polymer is hydroxypropyl methylcellulose. In certain embodiments, the dispersion consists of from about 5% to about 40%, about 10%) to about 30%>, or about 15% to about 25% by weight of Compound A and from about 60% to about 95%, about 70% to about 90%, or about 75% to about 85% by weight of hydrophilic polymer. In some embodiments, said dispersion consists of about 15% by weight of Compound A and about 85% by weight of hydrophilic polymer. In one embodiment, provided herein are oral dosage forms comprising the solid dispersion of Compound A provided herein and one or more pharmaceutically acceptable carriers or excipients. In some embodiments, the carrier or excipient of the oral dosage form is selected from the group consisting of microcrystalline cellulose, mannitol, sodium croscarmellose, calcium stearate, crospovidone, polyvinyl alcohol, magnesium stearate, anhydrous lactose, silicon dioxide, fructose, hydroxypropyl methylcellulose, polyvinylpyrrolidone, hydroxypropyl cellulose, and combinations thereof. In certain embodiments, the oral dosage forms comprise the dispersion at an amount of from about 20% to about 75%, about 30% to about 70%, or about 33% to about 67% percent by weight of the total dosage form. In certain embodiments, the oral dosage form comprises the dispersion at an amount of about 33% by weight of the total dosage form. In certain embodiments, Compound A, or a pharmaceutically acceptable prodrug, salt, solvate, or clathrate thereof, comprises from about 5 to about 10 weight percent of total weight of the composition. In another embodiment, Compound A, or a pharmaceutically acceptable prodrug, salt, solvate, or clathrate thereof, comprises about 5 weight percent of total weight of the composition. In certain embodiments, the carrier or excipient is microcrystalline cellulose. In some embodiments, the dosage forms provided herein comprise microcrystalline cellulose at an amount of from about 5% to about 40%, about 10% to 30%, about 15% to 25%, or about 10% to 20% by weight of the total dosage form. In one embodiment, the microcrystalline cellulose is present at an amount of about 10%, about 15%, about 20%, about 25%o, about 30%>, about 35% or about 40%> by weight of the total dosage form. In one embodiment, the microcrystalline cellulose is present at an amount of about 25% by weight of the total dosage form. In another embodiment, the microcrystalline cellulose is present at an amount of about 24% by weight of the total dosage form. In certain embodiments, microcrystalline cellulose used in connection with the dosage forms provided herein is a mixture of a maximum-compactibility grade microcrystalline cellulose (e.g., Ceolus KG- 1000 having a greater length to diameter ratio), and a different grade microcrystalline cellulose having more spherical particle shape and optionally a bigger particle size (e.g., Avicel PH-102). In some embodiment, maximum-compactibility grade microcrystalline cellulose is present at an amount of from about 1%) to 10%), about 5% to about 15%, about 10%> to about 20%>, about 15% to about 25%, about 20% to about 30%, about 25% to about 35%, or about 30% to about 40% by weight of the total dosage form, and the remaining portion of the microcrystalline cellulose consists of microcrystalline cellulose of a lower compactibility grade. In certain embodiments, the carrier or excipient is mannitol. In some embodiments, the dosage forms provided herein comprise mannitol at an amount of about 20% to about 60%, about 25% to about 50%, about 30% to about 40%, or about 35% to about 40%) by weight of the total dosage form. In certain embodiments, the carrier or excipient is a disintegrant such as, but not limited to, croscarmellose sodium. In some embodiments, the dosage forms provided herein comprise croscarmellose sodium at an amount of from about 1% to about 20%, about 3%) to about 15%, about 3% to about 7%, or about 5% to about 10% by weight of the total dosage form. In certain embodiments, the carrier or excipient is a lubricant such as, but not limited to, magnesium stearate, calcium stearate, stearic acid, vegetable oil, mineral oil, PEG and SLS. In one embodiment, the lubricant is calcium stearate. In some embodiments, the dosage forms provided herein comprise calcium stearate at an amount of from about 0.1% to about 3%, about 0.3% to about 2%, about 0.5% to about 1.5%, or about 0.7%) to about 1% by weight of the total dosage form. In one embodiment, provided herein is an oral dosage form comprising an amorphous solid dispersion of Compound A; microcrystalline cellulose; mannitol; a disintegrant; and a lubricant. In one embodiment, disintegrant is croscarmellose sodium. In another embodiment, lubricant is calcium stearate. In one embodiment, provided herein is an oral dosage form consisting of about 5% by weight of Compound A and about 28% by weight of hydroxypropyl methyl cellulose (which together comprise the dispersion that may be an amorphous sold dispersion and which optionally may be prepared by hot melt extrusion); about 24% by weight of microcrystalline cellulose; about 36% by weight of mannitol; about 5% by weight of sodium croscarmellose; and about 1% by weight of calcium stearate or magnesium stearate. In one embodiment, about 10% by weight of the about 24% by weight of microcrystalline cellulose in said oral dosage form consists of a maximum-compactibility grade microcrystalline cellulose (e.g., Ceolus KG- 1000 a greater length to diameter ratio), whereas the remaining about 14% by weight of microcrystalline cellulose is of a different grade having more spherical particle shape and optionally a bigger particle size (e.g., Avicel PH-102). In some embodiments, because it is typical to obtain Compound A, or a pharmaceutically acceptable prodrug, salt, solvate, or clathrate thereof, at a purity of less than 100%, the formulations and dosage forms provided herein may be defined as compositions, formulations, or dosage forms that comprise Compound A, or a pharmaceutically acceptable prodrug, salt, solvate, or clathrate thereof, at an amount that provides the potency of a specified amount of 100% pure Compound A. In certain embodiments, provided herein are anhydrous pharmaceutical compositions and dosage forms including an active ingredient, since water can facilitate the degradation of some compounds. For example, the addition of water (e.g., 5 percent) is widely accepted in the pharmaceutical arts as a means of simulating shelf- life, i.e., long-term storage in order to determine characteristics such as shelf-life or the stability of formulations over time. See, e.g., Jens T. Carstensen, Drug Stability: Principles & Practice, 2d. Ed., Marcel Dekker, NY, NY, 1995, pp. 379-80. In effect, water and heat accelerate decomposition. Thus, the effect of water on a formulation can be of great significance since moisture and/or humidity are commonly encountered during manufacture, handling, packaging, storage, shipment, and use of formulations. An anhydrous pharmaceutical composition should be prepared and stored such that the anhydrous nature is maintained. Accordingly, in some embodiments, anhydrous compositions are packaged using materials known to prevent exposure to water such that they can be included in suitable formulary kits. Examples of suitable packaging include, but are not limited to, hermetically sealed foils, plastic or the like, unit dose containers, blister packs, and strip packs. In this regard, also provided herein is a method of preparing a solid pharmaceutical formulation including an active ingredient through admixing the active ingredient and an excipient under anhydrous or low moisture/humidity conditions, wherein the ingredients are substantially free of water. The method can further include packaging the anhydrous or non-hygroscopic solid formulation under low moisture conditions. By using such conditions, the risk of contact with water is reduced and the degradation of the active ingredient can be prevented or substantially reduced.

*Second Active Agents:* In certain embodiments, provided herein are compositions and dosage form of Compound A, or a pharmaceutically acceptable stereoisomer, prodrug, salt, solvate, or clathrate thereof, which may further comprise one or more secondary active ingredients. Certain combinations may work synergistically in the treatment of particular types diseases or disorders, and conditions and symptoms associated with such diseases or disorders. Compound A, or a pharmaceutically acceptable stereoisomer, prodrug, salt, solvate, or clathrate thereof, can also work to alleviate adverse effects associated with certain second active agents, and vice versa. Specific second active compounds that can be contained in the formulations and dosage forms provided herein vary depending on the specific indication to be treated, prevented or managed. For instance, for the treatment, prevention or management of cancer, second active agents include, but are not limited to: semaxanib; cyclosporin; etanercept; doxycycline; bortezomib; acivicin; aclarubicin; acodazole hydrochloride; acronine; adozelesin; aldesleukin; altretamine; ambomycin; ametantrone acetate; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other second agents include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein- 1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (Gleevec^{®}), imiquimod; immunostimulant peptides; insulin- like growth factor- 1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; Erbitux, human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; nilutamide; nisamycin; nitric oxide modulators; nitroxide antioxidant; nitrullyn; oblimersen (Genasense^{®}); 06-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perfiubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safmgol; saintopin; SarCNU; sarcophytol A; sargramostim; Sdi 1 mimetics; semustine; senescence derived inhibitor 1; sense oligonucleotides; signal transduction inhibitors; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; velaresol; veramine; verdins; verteporfm; vinorelbine; vinxaltine; vitaxin; vorozole; zanoterone; zeniplatin; zilascorb; and zinostatin stimalamer.

Yet other second active agents include, but are not limited to, 2-methoxyestradiol, telomestatin, inducers of apoptosis in mutiple myeloma cells (such as, for example, TRAIL), statins, semaxanib, cyclosporin, etanercept, doxycycline, bortezomib, oblimersen (Genasense^{®}), remicade, docetaxel, celecoxib, melphalan, dexamethasone (Decadron^{®}), steroids, gemcitabine, cisplatinum, temozolomide, etoposide, cyclophosphamide, temodar, carboplatin, procarbazine, gliadel, tamoxifen, topotecan, methotrexate, Arisa^{®}, taxol, taxotere, fluorouracil, leucovorin, irinotecan, xeloda, CPT-11, interferon alpha, pegylated interferon alpha (e.g., PEG INTRON-A), capecitabine, cisplatin, thiotepa, fludarabine, carboplatin, liposomal daunorubicin, cytarabine, doxetaxol, pacilitaxel, vinblastine, IL-2, GM-CSF, dacarbazine, vinorelbine, zoledronic acid, palmitronate, biaxin, busulphan, prednisone, bisphosphonate, arsenic trioxide, vincristine, doxorubicin (Doxil^{®}), paclitaxel, ganciclovir, adriamycin, estramustine sodium phosphate (Emcyt^{®}), sulindac, and etoposide.

In another embodiment, examples of specific second agents according to the indications to be treated, prevented, or managed can be found in the following references, all of which are incorporated herein in their entireties: U.S. patent nos. 6,281,230 and 5,635,517; U.S. publication nos. 2004/0220144, 2004/0190609, 2004/0087546, 2005/0203142, 2004/0091455, 2005/0100529, 2005/0214328, 2005/0239842, 2006/0154880, 2006/0122228, and 2005/0143344; and U.S. provisional application no. 60/631,870.

Examples of second active agents that may be used for the treatment, prevention and/or management of pain include, but are not limited to, conventional therapeutics used to treat or prevent pain such as antidepressants, anticonvulsants, antihypertensives, anxiolytics, calcium channel blockers, muscle relaxants, non-narcotic analgesics, opioid analgesics, antiinflammatories, cox-2 inhibitors, immunomodulatory agents, alpha-adrenergic receptor agonists or antagonists, immunosuppressive agents, corticosteroids, hyperbaric oxygen, ketamine, other anesthetic agents, NMDA antagonists, and other therapeutics found, for example, in the Physician 's Desk Reference 2003. Specific examples include, but are not limited to, salicylic acid acetate (Aspirin^{®}), celecoxib (Celebrex^{®}), Enbreh^{®}, ketamine, gabapentin (Neurontin^{®}), phenytoin (Dilantin^{®}), carbamazepine (Tegretol^{®}), oxcarbazepine (Trileptal^{®}), valproic acid (Depakene^{®}), morphine sulfate, hydromorphone, prednisone, griseofulvin, penthonium, alendronate, dyphenhydramide, guanethidine, ketorolac (Acular^{®}), thyrocalcitonin, dimethylsulfoxide (DMSO), clonidine (Catapress^{®}), bretylium, ketanserin, reserpine, droperidol, atropine, phentolamine, bupivacaine, lidocaine, acetaminophen, nortriptyline (Pamelor^{®}), amitriptyline (Elavil^{®}), imipramine (Tofranil^{®}), doxepin (Sinequan^{®}), clomipramine (Anafranil^{®}), fluoxetine (Prozac^{®}), sertraline (Zoloft^{®}), naproxen, nefazodone (Serzone^{®}), venlafaxine (Effexor^{®}), trazodone (Desyrel^{®}), bupropion (Wellbutrin^{®}), mexiletine, nifedipine, propranolol, tramadol, lamotrigine, vioxx, ziconotide, ketamine, dextromethorphan, benzodiazepines, baclofen, tizanidine and phenoxybenzamine.

Examples of second active agents that may be used for the treatment, prevention and/or management of macular degeneration and related syndromes include, but are not limited to, a steroid, a light sensitizer, an integrin, an antioxidant, an interferon, a xanthine derivative, a growth hormone, a neutrotrophic factor, a regulator of neovascularization, an anti-VEGF antibody, a prostaglandin, an antibiotic, a phytoestrogen, an anti-inflammatory compound or an antiangiogenesis compound, or a combination thereof. Specific examples include, but are not limited to, verteporfm, purlytin, an angiostatic steroid, rhuFab, interferon-2a, pentoxifylline, tin etiopurpurin, motexafin, lucentis, lutetium, 9-fluoro- 11,21-dihydroxy- 16, 17-1-methylethylidinebis(oxy)pregna- 1,4-diene-3,20-dione, latanoprost (see U.S. Patent No. 6,225,348), tetracycline and its derivatives, rifamycin and its derivatives, macrolides, metronidazole (U.S. Patent Nos. 6,218,369 and 6,015,803), genistein, genistin, 6'-0-Mal genistin, 6'-0-Ac genistin, daidzein, daidzin, 6'-0-Mal daidzin, 6'-0-Ac daidzin, glycitein, glycitin, 6'-0-Mal glycitin, biochanin A, formononetin (U.S. Patent No. 6,001,368), triamcinolone acetomide, dexamethasone (U.S. Patent No. 5,770,589), thalidomide, glutathione (U.S. Patent No. 5,632,984), basic fibroblast growth factor (bFGF), transforming growth factor b (TGF-b), brain-derived neurotrophic factor (BDNF), plasminogen activator factor type 2 (PAI-2), EYE101 (Eyetech Pharmaceuticals), LY333531 (Eli Lilly), Miravant, and RETISERT implant (Bausch & Lomb). All of the references cited herein are incorporated in their entireties by reference.

Examples of second active agents that may be used for the treatment, prevention and/or management of skin diseases include, but are not limited to, keratolytics, retinoids, a-hydroxy acids, antibiotics, collagen, botulinum toxin, interferon, steroids, and immunomodulatory agents. Specific examples include, but are not limited to, 5-fluorouracil, masoprocol, trichloroacetic acid, salicylic acid, lactic acid, ammonium lactate, urea, tretinoin, isotretinoin, antibiotics, collagen, botulinum toxin, interferon, corticosteroid, transretinoic acid and collagens such as human placental collagen, animal placental collagen, Dermalogen, AlloDerm, Fascia, Cymetra, Autologen, Zyderm, Zyplast, Resoplast, and Isolagen.

Examples of second active agents that may be used for the treatment, prevention and/or management of pulmonary hepertension and related disorders include, but are not limited to, anticoagulants, diuretics, cardiac glycosides, calcium channel blockers, vasodilators, prostacyclin analogues, endothelin antagonists, phosphodiesterase inhibitors (e.g., PDE V inhibitors), endopeptidase inhibitors, lipid lowering agents, thromboxane inhibitors, and other therapeutics known to reduce pulmonary artery pressure. Specific examples include, but are not limited to, warfarin (Coumadin®), a diuretic, a cardiac glycoside, digoxin-oxygen, diltiazem, nifedipine, a vasodilator such as prostacyclin (e.g., prostaglandin 12 (PGI2), epoprostenol (EPO, Floran®), treprostinil (Remodulin®), nitric oxide (NO), bosentan (Tracleer®), amlodipine, epoprostenol (Floran®), treprostinil (Remodulin®), prostacyclin, tadalafil (Cialis®), simvastatin (Zocor®), omapatrilat (Vanlev®), irbesartan (Avapra®), pravastatin (Pravachol®), digoxin, L-arginine, iloprost, betaprost, and sildenafil (Viagra®).

Examples of second active agents that may be used for the treatment, prevention and/or management of asbestos-related disorders include, but are not limited to, anthracycline, platinum, alkylating agent, oblimersen (Genasense®), cisplatinum, cyclophosphamide, temodar, carboplatin, procarbazine, gliadel, tamoxifen, topotecan, methotrexate, taxotere, irinotecan, capecitabine, cisplatin, thiotepa, fludarabine, carboplatin, liposomal daunorubicin, cytarabine, doxetaxol, pacilitaxel, vinblastine, IL-2, GM-CSF, dacarbazine, vinorelbine, zoledronic acid, palmitronate, biaxin, busulphan, prednisone, bisphosphonate, arsenic trioxide, vincristine, doxorubicin (Doxil®), paclitaxel, ganciclovir, adriamycin, bleomycin, hyaluronidase, mitomycin C, mepacrine, thiotepa, tetracycline and gemcitabine.

Examples of second active agents that may be used for the treatment, prevention and/or management of parasitic diseases include, but are not limited to, chloroquine, quinine, quinidine, pyrimethamine, sulfadiazine, doxycycline, clindamycin, mefloquine, halofantrine, primaquine, hydroxychloroquine, proguanil, atovaquone, azithromycin, suramin, pentamidine, melarsoprol, nifurtimox, benznidazole, amphotericin B, pentavalent antimony compounds (e.g., sodium stiboglucuronate), interfereon gamma, itraconazole, a combination of dead promastigotes and BCG, leucovorin, corticosteroids, sulfonamide, spiramycin, IgG (serology), trimethoprim, and sulfamethoxazole.

Examples of second active agents that may be used for the treatment, prevention and/or management of immunodeficiency disorders include, but are not limited to: antibiotics (therapeutic or prophylactic) such as, but not limited to, ampicillin, tetracycline, penicillin, cephalosporins, streptomycin, kanamycin, and erythromycin; antivirals such as, but not limited to, amantadine, rimantadine, acyclovir, and ribavirin; immunoglobulin; plasma; immunologic enhancing drugs such as, but not limited to, levami sole and isoprinosine; biologies such as, but not limited to, gammaglobulin, transfer factor, interleukins, and interferons; hormones such as, but not limited to, thymic; and other immunologic agents such as, but not limited to, B cell stimulators (e.g., BAFF/BlyS), cytokines (e.g., IL-2, IL-4, and IL-5), growth factors (e.g., TGF-a), antibodies (e.g., anti-CD40 and IgM), oligonucleotides containing unmethylated CpG motifs, and vaccines (e.g., viral and tumor peptide vaccines).

Examples of second active agents that may be used for the treatment, prevention and/or management of CNS disorders include, but are not limited to: opioids; a dopamine agonist or antagonist, such as, but not limited to, Levodopa, L-DOPA, cocaine, a-methyl-tyrosine, reserpine, tetrabenazine, benzotropine, pargyline, fenodolpam mesylate, cabergoline, pramipexole dihydrochloride, ropinorole, amantadine hydrochloride, selegiline hydrochloride, carbidopa, pergolide mesylate, Sinemet CR, and Symmetrel; a MAO inhibitor, such as, but not limited to, iproniazid, clorgyline, phenelzine and isocarboxazid; a COMT inhibitor, such as, but not limited to, tolcapone and entacapone; a cholinesterase inhibitor, such as, but not limited to, physostigmine saliclate, physostigmine sulfate, physostigmine bromide, meostigmine bromide, neostigmine methylsulfate, ambenonim chloride, edrophonium chloride, tacrine, pralidoxime chloride, obidoxime chloride, trimedoxime bromide, diacetyl monoxim, endrophonium, pyridostigmine, and demecarium; an anti-inflammatory agent, such as, but not limited to, naproxen sodium, diclofenac sodium, diclofenac potassium, celecoxib, sulindac, oxaprozin, diflunisal, etodolac, meloxicam, ibuprofen, ketoprofen, nabumetone, refecoxib, methotrexate, leflunomide, sulfasalazine, gold salts, Rho-D Immune Globulin, mycophenylate mofetil, cyclosporine, azathioprine, tacrolimus, basiliximab, daclizumab, salicylic acid, acetylsalicylic acid, methyl salicylate, diflunisal, salsalate, olsalazine, sulfasalazine, acetaminophen, indomethacin, sulindac, mefenamic acid, meclofenamate sodium, tolmetin, ketorolac, dichlofenac, flurbinprofen, oxaprozin, piroxicam, meloxicam, ampiroxicam, droxicam, pivoxicam, tenoxicam, phenylbutazone, oxyphenbutazone, antipyrine, aminopyrine, apazone, zileuton, aurothioglucose, gold sodium thiomalate, auranofm, methotrexate, colchicine, allopurinol, probenecid, sulfinpyrazone and benzbromarone or betamethasone and other glucocorticoids; and an antiemetic agent, such as, but not limited to, metoclopromide, domperidone, prochlorperazine, promethazine, chlorpromazine, trimethobenzamide, ondansetron, granisetron, hydroxyzine, acetylleucine monoethanolamine, alizapride, azasetron, benzquinamide, bietanautine, bromopride, buclizine, clebopride, cyclizine, dimenhydrinate, diphenidol, dolasetron, meclizine, methallatal, metopimazine, nabilone, oxyperndyl, pipamazine, scopolamine, sulpiride, tetrahydrocannabinol, thiethylperazine, thioproperazine, tropisetron, and a mixture thereof.

Examples of second active agents that may be used for the treatment, prevention and/or management of CNS injuries and related syndromes include, but are not limited to, immunomodulatory agents, immunosuppressive agents, antihypertensives, anticonvulsants, fibrinolytic agents, antiplatelet agents, antipsychotics, antidepressants, benzodiazepines, buspirone, amantadine, and other known or conventional agents used in patients with CNS injury/damage and related syndromes. Specific examples include, but are not limited to: steroids (e.g., glucocorticoids, such as, but not limited to, methylprednisalone, dexamethasone and betamethasone); an anti-inflammatory agent, including, but not limited to, naproxen sodium, diclofenac sodium, diclofenac potassium, celecoxib, sulindac, oxaprozin, diflunisal, etodolac, meloxicam, ibuprofen, ketoprofen, nabumetone, refecoxib, methotrexate, leflunomide, sulfasalazine, gold salts, RHo-D Immune Globulin, mycophenylate mofetil, cyclosporine, azathioprine, tacrolimus, basiliximab, daclizumab, salicylic acid, acetylsalicylic acid, methyl salicylate, diflunisal, salsalate, olsalazine, sulfasalazine, acetaminophen, indomethacin, sulindac, mefenamic acid, meclofenamate sodium, tolmetin, ketorolac, dichlofenac, flurbinprofen, oxaprozin, piroxicam, meloxicam, ampiroxicam, droxicam, pivoxicam, tenoxicam, phenylbutazone, oxyphenbutazone, antipyrine, aminopyrine, apazone, zileuton, aurothioglucose, gold sodium thiomalate, auranofm, methotrexate, colchicine, allopurinol, probenecid, sulfinpyrazone and benzbromarone; a cAMP analog including, but not limited to, db-cAMP; an agent comprising a methylphenidate drug, which comprises 1-threo-methylphenidate, d-threo-methylphenidate, dl-threo-methylphenidate, I-erythro-methylphenidate, d-erythro-methylphenidate, dl-erythro-methylphenidate, and a mixture thereof; and a diuretic agent such as, but not limited to, mannitol, furasemide, glycerol, and urea.

Examples of second active agent that may be used for the treatment, prevention and/or management of dysfunctional sleep and related syndromes include, but are not limited to, a tricyclic antidepressant agent, a selective serotonin reuptake inhibitor, an antiepileptic agent (gabapentin, pregabalin, carbamazepine, oxcarbazepine, levitiracetam, topiramate), an antiaryhthmic agent, a sodium channel blocking agent, a selective inflammatory mediator inhibitor, an opioid agent, a second immunomodulatory compound, a combination agent, and other known or conventional agents used in sleep therapy. Specific examples include, but are not limited to, Neurontin, oxycontin, morphine, topiramate, amitryptiline, nortryptiline, carbamazepine, Levodopa, L-DOPA, cocaine, a-methyl-tyrosine, reserpine, tetrabenazine, benzotropine, pargyline, fenodolpam mesylate, cabergoline, pramipexole dihydrochloride, ropinorole, amantadine hydrochloride, selegiline hydrochloride, carbidopa, pergolide mesylate, Sinemet CR, Symmetrel, iproniazid, clorgyline, phenelzine, isocarboxazid, tolcapone, entacapone, physostigmine saliclate, physostigmine sulfate, physostigmine bromide, meostigmine bromide, neostigmine methylsulfate, ambenonim chloride, edrophonium chloride, tacrine, pralidoxime chloride, obidoxime chloride, trimedoxime bromide, diacetyl monoxim, endrophonium, pyridostigmine, demecarium, naproxen sodium, diclofenac sodium, diclofenac potassium, celecoxib, sulindac, oxaprozin, diflunisal, etodolac, meloxicam, ibuprofen, ketoprofen, nabumetone, refecoxib, methotrexate, leflunomide, sulfasalazine, gold salts, RHo-D Immune Globulin, mycophenylate mofetil, cyclosporine, azathioprine, tacrolimus, basiliximab, daclizumab, salicylic acid, acetylsalicylic acid, methyl salicylate, diflunisal, salsalate, olsalazine, sulfasalazine, acetaminophen, indomethacin, sulindac, mefenamic acid, meclofenamate sodium, tolmetin, ketorolac, dichlofenac, flurbinprofen, oxaprozin, piroxicam, meloxicam, ampiroxicam, droxicam, pivoxicam, tenoxicam, phenylbutazone, oxyphenbutazone, antipyrine, aminopyrine, apazone, zileuton, aurothioglucose, gold sodium thiomalate, auranofm, methotrexate, colchicine, allopurinol, probenecid, sulfinpyrazone, benzbromarone, betamethasone and other glucocorticoids, metoclopromide, domperidone, prochlorperazine, promethazine, chlorpromazine, trimethobenzamide, ondansetron, granisetron, hydroxyzine, acetylleucine monoethanolamine, alizapride, azasetron, benzquinamide, bietanautine, bromopride, buclizine, clebopride, cyclizine, dimenhydrinate, diphenidol, dolasetron, meclizine, methallatal, metopimazine, nabilone, oxyperndyl, pipamazine, scopolamine, sulpiride, tetrahydrocannabinol, thiethylperazine, thioproperazine, tropisetron, and a mixture thereof.

Examples of second active agents that may be used for the treatment, prevention and/or management of hemoglobinopathy and related disorders include, but are not limited to: interleukins, such as IL-2 (including recombinant IL-II ("rIL2") and canarypox IL-2), IL-10, IL-12, and IL-18; interferons, such as interferon alfa-2a, interferon alfa-2b, interferon alfa-nl, interferon alfa-n3, interferon beta-I a, and interferon gamma-I b; and G-CSF; hydroxyurea; butyrates or butyrate derivatives; nitrous oxide; hydroxy urea; HEMOXIN™ (NIPRISAN™; see United States Patent No. 5,800,819); Gardos channel antagonists such as clotrimazole and triaryl methane derivatives; Deferoxamine; protein C; and transfusions of blood, or of a blood substitute such as Hemospan™ or Hemospan™ PS (Sangart).

*Process far Making Dosage Forms:* Dosage forms provided herein can be prepared by any of the methods of pharmacy, but all methods include the step of bringing the active ingredient into association with the excipient, which constitutes one or more necessary ingredients. In general, the compositions are prepared by uniformly admixing (e.g., direct blend) the active ingredient with liquid excipients or finely divided solid excipients or both, and then, if necessary, shaping the product into the desired presentation (e.g., compaction such as roller-compaction). If desired, tablets can be coated by standard aqueous or non-aqueous techniques. A dosage form provided herein can be prepared by compression or molding, optionally with one or more accessory ingredients. Compressed tablets can be prepared by compressing in a suitable machine the active ingredient in a free-flowing form such as powder or granules, optionally mixed with an excipient as above and/or a surface active or dispersing agent. Molded tablets can be made by molding in a suitable machine a mixture of the powdered compound moistened with an inert liquid diluent. Encapsulation of the dosage forms provided herein can be done using capsules of methylcellulose, calcium alginate, or gelatin. In some embodiments, the active ingredients and excipients are directly blended and loaded into, for example, a capsule, or compressed directly into tablets. A direct-blended dosage form may be more advantageous than a compacted (e.g., roller-compacted) dosage form in certain instances, since direct-blending can reduce or eliminate the harmful health effects that may be caused by airborne particles of ingredients during the manufacture using compaction process. Direct blend formulations may be advantageous in certain instances because they require only one blending step, that of the active and excipients, before being processed into the final dosage form, e.g., tablet or capsule. This can reduce the production of airborne particle or dust to a minimum, while roller-compaction processes may be prone to produce dust. In roller-compaction process, the compacted material is often milled into smaller particles for further processing. The milling operation can produce significant amounts of airborne particles, since the purpose for this step in manufacturing is to reduce the materials particle size. The milled material is then blended with other ingredients prior to manufacturing the final dosage form. For certain active ingredients, in particular for a compound with a low solubility, the active ingredient's particle size is reduced to a fine powder in order to help increase the active ingredient's rate of solubilization. The increase in the rate of solubilization is often necessary for the active ingredient to be effectively absorbed in the gastrointestinal tract. However for fine powders to be directly-blended and loaded onto capsules, the excipients should preferably provide certain characteristics which render the ingredients suitable for the direct-blend process. Examples of such characteristics include, but are not limited to, acceptable flow characteristics. In one embodiment, therefore, provided herein is the use of, and compositions comprising, excipients which may provide characteristics, which render the resulting mixture suitable for direct-blend process, e.g., good flow characteristics. In certain embodiments, a dry blend tablet formulation is the preferred way of making the tablets provided herein.

*Screening:* The process for making the pharmaceutical compositions of the invention preferably includes the screening of the active ingredient and the excipient(s). In one embodiment, the active ingredient is passed through a screen having openings of about 200 microns to about 750 microns. In another embodiment, the active ingredient is passed through a screen with openings of about 200 microns to about 400 microns. In one embodiment, the active ingredient is passed through a screen having openings of about 300 to about 400 microns. Depending on the excipient(s) used, the screen openings vary. For example, disintegrants and binders are passed through openings of about 430 microns to about 750 microns, from about 600 microns to about 720 microns, or about 710 microns. Lubricants are typically passed through smaller openings, e.g., about 150 microns to about 250 microns screen. In one embodiment, the lubricant is passed through a screen opening of about 210 microns.

*Pre-blending:* After the ingredients are screened, the excipient and active ingredient are mixed in a diffusion mixer. In one embodiment, the mixing time is from about 1 minute to about 50 minutes, from about 5 minutes to about 45 minutes, from about 10 minutes to about 40 minutes, or from about 10 minutes to about 25 minutes. In another embodiment, the mixing time is about 15 minutes. When more than one excipient is used, the excipients may be admixed in a tumble blender for about 1 minute to about 20 minutes, or for about 5 minutes to about 10 minutes, prior to mixing with the active ingredient.

*Roller Compaction:* In one embodiment, the pre-blend may optionally be passed through a roller compactor with a hammer mill attached at the discharge of the compactor.

*Final Blend:* When a lubricant, e.g., sodium stearyl fumarate, is used, the lubricant is mixed with the pre-blend at the end of the process to complete the pharmaceutical composition. This additional mixing is from about I minute to about 10 minutes, or from about 3 minutes to about 5 minutes.

*Encapsulation:* The formulation mixture is then optionally encapsulated into the desired size capsule shell using, for example, a capsule filling machine or a rotary tablet press.

*Tableting:* The formulation mixture can also be tableted {e.g., via compaction, compression, or molding) into the desired size and shape tablet using, for example, a tablet press or other conventional tableting equipment and standard techniques.

*Kits:* Pharmaceutical packs or kits which comprise pharmaceutical compositions or dosage forms provided herein are also provided. An example of a kit comprises notice in the form prescribed by a governmental agency regulating the manufacture, use or sale of pharmaceuticals or biological products, which notice reflects approval by the agency of manufacture, use or sale for human administration.

*Methods of Treatment, Prevention, and Management:* Provided herein are methods of treating, preventing, and/or managing certain diseases or disorders using the formulations, compositions, or dosage forms provided herein. Examples of diseases or disorders include, but are not limited to, cancer, disorders associated with angiogenesis, pain including, but not limited to, Complex Regional Pain Syndrome ("CRPS"), Macular Degeneration ("MD") and related syndromes, skin diseases, pulmonary disorders, asbestos-related disorders, parasitic diseases, immunodeficiency disorders, CNS disorders, CNS injury, atherosclerosis and related disorders, dysfunctional sleep and related disorders, hemoglobinopathy and related disorders (e.g., anemia), tuberculosis and related disorders, TNFa related disorders, and other various diseases and disorders.

Examples of cancer and precancerous conditions include, but are not limited to, those described in U.S. patent nos. 6,281,230 and 5,635,517 to Muller et al., in various U.S. patent publications to Zeldis, including publication nos. 2004/0220144A1, published November 4, 2004 (Treatment of Myelodysplasia Syndrome); 2004/0029832A1, published February 12, 2004 (Treatment of Various Types of Cancer); and 2004/0087546, published May 6, 2004 (Treatment of Myeloproliferative Diseases). Examples also include those described in WO 2004/103274, published December 2, 2004. All of these references are incorporated herein in their entireties by reference.

Certain examples of cancer include, but are not limited to, cancers of the skin, such as melanoma; lymph node; breast; cervix; uterus; gastrointestinal tract; lung; ovary; prostate; colon; rectum; mouth; brain; head and neck; throat; testes; kidney; pancreas; bone; spleen; liver; bladder; larynx; nasal passages; and AIDS-related cancers. The compounds are also useful for treating cancers of the blood and bone marrow, such as multiple myeloma and acute and chronic leukemias, for example, lymphoblastic, myelogenous, lymphocytic, and myelocytic leukemias. The compounds provided herein can be used for treating, preventing or managing either primary or metastatic tumors. Other cancers include, but are not limited to, advanced malignancy, amyloidosis, neuroblastoma, meningioma, hemangiopericytoma, multiple brain metastase, glioblastoma multiforms, glioblastoma, brain stem glioma, poor prognosis malignant brain tumor, malignant glioma, recurrent malignant glioma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, chronic lymphocytic leukemia (CLL), Hodgkin's lymphoma, non-Hodgkin's lymphoma, cutaneous T-Cell lymphoma, cutaneous B-Cell lymphoma, diffuse large B-Cell lymphoma, low grade follicular lymphoma, metastatic melanoma (localized melanoma, including, but not limited to, ocular melanoma), malignant mesothelioma, malignant pleural effusion mesothelioma syndrome, peritoneal carcinoma, papillary serous carcinoma, gynecologic sarcoma, soft tissue sarcoma, scleroderma, cutaneous vasculitis, Langerhans cell histiocytosis, leiomyosarcoma, fibrodysplasia ossificans progressive, hormone refractory prostate cancer, resected high-risk soft tissue sarcoma, unrescectable hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone -insensitive prostate cancer, chemotherapy-insensitive prostate cancer, papillary thyroid carcinoma, follicular thyroid carcinoma, medullary thyroid carcinoma, and leiomyoma. In a specific embodiment, the cancer is metastatic. In another embodiment, the cancer is refractory or resistance to chemotherapy or radiation. In one embodiment, the diseases or disorders are various forms of leukemias such as chronic lymphocytic leukemia, chronic myelocytic leukemia, acute lymphoblastic leukemia, acute myelogenous leukemia and acute myeloblastic leukemia, including leukemias that are relapsed, refractory or resistant, as disclosed in U.S. publication no. 2006/0030594, published February 9, 2006, which is incorporated in its entirety by reference. The term "leukemia" refers malignant neoplasms of the blood-forming tissues. The leukemia includes, but is not limited to, chronic lymphocytic leukemia, chronic myelocytic leukemia, acute lymphoblastic leukemia, acute myelogenous leukemia and acute myeloblastic leukemia. The leukemia can be relapsed, refractory or resistant to conventional therapy. The term "relapsed" refers to a situation where patients who have had a remission of leukemia after therapy have a return of leukemia cells in the marrow and a decrease in normal blood cells. The term "refractory or resistant" refers to a circumstance where patients, even after intensive treatment, have residual leukemia cells in their marrow. In another embodiment, the diseases or disorders are various types of lymphomas, including Non-Hodgkin's lymphoma (NHL). The term "lymphoma" refers a heterogenous group of neoplasms arising in the reticuloendothelial and lymphatic systems. "NHL" refers to malignant monoclonal proliferation of lymphoid cells in sites of the immune system, including lymph nodes, bone marrow, spleen, liver and gastrointestinal tract. Examples of NHL include, but are not limited to, mantle cell lymphoma (MCL), lymphocytic lymphoma of intermediate differentiation, intermediate lymphocytic lymphoma (ILL), diffuse poorly differentiated lymphocytic lymphoma (PDL), centrocytic lymphoma, diffuse small-cleaved cell lymphoma (DSCCL), follicular lymphoma, and any type of the mantle cell lymphomas that can be seen under the microscope (nodular, diffuse, blastic and mentle zone lymphoma).

Examples of diseases and disorders associated with, or characterized by, undesired angiogenesis include, but are not limited to, inflammatory diseases, autoimmune diseases, viral diseases, genetic diseases, allergic diseases, bacterial diseases, ocular neovascular diseases, choroidal neovascular diseases, retina neovascular diseases, and rubeosis (neovascularization of the angle). Specific examples of the diseases and disorders associated with, or characterized by, undesired angiogenesis include, but are not limited to, arthritis, endometriosis, Crohn's disease, heart failure, advanced heart failure, renal impairment, endotoxemia, toxic shock syndrome, osteoarthritis, retrovirus replication, wasting, meningitis, silica-induced fibrosis, asbestos-induced fibrosis, veterinary disorder, malignancy-associated hypercalcemia, stroke, circulatory shock, periodontitis, gingivitis, macrocytic anemia, refractory anemia, and 5q-deletion syndrome.

Examples of pain include, but are not limited to those described in U.S. patent publication no. 2005/0203142, published September 15, 2005, which is incorporated herein by reference. Specific types of pain include, but are not limited to, nociceptive pain, neuropathic pain, mixed pain of nociceptive and neuropathic pain, visceral pain, migraine, headache and post-operative pain. Examples of nociceptive pain include, but are not limited to, pain associated with chemical or thermal burns, cuts of the skin, contusions of the skin, osteoarthritis, rheumatoid arthritis, tendonitis, and myofascial pain. Examples of neuropathic pain include, but are not limited to, CRPS type I, CRPS type II, reflex sympathetic dystrophy (RSD), reflex neurovascular dystrophy, reflex dystrophy, sympathetically maintained pain syndrome, causalgia, Sudeck atrophy of bone, algoneurodystrophy, shoulder hand syndrome, post-traumatic dystrophy, trigeminal neuralgia, post herpetic neuralgia, cancer related pain, phantom limb pain, fibromyalgia, chronic fatigue syndrome, spinal cord injury pain, central post-stroke pain, radiculopathy, diabetic neuropathy, post-stroke pain, luetic neuropathy, and other painful neuropathic conditions such as those induced by drugs such as vincristine and velcade.

As used herein, the terms "complex regional pain syndrome," "CRPS" and "CRPS and related syndromes" mean a chronic pain disorder characterized by one or more of the following: pain, whether spontaneous or evoked, including allodynia (painful response to a stimulus that is not usually painful) and hyperalgesia (exaggerated response to a stimulus that is usually only mildly painful); pain that is disproportionate to the inciting event (e.g., years of severe pain after an ankle sprain); regional pain that is not limited to a single peripheral nerve distribution; and autonomic dysregulation (e.g., edema, alteration in blood flow and hyperhidrosis) associated with trophic skin changes (hair and nail growth abnormalities and cutaneous ulceration).

Examples of MD and related syndromes include, but are not limited to, those described in U.S. patent publication no. 2004/0091455, published May 13, 2004, which is incorporated herein by reference. Specific examples include, but are not limited to, atrophic (dry) MD, exudative (wet) MD, age-related maculopathy (ARM), choroidal neovascularisation (CNVM), retinal pigment epithelium detachment (PED), and atrophy of retinal pigment epithelium (RPE).

Examples of skin diseases include, but are not limited to, those described in U.S. publication no. 2005/0214328A1, published September 29, 2005, which is incorporated herein by reference. Specific examples include, but are not limited to, keratoses and related symptoms, skin diseases or disorders characterized with overgrowths of the epidermis, acne, and wrinkles.

As used herein, the term "keratosis" refers to any lesion on the epidermis marked by the presence of circumscribed overgrowths of the horny layer, including but not limited to actinic keratosis, seborrheic keratosis, keratoacanthoma, keratosis follicularis (Darier disease), inverted follicular keratosis, palmoplantar keratoderma (PPK, keratosis palmaris et plantaris), keratosis pilaris, and stucco keratosis. The term "actinic keratosis" also refers to senile keratosis, keratosis senilis, verruca senilis, plana senilis, solar keratosis, keratoderma or keratoma. The term "seborrheic keratosis" also refers to seborrheic wart, senile wart, or basal cell papilloma. Keratosis is characterized by one or more of the following symptoms: rough appearing, scaly, erythematous papules, plaques, spicules or nodules on exposed surfaces (e.g., face, hands, ears, neck, legs and thorax), excrescences of keratin referred to as cutaneous horns, hyperkeratosis, telangiectasias, elastosis, pigmented lentigines, acanthosis, parakeratosis, dyskeratoses, papillomatosis, hyperpigmentation of the basal cells, cellular atypia, mitotic figures, abnormal cell-cell adhesion, dense inflammatory infiltrates and small prevalence of squamous cell carcinomas.

Examples of skin diseases or disorders characterized with overgrowths of the epidermis include, but are not limited to, any conditions, diseases or disorders marked by the presence of overgrowths of the epidermis, including but not limited to, infections associated with papilloma virus, arsenical keratoses, sign of Leser-Trelat, warty dyskeratoma (WD), trichostasis spinulosa (TS), erythrokeratodermia variabilis (EKV), ichthyosis fetalis (harlequin ichthyosis), knuckle pads, cutaneous melanoacanthoma, porokeratosis, psoriasis, squamous cell carcinoma, confluent and reticulated papillomatosis (CRP), acrochordons, cutaneous horn, cowden disease (multiple hamartoma syndrome), dermatosis papulosa nigra (DPN), epidermal nevus syndrome (ENS), ichthyosis vulgaris, molluscum contagiosum, prurigo nodularis, and acanthosis nigricans (AN). Examples of pulmonary disorders include, but are not limited to, those described in U.S. publication no. 2005/0239842A1, published October 27, 2005, which is incorporated herein by reference. Specific examples include pulmonary hypertension and related disorders. Examples of pulmonary hypertension and related disorders include, but are not limited to: primary pulmonary hypertension (PPH); secondary pulmonary hypertension (SPH); familial PPH; sporadic PPH; precapillary pulmonary hypertension; pulmonary arterial hypertension (PAH); pulmonary artery hypertension; idiopathic pulmonary hypertension; thrombotic pulmonary arteriopathy (TP A); plexogenic pulmonary arteriopathy; functional classes I to IV pulmonary hypertension; and pulmonary hypertension associated with, related to, or secondary to, left ventricular dysfunction, mitral valvular disease, constrictive pericarditis, aortic stenosis, cardiomyopathy, mediastinal fibrosis, anomalous pulmonary venous drainage, pulmonary venoocclusive disease, collagen vasular disease, congenital heart disease, HIV virus infection, drugs and toxins such as fenfluramines, congenital heart disease, pulmonary venous hypertension, chronic obstructive pulmonary disease, interstitial lung disease, sleep-disordered breathing, alveolar hypoventilation disorder, chronic exposure to high altitude, neonatal lung disease, alveolar-capillary dysplasia, sickle cell disease, other coagulation disorder, chronic thromboemboli, connective tissue disease, lupus including systemic and cutaneous lupus, schistosomiasis, sarcoidosis or pulmonary capillary hemangiomatosis.

Examples of asbestos-related disorders include, but not limited to, those described in U.S. publication no. 2005/0100529, published May 12, 2005, which is incorporated herein by reference. Specific examples include, but are not limited to, mesothelioma, asbestosis, malignant pleural effusion, benign exudative effusion, pleural plaques, pleural calcification, diffuse pleural thickening, rounded atelectasis, fibrotic masses, and lung cancer.

Examples of parasitic diseases include, but are not limited to, those described in U.S. publication no. 2006/0154880, published July 13, 2006, which is incorporated herein by reference. Parasitic diseases include diseases and disorders caused by human intracellular parasites such as, but not limited to, P. falcifarium, P. ovale, P. vivax, P. malariae, L. donovari, L. infantum, L. aethiopica, L. major, L. tropica, L. mexicana, L. braziliensis, T. Gondii, B. microti, B. divergens, B. coli, C. parvum, C. cayetanensis, E. histolytica, I. belli, S. mansonii, S. haematobium, Trypanosoma ssp., Toxoplasma ssp., and O. volvulus. Other diseases and disorders caused by non-human intracellular parasites such as, but not limited to, Babesia bovis, Babesia canis, Banesia Gibsoni, Besnoitia darlingi, Cytauxzoon felis, Eimeria ssp., Hammondia ssp., and Theileria ssp., are also encompassed. Specific examples include, but are not limited to, malaria, babesiosis, trypanosomiasis, leishmaniasis, toxoplasmosis, meningoencephalitis, keratitis, amebiasis, giardiasis, cryptosporidiosis, isosporiasis, cyclosporiasis, microsporidiosis, ascariasis, trichuriasis, ancylostomiasis, strongyloidiasis, toxocariasis, trichinosis, lymphatic filariasis, onchocerciasis, filariasis, schistosomiasis, and dermatitis caused by animal schistosomes.

Examples of immunodeficiency disorders include, but are not limited to, those described in U.S. application no. 11/289,723, filed November 30, 2005. Specific examples include, but not limited to, adenosine deaminase deficiency, antibody deficiency with normal or elevated Igs, ataxia-tenlangiectasia, bare lymphocyte syndrome, common variable immunodeficiency, Ig deficiency with hyper-IgM, Ig heavy chain deletions, IgA deficiency, immunodeficiency with thymoma, reticular dysgenesis, Nezelof syndrome, selective IgG subclass deficiency, transient hypogammaglobulinemia of infancy, Wistcott-Aldrich syndrome, X-linked agammaglobulinemia, X-linked severe combined immunodeficiency.

Examples of CNS disorders include, but are not limited to, those described in U.S. publication no. 2005/0143344, published June 30, 2005, which is incorporated herein by reference. Specific examples include, but are not limited to, include, but are not limited to, Amyotrophic Lateral Sclerosis, Alzheimer Disease, Parkinson Disease, Huntington's Disease, Multiple Sclerosis other neuroimmunological disorders such as Tourette Syndrome, delerium, or disturbances in consciousness that occur over a short period of time, and amnestic disorder, or discreet memory impairments that occur in the absence of other central nervous system impairments.

Examples of CNS injuries and related syndromes include, but are not limited to, those described in U.S. publication no. 2006/0122228, published June 8, 2006, which is incorporated herein by reference. Specific examples include, but are not limited to, CNS injury/damage and related syndromes, include, but are not limited to, primary brain injury, secondary brain injury, traumatic brain injury, focal brain injury, diffuse axonal injury, head injury, concussion, post-concussion syndrome, cerebral contusion and laceration, subdural hematoma, epidermal hematoma, post-traumatic epilepsy, chronic vegetative state, complete SCI, incomplete SCI, acute SCI, subacute SCI, chronic SCI, central cord syndrome, Brown-Sequard syndrome, anterior cord syndrome, conus medullaris syndrome, cauda equina syndrome, neurogenic shock, spinal shock, altered level of consciousness, headache, nausea, emesis, memory loss, dizziness, diplopia, blurred vision, emotional lability, sleep disturbances, irritability, inability to concentrate, nervousness, behavioral impairment, cognitive deficit, and seizure. Other disease or disorders include, but not limited to, viral, genetic, allergic, and autoimmune diseases. Specific examples include, but not limited to, HIV, hepatitis, adult respiratory distress syndrome, bone resorption diseases, chronic pulmonary inflammatory diseases, dermatitis, cystic fibrosis, septic shock, sepsis, endotoxic shock, hemodynamic shock, sepsis syndrome, post ischemic reperfusion injury, meningitis, psoriasis, fibrotic disease, cachexia, graft versus host disease, graft rejection, auto-immune disease, rheumatoid spondylitis, Crohn's disease, ulcerative colitis, inflammatory-bowel disease, multiple sclerosis, systemic lupus erythrematosus, ENL in leprosy, radiation damage, cancer, asthma, or hyperoxic alveolar injury.

Examples of atherosclerosis and related conditions include, but are not limited to, those disclosed in U.S. publication no. 2002/0054899, published May 9, 2002, which is incorporated herein by reference. Specific examples include, but are not limited to, all forms of conditions involving atherosclerosis, including restenosis after vascular intervention such as angioplasty, stenting, atherectomy and grafting. All forms of vascular intervention are contemplated herein, including diseases of the cardiovascular and renal system, such as, but not limited to, renal angioplasty, percutaneous coronary intervention (PCI), percutaneous transluminal coronary angioplasty (PTCA), carotid percutaneous transluminal angioplasty (PTA), coronary by-pass grafting, angioplasty with stent implantation, peripheral percutaneous transluminal intervention of the iliac, femoral or popliteal arteries, and surgical intervention using impregnated artificial grafts. The following chart provides a listing of the major systemic arteries that may be in need of treatment, all of which are contemplated herein: Artery - Body Areas Supplied; Axillary - Shoulder and axilla; Brachial - Upper arm; Brachiocephalic - Head, neck, and arm; Celiac - Divides into left gastric, splenic, and hepatic arteries; Common carotid - Neck; Common iliac - Divides into external and internal iliac arteries; Coronary - Heart; Deep femoral - Thigh; Digital - Fingers; Dorsalis pedis - Foot; External carotid - Neck and external head regions; External iliac - Femoral artery; Femoral - Thigh; Gastric - Stomach; Hepatic - Liver, gallbladder, pancreas, and duodenum; Inferior mesenteric - Descending colon, rectum, and pelvic wall; Internal carotid - Neck and internal head regions; Internal iliac - Rectum, urinary bladder, external genitalia, buttocks muscles, uterus and vagina; Left gastric - Esophagus and stomach; Middle sacral - Sacrum; Ovarian - Ovaries; Palmar arch - Hand; Peroneal - Calf; Popliteal - Knee; Posterior tibial - Calf; Pulmonary - Lungs; Radial - Forearm; Renal - Kidney; Splenic - Stomach, pancreas, and spleen; Subclavian - Shoulder; Superior mesenteric - Pancreas, small intestine, ascending and transverse colon; Testicular - Testes; and Ulnar - Forearm.

Examples of dysfunctional sleep and related syndromes include, but are not limited to, those disclosed in U.S. publication no. 2005/0222209A1, published October 6, 2005, which is incorporated herein by reference. Specific examples include, but are not limited to, snoring, sleep apnea, insomnia, narcolepsy, restless leg syndrome, sleep terrors, sleep walking sleep eating, and dysfunctional sleep associated with chronic neurological or inflammatory conditions. Chronic neurological or inflammatory conditions, include, but are not limited to, Complex Regional Pain Syndrome, chronic low back pain, musculoskeletal pain, arthritis, radiculopathy, pain associated with cancer, fibromyalgia, chronic fatigue syndrome, visceral pain, bladder pain, chronic pancreatitis, neuropathies (diabetic, post-herpetic, traumatic or inflammatory), and neurodegenerative disorders such as Parkinson's Disease, Alzheimer's Disease, amyotrophic lateral sclerosis, multiple sclerosis, Huntington's Disease, bradykinesia; muscle rigidity; parkinsonian tremor; parkinsonian gait; motion freezing; depression; defective long-term memory, Rubinstein-Taybi syndrome (RTS); dementia; postural instability; hypokinetic disorders; synuclein disorders; multiple system atrophies; striatonigral degeneration; olivopontocerebellar atrophy; Shy-Drager syndrome; motor neuron disease with parkinsonian features; Lewy body dementia; Tau pathology disorders; progressive supranuclear palsy; corticobasal degeneration; frontotemporal dementia; amyloid pathology disorders; mild cognitive impairment; Alzheimer disease with parkinsonism; Wilson disease; Hallervorden-Spatz disease; Chediak-Hagashi disease; SCA-3 spinocerebellar ataxia; X-linked dystonia parkinsonism; prion disease; hyperkinetic disorders; chorea; ballismus; dystonia tremors; Amyotrophic Lateral Sclerosis (ALS); CNS trauma and myoclonus.

Examples of hemoglobinopathy and related disorders include, but are not limited to, those described in U.S. publication no. 2005/0143420A1, published June 30, 2005, which is incorporated herein by reference. Specific examples include, but are not limited to, hemoglobinopathy, sickle cell anemia, and any other disorders related to the differentiation of CD34+ cells.

Examples of tuberculosis (TB) and related disorders include, but are not limited to, those described in PCT publication no. WO 2010/093588, published February 9, 2010, which is incorporated herein by reference. Specific examples include, but are not limited to, pulmonary TB and extrapulmonary TB (remote TB lesions) such as, but not limited to, genitourinary TB (e.g., kidney TB), tuberculous meningitis, military TB, tuberculous peritonitis, tuberculous pericarditis, tuberculous lymphadentitis, TB of bones and joints, gastrointestinal TB, and TB of the liver. In certain embodiments, provided herein are methods of treating, preventing, and/or managing the symptoms associated with TB. Examples include, but are not limited to, cough, dyspnea, hilar lymphadenopathy, segmental atelectasis, swelling of the nodes, lobar atelectasis, pulmonary caviation, fever, unremitting headache, nausea, drowsiness, stupor, coma, stiff neck, weakness, and malaise.

Disorders related to TB often include other mycobacterial infections, symptom of which resemble those of TB. Examples of such disorders include, but are not limited to, disorders caused by M. avium complex (MAC; M. avium and M. intracellulare), M. kansasii, M. xenopy, M. marinum, M. ulcerans, M. leprae, and M fortuitum complex (M fortuitum and M. chelonei). Examples of disorders caused by these mycobacteria include, but are not limited to, pulmonary diseases, lymphadenitis, cutaneous diseases, wounds, and foreign body infections. In certain embodiments, treatment, prevention and/or management of other granulomatous diseases are also encompassed herein. Examples of such diseases include, but are not limited to: infectious agents caused diseases such as histoplasmosis, cryptococcus, schitosomiasis, and leishmaniasis; allergic reactions caused diseases such as berylliosis; non-infectious agents caused diseases such as aspiration pneumonia and foreign body reaction; genetically caused diseases such as chronic granulomatous disease; and diseases of unknown causes such as sarcoidosis, Crohn's disease and cat-scratch fever.

Examples of TNFa related disorders include, but are not limited to, those described in WO 98/03502 and WO 98/54170, both of which are incorporated herein in their entireties by reference. Specific examples include, but are not limited to: endotoxemia or toxic shock syndrome; cachexia; adult respiratory distress syndrome; bone resorption diseases such as arthritis; hypercalcemia; Graft versus Host Reaction; cerebral malaria; inflammation; tumor growth; chronic pulmonary inflammatory diseases; reperfusion injury; myocardial infarction; stroke; circulatory shock; rheumatoid arthritis; Crohn's disease; HIV infection and AIDS; other disorders such as rheumatoid arthritis, rheumatoid spondylitis, osteoarthritis, psoriatic arthritis and other arthritic conditions, septic shock, septis, endotoxic shock, graft versus host disease, wasting, Crohn's disease, ulcerative colitis, multiple sclerosis, systemic lupus erythromatosis, ENL in leprosy, HIV, AIDS, and opportunistic infections in AIDS; disorders such as septic shock, sepsis, endotoxic shock, hemodynamic shock and sepsis syndrome, post ischemic reperfusion injury, malaria, mycobacterial infection, meningitis, psoriasis, congestive heart failure, fibrotic disease, cachexia, graft rejection, oncogenic or cancerous conditions, asthma, autoimmune disease, radiation damages, and hyperoxic alveolar injury; viral infections, such as those caused by the herpes viruses; viral conjunctivitis; or atopic dermatitis.

In other embodiments, the use of formulations, compositions or dosage forms provided herein in various immunological applications, in particular, as vaccine adjuvants, particularly anticancer vaccine adjuvants, as disclosed in U.S. Publication No. 2007/0048327, published March 1, 2007, which is incorporated herein in its entirety by reference, is also encompassed. These embodiments also relate to the uses of the compositions, formulations, or dosage forms provided herein in combination with vaccines to treat or prevent cancer or infectious diseases, and other various uses such as reduction or desensitization of allergic reactions.

The following examples are described in WO2013/040120 and are reiterated hereinafter:

Embodiments provided herein may be more fully understood by reference to the following examples. These examples are meant to be illustrative of pharmaceutical compositions and dosage forms provided herein, but are not in any way limiting. *Formulation of Compound A Dosage Tablet:* Table 1 illustrates a batch formulation and oral dosage formulation for an amorphous solid dispersion of Compound A.

| Trade Name | Common Name | Weight Percent |
|---|---|---|
| **Tablet Formulation** | | |
| Ceolus KG-1000* | Microcrystallinc Cellulose* | 10.0% |
| Avicel PH-102 | Microcrystallinc Cellulose | 14.3% |
| Partcck M200 | Mannitol | 36.4% |
| Ac-Di-Sol | Sodium croscarmellose | 5.0% |
| | Calcium stearate^{†} | 1.0% |
| | Hot Melt Formulation | 33.3% |
| | | 100.0% |

| **Hot Melt Formulation** | | |
|---|---|---|
| | **Compound A** | **15.0%** |
| **Methocel E3 LV** | **Hydroxypropyl methylcellulose** | **85.0%** |
| | | **100.0%** |

| | | |
|---|---|---|
| Maximum-compactibility grade microcrystalline cellulose. ^{†}Magnesium stearate may be used in the alternative. | | |

An amorphous solid dispersion of Compound A in HPMC was prepared as a milled hot melt extruded powder, then dry blended with the remaining excipients and carriers.

*Stability of Formulation:* Accelerated stability was assessed at room temperature, 40°C/75%RH, and 50°C/80%RH. Samples were pulled for hardness testing, assay and dissolution at 1 and 3 months. As depicted in Figure 1 of WO2013/040120, there was no decrease in hardness at room temperature over time. As depicted in Figures 2 and 4 of WO2013/040120, in tablets with protective packaging, substantially all of the active pharmaceutical ingredient was released within 10 minutes, and there was no change in dissolution over time up to 1 year period, even under accelerated conditions. As shown in Figure 3 of WO2013/040120, substantially similar stability profile (up to 3 months) was also observed in tablets without protective packaging. As illustrated in Table 2, although assay showed a decrease by approximately 5% for all conditions, the purity remained constant at 99.6% for all conditions.

| Time (Weeks) | RT | 40°C/75% | 50°C/80% |
|---|---|---|---|
| 0 | 108.4% | 108.4% | 108.4% |
| 4 | 109.3% | 107.9% | 106.7% |
| 12 | 104.1% | 103.8% | 103.2% |

These results show that the formulations provided herein have adequate stability for clinical and other uses, which meet the desired criteria of high hardness (>70 N) and fast disintegration (<10 min). Using AI-AI packing, no change in dissolution or purity of the active pharmaceutical ingredient ("Compound A") was observed, indicating stable formulation by hot melt extrusion. The unique combination of excipients in the formulation helps give the tablet a low friability and fast disintegrating properties. Without limited by a particular theory, Ceolus KG- 1000 may play an important role in creating the properties shown by the formulation due to its high compactibility. While examples of certain particular embodiments are provided herein, it will be apparent to those skilled in the art that various changes and modifications may be made. Such modifications are also intended to fall within the scope of the appended claims.

### SPECIFIC INORMATION CONCERNING ASPECT (IX) OF THE INVENTION

Aspect (ix) of the invention concerns forms of {drug9}, especially an dministration unit comprising 4-amino-5-fluoro-3-[6-(4-methylpiperazin-1-yl)-1H-benzimidazol-2-yl]-1H-quinolin-2-one for the treatment of cancer in moderate hepatic impaired patients ({drug9}) and / or crystalline Form B of lactate salt form of 4-amino-5-fluoro-3-[6-(4-methylpiperazin-1-yl)-1H-benzimidazol-2-yl]-1H-quinolin-2-one for the treatment of cancer in moderate hepatic impaired patients ({drug9a}). Aspect (ix) of the invention relates to a form of 4-amino-5-fluoro-3-[6-(4-methylpiperazin-1-yl)-1H-benzimidazol-2-yl]-1H-quinolin-2-one for (use in) the treatment of cancer in moderate hepatic impaired patients , namely to crystalline Form B of lactate salt form of 4-amino-5-fluoro-3-[6-(4-methylpiperazin-1-yl)-1H-benzimidazol-2-yl]-1H-quinolin-2-one for (use in) the treatment of cancer in moderate hepatic impaired patients , which is useful for treating Cancers including solid tumors, such as renal, breast, bladder, prostate cancer, multiple myeloma whereas the moderate hepatic impaired patient has the following plasma characteristics: 1.5 x ULN < TBL < 3.0 x ULN and/or AST and ALT < 5 x ULN.

4-amino-5-fluoro-3-[6-(4-methylpiperazin-1-yl)-1H-benzimidazol-2-yl]-1H-quinolin-2-one for the treatment of cancer in moderate hepatic impaired patients and / or crystalline Form B of lactate salt form of 4-amino-5-fluoro-3-[6-(4-methylpiperazin-1-yl)-1H-benzimidazol-2-yl]-1H-quinolin-2-one for the treatment of cancer in moderate hepatic impaired patients is described in WO2013/039764, which is incorporated by reference.

The invention relates now to an administration unit comprising 4-amino-5-fluoro-3-[6-(4-methylpiperazin-1-yl)-1H-benzimidazol-2-yl]-1H-quinolin-2-one for the treatment of cancer in moderate hepatic impaired patients and / or crystalline Form B of lactate salt form of 4-amino-5-fluoro-3-[6-(4-methylpiperazin-1-yl)-1H-benzimidazol-2-yl]-1H-quinolin-2-one for the treatment of cancer in moderate hepatic impaired patients. The administration unit according to the invention is useful for treating various diseases and disorders which include but are not limited to Cancers including solid tumors, such as renal, breast, bladder, prostate cancer, multiple myeloma whereas the moderate hepatic impaired patient has the following plasma characteristics: 1.5 x ULN < TBL < 3.0 x ULN and/or AST and ALT < 5 x ULN.

Preferred embodiments of the administration unit are described in WO2013/039764 and are reiterated hereinafter:

Aspect (ix) of the present invention relates to a method of treating a cancer in a patient in need thereof by administering 4-amino-5-fluoro-3-[6-(4-methylpiperazin-1-yl)-1H-benzimidazol-2-yl]-1H-quinolin-2-one, or a tautomer thereof, or a salt of any one of them (Compound [1]), wherein the patient is a moderate hepatic impaired patient. The following description is provided to assist the understanding of the reader. None of the information provided or references cited herein are admitted to be prior art to the present technology. 4-Amino-5-fluoro-3-[6-(4-methylpiperazin-1-yl)-1H-benzimidazol-2-yl]-1H-quinolin-2-one has the structure shown in Formula [1]:

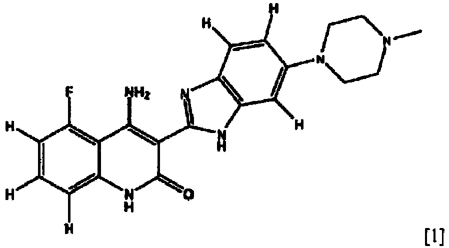

The compound of Formula [1] (in the following also referred to as "Compound [1]") inhibits various protein kinases, such as tyrosine receptor protein kinases. Use and preparation of this compound and its salts, including the mono-lactic acid salt, are described in U.S. Patent Nos. 6,605,617, 6,774,237, 7,335,774, and 7,470,709, and in U.S. Patent Application Serial Nos. 10/982,757, 10/982,543, and 10/706,328, and in the published PCT applications WO2006/127926 published on November 30, 2006 and WO2009/115562 published on September 24th, 2009, each of which is incorporated herein by reference in its entirety. Crystalline forms and their preparations are described in US11/915005, in particular Form B. Based on PK and safety data of Compound [1] from Phase I and II studies, the 500 mg/day on a 5 days on/2 days off dosing schedule has been selected for Treatment Groups 1 (normal hepatic function) and 2 (mild hepatic impairment) to ensure optimal systemic exposure for the cancer patients of these two groups. Indeed due to its properties, Compound [1] showed a time dependent plasma pharmacokinetic across all doses tested from 25 to 600 mg following continuous daily dosing regimen and the prolonged over-proportional exposure of Compound [1] was observed at the doses of 400 mg/day and above. The 5 days on/2 days off dosing schedule was introduced to prevent such prolonged and over proportional accumulation in Compound [1] exposure with dose escalation. For patients having moderate hepatic impairment, there is no data available as whether Compound [1] can be administered to those patients at the same dose as the normal hepatic and mild hepatic impaired patients. Patient with hepatic impairment might be at higher risk to have a decreased ability to eliminate Compound [1]. Decrease drug clearance as a result of impaired organ function can lead to an increased systemic exposure and possible toxicity. Aspect (ix) of the present invention pertains to Compound [1] for use in the treatment of cancer in a patient in need thereof wherein said patient a moderate hepatic impaired patient and wherein the dose administered is 400 mg per day and the dose schedule is 5 days on and 2 days off. Aspect (ix) of the present invention pertains to Compound [1] for use in the treatment of cancer in a patient in need thereof wherein said patient a moderate hepatic impaired patient and wherein the dose administered is 500 mg per day and the dose schedule is 5 days on and 2 days off. Aspect (ix) of the present invention pertains to Compound [1] for use in the treatment of cancer in a patient in need thereof wherein said patient a moderate hepatic impaired patient and wherein the dose administered is 300 mg per day and the dose schedule is 5 days on and 2 days off. Aspect (ix) of the present invention pertains to methods of treating a patient having a cancer by administering 4-amino-5-fluoro-3-[6-(4-methylpiperazin-1-yl)-1H-benzimidazol-2-yl]-1H-quinolin-2-one or a tautomer or a salt of either of them, wherein the patient is a moderate hepatic impaired patient and wherein the dose is 300 mg, 400mg or 500 mg per day and the dose schedule is 5 days on and 2 days off. Aspect (ix) of the present invention pertains to the use of 4-amino-5-fluoro-3-[6-(4-methylpiperazin-1-yl)-1H-benzimidazol-2-yl]-1H-quinolin-2-one or a tautomer or a salt of either of them for the preparation of a medicament for the treatment of cancer wherein the patient is moderate hepatic impaired patient and the dose administered is 300 mg or 400 mg or 500 mg per day and the dose schedule is 5 days on and 2 days off.

Cancers treated according to the present invention include solid tumors, such as renal, breast, bladder, prostate cancer, and multiple myeloma. According to aspect (ix) of the present invention, a moderate hepatic impaired patient is a patient having the following plasma characteristics: 1.5 x ULN < TBL < 3.0 x ULN and/or AST and ALT < 5 x ULN wherein ULN means upper limit normal, TBL means total bilirubin, AST means aspartate transaminase, ALT means alanine transaminase. *Alanine transaminase (ALT), also called Serum Glutamic Pyruvate Transaminase:* (SGPT) or Alanine aminotransferase (ALAT) is an enzyme present in hepatocytes (liver cells). When a cell is damaged, it leaks this enzyme into the blood, where it is measured. ALT rises dramatically in acute liver damage, such as viral hepatitis or paracetamol (acetaminophen) overdose. Elevations are often measured in multiples of the upper limit of normal (ULN). The reference range for ALT is 9 to 40 IU/L (international units per liter).

*Aspartate transaminase (AST) also called Serum Glutamic Oxaloacetic:* Transaminase (SGOT) or aspartate aminotransferase (ASAT) is similar to ALT in that it is another enzyme associated with liver parenchymal cells. It is raised in acute liver damage. The reference range for AST id 10 to 35 IU/L.

Bilirubin is a breakdown product of heme (a part of hemoglobin in red blood cells). The liver is responsible for clearing the blood of bilirubin. It does this by the following mechanism: Bilirubin is taken up into hepatocytes, conjugated (modified to make it water-soluble), and secreted into the bile, which is excreted into the intestine. Increased total bilirubin causes jaundice, and can signal a number of problems. Problems with the liver, which are reflected as deficiencies in bilirubin metabolism (e.g., reduced hepatocyte uptake, impaired conjugation of bilirubin, and reduced hepatocyte secretion of bilirubin). The normal level of total bilirubin is for example, the reference range of 0.2-1.2 mg/dL.

The person skilled in the art knows means, measurement methods and kits to measure the blood and/or plasma levels of AST, ALT and/or TBL, e.g. according to the FDA and/or the EMEA standard and knows how to stratify a patient as moderate impaired patient based on the results of the measurements of AST, ALT and/or TBL.

When Compound [1] is administered as a salt, the dose represents milligrams of 4-amino-5-fluoro-3-[6-(4-methylpiperazin-1-yl)-1H-benzimidazol-2-yl]-1H-quinolin-2-one. Suitable salts include any pharmaceutically acceptable salt, especially the lactate salt form, such as the mono-lactic acid salt form. Additional pharmaceutically acceptable salts are known to those of skill in the art. According to the present invention the lactate salt of Compound [1] can be 4- amino-5-fluoro-3-[6-(4-methylpiperazin-1-yl)-1H-benzimidazol-2-yl]-1H-quinolin-2-one lactate in crystalline Form B, as described in US 11/915005. The Examples of WO2013/039764 are reiterated hereinafter:

### Example 1: Pharmacokinetics of Compound 1 (4-amino-5-fluoro-3-[6-(4-methylpiperazin-1-yl)-1H-benzimidazol-2-yI]-lH-quinolin-2-one or a tautomer thereof e.g. in the lactic acid salt form thereof), in adult cancer patients with normal and moderate impaired hepatic functions.

*Primary objective:* To evaluate the effects of mild or moderate hepatic impairment versus normal hepatic function on the pharmacokinetics of Compound [1] in patients with advanced solid tumor.

*Secondary objectives:* To assess the safety and tolerability of Compound [1] administration on a 5 days on/2 days off dosing schedule in adult cancer patients with mild or moderate hepatic impairment compared to patients with normal hepatic function; to assess pharmacokinetics (PK), safety and tolerability of Compound [1] administration on a 5 days on/2 days off dosing schedule in adult cancer patients with moderate hepatic impairment, To explore the relationship between PK and hepatic functional abnormalities (i.e. bilirubin, (alanine aminotransferease/glutamic pyruvic transaminase/ GPT)/ ALT/AST, and Child-Pugh classification) using regression analysis as appropriate. To evaluate the preliminary anti-tumor activity of Compound [I] in the studied patient population.

*Exploratory objectives:* To determine the plasma protein-binding fraction of Compound [1] (pre-dose sample) in patients with mild, moderate or severe hepatic impairment versus normal hepatic function.

*Study population:* Adult patients who have advanced solid tumor (except breast cancer and lymphoma) that is either refractory to the standard therapy or has no available therapies with varying degrees of hepatic impairment according to NCI guidelines and matching cancer patients with normal hepatic function. Number of patients: Approximately 18-48 patients *Overview of study design:* This is a multi-center, open-label study to assess PK of Compound [1] at single-dose and steady state in adult cancer patients with mild or moderate hepatic impairment relative to patients with normal hepatic function. The study also assesses PK, safety and tolerability of Compound [1] in patients with moderate hepatic impairment, if it is considered safe to evaluate this group based on the evaluation and safety outcome of the mild and moderate hepatic impairment groups. A single dose PK of Compound [1] is collected from patients with normal and impaired hepatic function. Due to the time-dependent PK of Compound [1] (auto-induction of CYP1A1/A2), steady state PK of Compound [1] is also determined following multiple dosing of Compound [1] for 3 weeks or 4 weeks (in case a patient missed or was unable to have a satisfying PK sampling following 3 weeks of dosing) in these patients. The study consists of 4 treatment groups, including Treatment Groups 1 (normal hepatic function) and 2 (mild hepatic impairment), 3 (moderate hepatic impairment). All treatment groups enroll patients with any solid tumor except breast cancer and lymphoma. Treatment Group 3 (moderate hepatic dysfunction) by selecting 400 mg/day (5 days on/2 days off dose schedule) as the initial dose. However, other dose levels (500 mg/day or 300 mg/day) may be explored in patients with moderate hepatic dysfunction based on safety, tolerability, and PK data obtained during this study. Treatment duration includes a single-dose PK period with a single dose of Compound [1] administrated on Day 1 of Week 1 and multiple-dose treatment period with Compound [1], e.g. on a 5 days on/2 days off dosing regimen, starting on Day 4 of Week 1 until disease progression, e.g. assessed by RECIST 1.1, unacceptable toxicity, death or discontinuation from the study treatment for any other reason. Approximately 18-48 patients are enrolled in 3 treatments groups based on their total bilirubin and AST/ ALT levels at baseline/screening. Table below details patient allocation and treatment dose for each treatment group. The enrollment to the Treatment Groups 1-3 are in parallel, with at least 6 evaluable patients per treatment group. The mild hepatic impairment group requires 6-12 evaluable patients. The moderate hepatic impairment group may require 6-18 evaluable patients. At least 6 patients are evaluated for PK at the identified tolerated Compound [1] dose. To the extent possible, the enrollment to Treatment Group 1 (control) is comparable (or similar) to the enrollment to Treatment Groups 2 (mild hepatic impairment) and 3 (moderate hepatic impairment), with respect to age (±10 years) and body weight (±10 Kg). Therefore, the enrollment of Treatment Group 1 (normal hepatic function) remain open until the enrollment in the mild and moderate hepatic impairment groups is complete, and a sufficient number of matching controls has been achieved for comparison, with a minimum of 6 evaluable patients in this treatment group. In case the starting dose is not tolerated, a dose de-escalation to 400 mg is allowed for Treatment Group 2 (mild hepatic impairment), and to 300 mg for Treatment Group 3 (moderate hepatic impairment). Dose-limiting toxicities (DLT) criteria is used to guide dose escalation or dose de-escalation to identify the tolerated Compound [1] dose in Treatment Groups 2 and 3 based on the dose levels assessed in this study. An evaluable patient is required to complete both safety (DLT evaluation) and PK assessments. A patient is considered PK evaluable if a complete PK profile is obtained at the single dose PK period and at steady state. A patient is considered evaluable for DLT if the patient completed 4 weeks of Compound [1] treatment receiving at least 16 doses of the planned dose on a 5 days on /2 days off dosing regimen, or is discontinued from Compound [1] treatment due to the DLT within 4 weeks on a 5 days on/2 days off dosing regimen.

| **Treatment Group** | **Degree of Hepatic Impairment** | **Patient population** | **Total Bilirubin Level (TBL) (Day 1 predose)** | **ALT/AST Level (Day 1 pre-dose)** | **Dose Level 1 (mg/day) (5 days on / 2 days off)** | **Dose Level 2 (mg/day) (5 days on *l*2 days off)** |
|---|---|---|---|---|---|---|
| 1 | Normal hepatic function (control group) | Any solid tumors* | TBL ≤ ULN | AST and ALT ≤ ULN | 500 mg | N/A |
| 2 | Mild hepatic Impairment | Any solid tumors* | TBL ≤ ULN | ALT and/or AST > ULN (with both AST and ALT ≤ 5 x ULN) | 500 mg | 400 mg |
| | | Any solid tumors* | 1.0 x ULN < TBL ≤ 1.5 x ULN | AST and ALT ≤ 5 x ULN | 500 mg | 400 mg |
| 3 | Moderate hepatic impairment | Any solid tumors* | 1.5 x ULN < TBL ≤ 3.0 x ULN | AST and ALT ≤ 5 x ULN | 400 mg | 300 mg or 500 mg |
| | | | | | | |
| *any solid tumor except breast cancer and lymphoma | | | | | | |

| | | | | | | |
|---|---|---|---|---|---|---|
| *Pharmacokinetic (PK) and protein binding assessments:* For PK assessment: A total of 17 time points and approximately 34 mL of blood per patient is collected after starting Compound [1] treatment. Blood samples (2 mL per sample) is taken at the following time-points: • Single-dose PK period (Day 1 of Week 1 following a single dose): predose, 2, 4, 6, 8, 24, 48, and 72 hr post dose; • Steady State (Day 1 Week 4 following multiple doses on a 3^{rd} 5 days on/2 days off dosing schedule): predose, 2, 4, 6, 8, 24, 48, and 72 hr post dose. • In case the patient missed or was not able to have a satisfying Week 4 PK sampling (e.g., due to dose interruption, patient vomiting, etc.), the steady state PK can be collected on Day I to Day 4 Week 5 following multiple dosing on a 4^{th} 5 days on/ 2 days off dosing schedule): predose, 2, 4, 6, 8, 24, 48, and 72 hr post dose. • Trough concentration (Day 1 Week 7): pre-dose | | | | | | |

For protein binding assessment: Sample is collected at predose on Week 1 Day 1 and at predose on Week 4 Day I (or Week 5 Day 1, if applicable)

### Safety and tolerability assessments:

- Routine safety monitoring of adverse events (AEs) and serious adverse events (SAEs);
- Laboratory testing of hematology (hemoglobin, WBC, platelets, and differentials), serum chemistry (urea/BUN, creatinine, total and direct bilirubin, ALT, AST, alkaline phosphatase, gamma glutamyl transpeptidase, albumin, calcium, sodium, magnesium, phosphorus, glucose, LDH, amylase, and lipase), coagulation (PT/INR, PTT and fibrinogen), and urinalysis;
- Measurement of vital signs, performance status, and physical examination;
- ECG evaluation (predose (triplicate with 5-10 minutes apart), and within 1 hour after 2h and 6h postdose PK sampling);
- MUGA/ECHO scan, and HBV and HCV screening in HCC patients;
- Efficacy assessments is performed every 8 weeks based on the radiological tumor measurements (e.g. computed tomography or magnetic resonance imaging scan) using RECIST 1.1
- Other assessments include evaluation of plasma biomarkers for FGFR and VEGFR inhibition, and identification of patients who might benefit from treatment via biomarker analyses from blood plasma samples.

*Data analysis:* Non compartmental analysis is conducted on full PK profile of Compound [1]. Summary statistics and mean (SD) plots is presented for Compound [1] plasma concentration at each scheduled time point by hepatic group and dose for both single dose and steady state. Pharmacokinetic parameters such as Cmax, Tmax, AUClast, single-dose AUCinf, HL, Vz/F, CL/F is summarized by hepatic group and dose. Mild and moderate hepatic impairment groups is compared to the control group (normal hepatic function) for the PK parameters including Cmax, Tmax, AUClast, and single-dose AUCinf. A fixed effect ANOVA model is fit to the log-transformed dose-normalized primary PK parameters (Cmax, AUClast and single-dose AUCinf) from control, mild and moderate hepatic impairment groups, with hepatic function as the fixed effect if dose proportionality (DP) is a reasonable assumption. A point estimate and the corresponding 90% confidence interval for the mean difference between the control group and each hepatic impairment group is calculated. This is anti-logged to obtain the point estimate and 90% confidence interval for the ratio of the geometric means on the untransformed scale. The primary analysis is performed as described if there is further evidence for the DP assumption. Otherwise, other linear and/or nonlinear model based analysis to assess the impact of hepatic function on Compound [1] PK parameters may be employed as appropriate. The effect of baseline covariates such as age and weight is investigated, as necessary. In addition, PK data generated from this study contribute to the future meta-analysis for exploratory population PK of Compound [1] in patients with normal or moderate hepatic function. Safety analysis consist of AE summaries (frequency tables) and listings, laboratory abnormalities summary (shift tables for baseline to worst post-baseline) and flagging of notable values in listings, and listing of other tests (e.g., electrocardiogram or vital signs). The Safety Set is used for safety analysis. DLTs is listed using the dose-determining set.

*Statistical considerations* / *sample size calculation:* Sample size for this study is primarily driven by feasiblity and simple dose finding procedure to identify the tolerated Compound [1] dose in Treatment Groups 2 and 3 based on the dose levels assessed in this study. Approximately 18-48 patients are enrolled in study, with a minimum of 6 evaluable patients in Treatment Groups 1, 2 and 3. The inter-subject coefficient of variation (CV%) for AUC was around 50% on the 500 mg/day on a 5 days on/2 days off dosing schedule. Using an inter-subject CV% estimate of 50%> and a sample size of 6 per treatment group, the precision or half-width of 90% confidence intervals (CI) for (impaired hepatic function)-(normal hepatic function) comparison on the log scale extends 0.494 from the observed difference in means. This calculation is based on a two sample t-test with a type 1 error rate of 10%. No adjustments were made for multiple comparisons. The above half width translates into the following 90% confidence intervals (CI) of pharmacokinetic parameter ratios assuming different observed ratios.

| Ratio | Lower limit of 90% CI | Upper limit of 90% CI |
|---|---|---|
| 0.5 | **0.31** | **0.82** |
| 1.00 | **0.61** | **1.64** |
| 1.50 | **0.92** | **2.46** |
| 2.00 | **1.22** | **3.28** |

While certain embodiments have been illustrated and described, it should be understood that changes and modifications can be made therein in accordance with ordinary skill in the art without departing from the present technology in its broader aspects as defined in the following claims.

### SPECIFIC INORMATION CONCERNING ASPECT (X) OF THE INVENTION

Aspect (x) of the invention concerns forms of {drug10}, especially an administration unit comprising crystalline form of 6-carboxy-2-(3,5-dichlorophenyl)-benzoxazole meglumine. The invention relates to a solid form of 6-carboxy-2-(3,5-dichlorophenyl)-benzoxazole meglumine ({drug10}), namely to crystalline form of 6-carboxy-2-(3,5-dichlorophenyl)-benzoxazole meglumine ({drug10a}), which is useful for treating transthyretin amyloid diseases, such as senile systemic amyloidosis (SSA), familial amyloid polyneuropathy (FAP) and familial amyloid cardiomyopathy (FAC), in mammals. Preferably, the crystalline form of 6-carboxy-2-(3,5-dichlorophenyl)-benzoxazole meglumine has a powder X-ray diffraction pattern comprising peaks at diffraction angles (2Θ) of 10.7 ± 0.2, 11.8 ± 0.2, and 13.3 ± 0.2 ({drug10b}). Crystalline form of 6-carboxy-2-(3,5-dichlorophenyl)-benzoxazole meglumine is described in WO2013/038351, which is incorporated by reference. As used herein, "6-carboxy-2-(3,5-dichlorophenyl)-benzoxazole" can be represented by the following chemical structure:

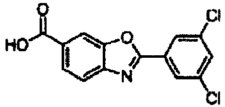

As used herein, "meglumine" can be represented by the following chemical structure:

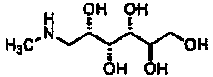

N-methyl-D-glucamine (meglumine) salt of 6-carboxy-2-(3,5-dichlorophenyl)-benzoxazole = 6-carboxy-2-(3,5-dichlorophenyl)-benzoxazole meglumine is also referred to as "Compound 1 ". As used herein, crystalline form of 6-carboxy-2-(3,5-dichlorophenyl)-benzoxazole meglumine is a crystalline form of 6-carboxy-2-(3,5-dichlorophenyl)-benzoxazole meglumine. Preferred embodiments of aspect (x) of the invention are described in the claims of WO2013/038351 and are reiterated as items 1 to 11 hereinafter:
[Item 1]: A crystalline form of 6-carboxy-2-(3,5-dichlorophenyl)-benzoxazole meglumine, wherein said crystalline form has a powder X-ray diffraction pattern comprising peaks at diffraction angles (2Θ) of 10.7 ± 0.2, 11.8 ± 0.2, and 13.3 ± 0.2. [Item 2]: A crystalline form of 6-carboxy-2-(3,5-dichlorophenyl)-benzoxazole meglumine, wherein said crystalline form has a powder X-ray diffraction pattern comprising peaks at diffraction angles (2Θ) of 10.7 ± 0.2, 11.8 ± 0.2, 13.3 ± 0.2, and 14.8 ± 0.2. [Item 3]: A crystalline form of 6-carboxy-2-(3,5-dichlorophenyl)-benzoxazole meglumine, wherein said crystalline form has a powder X-ray diffraction pattern comprising peaks at diffraction angles (2Θ) of 10.7 ± 0.2, 11.8 ± 0.2, 13.3 ± 0.2, 14.8 ± 0.2 and 21.7 ± 0.2. [Item 4]: A crystalline form of 6-carboxy-2-(3,5-dichlorophenyl)-benzoxazole meglumine, wherein said crystalline form has a Raman spectrum comprising Raman shift peaks (cm-1) at 1625 ± 2, 1596 ± 2, and 1548 ± 2. [Item 5]: A crystalline form of 6-carboxy-2-(3,5-dichlorophenyl)-benzoxazole meglumine, wherein said crystalline form has a Raman spectrum comprising Raman shift peaks (cm-1) at 1625 ± 2, 1616 ± 2, 1596 ± 2, and 1548 ± 2. [Item 6]: A crystalline form of 6-carboxy-2-(3,5-dichlorophenyl)-benzoxazole meglumine, wherein said crystalline form has an infrared spectrum comprising infrared shift peaks (cm-1) at 1581 ± 2, 1273 ± 2, 1010 ± 2, 906 ± 2 and 873 ± 2. [Item 7]: A crystalline form of 6-carboxy-2-(3,5-dichlorophenyl)-benzoxazole meglumine, wherein said crystalline form has a solid state NMR spectrum comprising 13C chemical shifts (ppm) at 1 12.6 ± 0.2, 133.9 ± 0.2, and 171.5 ± 0.2. [Item 8]: A crystalline form of 6-carboxy-2-(3,5-dichlorophenyl)-benzoxazole meglumine, wherein said crystalline form has a Raman spectrum comprising a Raman shift peak (cm-1) at 1625 cm ± 2; and a solid state NMR spectrum comprising a 13C chemical shift (ppm) at 133.9 ± 0.2. [Item 9]: A crystalline form of 6-carboxy-2-(3,5-dichlorophenyl)-benzoxazole meglumine, wherein said crystalline form has a Raman spectrum comprising a Raman shift peak (cm-1) at 1625 cm ± 2; and a solid state NMR spectrum comprising a 13C chemical shift (ppm) at 171.5 ± 0.2. [Item 10]: The crystalline form of any one of items 1-9, wherein said form is non-hygroscopic and anhydrous. [Item 11]: The crystalline form of any one of items 1-10, wherein said form is substantially pure. The crystalline form of 6-carboxy-2-(3,5-dichlorophenyl)-benzoxazole meglumine can be obtained as described in WO2013/038351, e.g. by the following examples:

The examples which follow will further illustrate the preparation of the distinct forms of the invention, i.e. a crystalline form, a two-dimensionally ordered liquid crystalline form and an amorphous form, but are not intended to limit the scope of the invention as defined herein or as claimed below.
Example 1 *- Preparation of Crystalline 1:* 6-Carboxy-2-(3,5-dichlorophenyl)-benzoxazole free acid (2.5 g, 8.1 mmol) and 2-propanol (49 ml.) were charged to a 100 mL jacketed, 2-neck round bottom flask with magnetic stirrer. The resulting slurry was warmed to 70 °C with stirring. Water (8.8 mL) was then charged. In a separate 15 mL round bottom flask a solution of N-methyl-D-glucamine (1.58 g, 8.1 mmol) in 5 mL water was prepared and dissolved with stirring. The aqueous N-methyl-D-glucamine solution was then transferred to the reaction flask over 2 min. Most (but not all) of the solids dissolved by the end of this addition. After 5 min stirring and warming to 79 °C, a clear, pale yellow solution resulted. The solution was filtered through a bed of Celite™, cooled to 60 °C, then cooled to 10 °C over 2 h. The resulting solids were collected by filtration, washing with 10 mL of 2-propanol. 3.35 g product was obtained (82% yield).
Example 2 *- Preparation of Compound 1 Liquid Crystal:* Crystalline Compound 1 (505 mg) was dissolved in 60 mL water at room temperature. The solution was transferred to a lyophlization vessel and frozen while rotated in an acetone/dry ice bath. The vessel was transferred to a bench-top freeze dryer and allowed to dry under vacuum for approximately 19 hours, producing a white solid.
Example 3 *- Preparation of Amorphous 1:* Crystalline Compound 1 (approximately 500 mg) was transferred to an aluminum pan and placed on a 200 °C hot plate. Melting occurred within I minute, at which point the pan was removed from the hot plate and immediately placed in liquid nitrogen. A glassy solid resulted.

Aspect (x) of the invention relates to an administration unit comprising crystalline form of 6-carboxy-2-(3,5-dichlorophenyl)-benzoxazole meglumine. The administration unit according to the invention is useful for treating various diseases and disorders which include but are not limited to transthyretin amyloid diseases, such as senile systemic amyloidosis (SSA), familial amyloid polyneuropathy (FAP) and familial amyloid cardiomyopathy (FAC), in mammals.

### PREFERRED EMBODIMENTS CONCERNING ASPECTS (I), (II), (III), (IV), (V), (VI), (VII), (VIII), (IX), AND (X) OF THE INVENTION

The invention relates to an administration unit comprising {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.

Preferably, the administration unit according to the invention comprises a therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. wherein at least 10 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are present as (i) {drug1a} or (drug1b); (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.Preferably, at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are present as (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.Preferably, at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are present as (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.

The total content of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} as well as the relative content of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i); (v) {drug5a}; (vi) {drug6a}; (vii) {drug7a}; (viii) {drug8a}; (ix) {drug9a}; or (x) {drug10a} or {drug10b} can be determined by standard analysis known to the skilled artisan. Suitable methods include but are not limited to thermal analysis, HPLC and XRPD. In a preferred embodiment of the administration unit according to the invention, not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are amorphous. Preferably, not more than 80% by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are amorphous. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are amorphous.

The total content of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} as well as the relative content of amorphous (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} can be determined by standard analysis known to the skilled artisan. Suitable methods include but are not limited to thermal analysis, HPLC and XRPD (see e.g. K.D. Harris, Powder diffraction crystallography of molecular solids, Top Curr Chem., 2012, 315:133-77). In a preferred embodiment of the administration unit according to the invention, not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are polymorphs of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} other than (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are polymorphs of (i) (drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} other than (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are polymorphs of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} other than (i) {drug1a} or {drug1b); (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.

The total content of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} as well as the relative content of polymorphs other than (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} can be determined by standard analysis known to the skilled artisan. Suitable methods include but are not limited to thermal analysis, HPLC and XRPD (see e.g. N. Chieng, T. Rades, J.Aaltonen, An overview of recent studies on the analysis of pharmaceutical polymorphs, J Pharm Biomed Anal. 2011, 25:55(4):618-44; R. Hilfiker, Polymorphism, Wiley-VCH, 1st ed. 2006; H.G. Brittain, Polymorphism in Pharmaceutical Solids (Drugs and the Pharmaceutical Sciences), Informa Healthcare, 2nd ed. 2009).

In a preferred embodiment of the administration unit according to the invention, the therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.1 µg to 2 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Preferably, the therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 1 µg to 2.5 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 2.5 µg to 5 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 5 µg to 10 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 10 µg to 25 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 25 µg to 50 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 50 µg to 100 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 100 µg to 250 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 250 µg to 500 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 500 µg to 1 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1 mg to 2.5 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 2.5 mg to 5 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 5 mg to 10 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 10 mg to 25 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 25 mg to 50 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 50 mg to 100 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 100 mg to 250 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 250 mg to 500 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 500 mg to 1 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1 g to 1.25 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1.25 g to 1.5 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1.5 g to 1.75 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1.75 g to 2 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

Preferably, the administration unit according to the invention comprises 0.1 µg to 2 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}. Preferably, it comprises 1 µg to 2.5 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 2.5 µg to 5 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 5 µg to 10 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 10 µg to 25 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 25 µg to 50 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 50 µg to 100 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 100 µg to 250 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 250 µg to 500 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 500 µg to 1 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1 mg to 2.5 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 2.5 mg to 5 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 5 mg to 10 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 10 mg to 25 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 25 mg to 50 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 50 mg to 100 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 100 mg to 250 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 250 mg to 500 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 500 mg to 1 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1 g to 1.25 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1.25 g to 1.5 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1.5 g to 1.75 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1.75 g to 2 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}. Preferably, the administration unit according to the invention contains (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} as substantially the only form of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, i.e. preferably contains neither non-crystalline forms of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} nor crystalline forms other than (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.

The administration unit according to the invention and its constituents, i.e. (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} and (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, respectively, as well as pharmaceutical excipients, can be characterized by analytic methods that are known to the skilled artisan and described in Ph. Eur. and USP (see e.g. H.G. Brittain, S.J. Bodanowich, D.E. Bugay, J. DeVoncentis, G. Lewen, A.W. Newman, Physical characterization of pharmaceutical solids, Pharm Res. 1991, 8(8):963-73; D.E. Bugay, Characterization of the solid-state: spoectroscopic techniques, Adv Drug Deliv Rev., 2001, 48(1):43-65; P.A. Tishmack, D.E. Bugay, S.R. Byrn, Solid-state nuclear magnetic resonance spectroscopy - pharmaceutical application, J Pharm Sci, 2003, 92(3):441-74; C.J. Lee, C.J. Strachan, P.J. Manson, T. Rades, Characterization of the bulk properties of pharmaceutical solids using nonlinear optics - a review, J Pharm Pharmacol., 2007, 59(2):241-50); R.A. Storey, Solid State Characterization of Pharmaceuticals, Wiley-Blackwell, 2011).

In a preferred embodiment, the administration unit according to the invention contains (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} in form of particles, wherein the particle size distribution of X90 is about 500 µm or less, i.e. a particle size distribution (preferably by volume) such that at least 90 % of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} has a particle size smaller than 500 µm. Preferably, for (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the administration unit according to the invention contains (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} in form of particles, wherein the particle size distribution of X50 is about 400 µm or less, i.e. a particle size distribution (preferably by volume) such that at least 50 % of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} has a particle size smaller than 400 µm. Preferably, for (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

According to USP, the following parameters may be defined based on the cumulative distribution. QR(X) = cumulative distribution of particles with a dimension less than or equal to X [µm] where the subscript R reflects the distribution type: 0 Number, 1 Length, 2 Area, 3 Volume. Therefore, by definition: QR(X) = 0.90 when X = X90; QR(X) = 0.50 when X = X50; and QR(X) = 0.10 when X = X10.

In preferred embodiments, the administration unit according to the invention contains (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} in form of particles having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the administration unit according to the invention contains (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} in form of particles having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125)≥0.50; or Q3(98) <0.50 and Q3(111) ≥0.50; or Q3(98) ≥0.50.

In preferred embodiments, the administration unit according to the invention contains (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} in form of particles having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Preferably, the administration unit according to the invention is solid, semisolid or liquid.

Preferably, the administration unit according to the invention is for administration once daily, or twice daily, or thrice daily, or four times daily, or five times daily, or six times daily.

Preferably, the administration unit according to the invention is monolithic or multiparticulate.

In a preferred embodiment of the administration unit according to the invention, (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} is homogeneously distributed over the administration unit.

Excipient composition and homogeneity can be determined by standard analysis known to the skilled artisan. Suitable methods include but are not limited to near-infrared chemical imaging.

In another preferred embodiment of the administration unit according to the invention, (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} is not homogeneously distributed over the administration unit.

In a preferred embodiment, the administration unit according to the invention provides controlled release of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Preferably, the administration unit according to the invention comprises a controlled release matrix or a controlled release coating.

In a preferred embodiment, under in vitro conditions (paddle apparatus, 75 rpm, 900 mL artificial gastric juice, pH 1.2) the administration unit according to the invention has released after 1 hour not more than 90 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Preferably, the administration unit according to the invention has released after 1 hour not more than 80 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 70 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 60 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 50 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 40 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 30 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 20 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 10 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

In a preferred embodiment, under in vitro conditions (paddle apparatus, 75 rpm, 900 mL artificial gastric juice, pH 1.2) the administration unit according to the invention has released after 2 hours not more than 90 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Preferably, the administration unit according to the invention has released after 2 hours not more than 80 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 70 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 60 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 50 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 40 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 30 % of (i) {drug1}, (ii) {drug2}, (iii) {drug3}, (iv) {drug4}, (v) {drug5},(vi) {drug6}, (vii) {drug7}, (viii) {drug8}, (ix) {drug9}, or (x) {drug10}, or not more than 20 % of (i) {drug1}, (ii) {drug2}, (iii) {drug3}, (iv) {drug4}, (v) {drug9}, (vi) {drug6}, (vii) {drug7}, (viii) {drug8}, (ix) {drug9}, or (x) {drug10}. or not more than 10 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

In a preferred embodiment, under in vitro conditions (paddle apparatus, 75 rpm, 900 mL artificial gastric juice, pH 1.2) the administration unit according to the invention has released after 4 hours not more than 90 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Preferably, the administration unit according to the invention has released after 4 hours not more than 80 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 70 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 60 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 50 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 40 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 30 % of (i) {drug1}, (ii) {drug2}, (iii) {drug3}, (iv) {drug4}, (v) {drug5},(vi) {drug6}, (vii) {drug7}, (viii) {drug5},(ix) {drug9}, or (x) {drug10}, or not more than 20 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 10 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

In a preferred embodiment, under in vitro conditions (paddle apparatus, 75 rpm, 900 mL artificial gastric juice, pH 1.2) the administration unit according to the invention has released after 8 hours not more than 90 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Preferably, the administration unit according to the invention has released after 8 hours not more than 80 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10},or not more than 70 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 60 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 50 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10},or not more than 40 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 30 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or not more than 20 % of (i) {drug1}, (ii)
{drug2}, (iii) {drug3}, (iv) {drug4}, (v) {drug5}, (vi) {drug6}, (vii) {drug7}, (viii) {drug8}, (ix) {drug9}, or (x) {drug10}, or not more than 10 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

In another preferred embodiment, the administration unit according to the invention provides immediate release of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

In a preferred embodiment, under in vitro conditions (paddle apparatus, 75 rpm, 900 mL artificial gastric juice, pH 1.2) the administration unit according to the invention has released after 0.5 hours at least 10 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Preferably, the administration unit according to the invention has released after 0.5 hours at least 20 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or at least 30 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or at least 40 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or at least 50 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or at least 60 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or at least 70 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or at least 80 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or at least 90 % of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or(x) {drug10}. Preferably, the administration unit according to the invention has a total weight of 10 mg to 3 g. Preferably, the administration unit according to the invention has a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

In a preferred embodiment, the administration unit according to the invention is for systemic or topical administration.

In another preferred embodiment, the administration unit according to the invention is for parenteral administration. Preferably, the administration unit according to the invention is for oral, buccal, opthalmic, nasal, rectal, transmucosal, or intestinal administration. Preferably, it is for oral administration.

Preferably, the administration unit according to the invention is selected from the group consisting of tablets, capsules, effervescent tablets, orodispersible tablets, dragees, sachets, drops, suspensions, and powders. Preferably, it is a tablet. Preferably, the administration unit according to the invention is round or oblong; and/or is planar or biconvex.

In a preferred embodiment, the administration unit according to the invention has a round cross-section with a diameter of 1 mm to 20 mm. Preferably, the diameter of the round cross-section is 1 mm to 3 mm, or 3 mm to 5 mm, or 5 mm to 8 mm, or 8 mm to 10 mm, or 10 mm to 13 mm, or 13 mm to 15 mm, or 15 mm to 18 mm, or 18 mm to 20 mm.

In a preferred embodiment, the administration unit according to the invention is oblong and has an aspect ratio of at least 1.1:1. Preferably, the aspect ratio is at least 4:3, or at least 21/2:1, or at least 3:2, or at least 16:10, or at least □:1, or at least 5:3, or at least 16:9.

In a preferred embodiment, the administration unit according to the invention has been manufactured by direct compression. In another preferred embodiment, the administration unit according to the invention has been manufactured by granulation and subsequent compression of granules. Preferably, granulation is wet granulation or dry granulation.

Preferably, the administration unit according to the invention has been compacted at a pressure of 10 MPa to 10,000 MPa. Preferably, the administration unit according to the invention has been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

In a preferred embodiment, the administration unit according to the invention comprises a film coating. Preferably, the film coating is enteric. Suitable enteric coating materials are known to the skilled artisan and include but are not limited to methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In a preferred embodiment of the administration unit according to the invention, the film coating comprises a cellulose ether (e.g. hydroxypropylmethyl cellulose) or a synthetic polymer (e.g. polyvinyl alcohol).

In a preferred embodiment of the administration unit according to the invention, the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

In a preferred embodiment, the administration unit according to the invention has a tablet porosity of not more than 90 %. Preferably, it has a tablet porosity of not more than 80 %, or not more than 70 %, or not more than 60 %, or not more than 50 %, or not more than 40 %, or not more than 30 %, or not more than 20 %, or not more than 10 %. Preferably, it has a tablet porosity of not more than 5 %, or not more than 4 %, or not more than 3 %, or not more than 2 %, or not more than 1 %, or not more than 0.5 %, or not more than 0.2 %, or not more than 0.1 %.

The most common strength test in pharmaceutical applications is the diametral compression test, which is used to calculate the radial tensile strength of a tablet (Fell, J.T., Newton, J.M., 1970. Determination of tablet strength by diametral compression test. J. Pharm. Sci. 59, 688-691). In order to calculate the radial tensile strength, the stress conditions have to be such that the tablet fails in tension. During radial tensile strength measurements, the fracture occurs through a predetermined diametral cross section of the tablet. Therefore, the radial tensile strength is likely to reflect the average strength of tablet rather than the strength of the weakest plane in the tablet.

In a preferred embodiment, the administration unit according to the invention has a radial tensile strength of 0.1 to 100 MPa. Preferably, it has a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

Another method for determining mechanical strength is to measure the axial tensile strength. The force necessary to break the tablet is obtained by pulling the tablet parallel to the applied force during the formation of the tablet and this force is then used to calculate the axial tensile strength (Nyström, C., Malmqvist, K., Mazur, J., Alex, W., Hölzer, A.W., 1978. Measurement of axial and radial tensile strength of tablets and their relation to capping. Acta Pharm. Suec. 15, 226-232). During axial tensile strength measurements, the fracture will occur through the weakest plane in the tablet. Consequently, this method allows detection of capping tendencies in a tablet.

In a preferred embodiment, the administration unit according to the invention has an axial tensile strength of 0.1 to 100 MPa. Preferably, it has an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

In a preferred embodiment, the administration unit according to the invention has an average pore size of 0.001 µm to 1000 µm. Preferably, it has an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to 1 µm, or I µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

The pore size distribution of a tablet may be assessed by methods that are known to the skilled artisan and include but are not limited to gas adsorption (e.g. Stanley-Wood, N.G., Johansson, M.E., 1980. Variation of intra- and inter-particle porosity with degree of compaction. Analyst 105, 1104-1112; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86) or mercury porosimetry (e.g. Stanley-Wood, N.G., Johansson, M.E., 1980. Variation of intra- and inter-particle porosity with degree of compaction. Analyst 105, 1104-1112; Juppo, A.M., 1996. Relationship between breaking force and pore structure of lactose, glucose and mannitol tablets. Int. J. Pharm.127, 95-102; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86). These techniques are complementary, in that mercury porosimetry can be used to measure larger pores (the lower size limit is about 0.003 µm in diameter) while gas adsorption allows measurement of smaller pores. For further details it is also referred to S. Lowell et al., Characterization of Porous Solids and Powders: Surface Area, Pore Size and Density (Particle Technology Series), Springer, 2010.

In a preferred embodiment, the administration unit according to the invention has a pore size distribution such that at least 10 % of the pores have a pore size of not more than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 0.1 µm.

In another preferred embodiment, the administration unit according to the invention has a pore size distribution such that at least 10 % of the pores have a pore size of not less than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 0.1 µm.

In preferred embodiment, the administration unit according to the invention has a pore size distribution such that at least 10 % of the pores have a pore size of not more than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 1 µm.

In another preferred embodiment, the administration unit according to the invention has a pore size distribution such that at least 10 % of the pores have a pore size of not less than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 1 µm.

In a preferred embodiment, the administration unit according to the invention has a pore size distribution such that at least 10 % of the pores have a pore size of not more than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 10 µm.

In another preferred embodiment, the administration unit according to the invention has a pore size distribution such that at least 10 % of the pores have a pore size of not less than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 10 µm.

In a preferred embodiment, the administration unit according to the invention has a water content of not more than 5 % by weight, relative to the total weight of the tablet. Preferably, it has a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

In a preferred embodiment, the administration unit according to the invention has a true density of 0.60 to 1.40 g cm-3. Preferably, it has a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention has an apparent density of 0.60 to 1.40 g cm-3. Preferably, it has an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention disintegrates within 6000 seconds. Preferably, it disintegrates within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

In a preferred embodiment, the administration unit according to the invention is a capsule. Preferably, it comprises a multitude of particulates comprising (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}. Preferably, it comprises at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

In a preferred embodiment of the administration unit according to the invention, the capsule material comprises hard gelatine. In a preferred embodiment, the administration unit according to the invention comprises at least 2 different types of particulates that differ from each other in at least one property selected from the group consisting of nature of excipients, content of excipients, content of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, content of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, water content, total weight, size, and shape.

In a preferred embodiment, the administration unit according to the invention passes the storage stability test according to Ph. Eur. under accelerated storage conditions at 40°C and 75 % rel. humidity. Preferably, it has a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

In a preferred embodiment, the administration unit according to the invention provides the peak plasma level within 0.1 hour to 36 hours after administration. Preferably, it provides the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

Preferably, the administration unit according to the invention comprises one or more excipients independently selected from the group consisting of antiadherents, binders, disintegrants, fillers, diluents, flavors, colors, lubricants, glidants, sorbents, surfactants, preservatives and sweeteners.

In a preferred embodiment of the administration unit according to the invention, the antiadherent is selected from the group consisting of silicon dioxide, talc, talc waxes, stearates (e.g. magnesium, calcium and sodium), stearic acid, stearowet, boric acid, sodium chloride, DL-leucine, sodium oleate, sodium benzoate, sodium acetate, sodium lauryl sulfate, and magnesium lauryl sulfate. Magnesium stearate is particularly preferred.

In a preferred embodiment of the administration unit according to the invention, the binder is selected from the group consisting of saccharides and their derivatives; proteins; gums; silicates; and synthetic polymers. Preferably, the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

In a preferred embodiment of the administration unit according to the invention, the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

In a preferred embodiment of the administration unit according to the invention, the filler (or diluent) is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

In a preferred embodiment of the administration unit according to the invention, the lubricant is hydrophilic or hydrophobic. Preferably, the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

In a preferred embodiment of the administration unit according to the invention, the glidant is selected from the group consisting of fumed silica, cornstarch, talc, and magnesium carbonate.

In a preferred embodiment of the administration unit according to the invention, the surfactant is selected from the group consisting of sodium lauryl sulfate, polyethylene-polypropylene glycol co-polymers, poly(oxyethylene)-poly(oxypropylene)-block-copolymers (e.g. poloxamers).

In a preferred embodiment of the administration unit according to the invention, the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

In a preferred embodiment, the administration unit according to the invention passes the friability test according to Ph. Eur. Preferably, the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

In a preferred embodiment, the administration unit according to the invention passes the test uniformity of content of single-dose preparations according to Ph. Eur.

In a preferred embodiment, the administration unit according to the invention does not comprise any active pharmaceutical ingredient other than (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

In a preferred embodiment, the administration unit according to the invention is for storage not above 50 °C. Preferably, it is for storage not above 25 °C.

In a preferred embodiment, the administration unit according to the invention is not identical with any of the administration units that are individualized in the experimental section of WO2013/018362, WO2013/017989, WO2013/021309, WO2013/028465, WO2013/028832, WO2013/030374, WO2013/030803, WO2013/040120, WO2013/039764, and WO2013/038351, respectively, which is incorporated by reference.

The invention also relates to the administration unit according to the invention for use in the treatment of a disorder or a disease in a mammal. Preferably, the mammal is a human. Preferably, the mammal is an adult.

Preferably, the mammal does not suffer from liver damage and/or kidney damage.

Preferably, the mammal is not pregnant.

Preferably, the mammal is not allergic against (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

In a preferred embodiment, the administration unit according to the invention is administered orally with a liquid. Preferably, the liquid is water, milk, juice or lemonade.

In a preferred embodiment of the administration unit according to the invention, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the administration unit according to the invention, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 5000 mg. Preferably, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

The invention also relates to a packaging comprising one or more administration units according to the invention.

In a preferred embodiment, the packaging according to the invention comprises a material selected from the group consisting of paper, cardboard, paperboard, metal foil and plastic foil.

In a preferred embodiment, the packaging according to the invention is a blister packaging.

A preferred blister packaging includes but is not limited to PVC-blister, PVDC-blister, PVC/PVDC-blister, moisture-proof packaging material such as aluminium foil blister pack, alu/alu blister, transparent or opaque polymer blister with pouch.

An alternative packaging according to the invention is a polypropylene tube, glass bottle and HDPE bottle optionally containing a child-resistant feature. The primary packaging material may comprise a desiccant such as molecular sieve or silica gel to improve chemical stability of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}. Opaque packaging such as colored blister materials, tubes, brown glass bottles or the like can be used to prolong shelflife of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} by reduction of photodegradation.

In a preferred embodiment, the packaging according to the invention comprises a multitude of at least 2, or at least 3, or at least 4, or at least 5, or at least 6, or at least 7, or at least 8, or at least 9, or at least 10 administration units according to the invention.

In a preferred embodiment of the packaging according to the invention, the administration units do not substantially differ in at least one property selected from the group consisting of nature of excipients, content of excipients, content of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, content of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, water content, total weight, release profile, disintegration time, size, shape, porosity, apparent density, true density, average pore size, pore size distribution, axial tensile strength, radial tensile strength, friability, and storage stability. Preferably, the administration units differ in said at least one property by not more than 5 %, or not more than 4 %, or not more than 3 %, or not more than 2 %, or not more than 1 %, or not more than 0.5 %, or not more than 0.1 %. Preferably, all administration units are substantially identical.

The invention also relates to the packaging according to the invention for use in the treatment of a disorder or a disease, wherein one or more administration units of said multitude are administered on a first day, and one or more administration units of said multitude are administered on a second day following said first day.

In a preferred embodiment of the packaging according to the invention, one or more administration units of said multitude are administered on one or more subsequent days following said second day. Preferably, the time interval between the administration of an administration unit and the administration of a subsequent administration unit is not more than 48 hours. Preferably, the time interval is not more than 36 hours, or not more than 24 hours, or not more than 12 hours, or not more than 8 hours, or not more than 6 hours, or not more than 4 hours.

In a preferred embodiment of the packaging according to the invention, the administration units are administered on not more than 30 subsequent days. Preferably, the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.

In a preferred embodiment of the packaging according to the invention, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight. In a preferred embodiment of the packaging according to the invention, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 5000 mg. Preferably, the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to I mg, or I mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

In a preferred embodiment, the packaging according to the invention does not comprise any administration unit comprising an active pharmaceutical ingredient other than (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.

The invention also relates to a method for manufacturing an administration unit according to the invention or a packaging according to the invention, comprising the steps:
(a) providing a quantity of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} that exceeds the dose of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} to be contained in a single administration unit by a factor of at least 10;
(b) mixing the quantity of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} with one or more pharmaceutical excipients;
(c) dividing the mixture obtained in step (b) in fractions each containing the dose of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} to be contained in a single administration unit;
(d) optionally, forming the fractions obtained in step (c) to administration units; and
(e) optionally, packing the fractions obtained in step (c) and the administration units obtained in step (d), respectively. In a preferred embodiment of the method according to the invention, in step (a) the quantity of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} exceeds the dose of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} to be contained in a single administration unit by a factor of at least 25, or at least 50, or at least 100, or at least 250, or at least 500, or at least 1000, or at least 2500, or at least 5000, or at least 10,000.

The invention also relates to particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} having a particle size distribution X90 of about 500 µm or less, i.e. a particle size distribution (preferably by volume) such that at least 90 % of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} has a particle size smaller than 500 µm. Preferably, for (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} according to the invention have a particle size distribution X50 of about 400 µm or less, i.e. a particle size distribution (preferably by volume) such that at least 50 % of (i) {drug1a} or {drug1b}: (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} has a particle size smaller than 400 µm. Preferably, for (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

In preferred embodiments, the particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} have a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥1.50.

In preferred embodiments, the particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} have a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} have a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Suitable milling and grinding techniques that are suitable for obtaining a specific particle size distribution are known to the skilled artisan (see e.g. N. Rasenack, B.W. Müller, Micron-size drug particles: common and novel micronization techniques, Pharm Dev Technol., 2004, 9(1):1-13; A. Martin, M.J. Cocero, Micronization processes with supercritical fluids: fundamentals and mechanisms, Adv Drug Deliv Rev. 2008, 60(3):339-50; S.C. Gad, Pharmaceutical Manufacturing Handbook: Production and Processes (Pharmaceutical Development Series), Wiley Interscience, 1st ed. 2008; A.J. Hickey, Pharmaceutical Process Engineering (Drugs and the Pharmaceutical Sciences), Informa Healthcare, 2nd ed. 2009; B. Nickerson, Sample Preparation of Pharmaceutical Dosage Forms: Challenges and Strategies for Sample Preparation and Extraction, Springer, 1st ed. 2011; D. Dragoman, Optical Characteriaztion of Solids, Springer, 1st ed. 2010; and D. Schulze; Powders and Bulk Solids: Behavior, Characterization, Storage and Flow, Springer, 2008).

### PREFERRED EMBODIMENTS OF THE INVENTION ACCORDING TO ITEMS 1 TO 137

1. An administration unit comprising (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
2. The administration unit according to item I, comprising a therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, wherein at least 10 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are present as (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
3. The administration unit according to item 2, wherein at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are present as (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
4. The administration unit according to any of items 2 to 3, wherein at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are present as (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
5. The administration unit according to any of items 2 to 4, wherein not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are amorphous.
6. The administration unit according to item 5, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are amorphous.
7. The administration unit according to item 5 or 6, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are amorphous.
8. The administration unit according to any of items 2 to 7, wherein not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are polymorphs of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} other than (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
9. The administration unit according to item 8, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are polymorphs of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} other than (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
10. The administration unit according to item 8 or 9, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} are polymorphs of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} other than (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
11. The administration unit according to any of items 2 to 10, wherein the therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.1 µg to 2 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or(x) {drug10}.
12. The administration unit according to item 11, wherein the therapeutically effective amount of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 1 µg to 2.5 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 2.5 µg to 5 µg of (i) {drug1}, (ii) {drug2}, (iii) {drug3}, (iv) {drug4}, (v) {drug5}, (vi) {drug6}, (vii) {drug7}, (viii) {drug8}; (ix) {drug9}, or (x) {drug10}, or 5 µg to 10 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 10 µg to 25 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 25 µg to 50 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 50 µg to 100 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 100 µg to 250 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 250 µg to 500 µg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 500 pg to 1 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1 mg to 2.5 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 2.5 mg to 5 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 5 mg to 10 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 10 mg to 25 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 25 mg to 50 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 50 mg to 100 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 100 mg to 250 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 250 mg to 500 mg of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 500 mg to 1 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1 g to 1.25 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1.25 g to 1.5 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1.5 g to 1.75 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, or 1.75 g to 2 g of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10).
13. The administration unit according to any of items 1 to 12, comprising 0.1 µg to 2 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
14. The administration unit according to item 13, comprising 1 µg to 2.5 pg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 2.5 µg to 5 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 5 µg to 10 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 10 µg to 25 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 25 µg to 50 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 50 µg to 100 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 100 µg to 250 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 250 µg to 500 µg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 500 µg to 1 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1 mg to 2.5 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 2.5 mg to 5 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 5 mg to 10 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 10 mg to 25 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 25 mg to 50 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 50 mg to 100 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 100 mg to 250 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 250 mg to 500 mg of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 500 mg to 1 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1 g to 1.25 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1.25 g to 1.5 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1.5 g to 1.75 g of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, or 1.75 g to 2 g of(i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}.
15. The administration unit according to any of items 1 to 14, which is solid, semisolid or liquid.
16. The administration unit according to any of items 1 to 15, which is for administration once daily, or twice daily, or thrice daily, or four times daily, or five times daily, or six times daily.
17. The administration unit according to any of items 1 to 16, which is monolithic or multiparticulate.
18. The administration unit according to any of items 1 to 17, wherein (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} is homogeneously distributed over the administration unit.
19. The administration unit according to any of items 1 to 17, wherein (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} is not homogeneously distributed over the administration unit.
20. The administration unit according to any of items 1 to 19, providing controlled release of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.
21. The administration unit according to item 20, comprising a controlled release matrix or a controlled release coating.
22. The administration unit according to any of items 1 to 19, providing immediate release of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.
23. The administration unit according to any of items 1 to 22, having a total weight of 10 mg to 3 g.
24. The administration unit according to item 23, having a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.
25. The administration unit according to any of items 1 to 24, which is for systemic or topical administration.
26. The administration unit according to any of items 1 to 25, which is for parenteral administration.
27. The administration unit according to any of items 1 to 26, which is for oral, buccal, opthalmic, nasal, rectal, transmucosal, or intestinal administration.
28. The administration unit according to any of items 1 to 27, which is for oral administration.
29. The administration unit according to item 28, which is selected from the group consisting of tablets, capsules, effervescent tablets, orodispersible tablets, dragees, sachets, drops, suspensions, and powders.
30. The administration unit according to any of items 1 to 29, which is a tablet.
31. The administration unit according to item 30, which is round or oblong; and/or which is planar or biconvex.
32. The administration unit according to any of items 30 to 31, having been manufactured by direct compression.
33. The administration unit according to any of items 30 to 31, having been manufactured by granulation and subsequent compression of granules.
34. The administration unit according to item 33, wherein granulation is wet granulation or dry granulation.
35. The administration unit according to any of items 30 to 34, having been compacted at a pressure of 10 MPa to 10,000 MPa.
36. The administration unit according to item 35, having been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.
37. The administration unit according to any of items 30 to 36, comprising a film coating.
38. The administration unit according to item 37, wherein the film coating is enteric.
39. The administration unit according to any of items 37 to 38, wherein the film coating comprises a cellulose ether (e.g. hydroxypropylmethyl cellulose) or a synthetic polymer (e.g. polyvinyl alcohol).
40. The administration unit according to any of items 37 to 39, wherein the film coating has an average thickness of 0.01 to 1000 µm.
41. The administration unit according to item 40, wherein the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 µm, or 500 µm to 1000 µm.
42. The administration unit according to any of items 30 to 41, having a tablet porosity of not more than 90 %.
43. The administration unit according to item 42, having a tablet porosity of not more than 80 %, or not more than 70 %, or not more than 60 %, or not more than 50 %, or not more than 40 %, or not more than 30 %, or not more than 20 %, or not more than 10 %.
44. The administration unit according to item 42 to 43, having a tablet porosity of not more than 5 %, or not more than 4 %, or not more than 3 %, or not more than 2 %, or not more than 1 %, or not more than 0.5 %, or not more than 0.2 %, or not more than 0.1 %.
45. The administration unit according to any of items 30 to 44, having a radial tensile strength of 0.1 to 100 MPa.
46. The administration unit according to item 45, having a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
47. The administration unit according to any of items 30 to 46, having an axial tensile strength of 0.1 to 100 MPa.
48. The administration unit according to item 47, having an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
49. The administration unit according to any of items 30 to 48, having an average pore size of 0.001 µm to 1000 µm.
50. The administration unit according to item 49, having an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to I µm, or 1 µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.
51. The administration unit according to any of items 30 to 50, having a pore size distribution such that at least 10 % of the pores have a pore size of not more than 0.1 µm.
52. The administration unit according to item 51, having a pore size distribution such that at least 20 %, or at least 30 %, or at least 40 %, or at least 50 %, or at least 60 %, or at least 70 %, or at least 80 %, or at least 90 % of the pores have a pore size of not more than 0.1 µm.
53. The administration unit according to any of items 30 to 52, having a pore size distribution such that at least 10 % of the pores have a pore size of not less than 0.1 µm.
54. The administration unit according to item 53, having a pore size distribution such that at least 20 %, or at least 30 %, or at least 40 %, or at least 50 %, or at least 60 %, or at least 70 %, or at least 80 %, or at least 90 % of the pores have a pore size of not less than 0.1 µm.
55. The administration unit according to any of items 30 to 54, having a pore size distribution such that at least 10 % of the pores have a pore size of not more than 1 µm.
56. The administration unit according to item 55, having a pore size distribution such that at least 20 %, or at least 30 %, or at least 40 %, or at least 50 %, or at least 60 %, or at least 70 %, or at least 80 %, or at least 90 % of the pores have a pore size of not more than 1 µm.
57. The administration unit according to any of items 30 to 56, having a pore size distribution such that at least 10 % of the pores have a pore size of not less than 1 µm.
58. The administration unit according to item 57, having a pore size distribution such that at least 20 %, or at least 30 %, or at least 40 %, or at least 50 %, or at least 60 %, or at least 70 %, or at least 80 %, or at least 90 % of the pores have a pore size of not less than 1 µm.
59. The administration unit according to any of items 30 to 58, having a pore size distribution such that at least 10 % of the pores have a pore size of not more than 10 µm.
60. The administration unit according to item 59, having a pore size distribution such that at least 20 %, or at least 30 %, or at least 40 %, or at least 50 %, or at least 60 %, or at least 70 %, or at least 80 %, or at least 90 % of the pores have a pore size of not more than 10 µm.
61. The administration unit according to any of items 30 to 60, having a pore size distribution such that at least 10 % of the pores have a pore size of not less than 10 µm.
62. The administration unit according to item 61, having a pore size distribution such that at least 20 %, or at least 30 %, or at least 40 %, or at least 50 %, or at least 60 %, or at least 70 %, or at least 80 %, or at least 90 % of the pores have a pore size of not less than 10 µm.
63. The administration unit according to any of items 30 to 62, having a water content of not more than 5 % by weight, relative to the total weight of the tablet.
64. The administration unit according to item 63, having a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.
65. The administration unit according to any of items 30 to 64, having a true density of 0.60 to 1.40 g cm-3.
66. The administration unit according to item 65, having a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
67. The administration unit according to any of items 30 to 66, having an apparent density of 0.60 to 1.40 g cm-3.
68. The administration unit according to item 67, having an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
69. The administration unit according to any of items 30 to 68, disintegrating within 6000 seconds.
70. The administration unit according to item 69, disintegrating within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.
71. The administration unit according to any of items 1 to 29, which is a capsule.
72. The administration unit according to item 71, comprising a multitude of particulates comprising (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {dnug10a} or {drug10b}.
73. The administration unit according to any of items 71 to 72, comprising at least 2 particulates, or at least 3 particulates, or at least 4 particulates, or at least 5 particulates, or at least 6 particulates, or at least 7 particulates, or at least 8 particulates, or at least 9 particulates, or at least 10 particulates.
74. The administration unit according to any of items 71 to 73, wherein the capsule material comprises hard gelatine.
75. The administration unit according to any of items 71 to 74, comprising at least 2 different types of particulates that differ from each other in at least one property selected from the group consisting of nature of excipients, content of excipients, content of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, content of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, water content, total weight, size, and shape.
76. The administration unit according to any of items 1 to 75, passing the storage stability test according to Ph. Eur. under accelerated storage conditions at 40°C and 75 % rel. humidity.
77. The administration unit according to item 76, having a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.
78. The administration unit according to any of items 1 to 77, providing the peak plasma level within 0.1 hour to 36 hours after administration.
79. The administration unit according to item 78, providing the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.
80. The administration unit according to any of items 1 to 79, comprising one or more excipients independently selected from the group consisting of antiadherents, binders, disintegrants, fillers, flavors, colors, lubricants, glidants, sorbents, surfactants, preservatives and sweeteners.
81. The administration unit according to item 80, wherein the antiadherent is selected from the group consisting of silicon dioxide, talc, talc waxes, stearates (e.g. magnesium, calcium or sodium), stearic acid, stearowet, boric acid, sodium chloride, DL-leucine, sodium oleate, sodium benzoate, sodium acetate, sodium lauryl sulfate, and magnesium lauryl sulfate.
82. The administration unit according to any of items 80 to 81, wherein the binder is selected from the group consisting of saccharides and their derivatives; proteins; gums; silicates; and synthetic polymers.
83. The administration unit according to item 82, wherein the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]}; alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.
84. The administration unit according to any of items 80 to 83, wherein the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).
85. The administration unit according to any of items 80 to 84, wherein the filler is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).
86. The administration unit according to any of items 80 to 85, wherein the lubricant is hydrophilic or hydrophobic.
87. The administration unit according to item 86, wherein the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.
88. The administration unit according to any of items 80 to 87, wherein the glidant is selected from the group consisting of fumed silica, cornstarch, talc, and magnesium carbonate.
89. The administration unit according to any of items 80 to 88, wherein the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.
90. The administration unit according to any of items 1 to 89, passing the friability test according to Ph. Eur.
91. The administration unit according to item 90, wherein the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.
92. The administration unit according to any of items 1 to 91, passing the test uniformity of content of single-dose preparations according to Ph. Eur.
93. The administration unit according to any of items 1 to 92, not comprising any active pharmaceutical ingredient other than (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.
94. The administration unit according to any of items 1 to 93, which is for storage not above 50 °C.
95. The administration unit according to item 94, which is for storage not above 25 °C.
96. The administration unit according to any of items 1 to 95, with the proviso that the administration unit is not identical with any of the administration units that are individualized in the experimental section of WO2013/018362, WO2013/017989, WO2013/021309, WO2013/028465, WO2013/028832, WO2013/030374, WO2013/030803, WO2013/040120, WO2013/039764, and WO2013/038351, respectively.
97. The administration unit according to any of items 1 to 96 for use in the treatment of a disorder or a disease in a mammal.
98. The administration unit according to item 97, wherein the mammal is a human.
99. The administration unit according to any of items 97 to 98, wherein the mammal is an adult.
100. The administration unit according to any of items 97 to 99 wherein the mammal does not suffer from liver damage.
101. The administration unit according to any of items 97 to 100, wherein the mammal does not suffer from kidney damage.
102. The administration unit according to any of items 97 to 101, wherein the mammal is not pregnant.
103. The administration unit according to any of items 97 to 102, wherein the mammal is not allergic against (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.
104. The administration unit according to any of items 97 to 103, which is administered orally with a liquid.
105. The administration unit according to item 104, wherein the liquid is water, milk, juice or lemonade.
106. The administration unit according to any of items 97 to 105, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 100 mg/kg body weight.
107. The administration unit according to item 106, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
108. The administration unit according to any of items 97 to 107, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 5000 mg.
109. The administration unit according to item 108, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 1999 mg.
110. A packaging comprising one or more administration units according to any of items 1 to 109.
111. The packaging according to item 110, comprising a material selected from the group consisting of paper, cardboard, paperboard, metal foil and plastic foil.
112. The packaging according to any of items 110 to 111, which is a blister packaging.
113. The packaging according to any of items 110 to 112, comprising a multitude of at least 2, or at least 3, or at least 4, or at least 5, or at least 6, or at least 7, or at least 8, or at least 9, or at least 10 administration units according to any of items 1 to 109.
114. The packaging according to item 113, wherein the administration units do not substantially differ in at least one property selected from the group consisting of nature of excipients, content of excipients, content of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b}, content of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}, water content, total weight, release profile, disintegration time, size, shape, porosity, apparent density, true density, average pore size, pore size distribution, axial tensile strength, radial tensile strength, friability, and storage stability.
115. The packaging according to item 114, wherein the administration units differ in said at least one property by not more than 5 %, or not more than 4 %, or not more than 3 %, or not more than 2 %, or not more than I %, or not more than 0.5 %, or not more than 0.1 %.
116. The packaging according to any of items 113 to 115, wherein all administration units are substantially identical.
117. The packaging according to any of items 113 to 116 for use in the treatment of a disorder or a disease, wherein one or more administration units of said multitude are administered on a first day, and one or more administration units of said multitude are administered on a second day following said first day.
118. The packaging according to item 117, wherein one or more administration units of said multitude are administered on one or more subsequent days following said second day.
119. The packaging according to any of items 117 to 118, wherein the time interval between the administration of an administration unit and the administration of a subsequent administration unit is not more than 48 hours.
120. The packaging according to item 119, wherein the time interval is not more than 36 hours, or not more than 24 hours, or not more than 12 hours, or not more than 8 hours, or not more than 6 hours, or not more than 4 hours.
121. The packaging according to any of items 117 to 120, wherein the administration units are administered on not more than 30 subsequent days.
122. The packaging according to item 121, wherein the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.
123. The packaging according to any of items 117 to 122, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 100 mg/kg body weight.
124. The packaging according to item 123, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
125. The packaging according to any of items 117 to 124, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 5000 mg.
126. The packaging according to item 125, wherein the administered daily dose of (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.
127. The packaging according to any of items 117 to 126, not comprising any administration unit comprising an active pharmaceutical ingredient other than (i) {drug1}, (ii) {drug2}, (iii) {drug3}, (iv) {drug4}, (v) {drug5}, (vi) {drug6}, (vii) {drug7}, (viii) {drug8}, (ix) {drug9}, or (x) {drug10}.
128. A method for manufacturing an administration unit according to any of items 1 to 109 or a packaging according to any of items 110 to 127, comprising the steps: (a) providing a quantity of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} that exceeds the dose of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} to be contained in a single administration unit by a factor of at least 10; (b) mixing the quantity of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} with one or more pharmaceutical excipients; (c) dividing the mixture obtained in step (b) in fractions each containing the dose of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} to be contained in a single administration unit; (d) optionally, forming the fractions obtained in step (c) to administration units; and (e) optionally, packing the fractions obtained in step (c) and the administration units obtained in step (d), respectively.
129. The method according to item 133, wherein in step (a) the quantity of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} exceeds the dose of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} to be contained in a single administration unit by a factor of at least 25, or at least 50, or at least 100, or at least 250, or at least 500, or at least 1000, or at least 2500, or at least 5000, or at least 10,000.
130. Particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} having a particle size distribution X90 of 500 µm or less.
131. The particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} according to item 130, having a particle size distribution X90 of 480 µm or less, or 460 µm or less, or 440 µm or less, or 420 µm or less, or 400 µm or less, of 380 µm or less, or 360 µm or less, or 340 µm or less, or 320 µm or less, or 300 µm or less, of 280 µm or less, or 260 µm or less, or 240 µm or less, or 220 µm or less, or 200 µm or less, of 180 µm or less, or 160 µm or less, or 140 µm or less, or 120 µm or less, or 100 µm or less, or 80 µm or less, or 60 µm or less, or 40 µm or less, or 20 µm or less.
132. The particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} according to any of items 130 to 131, having a particle size distribution X50 of 400 µm or less.
133. The particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} according to item 132, having a particle size distribution X50 of 380 µm or less, or 360 µm or less, or 340 µm or less, or 320 µm or less, or 300 µm or less, of 280 µm or less, or 260 µm or less, or 240 µm or less, or 220 µm or less, or 200 µm or less, 180 µm or less, or 160 µm or less, or 140 µm or less, or 120 µm or less, or 100 µm or less, or 80 µm or less, or 70 µm or less, or 60 µm or less, or 50 µm or less.
134. The particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} according to item 133, having a particle size distribution X50 of 45 µm or less, or 40 µm or less, or 35 µm or less, or 30 µm or less, or 25 µm or less, or 20 µm or less, or 15 µm or less, or 10 µm or less.
135. The particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} according to any of items 130 to 134, having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.
136. The particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {dnug10a} or {drug10b} according to any of items 130 to 135, having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.
137. The particles of (i) {drug1a} or {drug1b}; (ii) {drug2a}; (iii) {drug3a}; (iv) {drug4a}, {drug4b}, {drug4c}, {drug4d}, {drug4e}, {drug4f}, {drug4g}, {drug4h} or {drug4i}; (v) {drug5a}; (vi) {drug6a}; (vii) {drug7a}; (viii) {drug8a}; (ix) {drug9a}; or (x) {drug10a} or {drug10b} according to any of items 130 to 136, having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

## Claims

1. A packaging comprising one or more administration units comprising crystalline Form I of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}-methyl sulfamate hydrochloride salt.

2. The packaging according to claim 1, wherein Form I is **characterized by** an XRPD pattern having peaks at 2Θ angles of 4.5°, 15.2°, 21.3°, 21.8° and 24.0°.

3. The packaging according to claim 2, wherein Form I is **characterized by** an XRPD pattern having peaks at 2Θ angles 4.5°, 7.5°, 14.4°, 14.6°, 15.2°, 15.9°, 19.5°, 21.3°, 21.8°, 22.4°, 22.7°, 24.0° and 24.8°.

4. The packaging according to any of claims 1 to 3, comprising a material selected from the group consisting of paper, cardboard, paperboard, metal foil and plastic foil.

5. The packaging according to any of claims 1 to 4, comprising a multitude of at least 2, or at least 3, or at least 4, or at least 5, or at least 6, or at least 7, or at least 8, or at least 9, or at least 10 administration units according to any of claims 1 to 3.

6. The packaging according to claim 5, wherein the administration units do not substantially differ in at least one property selected from the group consisting of nature of excipients, content of excipients, content of crystalline form I of {(1S,2S,4R)-4-[(6-{[(1R,2S)-5-chloro-2-methoxy-2,3-dihydro-1H-inden-1-yl]amino}pyrimidin-4-yl)axy]-2-hydroxycyclopentyl}methyl sulfamate (I-216) hydrochloride salt, content of {(1S,2S,4R)-4-[(6-{[(1R,2S)-5-chloro-2-methoxy-2,3-dihydro-1H-inden-1-yl]amino}pyrimidin-4-yl)oxy]-2-hydroxycyclopentyl}methyl sulfamate (I-216) and pharmaceutically acceptable salts thereof, and prodrugs thereof, water content, total weight, release profile, disintegration time, size, shape, porosity, apparent density, true density, average pore size, pore size distribution, axial tensile strength, radial tensile strength, friability, and storage stability.

7. The packaging according to any of claims 5 to 6, wherein all administration units are substantially identical.

8. The packaging according to any of claims 1 to 7, wherein the administration unit is for oral, buccal, opthalmic, nasal, rectal, transmucosal, or intestinal administration.

9. The packaging according to any of claims I to 8 for use in the treatment of a disorder or a disease, wherein one or more administration units of said multitude are administered on a first day, and one or more administration units of said multitude are administered on a second day following said first day.

10. The packaging according to claim 9, wherein one or more administration units of said multitude are administered on one or more subsequent days following said second day.

11. The packaging according to any of claims 9 to 10, wherein the time interval between the administration of an administration unit and the administration of a subsequent administration unit is not more than 48 hours.

12. The packaging according to any of claims I to 11, wherein the administration unit is for use in the treatment of a disorder or a disease in a mammal.

13. The packaging according to claim 12, wherein the mammal is a human.

14. The packaging according to claim 12 or 13, wherein the disorder or disease is selected from disorders of cell proliferation, including cancers, and inflammatory disorders.

15. The packaging according to any of claims 1 to 14, wherein the administration unit is for use in the inhibition of the activity of NEDD8-activating enzyme.
